# EUROPEAN PATENT APPLICATION

(11) **EP 1 717 238 A1**
(43) Date of publication of application: **02.11.2006**
(21) Application number: 05719189.2
(22) Date of filing: 16.02.2005
(51) Int. Cl.: C07D 471/04, A61K 31/437, A61K 31/444, A61K 31/4745, A61K 31/496, A61P 31/00, A61P 31/10

(54) **FUNGICIDAL HETEROCYCLIC COMPOUNDS**

(30) Priority: 16.02.2004 JP 2004038918
(71) Applicant: DAIICHI PHARMACEUTICAL CO., LTD., Chuo-ku, Tokyo 103-8234 (JP)
(72) Inventor: KAWAKAKI, Katsuhiro, DAIICHI PHARMA. CO., LTD., Tokyo 134-8630 (JP); KANAI, Kazuo, DAIICHI PHARMA. CO., LTD., Tokyo 134-8630 (JP); FUJISAWA, Tetsunori, DAIICHI FINE CHEMICAL CO.LTD., Takaoka-shi, Toyama 933-8511 (JP); MORITA, Chikanori, DAIICHI FINE CHEMICAL CO., LTD., Takaoka-shi, Toyama 933-8511 (JP); SUZUKI, Takashi, DAIICHI FINE CHEMICAL CO., LTD., Toyama 933-8511 (JP)
(74) Representative: Reitstötter - Kinzebach
(86) International application number: PCT/JP2005/002337
(87) International publication number: WO 2005/077948

(57) **Abstract**

A compound which can specifically or selectively expresses an antifungal activity with a broad spectrum, based on the functional mechanism of 1,6-β-glucan synthesis inhibition, is provided, and an antifungal agent which comprises such a compound, a salt thereof or a solvate thereof is provided. A compound represented by the following formula (I), a salt thereof or a solvate thereof.

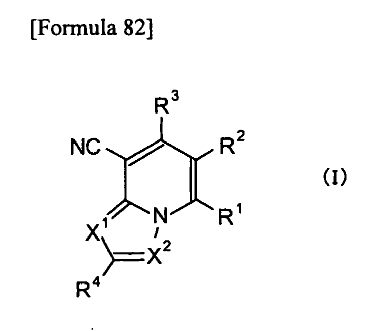

## Description

### Technical Field

The present invention relates to a compound which shows antifungal activity against pathogenic fungi, a salt thereof or a solvate thereof. It also relates to an antifungal agent comprising the same.

### Background Art

It is known that fungi cause infection on humans, animals, plants and the like and thereby induces various diseases therein.
For example, they induce superficial mycosis in various human tissues such as epidermic corneal layers of skins, keratinous tissues such as nails and hairs, and mucosal epitherlia in oral cavities, and also induce subcutaneous mycosis even in deep skin tissues existing in depth from the body surface, and cause deep-seated mycosis even in deep tissues in esophagi, internal organs and the brains. The genera *Candida, Cryptococcus,* and *Aspergillus* and the like are known as the typical pathogenic fungi which cause deep-seated mycosis in humans; and in the case of the superficial mycosis, the genus *Candida* which infect skins, oral cavities and vagina; and genus *Trichophyton* that infect the skins of hands and feet are considered as the main pathogens. Apart from these, it is considered that various other fungi are present that infect animals and plants.

With the rapid progress of research and development since in and after 1950 on antibiotics and chemotherapeutic drugs and broad popularization thereof, a large number of therapeutic drugs for bacterial infections have been developed. In the same manner, great efforts have also been made toward the development of antifungal agents, but in comparison with the development of antibacterial chemotherapeutic agents, such compounds so far subjected to the clinical field are not so many. On the other hand, deep-seated mycosis has been increasing and causing problems in recent years, due to the frequent use of antibacterial drugs (antibiotics and chemotherapeutic agent) in the actual crinical site, and increase of the compromised hosts having reduced immunity caused by malignant tumor, leukemia, organ or bone marrow transplantation, acquired immunodeficiency syndrome and the like.

As the main antifungal agents used in the current clinical field, there are polyene macrolides, fluoropyrimidines, azoles and the like. For the treatment of superficial mycosis, external preparation are used therefore, various azole-type medicines, and polyenemacrolide-type nystatin, griseofulvin, terbinafine hydrochloride, butenafine hydrochloride, amorolfine hydrochloride and the like are used therein. On the other hand, being highly safe in comparison with other drugs, azole-type fluconazole and itraconazole are frequently used in the treatment of deep-seated mycosis which has been significantly increasing in recent years, but their narrow antifungal spectrum is considered to be a problem. Also, a polyenemacrolide-type amphotericin B, has broad antifungal spectrum and high efficacy, but has a problem in view of its toxicity (side effects). In addition, a floropyrimidine-type medicine, flucytosine, has low toxicity but easily causes fungal resistance. Thus, among the drugs currently used in the treatment of deep-seated mycosis, those which have high medically satisfactory degree in view of the antifungal spectrum, efficacy, safety and the like are markedly few. In addition, fluconazole which is most frequently used among these anti-deep-seated mycosis agents has low sensitivity to, for example, *Candida glabrata, Candida tropicalis, Candida krusei* and the like, and their resistant strains have beenemerging. Accordingly, great concern has been directed in the clinical field toward the development of a novel antifungal drug which overcame these problems.

On the other hand, toward the advancement of mycosis treating methods and the development of novel antifungal agents in recent years, test methods for scientifically evaluating their usefulness have been established, so that, combined with the advance of studies on functional mechanisms, development of more effective and safe drugs is in demand. From the point of overcoming the problem of drug-registant fungi, it is much desired to develop antifungal agents having novel mechanisms.
In addition, different from bacteria (procaryotic cells), fungi are eucaryotic cells similar to the case of human, so that it is necessary from the viewpoint of safety to develop a compound which injure specifically (selectively) to eucaryotic cells.

Under these circumstances, a drug which inhibits synthesis of principal cell wall composing components of fungi, so-called cell wall polysaccharide synthesis system, namely an antifungal agent aiming a synthase of the cell wall polysaccharide system specifically existing in fungi, is expected from the viewpoint of the novelty of functional mechanism and selective toxicity. As the polysaccharides constituting fungal cell wall, β-glucan, chitin or chitosan and mannan are known, and the β-glucan as a principal composing component of fungal cell wall is groupified into 1,3-β-glucan and 1,6-β-glucan.

As 1,3-β-glucan synthase inhibitors, papulacandins (Non-Patent Reference 1), echinocandins (Non-Patent Reference 2), pneumocandins (Non-Patent Reference 3), aculeacins (Non-Patent Reference 4) and the like have been reported.
As 1,6-β-glucan synthase inhibitors, tricyclic system imidazo[1,2-a]pyridine derivatives have been reported (Patent Reference 1), but it is necessary to develop a 1,6-β-glucan synthase inhibitor which shows more strong growth inhibition and broad objective pathogenic fungi spectrum.

On the other hand, it is known that imidazopyridine, triazolopyridine, pyrazolopyridine and derivatives thereof, as pyridine derivatives having a bicyclic skeleton, have pharmacological activities over a markedly broad ranges, and there are reports stating that imidazopyridine and pyrazolopyridine derivatives show antifungal activity upon the fungi which cause plant diseases (Patent Reference 2, Non-patent Reference 5).
Patent Reference 1: Patent Application No. 2002-022767 (International Application No. PCT/JP 03/00912)
Patent Reference 2: International Publication 03/022850
Non-patent Reference 1: Journal of Antibiotics, vol. 36, p. 1539 (1983)
Non-patent Reference 2: Journal of Medicinal Chemistry, vol. 38, p. 3271 (1995)
Non-patent Reference 3: Journal of Antibiotics, vol. 45, p. 1875 (1992)
Non-patent Reference 4: Journal of Biochemistry, vol. 105, p. 606 (1989)
Non-patent Reference 5: Journal of Medicinal Chemistry, vol. 18, p. 1253 (1975)

### Disclosure of the Invention

### Problems that the Invention is to Solve

The object of the present invention is to provide a compound capable of expressing an antifungal activity based on the functional mechanism of 1,6-β-glucan synthesis inhibition, with a broad spectrum and specifically or selectively, and to provide an antifungal agent which comprises such a compound, a salt thereof or a solvate thereof.

### Means for Solving the Problems

With the aim of obtaining a compound which shows an antifungal activity by the inhibition of 1,6-β-glucan synthase, the present inventors have carried out screening of compounds and found a compound that shows a 1,6-β-glucan synthase inhibitory action by a biopolymer synthesis inhibition experiment based on [¹⁴C]-glucose inake as the index. In addition, whether a group of compounds structurally resembling to this compound show antifungal activity upon fungi was verified. As a result, the present invention was accomplished by finding that imidazopyridine, triazolopyridine and pyrazolopyridine derivatives having a basic substituent as a substituent, represented by a formula (1), salts thereof and solvates thereof show broad and potent antifungal activity based on the 1,6-β-glucan synthesis inhibition as the functional mechanism, and particularly show the antifungal activity upon the genus *Candida,* the genus *Cryptococcus* and the genus *Aspergillus* as typical causal fungi of deep-seated mycosis.

That is, the present invention includes the following embodiments.
1. A compound represented by the following formula (I), a salt thereof, or a solvate thereof 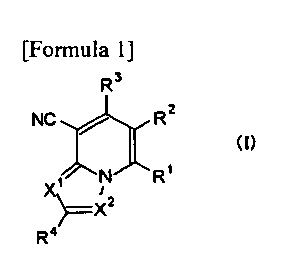
[in the formula,
R¹ means a basic group which may have a substituent,
R² means
hydrogen atom,
halogen atom,
carboxy group,
a group represented by the following formula 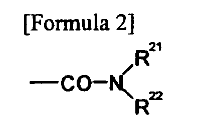 (in the formula, R²¹ and R²² each independently represents hydrogen atom, an alkyl group having from 1 to 6 carbon atoms or an aryl group having from 6 to 10 carbon atoms),
an alkyl group having from 1 to 6 carbon atoms,
an alkenyl group having from 2 to 6 carbon atoms,
an alkynyl group having from 2 to 6 carbon atoms,
an acyl group having from 2 to 7 carbon atoms,
an alkoxycarbonyl group having from 2 to 7 carbon atoms,
a cycloalkyl group having from 3 to 6 carbon atoms,
a cycloalkenyl group having 5 or 6 carbon atoms,
a cycloalkylalkyl group having from 4 to 12 carbon atoms,
an aryl group having from 6 to 10 carbon atoms,
an aralkyl group having from 7 to 12 carbon atoms,
a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom),
a heteroaryl group having from 3 to 10 carbon atoms, or
a heteroarylalkyl group having from 3 to 12 carbon atoms,
wherein when R² is an alkyl group, an alkenyl group, an alkynyl group, an acyl group or an alkoxycarbonyl group, these may have 1 or more groups of 1 or more species selected from [substituent group 2-1] as the substituent;
[substituent group 2-1]
halogen atom,
amino group,
imino group,
nitro group,
hydroxy group,
mercapto group,
carboxy group,
cyano group,
sulfo group,
a dialkyl phosphoryl group,
a group represented by the following formula 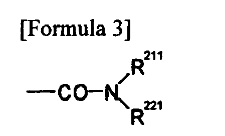 (in the formula, R²¹¹ and R²²¹ each independently represents hydrogen atom, an alkyl group having from 1 to 6 carbon atoms or an aryl group having from 6 to 10 carbon atoms),
an alkoxy group having from 1 to 6 carbon atoms,
an alkylthio group having from 1 to 6 carbon atoms,
an acyl group having from 2 to 7 carbon atoms,
an alkoxycarbonyl group having from 2 to 7 carbon atoms,
a cycloalkyl group having from 3 to 6 carbon atoms,
an aryl group having from 6 to 10 carbon atoms, and
an arylthio group having from 6 to 10 carbon atoms
wherein amino group of the [substituent group 2-1] may have 1 or 2 groups, as the substituent, selected from the group consisting of formyl group, an alkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, an aralkyl group having from 7 to 12 carbon atoms, an aromatic heterocyclic group, an alkylsulfonyl group having from 1 to 6 carbon atoms and an arylsulfonyl group having from 6 to 10 carbon atoms, in addition, when said amino group has 2 substituents, they may be bonded together to form a cyclic structure;
hydroxy group of the [substituent group 2-1] or mercapto group of the [substituent group 2-1] may have a substituent selected from the group consisting of an alkyl group having from 1 to 6 carbon atoms, an aminoalkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, a cycloalkyl group having from 3 to 6 carbon atoms, an aryl group having from 6 to 10 carbon atoms, an aralkyl group having from 7 to 12 carbon atoms and an aromatic heterocyclic group;
when R² is a cycloalkyl group, these may have 1 or more groups of 1 or more species selected from [substituent group 2-2] as the substituent;
[substituent group 2-2]:
halogen atom,
amino group,
imino group,
nitro group,
hydroxy group,
mercapto group,
carboxy group,
cyano group,
sulfo group,
a group represented by the following formula 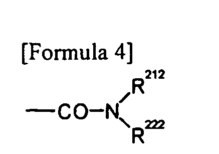
(in the formula, R²¹² and R²²² each independently represents hydrogen atom, an alkyl group having from 1 to 6 carbon atoms or an aryl group having from 6 to 10 carbon atoms),
an alkoxy group having from 1 to 6 carbon atoms,
an alkylthio group having from 1 to 6 carbon atoms,
an acyl group having from 2 to 7 carbon atoms, and
an alkoxycarbonyl group having from 2 to 7 carbon atoms;
amino group of the [substituent group 2-2] may have 1 or 2 groups, as the substituent, selected from the group consisting of formyl group, an alkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, an aralkyl group having from 7 to 12 carbon atoms, an aromatic heterocyclic group, an alkylsulfonyl group having from 1 to 6 carbon atoms and an arylsulfonyl group having from 6 to 10 carbon atoms, in addition, when said amino group has 2 substituents, they may be bonded together to form a cyclic structure;
when R² is an aryl group, an aralkyl group, a heteroaryl group or a heteroarylalkyl group, these may have 1 or more groups of 1 or more species selected from [substituent group 2-3] as the
substituent;
[substituent group 2-3]:
halogen atom,
amino group,
imino group,
nitro group,
hydroxy group,
mercapto group,
carboxy group,
cyano group,
sulfo group,
a group represented by the following formula 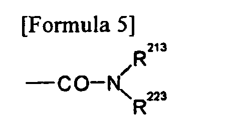
(in the formula, R²¹³ and R²²³ each independently represents hydrogen atom, an alkyl group having from 1 to 6 carbon atoms or an aryl group having from 6 to 10 carbon atoms), an alkoxy group having from 1 to 6 carbon atoms, an alkylthio group having from 1 to 6 carbon atoms, an acyl group having from 2 to 7 carbon atoms, an alkoxycarbonyl group having from 2 to 7 carbon atoms, an aralkyloxy group having from 7 to 12 carbon atoms, an aralkyloxycarbonyl group having from 8 to 15 carbon atoms, an aryl group and a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom);
amino group of the [substituent group 2-3] may have 1 or 2 groups, as the substituent, selected from the group consisting of formyl group, an alkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, an aralkyl group having from 7 to 12 carbon atoms, an aromatic heterocyclic group, an alkylsulfonyl group having from 1 to 6 carbon atoms and an arylsulfonyl group having from 6 to 10 carbon atoms, in addition, when said amino group has 2 substituents, they may be bonded together to form a cyclic structure;
when R² is a heterocyclic group, it may have 1 or 2 groups selected from the next [substituent group 2-4] as the substituent;
[substituent group 2-4]:
halogen atom,
amino group,
hydroxy group,
mercapto group,
carboxy group,
sulfo group,
a group represented by the following formula 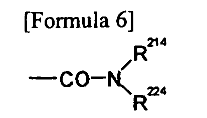
(in the formula, R²¹⁴ and R²²⁴ each independently represents hydrogen atom, an alkyl group having from 1 to 6 carbon atoms or an aryl group having from 6 to 10 carbon atoms),
an alkyl group having from 1 to 6 carbon atoms,
an alkenyl group having from 2 to 6 carbon atoms,
an alkynyl group having from 2 to 6 carbon atoms,
an alkoxy group having from 1 to 6 carbon atoms,
an alkylthio group having from 1 to 6 carbon atoms,
a halogenoalkyl group having from 1 to 6 carbon atoms,
an acyl group having from 2 to 7 carbon atoms,
an alkoxycarbonyl group having from 2 to 7 carbon atoms, and
an aryl group having from 6 to 10 carbon atoms;
wherein amino group of the [substituent group 2-4] may have 1 or 2 groups, as the substituent, selected from the group consisting of formyl group, an alkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, an aralkyl group having from 7 to 12 carbon atoms, a monocyclic, bicyclic or spiro cyclic heterocyclic group having from 2 to 10 carbon atoms
(contains from 1 to 4 hetero atoms of 1 or more species selected from the group consisting of nitrogen atom, oxygen atom and sulfur atom), an aromatic heterocyclic group, an alkylsulfonyl group having from 1 to 6 carbon atoms and an arylsulfonyl group having from 6 to 10 carbon atoms, in addition, when said amino group has 2 substituents, they may be bonded together to form a cyclic structure;
in addition, R¹ and R² may together form a cyclic structure including the carbon atoms to which these are bonded, wherein this ring contains 1 or 2 hetero atoms of 1 or more species selected from the group consisting of nitrogen atom, oxygen atom and sulfur atom, and the structural moiety to be formed herein may be saturated or unsaturated;
R³ means
hydrogen atom,
halogen atom,
amino group,
hydroxy group,
mercapto group,
nitro group,
cyano group,
formyl group,
carboxy group,
a group represented by the following formula

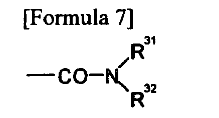

(in the formula, R³¹ and R³² each independently represents hydrogen atom, an alkyl group having from 1 to 6 carbon atoms or an aryl group having from 6 to 10 carbon atoms),
an alkyl group having from 1 to 6 carbon atoms,
an alkenyl group having from 2 to 6 carbon atoms,
an alkynyl group having from 2 to 6 carbon atoms,
an alkoxy group having from 1 to 6 carbon atoms,
an alkylthio group having from 1 to 6 carbon atoms
an acyl group having from 2 to 5 carbon atoms,
an alkoxycarbonyl group having from 2 to 5 carbon atoms,
a cycloalkyl group having from 3 to 7 carbon atoms,
a cycloalkenyl group having from 4 to 7 carbon atoms,
an aryl group having from 6 to 10 carbon atoms,
an aralkyl group having from 7 to 12 carbon atoms,
a heteroaryl group having from 3 to 10 carbon atoms;
wherein said amino group, said hydroxy group or said mercapto group may be protected by a protecting group;
when R³ is an alkyl group, an alkenyl group, an alkynyl group, an alkoxy group, an alkylthio group, an acyl group, an alkoxycarbonyl group, a cycloalkyl group, a cycloalkenyl group, an aryl group, an aralkyl group or a heteroaryl group, these may have 1 or more groups of 1 or more species selected from [substituent group 3-1] as the substituent;
[substituent group 3-1]:
amino group,
hydroxy group,
mercapto group,
halogen atom,
an alkoxy group having from 1 to 6 carbon atoms,
an alkylthio group having from 1 to 6 carbon atoms,
an acyl group having from 2 to 5 carbon atoms, and
an alkoxycarbonyl group having from 2 to 5 carbon atoms;
amino group of the [substituent group 3-1] may have 1 or 2 groups, as the substituent, selected from the group consisting of formyl group, an alkyl group having from 1 to 6 carbon atoms, a cycloalkyl group having from 3 to 6 carbon atoms, an aryl group having from 6 to 10 carbon atoms, an aromatic heterocyclic group, an acyl group having from 2 to 5 carbon atoms and an alkoxycarbonyl group having from 2 to 5 carbon atoms, wherein when said amino group has 2 substituents, they may be bonded together to form a cyclic structure;
in addition, R² and R³ may together form a polymethylene chain structure and form a 5-membered or 6-membered cyclic structure by including the carbon atoms to which R² and R³ are to be bonded, this polymethylene chain may contain 1 or 2 hetero atoms of 1 or more species selected from the group consisting of nitrogen atom, oxygen atom and sulfur atom, and the polymethylene chain formed herein may have 1 or more groups of 1 or more species selected from [substituent group 3-2] as the substituent;
[substituent group 3-2]:
amino group,
hydroxy group,
mercapto group,
halogen atom,
an alkoxy group having from 1 to 6 carbon atoms,
an alkylthio group having from 1 to 6 carbon atoms,
an acyl group having from 2 to 5 carbon atoms, and
an alkoxycarbonyl group having from 2 to 5 carbon atoms;
amino group of the [substituent group 3-2] may have 1 or 2 groups, as the substituent, selected from the group consisting of formyl group, an alkyl group having from 1 to 6 carbon atoms, a cycloalkyl group having from 1 to 6 carbon atoms, an aryl group having from 6 to 10 carbon atoms, an aromatic heterocyclic group, an acyl group having from 2 to 5 carbon atoms and an alkoxycarbonyl group having from 2 to 5 carbon atoms, wherein when said amino group has 2 substituents, they may be bonded together to form a cyclic structure;
in addition, R² and R³ may together form a polymethylene chain structure and form a 5-membered or 6-membered cyclic structure by including the carbon atoms to which R² and R³ are to be bonded, and this polymethylene chain may contain 1 or 2 hetero atoms of 1 or more species selected from the group consisting of nitrogen atom, oxygen atom and sulfur atom,
wherein the polymethylene chain formed herein may have 1 or more groups of 1 or more species selected from [substituent group 3-2] as the substituent;
[substituent group 3-2]: amino group, hydroxy group, mercapto group, halogen atom, an alkoxy group having from 1 to 6 carbon atoms, an alkylthio group having from 1 to 6 carbon atoms, an acyl group having from 2 to 5 carbon atoms, and an alkoxycarbonyl group having from 2 to 5 carbon atoms;
amino group of the [substituent group 3-2] may have 1 or 2 groups, as the substituent, selected from the group consisting of formyl group, an alkyl group having from 1 to 6 carbon atoms, a cycloalkyl group having from 1 to 6 carbon atoms, an aryl group having from 6 to 10 carbon atoms, an aromatic heterocyclic group, an acyl group having from 2 to 5 carbon atoms and an alkoxycarbonyl group having from 2 to 5 carbon atoms, wherein when said amino group has 2 substituents, they may be bonded together to form a cyclic structure; and
R⁴ means
hydrogen atom,
halogen atom,
amino group,
hydroxy group,
mercapto group,
nitro group,
cyano group,
formyl group,
carboxy group,
a group represented by the following formula 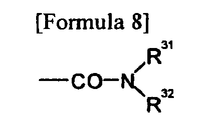 (in the formula, R³¹ and R³² each independently represents hydrogen atom, an alkyl group having from 1 to 6 carbon atoms or an aryl group having from 6 to 10 carbon atoms),
an alkyl group having from 1 to 4 carbon atoms,
an cyclic alkyl group having from 3 to 8 carbon atoms,
an aryl group having from 6 to 10 carbon atoms,
a heteroaryl group having from 5 to 9 carbon atoms,
an alkynyl group having from 2 to 6 carbon atoms, or
a group represented by 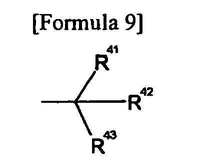 (in the formula, R⁴¹ and R⁴² each independently represents hydrogen atom, an alkyl group having from 1 to 6 carbon atoms or an alkoxy group having from 1 to 6 carbon atoms, or both may together form an exomethylene structure, and this exomethylene structure may further have an alkyl group having from 1 to 6 carbon atoms, an alkoxy group having from 1 to 6 carbon atoms or a halogenoalkyl group having from 1 to 6 carbon atoms, as a substituent, and
R⁴³ means hydrogen atom, a halogen atom, hydroxy group, mercapto group, nitrile group, nitro group, carboxy group, an alkoxycarbonyl group having from 2 to 7 carbon atoms, an alkylaminocarbonyl group having from 2 to 7 carbon atoms, an arylaminocarbonyl group having from 7 to 11 carbon atoms, a cycloalkylaminocarbonyl group having from 4 to 7 carbon atoms, an aralkylaminocarbonyl group having from 8 to 12 carbon atoms, an alkyl group having from 1 to 6 carbon atoms, a halogenoalkyl group having from 1 to 6 carbon atoms, a hydroxyalkyl group having from 1 to 6 carbon atoms, an aminoalkyl group having from 1 to 6 carbon atoms, an alkoxy group having from 1 to 6 carbon atoms, a cycloalkyl group having from 3 to 8 carbon atoms, an aralkyl group having from 7 to 11 carbon atoms, or an aralkyloxy group having from 7 to 11 carbon atoms);
when R⁴ is an alkyl group, a cyclic alkyl group, an aryl group or a heteroaryl group, and when R⁴³ is an alkyl group, these may have 1 or more groups of 1 or more species selected from [substituent group 4] as the substituent;
[substituent group 4]:
halogen atom,
amino group,
nitro group,
hydroxy group,
mercapto group,
carboxy group,
cyano group,
sulfo group,
a group represented by the following formula 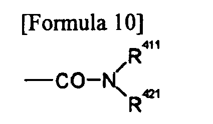
(in the formula, R⁴¹¹ and R⁴²¹ each independently mean hydrogen atom, an alkyl group having from 1 to 6 carbon atoms or an aryl group having from 6 to 10 carbon atoms),
an alkoxy group having from 1 to 6 carbon atoms,
an alkylthio group having from 1 to 6 carbon atoms,
an acyl group having from 2 to 7 carbon atoms,
an alkoxycarbonyl group having from 2 to 7 carbon atoms,
an aralkyloxy group having from 7 to 12 carbon atoms,
an aralkyloxycarbonyl group having from 8 to 15 carbon atoms,
an aryl group having from 6 to 10 carbon atoms, and
a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom);
amino group of the [substituent group 4] may have 1 or 2 groups, as the substituent, selected from the group consisting of formyl group, an alkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, an aralkyl group having from 7 to 12 carbon atoms, an aromatic heterocyclic group, an alkylsulfonyl group having from 1 to 6 carbon atoms and an arylsulfonyl group having from 6 to 10 carbon atoms, wherein when said amino group has 2 substituents, they may be bonded together to form a cyclic structure;
hydroxy group or mercapto group of the [substituent group 4] may have a substituent selected from the group consisting of an alkyl group having from 1 to 6 carbon atoms, an aminoalkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, a cycloalkyl group having from 3 to 6 carbon atoms, an aryl group having from 6 to 10 carbon atoms, an aralkyl group having from 7 to 12 carbon atoms and an aromatic heterocyclic group, wherein when R⁴ is an alkynyl group, it may have an alkyl group having from 1 to 6 carbon atoms, an alkoxyalkyl group having from 1 to 6 carbon atoms, a halogenoalkyl group having from 1 to 6 carbon atoms or carboxy group as a substituent;
X¹ and X² each independently means
nitrogen atom or
carbon atom which may be substituted with
a halogen atom,
an alkoxy group having from 1 to 6 carbon atoms,
an alkyl group having from 1 to 6 carbon atoms which may have a substituent,
an ester group, wherein either one of X¹ and X² is nitrogen atom;
wherein the substituent of alkyl group is 1 or 1 or more groups selected from the following group of substituents;
halogen atom,
amino group,
nitro group,
hydroxy group,
mercapto group,
carboxy group,
cyano group,
an alkoxy group having from 1 to 6 carbon atoms,
an alkylthio group having from 1 to 6 carbon atoms,
an acyl group having from 2 to 7 carbon atoms,
an alkoxycarbonyl group having from 2 to 7 carbon atoms,
a cycloalkyl group having from 3 to 6 carbon atoms, and
an aryl group having from 6 to 10 carbon atoms;
when the substituents on carbon atoms are esters, these may be
an alkyl ester having from 1 to 6 carbon atoms,
an aryl ester having from 6 to 10 carbon atoms,
or an aralkyl ester consisting of an alkyl group having from 1 to 6 carbon atoms and an aryl group having from 6 to 10 carbon atoms;
in addition, the aryl moiety of these aryl esters and aralkyl groups may be substituted with 1 or 1 or more groups selected from the following group of substituents;
halogen atom,
amino group,
nitro group,
hydroxy group,
mercapto group,
carboxy group,
cyano group,
an alkyl group having from 1 to 6 carbon atoms,
an alkoxy group having from 1 to 6 carbon atoms,
an alkylthio group having from 1 to 6 carbon atoms,
an acyl group having from 2 to 7 carbon atoms,
an alkoxycarbonyl group having from 2 to 7 carbon atoms,
a cycloalkyl group having from 3 to 6 carbon atoms, and
an aryl group having from 6 to 10 carbon atoms].
2. The compound, a salt thereof, or a solvate thereof described in the aforementioned 1, wherein the basic group of R¹ is
   (1) an amino substituted alkyl group having from 1 to 6 carbon atoms, which may have a substituent,
   (2) an amino substituted cyclic alkyl group having from 3 to 6 carbon atoms, which may have a substituent,
   (3) an aminocycloalkenyl group having from 3 to 6 carbon atoms, which may have a substituent,
   (4) an amino substituted aralkyl group wherein the binding region with the bicyclic nucleus is an aromatic ring, which may have a substituent,
   (5) an aminoalkyl substituted amino group having from 1 to 6 carbon atoms, which may have a substituent,
   (6) an amino substituted cyclic alkylamino group having from 3 to 6 carbon atoms, which may have a substituent,
   (7) an aminocycloalkenylamino group having from 3 to 6 carbon atoms, which may have a substituent,
   (8) an amino substituted aralkylamino group wherein the binding region with the bicyclic nucleus is an aromatic ring, which may have a substituent, or
   (9) a nitrogen-containing heterocyclic substituent, which may have a substituent;
wherein the amino group as the basic nature expressing group in the substituents of (1) to (8) may have 1 or 2 (may be the same or different when 2) of the substituents selected from the following substituent group [1-1]; substituent group [1-1]:
an alkyl group having from 1 to 6 carbon atoms, an alkenyl group having from 2 to 6 carbon atoms, an alkynyl group having from 2 to 6 carbon atoms, an alkoxycarbonyl group having from 2 to 7 carbon atoms, a cycloalkyl group having from 3 to 10 carbon atoms, a cycloalkenyl group having from 4 to 10 carbon atoms, and a group derived from an amino acid, a dipeptide or a polypeptide consisting of 3 to 5 amino acids;
also, when the substituent selected from the substituent group [1-1] is an alkyl group, an alkenyl group, an alkynyl group, an alkoxycarbonyl group, a cycloalkyl group or a cycloalkenyl group, these may have 1 or more of 1 or more groups selected from [substituent group 1-1-1]; [substituent group 1-1-1]: hydroxy group, a halogen atom, an alkoxy group having from 1 to 6 carbon atoms, an alkylthio group having from 1 to 6 carbon atoms and a cycloalkyl group having from 3 to 10 carbon atoms;
in addition, the nitrogen-containing heterocyclic substituent of (9) preferably uses a carbon atom as the binding position, is saturated or partially saturated, and is a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom), and the substituent on this heterocyclic group may be selected from [substituent group 1-2]; [substituent group 1-2]: a halogen atom, amino group, hydroxy group, oxo group, a group represented by the following formula 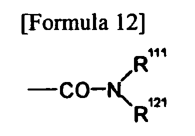
(in the formula, R¹¹¹ and R¹²¹ each independently represents hydrogen atom, an alkyl group having from 1 to 6 carbon atoms or an aryl group having from 6 to 10 carbon atoms), an alkyl group having from 1 to 6 carbon atoms, an aminoalkyl group having from 1 to 8 carbon atoms, an aminocycloalkyl group having from 3 to 8 carbon atoms, an alkoxy group having from 1 to 6 carbon atoms, an alkylthio group having from 1 to 6 carbon atoms, a halogenoalkyl group having from 1 to 6 carbon atoms and an alkylamino group having from 1 to 6 carbon atoms;
wherein the alkyl moiety of the alkyl group, alkylamino group, cycloalkylamino group, alkoxy group, alkylthio group, halogenoalkyl group or aminoalkyl group of the [substituent group 1-2] may have 1 or more groups of 1 or more species selected from [substituent group 1-2-1]; [substituent group 1-2-1]: a halogen atom, hydroxy group, an alkyl group having from 1 to 6 carbon atoms, an alkoxy group having from 1 to 6 carbon atoms, an alkoxycarbonyl group having from 2 to 7 carbon atoms, an alkylcarbonylamino group having from 2 to 7 carbon atoms and an aryl group having from 6 to 10 carbon atoms;
wherein the amino group moiety of the amino group, aminoalkyl group, aminocycloalkyl group and alkylamino group of the [substituent group 1-2] may be protected with a protecting group, and also may have 1 or 2 of alkyl groups having from 1 to 6 carbon atoms (may have 1 or more groups of 1 or more species selected from the group of groups consisting of hydroxy group, a halogen atom, and an alkoxy group and alkylthio group having from 1 to 6 carbon atoms) as the substituent, and also, an amino acid, a dipeptide or a polypeptide consisting of 3 to 5 amino acids may be bonded thereto.
3. The compound, a salt thereof, or a solvate thereof described in the aforementioned 2, wherein R¹ is a nitrogen-containing heterocyclic group which may have a substituent.
4. The compound, a salt thereof, or a solvate thereof described in the aforementioned 3, wherein R¹ is a nitrogen-containing heterocyclic group which may have a substituent, and said nitrogen-containing heterocyclic group is a saturate or partially saturated nitrogen-containing heterocyclic group.
5. The compound, a salt thereof, or a solvate thereof described in the aforementioned 4, wherein R¹ is a group represented by the following formula; 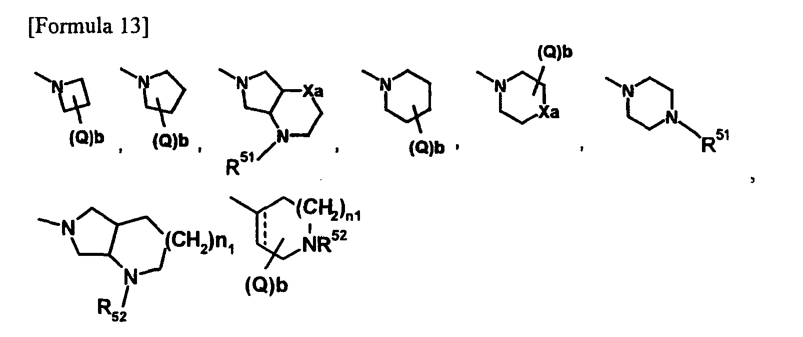 [in the formula, Xa means oxygen atom, sulfur atom, a substituent or NR⁵²,
   R⁵¹ and R⁵² each independently means hydrogen atom, an alkyl group having from 1 to 6 carbon atoms, a halogenoalkyl group having from 1 to 6 carbon atoms or a cycloalkyl group having from 3 to 6 carbon atoms,
   the substituent Q means a substituent represented by the following formula, 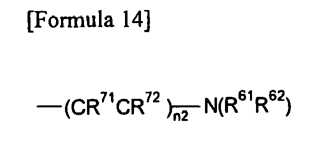 b means an integer of 0, 1 or 2,
   n1 means an integer of 0 or 1,
   n2 means an integer of 0, 1 or 2,
   R⁶¹ and R⁶² each independently means hydrogen atom, an alkyl group having from 1 to 6 carbon atoms or a halogenoalkyl group having from 1 to 6 carbon atoms, or a group derived from an amino acid, a dipeptide or a polypeptide consisting of 3 to 5 amino acids,
   R⁷¹ and R⁷² each independently means hydrogen atom, an alkyl group having from 1 to 6 carbon atoms, a halogenoalkyl group having from 1 to 6 carbon atoms, a hydroxyalkyl group having from 3 to 6 carbon atoms, an aminoalkyl group having from 1 to 6 carbon atoms, an alkoxyalkyl group having from 2 to 12 carbon atoms, a cycloalkyl group having from 3 to 6 carbon atoms, a phenyl group which may have a substituent or a heteroaryl group having from 3 to 10 carbon atoms, and the dotted line means that said binding region may form a double bond].
6. The compound, a salt thereof, or a solvate thereof described in any one of the aforementioned 1 to 5, wherein R² is an aryl group having from 6 to 10 carbon atoms or a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom).
7. The compound, a salt thereof, or a solvate thereof described in the aforementioned 6, wherein R² is a group represented by the following formula;

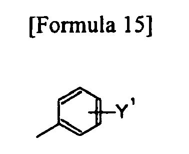

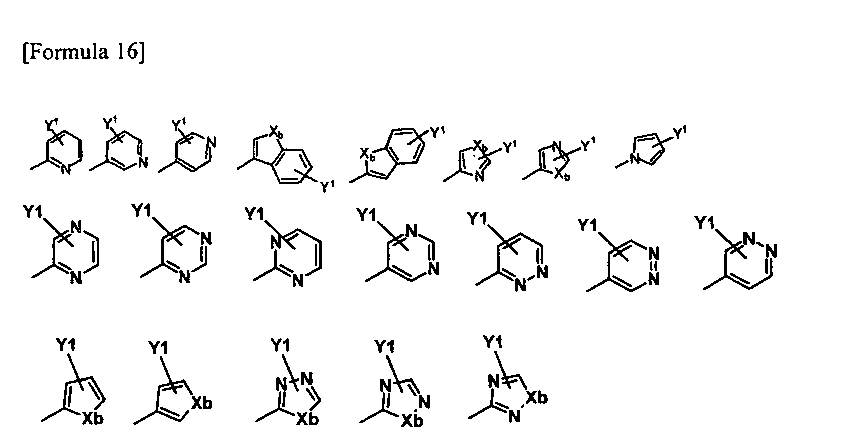

(in the formula, Xb means oxygen atom, sulfur atom, a substituent or NR⁸, wherein R⁸ means hydrogen atom, an alkyl group having from 1 to 6 carbon atoms or a halogenoalkyl group having from 1 to 6 carbon atoms, and the substituent Y¹ has the same meaning as described in the aforementioned [substituent group 2-2]).
8. The compound, a salt thereof, or a solvate thereof described in the aforementioned 7, wherein R³ is a halogen atom, amino group, hydroxy group, mercapto group, an alkyl group having from 1 to 4 carbon atoms which may have a substituent, an alkoxy group having from 1 to 6 carbon atoms which may have a substituent, an alkylthio group having from 1 to 6 carbon atoms, an acyl group having from 2 to 5 carbon atoms or an alkoxycarbonyl group having from 2 to 5 carbon atoms;
   wherein the amino group among them may have 1 or 2 groups, as the substituent, selected from the group consisting of formyl group, an alkyl group having from 1 to 6 carbon atoms, a cycloalkyl group having from 1 to 6 carbon atoms, an aryl group having from 6 to 10 carbon atoms, an acyl group having from 2 to 5 carbon atoms and an alkoxycarbonyl group having from 2 to 5 carbon atoms, and when said amino group has 2 substituents, they may be bonded together to form a cyclic structure.
9. The compound, a salt thereof, or a solvate thereof described in the aforementioned 7, wherein R³ is a group represented by the following formula; 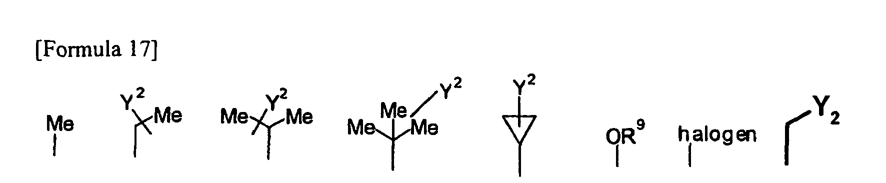 (in the formula, R⁹ means hydrogen atom, an alkyl group having from 1 to 6 carbon atoms, a cycloalkyl group having from 3 to 7 carbon atoms, an aryl group having from 6 to 10 carbon atoms, an aralkyl group having from 7 to 12 carbon atoms or an aromatic heterocyclic group, and the substituent Y² means amino group, hydroxy group, mercapto group, a halogen atom, an alkoxy group having from 1 to 6 carbon atoms, an alkylthio group having from 1 to 6 carbon atoms, an acyl group having from 2 to 5 carbon atoms or an alkoxycarbonyl group having from 2 to 5 carbon atoms, wherein the amino group among them may have 1 or 2 groups, as the substituent, selected from the group consisting of formyl group, an alkyl group having from 1 to 6 carbon atoms, a cycloalkyl group having from 1 to 6 carbon atoms, an aryl group having from 6 to 10 carbon atoms, an aromatic heterocyclic group, an acyl group having from 2 to 5 carbon atoms and an alkoxycarbonyl group having from 2 to 5 carbon atoms, and when said amino group has 2 substituents, they may be bonded together to form a cyclic structure).
10. The compound, a salt thereof, or a solvate thereof described in the aforementioned 7, wherein R³ is a group represented by the following formula; 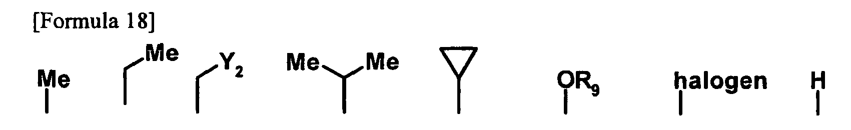 (in the formula, R⁹ means hydrogen atom, an alkyl group having from 1 to 6 carbon atoms, a cycloalkyl group having from 3 to 7 carbon atoms, an aryl group having from 6 to 10 carbon atoms, an aralkyl group having from 7 to 12 carbon atoms or an aromatic heterocyclic group, and the substituent Y² means amino group, hydroxy group, mercapto group, a halogen atom, an alkoxy group having from 1 to 6 carbon atoms, an alkylthio group having from 1 to 6 carbon atoms, an acyl group having from 2 to 5 carbon atoms or an alkoxycarbonyl group having from 2 to 5 carbon atoms, wherein the amino group among them may have 1 or 2 groups, as the substituent, selected from the group consisting of formyl group, an alkyl group having from 1 to 6 carbon atoms, a cycloalkyl group having from 1 to 6 carbon atoms, an aryl group having from 6 to 10 carbon atoms, an aromatic heterocyclic group, an acyl group having from 2 to 5 carbon atoms and an alkoxycarbonyl group having from 2 to 5 carbon atoms, and when said amino group has 2 substituents, they may be bonded together to form a cyclic structure).
11. The compound, a salt thereof, or a solvate thereof described in the aforementioned 9. or 10, wherein Y² is a halogen atom, alkoxy group having from 1 to 6 carbon atoms, hydroxy group or amino group, and R⁹ is hydrogen atom, an alkyl group having from 1 to 6 carbon atoms, a cycloalkyl group having from 3 to 7 carbon atoms, an aryl group having from 6 to 10 carbon atoms or an aralkyl group having from 7 to 12 carbon atoms.
12. The compound described in the aforementioned 9. or 10, wherein Y² is fluorine atom, chlorine atom, methoxy group or hydroxy group, and R⁹ is hydrogen atom, methyl group, ethyl group or isopropyl group.
13. The compound, a salt thereof, or a solvate thereof described in any one of the aforementioned 1. to 12, wherein R⁴ is an alkyl group having from 1 to 4 carbon atoms which may have a substituent, or a compound represented by the following formula; 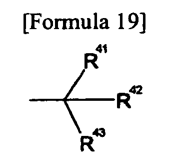 (R⁴¹, R⁴² and R⁴³ are as defined in the foregoing).
14. The compound, a salt thereof, or a solvate thereof described in any one of the aforementioned 1. to 12, wherein R⁴ is a substituent having a structure represented by the following formula; 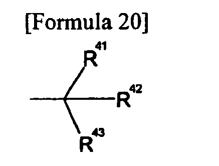 (R⁴¹ , R⁴² and R⁴³ are as defined in the foregoing).
15. A compound, a salt thereof, or a solvate thereof, which is a compound represented by the formula (I) having a combination in which
   R² is an aryl group;
   R¹ is a cyclic substituent having a saturated or partially saturated substituent;
   R³ is an alkyl group having from 1 to 3 carbon atoms;
   R⁴ is a substituent selected from the group consisting of (1) an alkyl or alkylene group having from 2 to 5 carbon atoms which may take a branched chain form, (2) a cyclic alkyl group having 3 or 4 carbon atoms, (3) an alkyl group having from 2 to 5 carbon atoms having fluorine atom or chlorine atom, which may take a branched chain form, (4) an alkoxyalkyl group having from 2 to 5 carbon atoms, and (6) a substituted benzyloxyethyl group which may have 1 or 2 methyl groups on the ethyl group.
16. A compound, a salt thereof, or a solvate thereof, which is a compound represented by the formula (I) having a combination in which
   R² is an aryl group;
   R¹ is a saturated or partially saturated nitrogen-containing heterocyclic group substituted with amino group, an alkylamino group or a dialkylamino group;
   R³ is an alkyl group having from 1 to 3 carbon atoms;
   R⁴ is a substituent selected from the group consisting of (1) an alkyl or alkylene group having from 2 to 5 carbon atoms which may take a branched chain form, (2) a cyclic alkyl group having 3 or 4 carbon atoms, (3) an alkyl group having from 2 to 5 carbon atoms having fluorine atom or chlorine atom, which may take a branched chain form, (4) an alkoxyalkyl group having from 2 to 5 carbon atoms, and (6) a substituted benzyloxyethyl group which may have 1 or 2 methyl groups on the ethyl group.
17. A compound, a salt thereof, or a solvate thereof, which is a compound represented by the formula (I) having a combination in which
   R² is phenyl group;
   R¹ is pyrrolidinyl group substituted with amino group, an alkylamino group or a dialkylamino group;
   R³ is methyl group;
   R⁴ is a substituent selected from the group consisting of ethyl group, isopropyl group, normal butyl group, tertiary butyl group, cyclopropyl group, propylen-2-yl group, methoxymethyl group, fluoromethyl group, 2-chloroethyl group, 2-hydroxyethyl group, 1,1-dimethyl-2-hydroxyethyl group, 2-benzyloxyethyl group, 2-benzyloxy-1,1-dimethyl-ethyl group and 2-(4-fluorophenylmethyl)oxyethyl group.
18. A compound, a salt thereof, or a solvate thereof, which is a compound represented by the formula (I) having a combination in which
   R² is phenyl group;
   R¹ is pyrrolidinyl group substituted with amino group, methylamino group or dimethylamino group;
   R³ is methyl group;
   R⁴ is a substituent selected from the group consisting of ethyl group, isopropyl group, normal butyl group, tertiary butyl group, cyclopropyl group, propylen-2-yl group, methoxymethyl group, fluoromethyl group, 2-chloroethyl group, 2-hydroxyethyl group, 1,1-dimethyl-2-hydroxyethyl group, 2-benzyloxyethyl group, 2-benzyloxy-1,1-dimethyl-ethyl group and 2-(4-fluorophenylmethyl)oxyethyl group.
19. A medicine which comprises the compound, a salt thereof, or a solvate thereof described in any one of the aforementioned 1. to 18.
20. An infection treating agent which comprises the compound, a salt thereof, or a solvate thereof described in any one of the aforementioned 1. to 18.
21. An antifungal agent which comprises the compound, a salt thereof, or a solvate thereof described in any one of the aforementioned 1. to 18.
22. A method for treating an infection, which uses the compound, a salt thereof, or a solvate thereof described in any one of the aforementioned 1. to 18.
23. Use of the compound, a salt thereof, or a solvate thereof described in any one of the aforementioned 1. to 18. for infection treatment.

### Effects of the Invention

The provides a compound capable of expressing an antifungal activity based on the functional mechanism of 1,6-β-glucan synthesis inhibition, with a broad spectrum and specifically or selectively, and provides an antifungal agent which comprises such a compound, a salt thereof or a solvate thereof.

### Best Mode for Carrying Out the Invention

Definitions of the terms used herein are as follows.
The "alkyl group" or the alkyl moiety of an alkyl moiety-containing substituent (e.g., an alkoxy group or the like) may be either straight chain or branched chain. Illustratively, methyl group, ethyl group, normal propyl group, normal butyl group, normal pentyl group, normal hexyl group, normal heptyl group, normal octyl group, normal nonyl group, normal undecyl group, normal dodecyl group, normal tridecyl group, normal tetradecyl group, normal pentadecyl group, normal hexadecyl group, normal heptadecyl group, normal octadecyl group, isopropyl group, isobutyl group, secondary butyl group, tertiary butyl group, isopentyl group, neopentyl group, tertiary pentyl group, isohexyl group, 1,1-dimethylpropyl group, n-heptyl group, n-octyl group and the like can be exemplified as the alkyl group.

The "cycloalkyl group" means a monocyclic or bicyclic cyclic alkyl group, and cyclopropyl group, cyclobutyl group, cyclopentyl group, cyclohexyl group, bicyclo[3.2.1]oct-2-yl group and the like can for example be cited.

The "alkenyl group" may be either straight chain or branched chain, and it has 1 or 2 or more of carbon-carbon double bond. Illustratively, vinyl group, propenyl group, buten-1-yl group, isobutenyl group, penten-1-yl group, 2-methylbuten-1-yl group, 3-methylbuten-1-yl group, hexen-1-yl group, hepten-1-yl group, octen-1-yl group and the like can be exemplified.

The "cycloalkenyl group" means a monocyclic or bicyclic cyclic alkenyl group, and 2-cyclopenten-1-yl group, 2,4-cyclopentadien-1-yl group, 5-norbomen-2-yl group and the like can for example be cited.

The "alkynyl group" may be either straight chain or branched chain, and it has 1 or 2 or more of carbon-carbon triple bond. Illustratively, ethynyl group, propynyl group and the like can be exemplified.

The "halogen atom" means fluorine atom, chlorine atom, bromine atom or iodine atom.

The "aryl group" means a monovalent group in which 1 hydrogen atom is removed from the aromatic ring of an aromatic hydrocarbon. The aromatic ring which constitutes the aryl group may be either a monocyclic ring or a condensed ring. For example, phenyl group, naphthyl group, anthryl group, azulenyl group and the like can be cited.

The "aralkyl group" means a group in which 1 or 2 or more hydrogen atoms of an alkyl group are replaced by the aforementioned aryl group. For example, benzyl group, benzhydryl group, trityl group and the like can be cited.

The "heterocyclic group" means a group derived from a saturated, partially saturated or unsaturated heterocyclic compound, which may be a monocyclic, bicyclic or spirocyclic. As the heterocyclic compound which gives a heterocyclic group, for example, aziridine, azetidine, pyrrole, furan, thiophene, pyrrolidine, tetrahydrofuran, tetrahydrothiophene, imidazole, pyrazole, imidazolidine, pyrazolidine, oxazole, isoxazole, thiazole, isothiazole, pyridine, dihydropyridine, tetrahydropyran, piperidine, pyridazine, pyrimidine, triazine, pyrazine, piperazine, pyrrolidone, dioxane, pyran, morpholine, benzofuran, indolizine, benzothiophene, indole, naphthyridine, quinoxaline, quinazoline, chroman and the like can be cited, and those which are represented by the following formulae can be further exemplified.

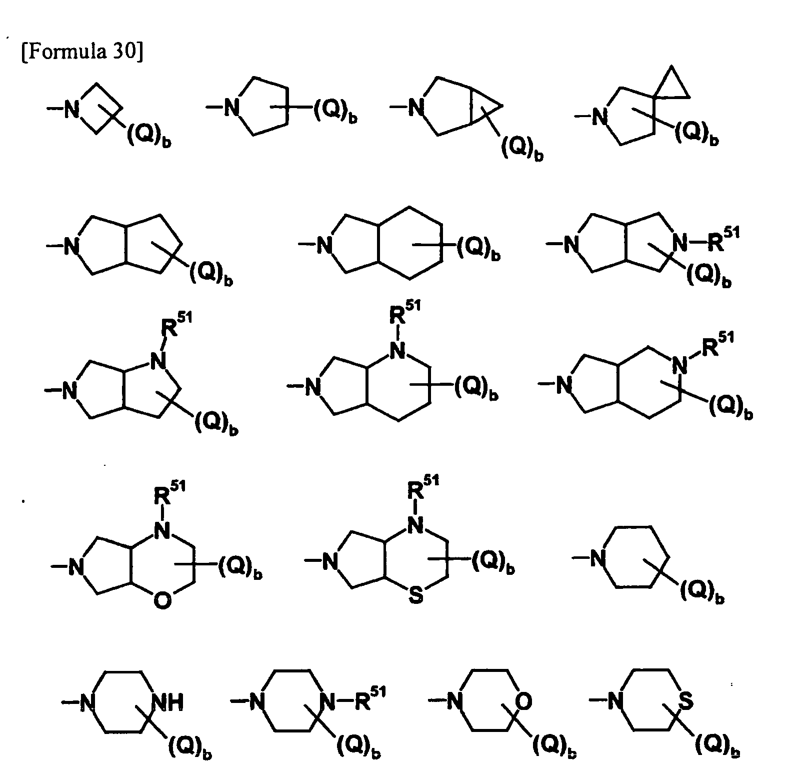

(In the formulae, R⁵¹ means hydrogen atom, an alkyl group having from 1 to 6 carbon atoms, a halogenoalkyl group having from 1 to 6 carbon atoms or a cycloalkyl group having from 3 to 6 carbon atoms, the substituent Q means a substituent represented by the following formula,

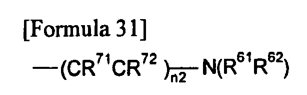

b means an integer of 0, 1 or 2, n2 means an integer of 0, 1 or 2, R⁶¹ and R⁶² each independently means hydrogen atom, an alkyl group having from 1 to 6 carbon atoms or a halogenoalkyl group having from 1 to 6 carbon atoms, or an amino acid, a dipeptide or a polypeptide consisting of 3 to 5 amino acids, and R⁷¹ and R⁷² each independently means hydrogen atom, an alkyl group having from 1 to 6 carbon atoms, a halogenoalkyl group having from 1 to 6 carbon atoms, a hydroxyalkyl group having from 3 to 6 carbon atoms, an aminoalkyl group having from 1 to 6 carbon atoms, an alkoxyalkyl group having from 2 to 12 carbon atoms, a cycloalkyl group having from 3 to 6 carbon atoms, a phenyl group which may have a substituent or a heteroaryl group having from 3 to 10 carbon atoms which may have a substituent.)

Hydrogen atom or an alkyl group is desirable as R⁵¹, and methyl group, ethyl group, normal propyl group or isopropyl group is desirable as the alkyl group.

Hydrogen atom or an alkyl group is desirable as R⁶¹ and R⁶², and methyl group, ethyl group, normal propyl group or isopropyl group is desirable as the alkyl group.

It is desirable that R⁷¹ and R⁷² are each independently hydrogen atom, an alkyl group, a halogenoalkyl group, an alkoxyalkyl group, a cycloalkyl group or phenyl group. Among them, hydrogen atom, methyl group, ethyl group, fluoromethyl group, trifluoromethyl group, 2-fluoroethyl group, methoxymethyl group, cyclopropyl group, cyclobutyl group or phenyl group is further desirable.

In addition, R⁷¹ and R⁷² may together form a cyclic structure having from 3 to 6 carbon atoms. Further, this ring may contain nitrogen atom as a ring constituting atom. As a preferred ring structure, cyclopropyl, cyclobutyl or cyclopentyl can be cited.

The "heteroaryl group" means particularly a group having an aromaticity (aromatic property) among the aforementioned heterocyclic groups, and means a group which is called "aromatic heterocycle". For example, a monocyclic 5-membered ring or 6-membered ring, a bicyclic benzo-fused ring system or heterocyclic -heterocyclic fused ring system, which is a 5-6 fused ring system or 6-6 fused ring system, and the like can be cited. For example, pyrrolyl group, furyl group, thienyl group, imidazolyl group, pyrazolyl group, oxazolyl group, isoxazolyl group, thiazolyl group, isothiazolyl group, pyridyl group, pyridazinyl group, pyrimidinyl group, triazinyl group, pyrazinyl group, benzofuryl indolyl group, naphthyridinyl group, quinoxalinyl group, quinazolinyl group and the like can be cited.
In addition, according to the description of the instant application, the "aromatic heterocyclic group" particularly means, among the aforementioned heteroaryl groups, a monocyclic 5-membered ring or 6-membered ring which contains from 1 to 4 hetero atoms of 1 or more species selected from the group consisting of nitrogen atom, oxygen atom and sulfur atom. For example, pyrrolyl group, furyl group, thienyl group, imidazolyl group, pyrazolyl group, oxazolyl group, isoxazolyl group, thiazolyl group, isothiazolyl group, pyridyl group, pyridazinyl group, pyrimidinyl group, triazinyl group, pyrazinyl group and the like can be cited.

The "protecting group" as described herein as "which may be protected with a protecting group", regarding amino group, hydroxy group, mercapto group or the like, is not particularly limited with the proviso that it is generally used in this field, and its examples include tertiary butoxycarbonyl group, 2,2,2-trichloroethoxycarbonyl group and the like alkoxycarbonyl groups; benzyloxycarbonyl group, paramethoxybenzyloxycarbonyl group, paranitrobenzyloxycarbonyl group and the like aralkyloxycarbonyl groups; acetyl group, methoxyacetyl group, trifluoroacetyl group, chloroacetyl group, pivaloyl group, formyl group, benzoyl group and the like acyl groups; tertiary butyl group, benzyl group, paranitrobenzyl group, paramethoxybenzyl group, triphenylmethyl group and the like alkyl groups, or aralkyl groups; methoxymethyl group, tertiary butoxymethyl group, tetrahydropyranyl group, 2,2,2-trichloroethoxymethyl group and the like ethers; and trimethylsilyl group, isopropyldimethylsilyl group, tertiary butyldimethylsilyl group, tribenzylsilyl group, tertiary butyldiphenylsilyl group and the like (alkyl and/or aralkyl) substituted silyl groups. In addition, amino group may be protected by forming phthalimide.
Examples of the "a group derived from an amino acid, a dipeptide or a polypeptide consisting of 3 to 5 amino acids" or "an amino acid, a dipeptide or a polypeptide consisting of 3 to 5 amino acids, which binds to amino group" include amino acids, dipeptides and tripeptides, or substituted carbonyl groups derived therefrom. That is, glycine, alanine, asparagine and the like amino acids, glycine-glycine, glycine-alanine, alanine-alanine and the like dipeptides and glycine-glycine-alanine, glycine-alanine-alanine and the like tripeptides, or substituted carbonyl groups derived therefrom, can be exemplified.

Partial structures and substituents of the compound of the present invention represented by the formula (I) are described.
In the compound represented by the following formula (I),

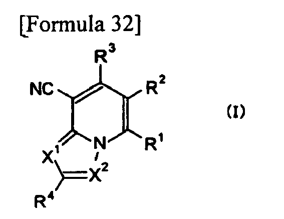

R¹ is a basic substituent. That is, it may be any substituent which has a basic property. As such a basic substituent;
(1) a wide variety of substituent having amino group as its substituent, and
(2) a nitrogen-containing heterocyclic substituent can be exemplified.
The wide variety of substituent having amino group as its substituent is a substituent having a structure in which the bicyclic nucleus and amino group are linked together through a wide variety of substituent as a linker.
Amino group is classified as a basic substituent, but its basic property is reduced when directly linked to the bicyclic nucleus like the instant application, so that it is desirably an amino group inserted with a certain substituent as the linker. Based on such a viewpoint, when an aromatic ring structure is used as the linker, a certain binding region is further required between it and amino group.
It is desirable that the nitrogen-containing heterocyclic ring substituent is a saturated or partially saturated nitrogen-containing heterocyclic substituent, rather than an aromatic heterocyclic substituent. It is desirable that this nitrogen-containing heterocyclic substituent is linked to the bicyclic nucleus through a carbon atom, and when it linked to the nucleus through the nitrogen atom, it is necessary that it further contains a nitrogen atom or has a basic substituent. In addition, it is desirable that this nitrogen-containing heterocyclic group also has a basic substituent, and amino group which may have a substituent is desirable as the basic substituent.
As the wide variety of substituent having amino group as the substituent;
(1) an amino substituted alkyl group having from 1 to 6 carbon atoms (aminoalkyl group; the alkyl group becomes the linker as alkylene group),
(2) an amino substituted cyclic alkyl group having from 3 to 6 carbon atoms (aminocycloalkyl group; the cycloalkyl group becomes the linker as cycloalkylene group),
(3) an aminocycloalkenyl group having from 3 to 6 carbon atoms (aminocycloalkyl group; the cycloalkenyl group becomes the linker),
(4) an amino substituted aralkyl group wherein the binding region with the bicyclic nucleus is an aromatic ring (aminoaralkyl group; the aralkyl group becomes the linker), and
(5) an aminoalkyl substituted amino group having from 1 to 6 carbon atoms (a structure in which an amino group is linked to the bicyclic nucleus, and this amino group is replaced by an amino group as a basic group using an alkylene group as the linker) can be exemplified.
As the amino substituted alkyl group among these, aminomethyl group, aminoethyl group, aminopropyl group and aminobutyl group are preferable, and aminoethyl group, aminopropyl group and aminomethyl group are more preferable.
As the amino substituted cyclic alkyl group, aminocyclopropyl group, aminocyclobutyl group, aminocyclopentyl group and aminocyclohexyl group are preferable.
As the amino substituted cycloalkenyl group, aminocyclobutenyl group and aminocyclohexenyl group can be exemplified as preferable groups. Cycloalkenyl group contains 1 unsaturated bond, but it is preferable that this unsaturated bond is present at a position where it does not conjugate with amino group.
As the amino substituted aralkyl group, aminomethylphenyl group, aminoethylphenyl group aminopropylphenyl group can be exemplified, of which aminomethylphenyl group and aminoethylphenyl are more preferable. It is preferable that the amino group is present on the alkyl group, more preferably at the terminus of the alkyl group. Position of the aminoalkyl group to the phenyl group has no particular limitation, and it may be any one of the ortho position, meta position and para position based on the binding posiiton of phenyl group to the bicyclic mother nucleus.
The cyclic alkyl group of amino substituted cyclic alkyl group and the cyclic cycloalkenyl group of amino substituted cycloalkenyl group may be substituted with 1 to 3 groups selected from the group consisting of hydrogen atom, a halogen atom, an alkyl group having from 1 to 6 carbon atoms, an alkoxy group having from 1 to 6 carbon atoms, a halogenoalkyl group having from 1 to 6 carbon atoms and a hydroxyalkyl group having from 1 to 6 carbon atoms. Preferred groups among them are methyl group, ethyl group, fluorine atom, chlorine atom, methoxy group, ethoxy group and hydroxymethyl group. More preferred are methyl group, fluorine atom, chlorine atom and hydroxymethyl group.
The aromatic ring of amino substituted aralkyl group may be substituted with 1 to 3 groups selected from the group consisting of hydroxy gruoup, a halogen atom, an alkyl group having from 1 to 6 carbon atoms, an alkoxy group having from 1 to 6 carbon atoms, a halogenoalkyl group having from 1 to 6 carbon atoms and a hydroxyalkyl group having from 1 to 6 carbon atoms. Preferred groups among them are methyl group, ethyl group, fluorine atom, chlorine atom, methoxy group, ethoxy group and hydroxymethyl group. More preferred are methyl group, methoxy group, fluorine atom, chlorine atom and hydroxymethyl group.
In addition, a basic substituent having a structure in which the aforementioned "wide variety of substituent having amino group as its substituent" is present on the amino group, and this amino group is further linked to the bicyclic mother nucleus, is also an example of the preferred substituent. Its examples include an aminoalkyl substituted amino group, an amino substituted cyclic alkylamino group, an aminocycloalkenylamino group and the like. In these groups, each of the amino group-substituted various substituents linked to the amino group which is bonded to the mother nucleus may be substituents having the same structure as described in the above.

In addition, the amino group (as a basic substituent) of the aminoalkyl group having from 1 to 8 carbon atoms, amino cyclic alkyl group having from 3 to 8 carbon atoms, amino substituted aralkyl group or amino alkyl substituted amino group of R¹ is an amino group which is protected by a protecting group or is possessed of 1 or 2 alkyl groups having from 1 to 6 carbon atoms (may have 1 or more substituents of 1 species or more of groups selected from the group consisting of hydroxy grouop, a halogen atom, an alkoxy group having from 1 to 6 carbon atoms and an alkylthio group, as the substituent) as the substituent, or to which an amino acid, a dipeptide or a polypeptide consisting of 3 to 5 amino acids may be bonded.

The amino group as the group for expressing the basic property may have a substituent but preferably 1 or 2 (may be the same or different when 2) of the substituents selected from the following substituent group [1-1];
substituent group [1-1]:
an alkyl group having from 1 to 6 carbon atoms, an alkenyl group having from 2 to 6 carbon atoms, an alkynyl group having from 2 to 6 carbon atoms, an alkoxycarbonyl group having from 2 to 7 carbon atoms, a cycloalkyl group having from 3 to 10 carbon atoms, a cycloalkenyl group having from 4 to 10 carbon atoms, and a group derived from an amino acid, a dipeptide or a polypeptide consisting of 3 to 5 amino acids.

Also, when the substituent selected from the substituent group [1-1] is an alkyl group, an alkenyl group, an alkynyl group, an alkoxycarbonyl group, a cycloalkyl group or a cycloalkenyl group, these may have 1 or more of 1 or more groups selected from [substituent group 1-1-1], as the substituent;

[substituent group 1-1-1]: hydroxy group, a mercapto group, a halogen atom, an alkoxy group having from 1 to 6 carbon atoms, an alkylthio group having from 1 to 6 carbon atoms and a cycloalkyl group having from 3 to 10 carbon atoms.
Preferred as the substituents to be selected from the substituent group [1-1] are an alkyl group having from 1 to 6 carbon atoms, a cycloalkyl group having from 3 to 10 carbon atoms or a cycloalkenyl group having from 4 to 10 carbon atoms, and for example, methyl group, ethyl group, propyl group, isopropyl group and a cycloalkyl group are more preferred groups. As the substituted amino group, a monoalkyl amino group and a dialkyl amino group are preferable. For example, methylamino group, ethylamino group, dimethylamino group, methylethylamino group, diethylamino group and the like are preferable, of which methylamino group, ethylamino group and dimethylamino group are more preferable.
Preferred as the groups to be selected from the substituent group [1-1-1] are hydroxy group, carboxyl group, a halogen atom, an alkoxy group having from 1 to 6 carbon atoms and an alkylthio group having from 1 to 6 carbon atoms, an alkylcarbonylamino group having from 2 to 7 carbon atoms, phenyl group which may have a substituent, or a heteroaryl group having from 3 to 10 carbon atoms which may have a substituent, and for example, fluorine atom, chlorine atom, methoxy group, ethoxy group, propoxy group, isopropoxy group, methylthio group, ethylthio group, acetylamino group, phenyl group and cyclopropyl group are preferable. More preferable among them are hydroxy group, fluorine atom and methoxy group.

When R¹ is a nitrogen-containing heterocyclic group which may have a substituent, the nitrogen-containing heterocyclic group is preferably a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom) which uses a carbon atom as the binding region and is saturated or partially saturated, and the substituent on this heterocyclic group may be selected from [substituent group 1-2];
[substituent group 1-2]: a halogen atom, amino group, hydroxy group, oxo group, a group represented by the following formula

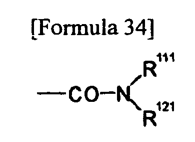

(in the formula, R¹¹¹ and R¹²¹ each independently represents hydrogen atom, an alkyl group having from 1 to 6 carbon atoms or an aryl group having from 6 to 10 carbon atoms), an alkyl group having from 1 to 6 carbon atoms, an aminoalkyl group having from 1 to 8 carbon atoms, an aminocycloalkyl group having from 3 to 8 carbon atoms, an alkoxy group having from 1 to 6 carbon atoms, an alkylthio group having from 1 to 6 carbon atoms, a halogenoalkyl group having from 1 to 6 carbon atoms and an alkylamino group having from 1 to 6 carbon atoms.

In this case, the alkyl moiety of the alkyl group, alkylamino group, cycloalkylamino group, alkoxy group, alkylthio group, halogenoalkyl group or aminoalkyl group of the [substituent group 1-2] may have 1 or more groups of 1 or more species selected from [substituent group 1-2-1];

[substituent group 1-2-1]: a halogen atom, hydroxy group, an alkyl group having from 1 to 6 carbon atoms, an alkoxy group having from 1 to 6 carbon atoms, an alkoxycarbonyl group having from 2 to 7 carbon atoms, an alkylcarbonylamino group having from 2 to 7 carbon atoms and an aryl group having from 6 to 10 carbon atoms.

In this case, the amino group moiety of the amino group, aminoalkyl group, aminocycloalkyl group and alkylamino group of the [substituent group 1-2] may be protected with a protecting group, and also may have 1 or 2 of alkyl groups having from 1 to 6 carbon atoms (may have 1 or more groups of 1 or more species selected from the group of groups consisting of hydroxy group, a halogen atom, and an alkoxy group and alkylthio group having from 1 to 6 carbon atoms, as the substituent), and also, an amino acid, a dipeptide or a polypeptide consisting of 3 to 5 amino acids may be linked thereto.

Also, it is a preferred embodiment of the present invention that R¹ is a nitrogen-containing heterocyclic group which may have a substituent, particularly a saturated or partially saturated (having 1 double bond) nitrogen-containing heterocyclic group. Illustratively, it is particularly desirable that R¹ is a group represented by the following formula.

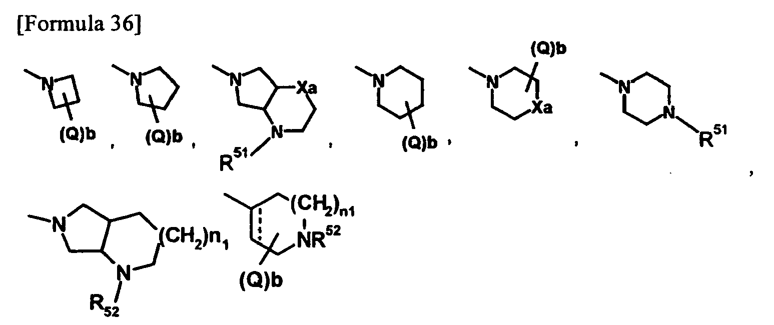

In the formula, Xa means oxygen atom, sulfur atom, a substituent or NR⁵², R⁵¹ and R⁵² each independently means hydrogen atom, an alkyl group having from 1 to 6 carbon atoms, a halogenoalkyl group having from 1 to 6 carbon atoms or a cycloalkyl group having from 3 to 6 carbon atoms, the substituent Q means a substituent represented by the following formula,

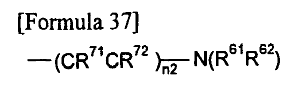

b means an integer of 0, 1 or 2, n1 means an integer of 0 or 1, n2 means an integer of 0, 1 or 2, R⁶¹ and R⁶² each independently means hydrogen atom, an alkyl group having from 1 to 6 carbon atoms or a halogenoalkyl group having from 1 to 6 carbon atoms, or a group derived from an amino acid, a dipeptide or a polypeptide consisting of 3 to 5 amino acids, R⁷¹ and R⁷² each independently mean hydrogen atom, an alkyl group having from 1 to 6 carbon atoms, a halogenoalkyl group having from 1 to 6 carbon atoms, a hydroxyalkyl group having from 1 to 6 carbon atoms, an aminoalkyl group having from 1 to 6 carbon atoms, an alkoxyalkyl group having from 2 to 12 carbon atoms, a cycloalkyl group having from 3 to 6 carbon atoms, a phenyl group which may have a substituent or a heteroaryl group having from 3 to 10 carbon atoms which may have a substituent.
In the aforementioned cyclic substituent, the presence of Q is essential when the nitrogen atom is the binding region to the bicyclic mother nucleus and other nitrogen atom is not present, and b is 1 or 2, but more preferably b is 1. When a carbon atom is the binding region of the cyclic substituent to the bicyclic mother nucleus, Q may not be present. When present, it is preferable that b is 1.
In either case, it is preferable that the respective parts which constitute Q are as follows. That is, n2 is most preferably 2, but may be 1 or 2. When n2 is 1, R⁷¹ and R⁷² in this part are each independently hydrogen atom or an alkyl group having from 1 to 6 carbon atoms (may have 1 or more groups of 1 or more species selected from the group consisting of hydroxy group, a halogen atom, an alkoxy group having from 1 to 6 carbon atoms and an alkylthio group, as the substituent), or R⁷¹ and R⁷² may be bonded with each other to form a ring structure (cycloalkyl having from 2 to 5 carbon atoms). Preferably, it is desirable that they are hydrogen atom, methyl group, ethyl group, isopropyl group, cyclopropane ring or cyclobutane ring.
It is preferable that R⁶¹ and R⁶² are a combination selected from hydrogen atom, methyl group, ethyl group and trifluoromethyl group, but it is more desirable that both are methyl group or only one of them is methyl group or ethyl group. In addition, when either one or both of R⁶¹ and R⁶² are a group derived from an amino acid or a derivative thereof, this is useful as a prodrug.
It is desirable that R⁵¹ and R⁵² are groups selected from hydrogen atom, methyl group, ethyl group and cyclopropyl group.
It is necessary that Xa is NR⁵² when Q is not present in the cyclic substituent, but it may be other hetero atom when Q is present. Preferably, Q is not present, that Xa is NR⁵², and this R⁵² is hydrogen atom or methyl group. (More preferred is a case of hydrogen atom.)
The dotted line means that said binding region may form a double bond.
A group containing a cyclic structure is preferable as R², so that preferred is a case in which it is an aryl group having from 6 to 10 carbon atoms, which may have a substituent, or a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom).

R² means hydrogen atom, halogen atom, carboxy group, a group represented by the following formula

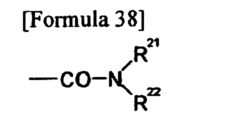

(in the formula, R²¹ and R²² each independently represents hydrogen atom, an alkyl group having from 1 to 6 carbon atoms or an aryl group having from 6 to 10 carbon atoms), an alkyl group having from 1 to 6 carbon atoms, an alkenyl group having from 2 to 6 carbon atoms, an alkynyl group having from 2 to 6 carbon atoms, an acyl group having from 2 to 7 carbon atoms, an alkoxycarbonyl group having from 2 to 7 carbon atoms, a cycloalkyl group having from 3 to 6 carbon atoms, a cycloalkenyl group having 5 or 6 carbon atoms, a cycloalkylalkyl group having from 4 to 12 carbon atoms, an aryl group having from 6 to 10 carbon atoms, an aralkyl group having from 7 to 12 carbon atoms, a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom), a heteroaryl group having from 3 to 10 carbon atoms, or a heteroarylalkyl group having from 3 to 12 carbon atoms.

In this case, when R² is an alkyl group, an alkenyl group, an alkynyl group, an acyl group or an alkoxycarbonyl group, these may have 1 or more groups of 1 or more species selected from [substituent group 2-1] as the substituent;
[substituent group 2-1]: halogen atom, amino group, imino group, nitro group, hydroxy group, mercapto group, carboxy group, cyano group, sulfo group, a dialkyl phosphoryl group, a group represented by the following formula

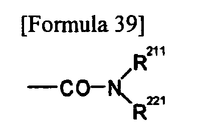

(in the formula, R²¹¹ and R²²¹ each independently represents hydrogen atom, an alkyl group having from 1 to 6 carbon atoms or an aryl group having from 6 to 10 carbon atoms), an alkoxy group having from 1 to 6 carbon atoms, an alkylthio group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, and an arylthio group having from 6 to 10 carbon atoms.

In this case, the amino group of the [substituent group 2-1] may have 1 or 2 groups, as the substituent, selected from the group consisting of formyl group, an alkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, an aralkyl group having from 7 to 12 carbon atoms, an aromatic heterocyclic group, an alkylsulfonyl group having from 1 to 6 carbon atoms and an arylsulfonyl group having from 6 to 10 carbon atoms, in addition, when said amino group has 2 substituents, they may be bonded together to form a cyclic structure.

Hydroxyl group of the [substituent group 2-1] or mercapto group of the [substituent group 2-1] may have a substituent selected from the group consisting of an alkyl group having from 1 to 6 carbon atoms, an aminoalkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, a cycloalkyl group having from 3 to 6 carbon atoms, an aryl group having from 6 to 10 carbon atoms, an aralkyl group having from 7 to 12 carbon atoms and an aromatic heterocyclic group.

When R² is a cycloalkyl group, these may have 1 or more groups of 1 or more species selected from [substituent group 2-2] as the substituent;
[substituent group 2-2]: halogen atom, amino group, imino group, nitro group, hydroxy group, mercapto group, carboxy group, cyano group, sulfo group, a group represented by the following formula

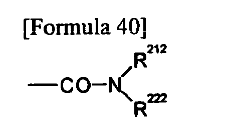

(in the formula, R²¹² and R²²² each independently represents hydrogen atom, an alkyl group having from 1 to 6 carbon atoms or an aryl group having from 6 to 10 carbon atoms), an alkoxy group having from 1 to 6 carbon atoms, an alkylthio group having from 1 to 6 carbon atoms, an acyl group having from 2 to 7 carbon atoms and an alkoxycarbonyl group having from 2 to 7 carbon atoms.

Amino group of the [substituent group 2-2] may have 1 or 2 groups, as the substituent, selected from the group consisting of formyl group, an alkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, an aralkyl group having from 7 to 12 carbon atoms, an aromatic heterocyclic group, an alkylsulfonyl group having from 1 to 6 carbon atoms and an arylsulfonyl group having from 6 to 10 carbon atoms, in addition, when said amino group has 2 substituents, they may be bonded together to form a cyclic structure.

When R² is an aryl group, an aralkyl group, a heteroaryl group or a heteroarylalkyl group, these may have 1 or more groups of 1 or more species selected from [substituent group 2-3] as the substituent;
[substituent group 2-3]: halogen atom, amino group, imino group, nitro group, hydroxy group, mercapto group, carboxy group, cyano group, sulfo group,
a group represented by the following formula

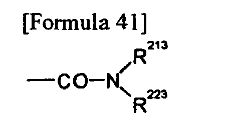

(in the formula, R²¹³ and R²²³ each independently represents hydrogen atom, an alkyl group having from 1 to 6 carbon atoms or an aryl group having from 6 to 10 carbon atoms), an alkoxy group having from 1 to 6 carbon atoms, an alkylthio group having from 1 to 6 carbon atoms, an acyl group having from 2 to 7 carbon atoms, an alkoxycarbonyl group having from 2 to 7 carbon atoms, an aralkyloxy group having from 7 to 12 carbon atoms, an aralkyloxycarbonyl group having from 8 to 15 carbon atoms, an aryl group and a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom).

Amino group of the [substituent group 2-3] may have 1 or 2 groups, as the substituent, selected from the group consisting of formyl group, an alkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, an aralkyl group having from 7 to 12 carbon atoms, an aromatic heterocyclic group, an alkylsulfonyl group having from 1 to 6 carbon atoms and an arylsulfonyl group having from 6 to 10 carbon atoms, in addition, when said amino group has 2 substituents, they may be bonded together to form a cyclic structure.

When R² is a heterocyclic group, it may have 1 or 2 groups selected from the next [substituent group 2-4] as the substituent;
[substituent group 2-4]: halogen atom, amino group, hydroxy group, mercapto group, carboxy group, sulfo group, a group represented by the following formula

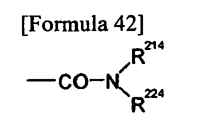

(in the formula, R²¹⁴ and R²²⁴ each independently represents hydrogen atom, an alkyl group having from 1 to 6 carbon atoms or an aryl group having from 6 to 10 carbon atoms), an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, an alkylthio group having from 1 to 6 carbon atoms, a halogenoalkyl group having from 1 to 6 carbon atoms, an acyl group having from 2 to 7 carbon atoms, an alkoxycarbonyl group having from 2 to 7 carbon atoms and an aryl group having from 6 to 10 carbon atoms.

In this case, the amino group of the [substituent group 2-4] may have 1 or 2 groups, as the substituent, selected from the group consisting of formyl group, an alkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, an aralkyl group having from 7 to 12 carbon atoms, a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom), an aromatic heterocyclic group, an alkylsulfonyl group having from 1 to 6 carbon atoms and an arylsulfonyl group having from 6 to 10 carbon atoms, in addition, when said amino group has 2 substituents, they may be bonded together to form a cyclic structure.

Among them, R² is preferably an aryl group having from 6 to 10 carbon atoms, which may have a substituent, or a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom), more preferably, R² is a group represented by the following formulae.

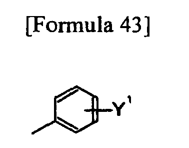

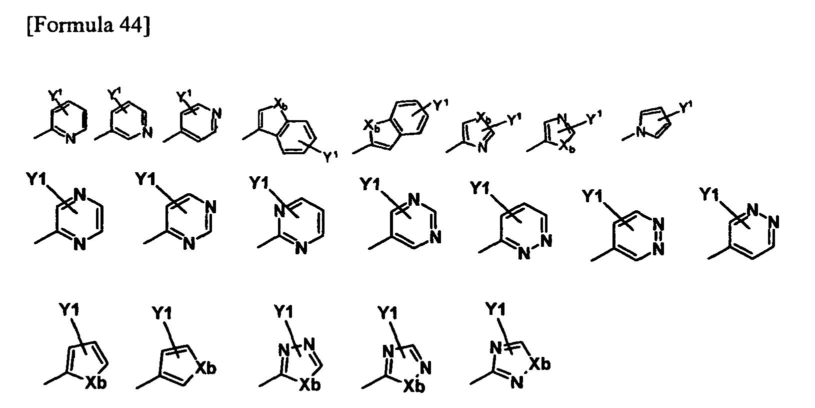

(In the formulae, Xb means oxygen atom, sulfur atom, a substituent or NR⁸, wherein R⁸ means hydrogen atom, an alkyl group having from 1 to 6 carbon atoms or a halogenoalkyl group having from 1 to 6 carbon atoms, and the substituent Y¹ has the same meaning as described in the aforementioned [substituent group 2-2].)
Among them, preferred as Y¹ is a halogen atom, amino group, nitro group, hydroxy group, cyano group, alkoxycarbonyl group, carboxyl group, a group represented by the following formula

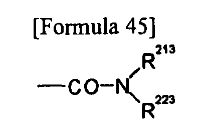

(in the formula, R²¹³ and R²²³ each independently represents hydrogen atom, an alkyl group having from 1 to 6 carbon atoms or an aryl group having from 6 to 10 carbon atoms), and an alkoxy group having from 1 to 6 carbon atoms.
More preferred among these substituents are a halogen atom, amino group, hydroxy group, cyano group, an alkyl group having from 1 to 3 carbon atoms and an alkoxy group having from 1 to 6 carbon atoms, and are illustratively fluorine atom, chlorine atom, amino group, hydroxy group, cyano group, methyl group and methoxy group.
Also, when Y¹ is amino group, this amino group may have 1 or 2 groups, as the substituent, selected from the group consisting of formyl group, an alkyl group having from 1 to 6 carbon atoms, an aminoalkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, an aralkyl group having from 7 to 12 carbon atoms, an aromatic heterocyclic group, an alkylsulfonyl group having from 1 to 6 carbon atoms and an arylsulfonyl group having from 6 to 10 carbon atoms. Among them, an alkyl group having from 1 to 6 carbon atoms and a cycloalkyl group having from 3 to 6 carbon atoms are preferable. Illustratively, methyl group, ethyl group, propyl group and cyclopropyl group are preferable. When the amino group has 2 substituents, they may be bonded together to form a cyclic structure.

In addition, R¹ and R² may together form a cyclic structure by including the carbon atoms to which these are bonded, wherein this ring contains 1 or 2 hetero atoms of 1 or more species selected from the group consisting of nitrogen atom, oxygen atom and sulfur atom, and the structural moiety to be formed herein may be saturated or unsaturated. As illustrative examples of the heterocyclic ring structure formed from R¹ and R², those which are represented by the following formula can for example be cited (in this connection, this drawing shows the structure to be formed as a partial structure, and the nitrogen atom "N" described in the partial structure is a nitrogen atom which is present in the 6-membered ring of the bicyclic mother nucleus and bonded with X²).

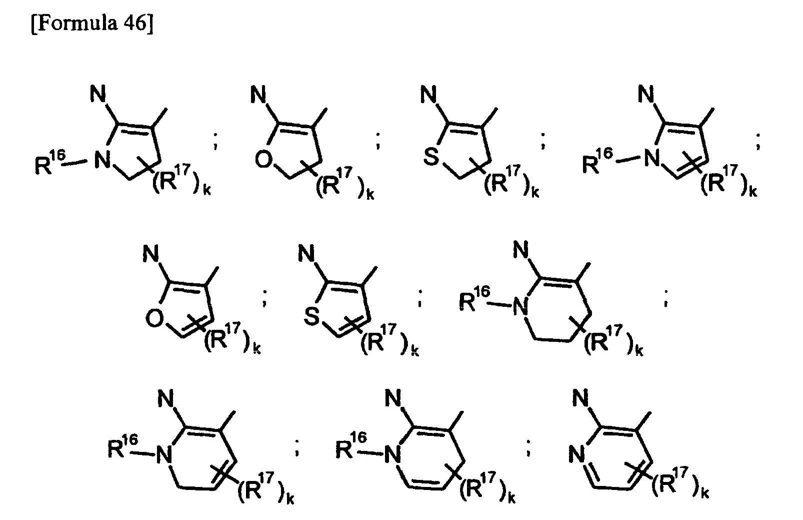

(In the formula, R¹⁶ means hydrogen atom, an alkyl group having from 1 to 6 carbon atoms, an alkenyl group having from 2 to 6 carbon atoms, an alkynyl group having from 2 to 6 carbon atoms, a halogenoalkyl group having from 1 to 6 carbon atoms or a cycloalkyl group having from 3 to 6 carbon atoms, R¹⁷ means hydrogen atom, a halogen atom, amino group which may have a substituent, hydroxy group, thiol group, an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, an alkylthio group having from 1 to 6 carbon atoms, a halogenoalkyl group having from 1 to 6 carbon atoms, a bicycloalkyl alkyl group having from 3 to 6 carbon atoms, which may have a halogen atom, or a spirocycloalkyl group having from 3 to 6 carbon atoms, which may have a halogen atom, and k means an integer of 1 or 2.)
Preferred as R¹⁶ are hydrogen atom, an alkyl group having from 1 to 6 carbon atoms, a halogenoalkyl group having from 1 to 6 carbon atoms and a cycloalkyl group having from 3 to 6 carbon atoms. Among them, hydrogen atom, an alkyl group having from 1 to 6 carbon atoms and a cycloalkyl group having from 3 to 6 carbon atoms are preferred. Illustratively, methyl group, ethyl group, propyl group, isopropyl group and cyclopropyl group can be exemplified.
Preferred as R¹⁷ is amino group which may have a substituent, and when this amino group has a substituent, an alkyl group and a cycloalkyl group are preferable. Illustratively, methyl group, ethyl group, propyl group, isopropyl group and cyclopropyl group can be exemplified. It is preferable that this has 1 or 2 of them.

R³ means hydrogen atom, halogen atom, amino group, hydroxy group, mercapto group, nitro group, cyano group, formyl group, carboxy group, a group represented by the following formula

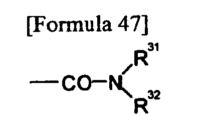

(in the formula, R³¹ and R³² each independently represents hydrogen atom, an alkyl group having from 1 to 6 carbon atoms or an aryl group having from 6 to 10 carbon atoms), an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, an alkylthio group having from 1 to 6 carbon atoms an acyl group having from 2 to 5 carbon atoms, an alkoxycarbonyl group having from 2 to 5 carbon atoms, a cycloalkyl group having from 3 to 7 carbon atoms, a cycloalkenyl group having from 4 to 7 carbon atoms, an aryl group having from 6 to 10 carbon atoms, an aralkyl group having from 7 to 12 carbon atoms, a heteroaryl group having from 3 to 10 carbon atoms, wherein said amino group, said hydroxy group or said mercapto group may be protected by a protecting group.

When R³ is an alkyl group, an alkenyl group, an alkynyl group, an alkoxy group, an alkylthio group, an acyl group, an alkoxycarbonyl group, a cycloalkyl group, a cycloalkenyl group, an aryl group, an aralkyl group or a heteroaryl group, these may have 1 or more groups of 1 or more species selected from [substituent group 3-1] as the substituent; [substituent group 3-1]: amino group, hydroxy group, mercapto group, a halogen atom, an alkoxy group having from 1 to 6 carbon atoms, an alkylthio group having from 1 to 6 carbon atoms, an acyl group having from 2 to 5 carbon atoms, and an alkoxycarbonyl group having from 2 to 5 carbon atoms.

The amino group of the [substituent group 3-1] may have 1 or 2 groups, as the substituent, selected from the group consisting of formyl group, an alkyl group having from 1 to 6 carbon atoms, a cycloalkyl group having from 3 to 6 carbon atoms, an aryl group having from 6 to 10 carbon atoms, an aromatic heterocyclic group, an acyl group having from 2 to 5 carbon atoms and an alkoxycarbonyl group having from 2 to 5 carbon atoms, wherein when said amino group has 2 substituents, they may be bonded together to form a cyclic structure.

Preferred R³ among them is a halogen atom, amino group, hydroxy group, mercapto group, an alkyl group having from 1 to 4 carbon atoms which may have a substituent, an alkoxy group having from 1 to 6 carbon atoms which may have a substituent, an alkylthio group having from 1 to 6 carbon atoms, an acyl group having from 2 to 5 carbon atoms or an alkoxycarbonyl group having from 2 to 5 carbon atoms. The amino group among them may have 1 or 2 groups, as the substituent, selected from the group consisting of formyl group, an alkyl group having from 1 to 6 carbon atoms, a cycloalkyl group having from 1 to 6 carbon atoms, an aryl group having from 6 to 10 carbon atoms, a heteroaryl group having from 3 to 10 carbon atoms, an acyl group having from 2 to 5 carbon atoms and an alkoxycarbonyl group having from 2 to 5 carbon atoms, and when said amino group has 2 substituents, they may be bonded together to form a cyclic structure. Illustratively desirable R³ are groups represented by the following formulae.

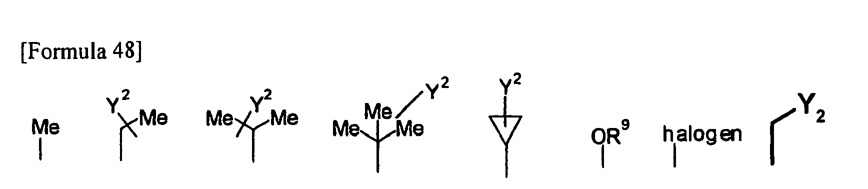

(In the formulae, Me means methyl group, halogen means a halogen atom, and R⁹ means hydrogen atom, an alkyl group having from 1 to 6 carbon atoms, a cycloalkyl group having from 3 to 7 carbon atoms, an aryl group having from 6 to 10 carbon atoms, an aralkyl group having from 7 to 12 carbon atoms or an aromatic heterocyclic group. Also, the substituent Y² means amino group, hydroxy group, mercapto group, a halogen atom, an alkoxy group having from 1 to 6 carbon atoms, an alkylthio group having from 1 to 6 carbon atoms, an acyl group having from 2 to 5 carbon atoms or an alkoxycarbonyl group having from 2 to 5 carbon atoms, wherein the amino group among them may have 1 or 2 groups, as the substituent, selected from the group consisting of formyl group, an alkyl group having from 1 to 6 carbon atoms, a cycloalkyl group having from 1 to 6 carbon atoms, an aryl group having from 6 to 10 carbon atoms, an aromatic heterocyclic group, an acyl group having from 2 to 5 carbon atoms and an alkoxycarbonyl group having from 2 to 5 carbon atoms, and when said amino group has 2 substituents, they may be bonded together to form a cyclic structure.)

More preferred substituents are the groups represented by the following formulae. 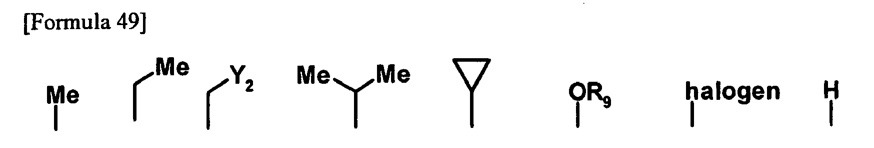 The substituents preferable as Y² are a halogen atom, an alkoxy group having from 1 to 6 carbon atoms, hydroxy group and amino group. More preferred groups are fluorine atom, chlorine atom, methoxy group and hydroxy group.
The substituents preferable as R⁹ are hydrogen atom, an alkyl group having from 1 to 6 carbon atoms, a cycloalkyl group having from 3 to 7 carbon atoms, an aryl group having from 6 to 10 carbon atoms and an aralkyl group having from 7 to 12 carbon atoms, and more preferably are hydrogen atom, methyl group, ethyl group and isopropyl group. (Preferred as the halogen atom is fluorine atom or chlorine atom.)
On the other hand, R² and R³ may together form a polymethylene chain structure and form a 5-membered or 6-membered cyclic structure by including the carbon atoms to which R² and R³ are to be bonded. In addition, this polymethylene chain may contain 1 or 2 hetero atoms of 1 or more species selected from the group consisting of nitrogen atom, oxygen atom and sulfur atom.
The polymethylene chain formed herein may have 1 or more groups of 1 or more species selected from [substituent group 3-2] as the substituent; [substituent group 3-2]: amino group, hydroxy group, mercapto group, a halogen atom, an alkoxy group having from 1 to 6 carbon atoms, an alkylthio group having from 1 to 6 carbon atoms, an acyl group having from 2 to 5 carbon atoms and an alkoxycarbonyl group having from 2 to 5 carbon atoms.

The amino group of the [substituent group 3-2] may have 1 or 2 groups, as the substituent, selected from the group consisting of formyl group, an alkyl group having from 1 to 6 carbon atoms, a cycloalkyl group having from 1 to 6 carbon atoms, an aryl group having from 6 to 10 carbon atoms, an aromatic heterocyclic group, an acyl group having from 2 to 5 carbon atoms and an alkoxycarbonyl group having from 2 to 5 carbon atoms, wherein when said amino group has 2 substituents, they may be bonded together to form a cyclic structure.

R⁴ means hydrogen atom, a halogen atom, amino group, hydroxy group, mercapto group, nitro group, cyano group, formyl group, carboxy group, a group represented by the following formula 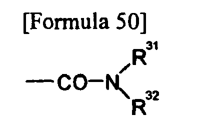 (in the formula, R³¹ and R³² each independently represents hydrogen atom, an alkyl group having from 1 to 6 carbon atoms or an aryl group having from 6 to 10 carbon atoms), an alkyl group having from 1 to 4 carbon atoms, an cyclic alkyl group having from 3 to 8 carbon atoms, an aryl group having from 6 to 10 carbon atoms, a heteroaryl group having from 5 to 9 carbon atoms, an alkynyl group having from 2 to 6 carbon atoms, or a group represented by the following formula

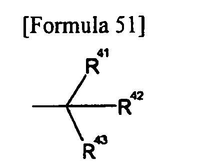

(in the formula, R⁴¹ and R⁴² each independently represents hydrogen atom, an alkyl group having from 1 to 6 carbon atoms or an alkoxy group having from 1 to 6 carbon atoms, or both may together form an exomethylene structure, and this exomethylene structure may further have an alkyl group having from 1 to 6 carbon atoms, an alkoxy group having from 1 to 6 carbon atoms or a halogenoalkyl group having from 1 to 6 carbon atoms, as a substituent.

R⁴³ means hydrogen atom, a halogen atom, hydroxy group, mercapto group, nitrile group, nitro group, carboxy group, an alkoxycarbonyl group having from 2 to 7 carbon atoms, an alkylaminocarbonyl group having from 2 to 7 carbon atoms, an arylaminocarbonyl group having from 7 to 11 carbon atoms, a cycloalkylaminocarbonyl group having from 2 to 7 carbon atoms, an aralkylaminocarbonyl group having from 8 to 12 carbon atoms, an alkyl group having from 1 to 6 carbon atoms, a halogenoalkyl group having from 1 to 6 carbon atoms, a hydroxyalkyl group having from 1 to 6 carbon atoms, an aminoalkyl group having from 1 to 6 carbon atoms, an alkoxy group having from 1 to 6 carbon atoms, a cycloalkyl group having from 3 to 8 carbon atoms, a cycloalkyloxy group having from 3 to 8 carbon atoms, an aralkyl group having from 7 to 11 carbon atoms, or an aralkyloxy group having from 7 to 11 carbon atoms).

When R⁴ is an alkyl group, a cyclic alkyl group, an aryl group or a heteroaryl group, and when R⁴³ is an alkyl group, these may have 1 or more groups of 1 or more species selected from [substituent group 4] as the substituent;
[substituent group 4]: halogen atom, amino group, nitro group, hydroxy group, mercapto group, carboxy group, cyano group, sulfo group, a group represented by the following formula

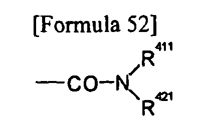

(in the formula, R⁴¹¹ and R⁴²¹ each independently means hydrogen atom, an alkyl group having from 1 to 6 carbon atoms or an aryl group having from 6 to 10 carbon atoms), an alkoxy group having from 1 to 6 carbon atoms, an alkylthio group having from 1 to 6 carbon atoms, an acyl group having from 2 to 7 carbon atoms, an alkoxycarbonyl group having from 2 to 7 carbon atoms, an aralkyloxy group having from 7 to 12 carbon atoms, an aralkyloxycarbonyl group having from 8 to 15 carbon atoms, an aryl group having from 6 to 10 carbon atoms, and a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom).

The amino group of the [substituent group 4] may have 1 or 2 groups, as the substituent, selected from the group consisting of formyl group, an alkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, an aralkyl group having from 7 to 12 carbon atoms, an aromatic heterocyclic group, an alkylsulfonyl group having from 1 to 6 carbon atoms and an arylsulfonyl group having from 6 to 10 carbon atoms, wherein when said amino group has 2 substituents, they may be bonded together to form a cyclic structure.

The hydroxy group or mercapto group of the [substituent group 4] may have a substituent selected from the group consisting of an alkyl group having from 1 to 6 carbon atoms, an aminoalkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, a cycloalkyl group having from 3 to 6 carbon atoms, an aryl group having from 6 to 10 carbon atoms, an aralkyl group having from 7 to 12 carbon atoms and an aromatic heterocyclic group, and when R⁴ is an alkynyl group, it may have an alkyl group having from 1 to 6 carbon atoms, an alkoxyalkyl group having from 2 to 12 carbon atoms, a halogenoalkyl group having from 1 to 6 carbon atoms or carboxy group as a substituent.

A preferred R⁴ among them is an alkyl group having from 1 to 4 carbon atoms, which may have a substituent, or a substituent having a structure represented by the following formula

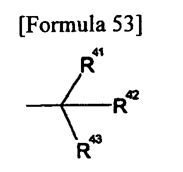

(R⁴¹, R⁴² and R⁴³ are as defined in the foregoing).
Those which are preferable when R⁴ is the substituent represented by the aforementioned formula are groups represented by the following formulae. 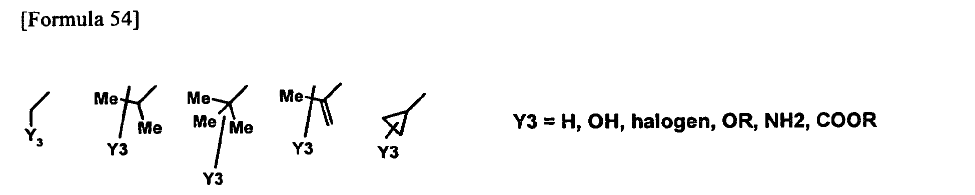 Preferred among them are groups represented by the following formulae. 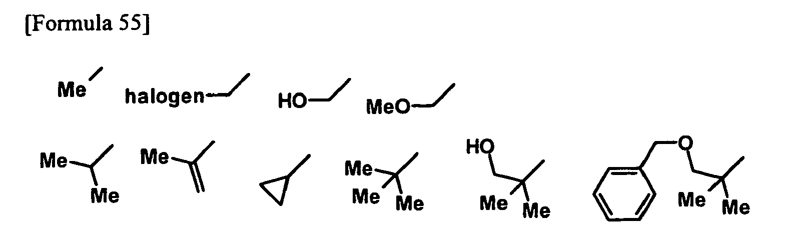 One of the preferred embodiments as R⁴ is,
(1) an alkyl group or alkylene group which may become a branched chain having from 2 to 5 carbon atoms, (2) a cyclic alkyl group having 3 or 4 carbon atoms, (3) an alkyl group which may become a branched chain having from 2 to 5 carbon atoms and have fluorine atom or chlorine atom, (4) an alkoxyalkyl group having from 2 to 5 carbon atoms, or (6) a substituted benzyloxyethyl group which may have 1 or 2 methyl groups on the ethyl group.
Illustratively, it is ethyl group, isopropyl group, normal butyl group, tertiary butyl group, cyclopropyl group, propylen-2-yl group, methoxymethyl group, fluoromethyl group, 2-chloroethyl group, 2-hydroxyethyl group, 1,1-dimethyl-2-hydroxyethyl group, 2-benzyloxyethyl group, 2-benryloxy-1,1-dimethyl-ethyl group or 2-(4-fluorophenylmethyl)oxyethyl group or the like.

Even when particularly clearly described, the aryl group, heteroaryl group or heterocyclic group in the description of the aforementioned substituents R¹, R², R³ and R⁴ may have, as the substituent, 1 or more groups of 1 or more species selected from the group consisting of a halogen atom, amino group, hydroxy group, mercapto group, nitro group, cyano group, carboxy group, sulfo group, a group represented by the following formula

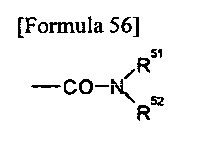

(in the formula, R⁵¹ and R⁵² each independently means hydrogen atom, an alkyl group having from 1 to 6 carbon atoms or an aryl group having from 6 to 10 carbon atoms), an alkyl group having from 1 to 10 carbon atoms, an alkenyl group having from 2 to 10 carbon atoms, an alkynyl group having from 2 to 10 carbon atoms, an alkoxy group having from 1 to 10 carbon atoms, an alkylthio group having from 1 to 10 carbon atoms, a halogenoalkyl group having from 1 to 10 carbon atoms, an acyl group having from 2 to 10 carbon atoms, an alkoxycarbonyl group having from 2 to 10 carbon atoms, an aryl group having from 6 to 10 carbon atoms and an aromatic heterocyclic group, wherein

said alkyl group, said alkoxy group, said alkylthio group, said acyl group, said alkoxycarbonyl group, said aryl group or said aromatic heterocyclic group may have 1 or more groups of 1 or more species selected from the group consisting of a halogen atom, hydroxy group, an alkoxy group having from 1 to 6 carbon atoms and an alkylthio group having from 1 to 6 carbon atoms, as the substituent, and

said amino group may have 1 or 2 groups, as the substituent, selected from the group consisting of formyl group, an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, an alkoxycarbonyl group having from 2 to 8 carbon atoms, a cycloalkyl group having from 3 to 10 carbon atoms, an aryl group having from 6 to 10 carbon atoms, an aralkyl group having from 7 to 16 carbon atoms, a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom), an aromatic heterocyclic group, an alkylsulfonyl group having from 1 to 10 carbon atoms and an arylsulfonyl group having from 6 to 10 carbon atoms, in addition, when said amino group has 2 substituents, they may be bonded together to form a cyclic structure. As such a cyclic structure, those which are represented by the following formulae can be exemplified.

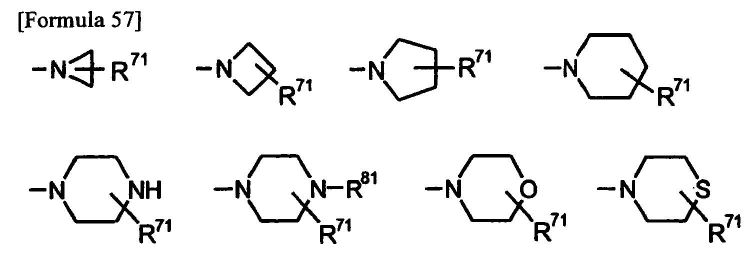

(In the formulae, R⁷¹ means hydrogen atom, a halogen atom, hydroxy group, thiol group, an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, an alkylthio group having from 1 to 6 carbon atoms, a halogenoalkyl group having from 1 to 6 carbon atoms, a bicycloalkyl group having from 3 to 6 carbon atoms, which may have a halogen atom, or a spirocycloalkyl group having from 3 to 6 carbon atoms, which may have a halogen atom, and R⁸¹ means an alkyl group having from 1 to 6 carbon atoms or a halogenoalkyl group having from 1 to 6 carbon atoms.)

Even when particularly clearly described, the amino group in the description of the aforementioned substituents R¹, R², R³ and R⁴ may have, as the substituent, 1 or 2 of the groups selected from the group consisting of formyl group, an alkyl group having from 1 to 6 carbon atoms, an acyl group having from 2 to 18 carbon atoms, an alkoxycarbonyl group having from 2 to 18 carbon atoms, an alkylsulfonyl group having from 1 to 18 carbon atoms and an arylsulfonyl group having from 6 to 10 carbon atoms, in addition, when said amino group has 2 substituents, they may be bonded together to form a cyclic structure. As such a cyclic structure, those which are represented by the following formulae can be exemplified.

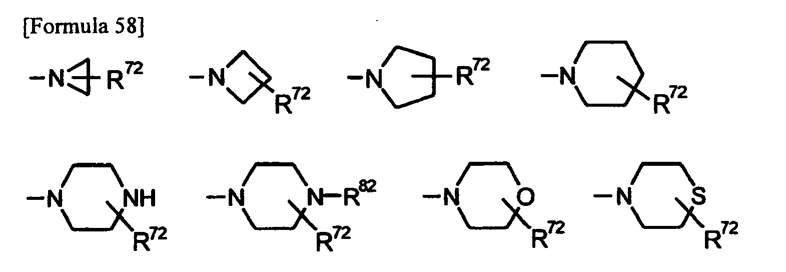

(In the formulae, R⁷² means hydrogen atom, a halogen atom, hydroxy group, thiol group, an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, an alkylthio group having from 1 to 6 carbon atoms, a halogenoalkyl group having from 1 to 6 carbon atoms, a bicycloalkyl group having from 3 to 6 carbon atoms, which may have a halogen atom, or a spirocycloalkyl group having from 3 to 6 carbon atoms, which may have a halogen atom, and R⁸² means an alkyl group having from 1 to 6 carbon atoms or a halogenoalkyl group having from 1 to 6 carbon atoms.)

X¹ and X² each independently means nitrogen atom or carbon atom which may be substituted by a halogen atom, an alkoxy group having from 1 to 6 carbon atoms, an alkyl group having from 1 to 6 carbon atoms, which may have a substituent, or an ester group, wherein either one of X¹ and X² is nitrogen atom.

In this case, the substituent of alkyl group is 1 or 1 or more groups selected from the following group of substituents;
a halogen atom, amino group, nitro group, hydroxy group, mercapto group, carboxy group, cyano group, an alkoxy group having from 1 to 6 carbon atoms, an alkylthio group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, and an aryl group having from 6 to 10 carbon atoms;

wherein when the substituents on carbon atoms are esters, these may be an alkyl ester having from 1 to 6 carbon atoms, an aryl ester having from 6 to 10 carbon atoms, or an aralkyl ester consisting of an alkyl group having from 1 to 6 carbon atoms and an aryl group having from 6 to 10 carbon atoms;

in addition, the aryl moiety of these aryl esters and aralkyl groups may be substituted with 1 or 1 or more of groups selected from the following group of substituents;
a halogen atom, amino group, nitro group, hydroxy group, mercapto group, carboxy group, cyano group, an alkyl group having from 1 to 6 carbon atoms, an alkoxy group having from 1 to 6 carbon atoms, an alkylthio group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms and an aryl group having from 6 to 10 carbon atoms.
It is preferable that X¹ and X² are both nitrogen atom.

One of the preferred embodiments of the combination of respective substituents of the compound of the present invention represented by the formula (I) is a combination in which R² is an aryl group; R¹ is a cyclic substituent having a saturated or partially saturated substituent; R³ is an alkyl group having from 1 to 3 carbon atoms; and R⁴ is a substituent selected from the group consisting of (1) an alkyl group or alkylene group which may become a branched chain having from 2 to 5 carbon atoms, (2) a cyclic alkyl group having 3 or 4 carbon atoms, (3) an alkyl group which may become a branched chain having from 2 to 5 carbon atoms and have fluorine atom or chlorine atom, (4) an alkoxyalkyl group having from 2 to 5 carbon atoms, and (6) a substituted benzyloxyethyl group which may have 1 or 2 methyl groups on the ethyl group.
One of the further preferred embodiments is a combination in which R² is an aryl group; R¹ is amino group, a saturated or partially saturated nitrogen-containing heterocyclic group substituted with alkylamino group or a dialkylamino group; R³ is an alkyl group having from 1 to 3 carbon atoms; and R⁴ is a substituent selected from the group consisting of (1) an alkyl group or alkylene group which may become a branched chain having from 2 to 5 carbon atoms, (2) a cyclic alkyl group having 3 or 4 carbon atoms, (3) an alkyl group which may become a branched chain having from 2 to 5 carbon atoms and have fluorine atom or chlorine atom, (4) an alkoxyalkyl group having from 2 to 5 carbon atoms, and (6) a substituted benzyloxyethyl group which may have 1 or 2 methyl groups on the ethyl group.
One of the illustrative embodiments of the preferred combination of respective substituents of the compound of the present invention represented by the formula (I) is a combination in which R² is phenyl group; R¹ is pyrrolidinyl group substituted with amino group, an alkylamino group or a dialkylamino group; R³ is methyl group; and R⁴ is a substituent selected from the group consisting of ethyl group, isopropyl group, normal butyl group, tertiary butyl group, cyclopropyl group, propylen-2-yl group, methoxymethyl group, fluoromethyl group, 2-chloroethyl group, 2-hydroxyethyl group, 1,1-dimethyl-2-hydroxyethyl group, 2-benzyloxyethyl group, 2-benzyloxy-1,1-dimethyl-ethyl group or 2-(4-fluorophenylmethyl)oxyethyl group.
One of the illustrative embodiments of the further preferred combination of respective substituents of the compound of the present invention represented by the formula (I) is a combination in which R² is phenyl group; R¹ is pyrrolidinyl group substituted with amino group, methylamino group or dimethylamino group; R³ is methyl group; and R⁴ is a substituent selected from the group consisting of ethyl group, isopropyl group, normal butyl group, tertiary butyl group, cyclopropyl group, propylen-2-yl group, methoxymethyl group, fluoromethyl group, 2-chloroethyl group, 2-hydroxyethyl group, 1,1-dimethyl-2-hydroxyethyl group, 2-benzyloxyethyl group, 2-benzyloxy-1,1-dimethyl-ethyl group or 2-(4-fluorophenylmethyl)oxyethyl group.

When the compound of the present invention represented by the aforementioned formula (I) has a structure in which enantiomers exist therein, all of the respective enantiomers, a racemic body as a 1:1 mixture thereof and an enantiomer mixture wherein the respective enantiomers are present at an optional mixing ratio and the optical purity is less than 100% are included in the compound of the present invention. In addition, when the compound represented by the formula (I) has a structure in which diastereomers exist therein, a single diastereomer and a mixture of diastereomers are included in the compound of the present invention.

When the compound represented by the formula (I) has a structure in which enantiomers exist therein, it is desirable to administer a preparation consisting of a pure enantiomer in administering the compound of the present invention to human or an animal. It should be understood that the term "consisting of a pure enantiomer" includes not only a case in which the other enantiomer is completely absent but also another case in which it can be generally said that this is chemically pure. That is, it should be understood that the other enantiomer can be included, with the proviso that its level is such a degree that it does not exert influence upon physical constants and physiological activities.

In addition, when the compound represented by the formula (I) has a structure in which diastereomers exist therein, it is desirable to administer a preparation consisting of a pure diastereomer in administering the compound of the present invention to human or an animal. It should be understood that the term "consisting of a pure diastereomer" includes not only a case in which the other diastereomer is completely absent but also another case in which it can be generally said that this is chemically pure. That is, it should be understood that the other diastereomer can be included, with the proviso that its level is such a degree that it does not exert influence upon physical constants and physiological activities.

In addition, the term "stereochemically pure" means that, when a compound or the like exists in an isomer relationship due to the presence of an asymmetric carbon, it is constituted from only one species thereof. Also in this case, this "pure" is also considered in the same manner as in the above.

When the compound represented by the formula (I) is an acid derivative having phenolic hydroxy group, carboxy group (carboxylic acid derivative) or sulfo group (sulfonic acid derivative) at an any substituent moiety, such an acid derivative may be the free form as such, or may be used as a salt of the phenolic hydroxy group, carboxy group or sulfo group.

These salts may be either inorganic salts or organic salts, such as a lithium salt, a sodium salt, a potassium salt or the like alkali metal salt, a magnesium salt, a calcium salt or the like alkaline earth metal salt, an ammonium salt, or a triethylamine salt, an N-methylglucamine salt, a tris-(hydroxymethyl)aminomethane salt and the like. In addition, the free forms and salts of these acid derivatives may be present as hydrates.

On the other hand, when the compound represented by the formula (I) is a basic derivative having amino group or amine structure at an any substituent moiety, such a basic derivative may be the free form as such, or may be used as an acid addition salt.

As examples when made into acid addition salts, hydrochloride, sulfate, nitrate, hydrobromide, hydroiodide, phosphate and the like inorganic acid salts or methanesulfonate, benzenesulfonate, paratoluenesulfonate (sulfonate), acetate, citrate, maleate, fumarate, lactate, tartarate (carboxylic acid salt) and the like organic acid salts can be cited. In addition, the free forms and salts of these basic derivatives may be present as hydrates in some cases.

When the compound represented by the formula (I) is a carboxylic acid compound, a derivative in which the carboxylic acid moiety became an ester is useful as a synthetic intermediate or a prodrug. For example, alkyl esters, benzyl esters, alkoxy alkyl esters, phenyl alkyl esters and phenyl esters are useful as synthesis intermediates.

Also, when the carboxylic acid compound of the present invention is used for an antifungal purpose, the ester to be used as a prodrug is an ester which is easily cleaved in the vivo and thereby forms a free form carboxylic acid, such as acetoxy methyl ester, pivaloyloxy methyl ester, ethoxycarbonyl ester, choline ester, dimethylaminoethyl ester, 5-indanyl ester and phthalidinyl ester, and 5-alkyl-2-oxo-1,3-dioxol-4-yl methyl ester, 3-acetoxy-2-oxo butyl ester or the like oxo alkyl ester.

In addition, when the compound represented by the formula (I) is a basic compound having amino group, its derivative in which an amino acid, a dipeptide or a tripeptide is linked to the amino group is useful as a prodrug.

The amino acid, dipeptide and tripeptide to be used as prodrugs are those in which the peptide bond formed from their carboxy group and the amino group of the compound of formula (I) of the present invention is easily cleaved in vivo and thereby forms free form of amine, such as glycine, alanine, asparagine and the like amino acids, glycine-glycine, glycine-alanine, alanine-alanine and the like dipeptides and glycine-glycine-alanine, glycine-alanine-alanine and the like tripeptides.

The compounds of the present invention represented by the formula (I) are produced by various methods. As their preferred examples, typical production methods are described based on the following reaction scheme, though not limited thereto. In this connection, in carrying out the reactions, substituents are protected with protecting groups if it is desireble, and the order of the conversion of respective substituents (functional groups) is not particularly limited thereto.

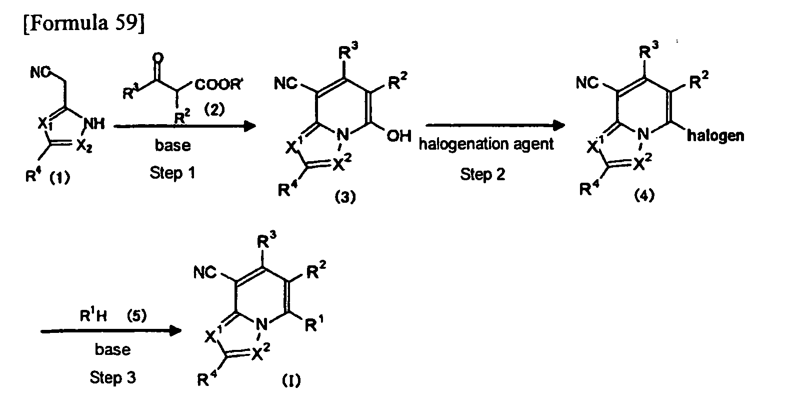

(In the reaction scheme, X¹ and X² each independently means nitrogen atom or carbon atom which may be substituted with a halogen atom, an alkoxy group having from 1 to 6 carbon atoms, an alkyl group having from 1 to 6 carbon atoms, which may have a substituent, or an ester group, wherein either one of X¹ and X² is nitrogen atom.)

The step 1 is a step in which the compound (3) is produced by allowing an acetonitrile derivative of imidazole, triazole or pyrazole, as the compound (1) and a β-keto ester derivative as the compound (2) to react with each other, to effect their condensation and cyclization.

The reaction can be carried out with or without a solvent. The solvent to be used in the reaction is not limited when it does not inhibit the reaction, and its examples include chloroform, dichloroethane, dimethylformamide, dimethylacetamide, dimethyl sulfoxide, benzene, toluene, chlorobenzene, dichlorobenzene, diethyl ether, tetrahydrofuran, 1,4-dioxane and diphenyl ether, or a mixture thereof. When one or both of the compound (1) and compound (2) as the starting materials is or are solution, it is preferable to carry out the reaction without using a solvent.

It is preferable to carry out the reaction in the presence of an inorganic base, organic base or the like acid acceptor, its examples including an inorganic base compound such as acetate, carbonate or hydrogencarbonate with an alkali metal, an alkaline earth metal or ammonia, and an organic base compound such as triethylamine, pyridine, 1,8-diazabicycloundecene, N-methylpiperidine, N,N-diisopropylethylamine. Among them, the use of ammonium acetate is particularly preferable, and it is preferabl that the base emplyed is optionally changed according to the reactivity.

The reaction temperature is generally within a range of from room temperature to 200°C, preferably within a range of from 25°C to reflux temperature when a solvent is used, or from 80°C to 150°C when a solvent is not used. The reaction time may be within a range of from 15 minutes to 48 hours, and the reaction is completed in generally about 30 minutes to 6 hours.

The step 2 is a step in which the compound (4) is produced by allowing the compound (3) to react with a halogenation agent.

The reaction can be carried out with or without a solvent. The solvent to be used in the reaction is not limited when it does not inhibit the reaction, and its examples include chloroform, dichloroethane, dimethylformamide, dimethylacetamide, dimethyl sulfoxide, benzene, toluene, chlorobenzene, dichlorobenzene, diethyl ether, tetrahydrofuran, 1,4-dioxane and diphenyl ether, or a mixture thereof. When the halogenation agent is a solution, it is preferable to carry out the reaction without using a solvent, but using excess amount of the halogenation agent which also serves as a solvent.

The halogenation agent has no particular limitation, with the proviso that it is generally used, for example, in case of the halogenation of alcohol. Its examples include thionyl chloride, thionyl bromide, thionyl iodide and the like thionyl halides, sulfuryl chloride, sulfuryl bromide, sulfuryl iodide and the like sulfuryl halides, phosphorus trichloride, phosphorus tribromide, phosphorus triiodide and the like phosphorus trihalides, phosphorus pentachloride, phosphorus pentabromide, phosphorus pentaiodide and the like phosphorus pentahalides, phosphorus oxychloride, phosphorus oxybromide, phosphorus oxyiodide and the like phosphorus oxyhalides, dialkyl aminosulfite fluorides represented by the following formula

[Formula 60] **(R**^{**35**}**)(R**^{**36**}**)NSF**_{**3**}

(in the formula, R³⁵ and R³⁶ may be the same or different and each represents an alkyl group having from 1 to 6 carbon atoms, or altogether an alkylene group having from 2 to 6 carbon atoms which may mediate oxygen atom), and a fluorination agent such as CF₃CHFCF₂N(C₂H₅)₂ or CF₃CF=CFN(C₂H₅)₂. Preferred are phosphorus oxychloride and the like chlorination agents.

The reaction temperature is generally within a range of from room temperature to 200°C, preferably within a range of from 25°C to reflux temperature when a solvent is used or the halogenating agent is a fluid, preferably from 50°C to 150°C. The reaction time may be within a range of from 15 minutes to 48 hours, and the reaction is completed in generally about 30 minutes to 2 hours.

The step 3 is a step in which the compound of the present invention, wherein the substituent R¹ shown in the formula (I) is substituted with (substituted) amino group, is produced by allowing the compound (4) to react with the amine derivative (5). In this reaction, the compound (1) of the present invention is formed through the nucleophilic recaction of halogenated compound (4) and amine derivative (5).

The reaction can be carried out with or without a solvent. The solvent to be used in the reaction is not limited when it does not inhibit the reaction, and its examples include dimethyl sulfoxide, pyridine, acetonitrile, ethanol, chloroform, dimethylformamide, dimethylacetamide, N-methylpyrrolidone, tetrahydrofuran, water and 3-methoxybutanol, or a mixture thereof.

It is desirable to carry out the reaction in the presence of an inorganic base, organic base or the like acid acceptor, and its examples include a carbonate or hydrogencarbonate of an alkali metal, an alkaline earth metal, and an organic base compound such as triethylamine, pyridine, 1,8-diazabicycloundecene, N-methylpiperidine, N,N-diisopropylethylamine.

The reaction temperature is generally within a range of from room temperature to 200°C, preferably within a range of from 25 to 150°C. The reaction time may be within a range of from 30 minutes to 48 hours, and the reaction is completed generally inabout 30 minutes to 18 hours.

When the amine derivative (5) to be used in the reaction has amino group, hydroxy group or thiol group, a compound in which such a substituent is protected with an appropriate protecting group can be used. In addition, when deprotection is necessary after completion of the reaction, the compound of interest represented by the formula (I) can be obtained by removing the protecting group under an appropriate condition corresponding to the protecting group.

The protecting group may be any protecting group generally used in this field, and its examples include tertiary butoxycarbonyl group, 2,2,2-trichloroethoxycarbonyl group and the like alkoxycarbonyl groups, benzyloxycarbonyl group, paramethoxybenzyloxycarbonyl group, paranitrobenzyloxycarbonyl group and the like aralkyloxycarbonyl groups, acetyl group, methoxyacetyl group, trifluoroacetyl group, chloroacetyl group, pivaloyl group, formyl group, benzoyl group and the like acyl groups, tertiary butyl group, benzyl group, paranitrobenzyl group, paramethoxybenzyl group, triphenylmethyl group and the like alkyl groups, or aralkyl groups, methoxymethyl group, tertiary butoxymethyl group, tetrahydropyranyl group, 2,2,2-trichloroethoxymethyl group and the like ethers, and trimethylsilyl group, isopropyldimethylsilyl group, tertiary butyldimethylsilyl group, tribenzylsilyl group, tertiary butyldiphenylsilyl group and the like silyl groups.

After completion of the reaction, the intended compound of step 3 is collected from the reaction mixture by a generally used method. For example, the compound of interest is obtained after completion of the reaction, by a method in which the compound of interest precipitated by adding an appropriate solvent is collected by filtration, or in which water is added to the reaction solution and a solvent which does not blend with water but dissolves the compound of interest is added thereto to extract the compound of interest, subsequently, the thus extracted organic layer is optionally subjected to water washing or the like operation and dried using anhydrous sodium sulfate, anhydrous magnesium sulfate or the like, and then the solvent is evaporated.

In addition, a condensed-cyclized compound (3-1) can also be obtained in the same manner as the case of the β-keto ester derivative (2), when an acrylic ester or an acrylonitrile derivative (2-1) is used in the aforementioned step 1 instead of the β-keto ester derivative (2). In this case, the condensed-cyclized compound can be obtained at a low temperature of -30°C or lower using lithium diisopropylamide or the like strong base as the base. The amino group of the condensed-cyclized compound (3-1) which is obtained when the acrylonitrile derivative is used can be replaced by a halogen atom through the Sandmeyer reaction or the like.

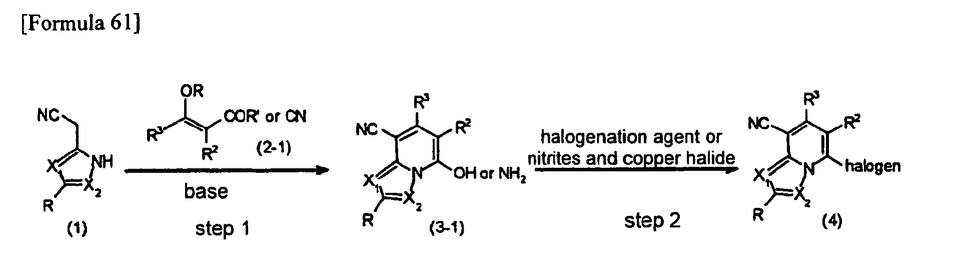

In addition, the compound of interest obtained in this manner can be purified as occasion demands by usually used techniques such as recrystallization, reprecipitation, chromatography and the like.

The compound of the present invention does not show antibacterial action and antitumor action but shows antifungal activity specifically (selectively), and is active against a broad range of fungi which cause various fungal infections, so that it can treat, prevent or alleviate diseases caused by these pathogens.

As the fungi for which the compound of the present invention is effective, *Candida albicans, Candida glabrata, Candida krusei, Candida tropicalis* and the like various species belonging to the genus *Candida,* the genus *Cryptococcus* such as *Cryptococcus neoformans* and the like, the genus *Aspergillus* such as *Aspergillus fumigatus, Aspergillus flavus* and the like, *Pneumocystis carinii,* the genus *Rhizopus,* the genus *Absidia,* the genus *Histoplasma* such as *Histoplasma capsulatum* and the like, the genus *Coccidioides* such as *Coccidioides immitis* and the like, the genus Blastomyces, the genus *Paracoccidioides* such as *Paracoccidioides brasiliensis* and the like, the genus *Penicillium,* the genus *Pseudallescheria,* the genus *Sporothrix,* a dematiaceae, the genus *Trichophyton,* the genus *Microsporum,* the genus *Epidermophyton,* the genus *Malassezia,* the genus *Honsenchaea,* the genus *Fusarium,* the genus *Paecilomyces,* the genus *Trichosporon* such as *Trichosporon cutaneum* and the like, the genus *Hyaphola, Cladosporium* and the like can be exemplified. In addition, *Saccharomyces cerevisiae, Candida albicans, Candida glabrata*, *Candida krusei, Candida tropicalis, Cryptococcus neoformans*, *Trichosporon cutaneum*, *Aspergillus fumigatus* and the like can be exemplified.

Also, regarding the diseases which are caused by these pathogens, candidiasis, cryptococcosis and aspergillosis (fungal diseases), actinomycosis (a ray fungal disease, nocardiosis and mucoymycosis (zygomycetes diseases), geotrichosis, histoplasmosis, coccidioidomycosis, paracoccidioidomycosis, blastomycosis, penicilliosis and the like, illustratively fungemia, mycosis of systema respiratorium, mycosis of digestive system, mycosis of urinary tract, fungal meningitis and the like, can be exemplified as intestinal organ mycoses (mycoses profunda), and sporotrichosis and chromomycosis (melanomycoses), mycetoma (mycetoma) and the like as deep skin mycoses, and a general disease type tinea, tines profunda, intractable tinea, nail tinea, pityriasis versicolor, skin candidiasis, buccal candidiasis and the like as superficial mycoses.
In addition, the compound of the present invention is also effective for various species of fungi which cause fungal infections in animals.

Making use of the antifungal activity of the compound of the present invention upon pathogenic fungi, it can be used as a medicine, an infection treating agent or an antifungal agent, which comprises the compound of the present invention, a salt thereof or a solvate thereof, and it is possible also to apply it to a drug for animals, a drug for fisheries or an antifungal preservative.

The compound of the present invention, a salt thereof or a solvate thereof may be used in the production of a medicine, an infection treating agent or an antifungal agent, which comprises the same. For example, the compound of the present invention, a salt thereof or a solvate thereof may be used in the production of injections, solutions and the like which are provided in the state of solution. In addition, a medicine, an infection treating agent or an antifungal agent can be produced by a conventional method for preparing pharmaceutical preparations, in which the compound of the present invention, a salt thereof or a solvate thereof is formulated and additive agents are optionally added thereto as occasion demands.

As the dosage forms of an antifungal agent which comprises the compound of the present invention, a salt thereof or a solvate thereof, for example, tablets, powders, granules or capsules, or solutions, syrups, elixirs, oily or aqueous suspensions, and the like can be exemplified as oral preparations.

Regarding the injections, an stabilizing agent, an antiseptic agent or a solubilizing agent may be used in the preparations, and solutions which may contain these auxiliary agents may be made into solid preparations to be prepared when used, by containing them in containers and then subjecting to freeze drying or the like.

In addition, solutions, suspensions, emulsions, ointments, gels, creams, lotions, or sprays, and the like can be exemplified as external preparations.

The solid preparations may contain pharmaceutically acceptable additive agents together with the compound of the present invention, a salt thereof or a solvate thereof and can be prepared, for example, by optionally selecting and mixing fillers, extenders, binders, disintegrators, solubilization accelerators, moistening agents, lubricants and the like as occasion demands.

As the liquid preparations, solutions, suspensions, emulsions and the like can be exemplified, and they may contain a suspending agent, an emulsifying agent and the like as additive agents.

Regarding the method for administering the compound of the present invention, a salt thereof or a solvate thereof to an animal, a method in which it is orally administered directly or by mixing with feed, a method in which it is made into a solution and then orally administered directly or by adding to drinking water or feed, a method in which it is administered by injection, and the like can be exemplified.

Regarding the pharmaceutical preparations for administering the compound of the present invention, a salt thereof or a solvate thereof to an animal, it can be optionally made into powders, fine subtilaes, soluble powders, syrups, solutions or injections by techniques generally used in this field.
Next, preparation formulation examples are shown below.

Preparation Example 1. (capsules)

| | |
|---|---|
| Compound of Example 1 | 100.0 mg |
| Corn starch | 23.0 mg |
| CMC calcium | 22.5 mg |
| Hydroxymethyl cellulose | 3.0 mg |
| Magnesium stearate | 1.5 mg |
| Total | 150.0 mg |

Preparation Example 2. (solutions)

| | |
|---|---|
| Compound of Example | 11 to 10g |
| Acetic acid or sodium hydroxide | 0.5 to 2 g |
| Ethyl parahydroxybenzoate | 0.1 g |
| Purified water | 88.9 to 98.4 g |
| Total | 100 g |

Preparation Example 3. (powders for feed mixing use)

| | |
|---|---|
| Compound of Example 1 | 1 to 10 g |
| Corn starch | 98.5 to 89.5 g |
| Light anhydrous silicic acid | 0.5 g |
| Total | 100 g |

Administration method, dose and administration frequency of the medicine of the present invention are not particularly limited and can be optionally selected in response to various conditions such as the kind of pathogenic fungus and age, body weight, symptoms and the like of the patient. Generally from 0.1 to 100 mg/kg may be administered to adult by oral or parenteral (injection, drip infusion or the like) administration, by dividing the dose into 1 to several doses.

Administration method, dose and administration frequency of the medicine of the present invention are not particularly limited and can be optionally selected in response to various conditions such as the kind of pathogenic fungus and age, body weight, symptoms and the like of the patient. Generally from 0.1 to 100 mg/kg may be administered to adult by oral or parenteral (injection, drip infusion or the like) administration, by dividing the dose into 1 to several doses.

### Examples

Next, the instant invention is described based on Examples and Reference Examples, but the present invention is not limited thereto.

### [Reference Example 1]

### 6-n-Butyl-7-methyl-5-oxo-1,5-dihydroimidazo[1,2-a]pyridine-8-carbonitrile (I-1)

A mixture of 311 mg (2.90 mmol) of (1H-imidazol-2-yl)acetonitrile synthesized in accordance with a conventionally known method (International Publication 94/06791) with 626 µl (3.19 mmol) of 2-acetylhexanoic acid ethyl ester and 448 mg (5.81 mmol) of ammonium acetate was heated at 140°C for 1.5 hours. After cooling, water was added to the reaction mixture and stirred, and then the resulting solid material was collected by filtration, washed with acetonitrile and then dried to obtain 288 mg (43%) of the title compound as a dark brown solid. ¹H-NMR (CDCl₃) d: 0.94 (3H, t, *J=* 7.1 Hz), 1.36-1.50 (4H, m), 2.45 (3H, s), 2.61-2.65 (2H, m), 7.08 (1H, d, *J=* 2.2 Hz), 7.67 (1H, d*, J=* 2.2 Hz).

### [Reference Example 2]

### 6-n-Butyl-5-chloro-7-methylimidazo[1,2-a]pyridine-8-carbonitrile (I-2)

A mixture of 288 mg (1.26 mmol) of 6-n-butyl-7-methyl-5-oxo-1,5-dihydroimidazo[1,2-a]pyridine-8-carbonitrile (I-1) with 2.0 ml (21.5 mmol) of phosphoryl chloride was heated under reflux for 1 hour. After cooling, the reaction mixture was concentrated under a reduced pressure, and the residue was mixed with ice water and then extracted with chloroform. The organic layer was washed with brine and then dried over anhydrous sodium sulfate. The solvent was evaporated under a reduced pressure, and the thus obtained crude product was dried to obtain 304 mg (97%) of the title compound as a dark brown solid.
MS(ESI)*m*/*z*: 247 (M⁺).
¹H-NMR (CDCl₃) d: 1.00 (3H, t, *J=* 7.1 Hz), 1.45-1.59 (4H, m), 2.69 (3H, s), 2.80-2.84 (2H, m), 7.73 (1H, d, *J*= 1.2 Hz), 7.78 (1H, d, *J* = 1.2 Hz).

### [Example 1]

### 6-n-Butyl-5-[(3S)-dimethylaminopyrrolidin-1-yl]7-methylimidazo[1,2-a]pyridine-8-carbonitrile (#1)

A 208 µl portion (1.33 mmol) of (3S)-dimethylaminopyrrolidine and 336 µl (2.41 mmol) of triethylamine were added to an N,N-dimethylformamide (4 ml) solution of 299 mg (1.21 mmol) of 6-n-Butyl-5-chloro-7-methylimidazo[1,2-a]pyridine-8-carbonitrile (I-2) and heated at 90°C for 5.5 hours. After concentration of the reaction mixture, the residue was diluted with chloroform, washed with saturated sodium bicarbonate aqueous solution and brine, and then dried over anhydrous sodium sulfate. The solvent was evaporated under a reduced pressure, and the residue was purified by a column chromatography. By eluting with a mixed solvent of chloroform-methanol (9:1, v/v), 271 mg (69%) of the title compound was obtained as a brown solid. MS(ESI)*m*/*z*: 326 (M+1)⁺.
¹H-NMR (CDCl₃) d: 0.95-1.30 (3H, m), 1.41-1.53 (4H, m), 2.10 (1H, m), 2.28 (1H, m), 2.33 (6H, s), 2.62 (3H, s), 2.66-2.70 (2H, m), 2.97 (1H, m), 3.32 (1H, m), 3.34-3.47 (3H, m), 7.59 (1H, broad s), 7.63 (1H, broad s).
IR (ATR): 2954, 2861, 2815, 2767, 2217, 1610, 1486 cm⁻¹.
Elemental analysis values: as C₁₉H₂₇N₅
Calcd.: C, 70.12%; H, 8.36%; N, 21.28%
Found: C, 69.81%; H, 8.33%; N, 21.28%

### [Reference Example 3]

### 7-Methyl-5-oxo-6-phenyl-1,5-dihydroimidazo[1,2-a]pyridine-8-carbonitrile (I-3)

A mixture of 160 mg (1.49 mmol) of (1H-imidazol-2-yl)acetonitrile, 308 mg (1.49 mmol) of 2-phenylacetoacetic acid ethyl ester and 230 mg (2.99 mmol) of ammonium acetate was heated at 140°C for 1.5 hours. After cooling, the reaction mixture was mixed with water and extracted with chloroform, and the organic layer was washed with brine and dried over anhydrous sodium sulfate. The solvent was evaporated under a reduced pressure, the residue was mixed with acetonitrile and stirred, and then the precipitated solid was collected by filtration and dried to obtain 158 mg (43%) of the title compound as a brown solid.
MS (ESI)*m*/*z*: 250 (M+1)⁺.
¹H-NMR (CDCl₃) d: 2.16 (3H, s), 7.22 (2H, d, *J=* 7.3 Hz), 7.30 (1H, t, *J* = 7.3 Hz), 7.39 (2H, t, *J* = 7.3 Hz), 7.61 (1H, d, *J =* 2.2 Hz), 7.77 (1H, d, *J =* 2.2 Hz).

### [Reference Example 4]

### 5-Chloro-7-methyl-6-phenylimidazo[1,2-a]pyridine-8-carbonitrile (I-4)

A mixture of 210 mg (0.84 mmol) of 7-methyl-5-oxo-6-phenyl-1,5-dihydroimidazo[1,2-a]pyridine-8-carbonitrile (I-3) and 2.0 ml (21.5 mmol) of phosphoryl chloride was heated under reflux for 1.5 hours. After cooling, the reaction mixture was concentrated under a reduced pressure, and the resulting residue was mixed with ice water, stirred, and then extracted with chloroform. The organic layer was washed with brine and then dried over anhydrous sodium sulfate. The solvent was evaporated under a reduced pressure, and the thus obtained crude product was dried to obtain 156 mg (69%) of the title compound as a dark brown solid.
MS(ESI)*m*/*z*: 268, 270 (M+1)⁺.
¹H-NMR (CDCl₃) d: 2.38 (3H, s), 7.23-7.25 (2H, m), 7.48-7.56 (3H, m), 7.80 (1H, m), 7.84 (1H, m).

### [Example 2]

### 5-[(3S)-Dimethylaminopyrrolidin-1-yl]-7-methyl-6-phenylimidazo[1,2-a]pyridine-8-carbonitrile (#2)

An 81.3 µl portion (0.64 mmol) of (3S)-dimethylaminopyrrolidine and 162 µl (1.17 mmol) of triethylamine were added to an N,N-dimethylformamide (3 ml) solution of 156 mg (0.58 mmol) of 5-chloro-7-methyl-6-phenylimidazo[1,2-a]pyridine-8-carbonitrile (I-4) and heated at 100°C for 18 hours. After concentration of the reaction mixture, the residue was diluted with chloroform, washed with saturated sodium bicarbonate aqueous solution and brine, and then dried over anhydrous sodium sulfate. The solvent was evaporated under a reduced pressure, and the residue was purified by a column chromatography. By eluting with a mixed solvent of chloroform-methanol (10:1, v/v), 53 mg (26%) of the title compound was obtained as a red solid. MS(ESI)*m*/*z*: 346 (M+1)⁺.
¹H-NMR (CDCl₃) d: 1.64 (1H, m), 1.95 (1H, m), 2.14 (6H, s), 2.29 (3H, s), 2.57 (1H, m), 2.82 (1H, dd, *J =* 9.3, 8.1 Hz), 2.98-3.04 (2H, m), 3.13 (1H, dd, *J* = 9.3, 7.1 Hz), 7.17 (1H, m), 7.21 (1H, m), 7.43-7.51 (3H, m), 7.56 (1H, d, *J* = 1.2 Hz), 7.68 (1H, d *J* = 1.2 Hz).
IR (ATR): 2958, 2863, 2759, 2217, 1602, 1533, 1469, 1440 cm⁻¹.
Elemental analysis values: as C₂₁H₂₃N₅0.25H₂O
Calcd.: C, 72.08%; H, 6.77%; N, 20.01%
Found: C, 72.44%; H, 6.64%; N, 20.09%

### [Reference Example 5]

### 7-Methyl-6-(2-methylthiazol-4-yl)-5-oxo-1,5-dihydroimidazo[1,2-a]pyridine-8-carbonitrile (I-5)

A mixture of 300 mg (2.80 mmol) of (1H-imidazol-2-yl)acetonitrile, 637 mg (2.80 mmol) of 2-(2-methylthiazol-4-yl)acetoacetic acid ethyl ester and 432 mg (5.60 mmol) of ammonium acetate was heated at 140°C for 1.5 hours. After cooling, the reaction mixture was mixed with water and extracted with chloroform, and the organic layer was washed with brine and dried over anhydrous sodium sulfate. The solvent was evaporated under a reduced pressure, the residue was mixed with acetonitrile and stirred, and then the precipitated solid was collected by filtration and dried to obtain 260 mg (34%) of the title compound as a brown solid.
MS (ESI)*m*/*z*: 271 (M+1)⁺.
¹H-NMR (CDCl₃) d: 2.43 (3H, s), 2.75 (3H, s), 7.11 (1H, d, *J=* 2.2 Hz), 7.75 (1H, d, *J=* 2.2 Hz).

### [Reference Example 6]

### 5-Chloro-7-methyl-6-(2-methylthiazol-4-yl)imidazo[1,2-a]pyridine-8-carbonitrile (I-6)

A mixture of 180 mg (0.66 mmol) of 7-methyl-6-(2-methylthiazol-4-yl)-5-oxo-1,5-dihydroimidazo[1,2-a]pyridine-8-carbonitrile (I-5) and 3.0 ml (32.2 mmol) of phosphoryl chloride was heated under reflux for 5 hours. After cooling, the reaction mixture was concentrated under a reduced pressure, and the resulting residue was mixed with ice water, stirred, and then extracted with chloroform. The organic layer was washed with brine and then dried over anhydrous sodium sulfate. The solvent was evaporated under a reduced pressure, and the thus obtained crude product was dried to obtain 150 mg (79%) of the title compound as a dark brown solid.
MS(ESI)*m*/*z*: 289, 291 (M+1)⁺.
¹H-NMR (CDCl₃) d: 2.45 (3H, s), 2.81 (3H, s), 7.25 (1H, s), 7.78 (1H, d, *J*= 1.5 Hz), 7.82 (1H, d, *J*= 1.5 Hz).

### [Example 3]

### 5-[(3S)-Dimethylaminopyrrolidin-1-yl]-7-methyl-6-(2-methylthiazol-4-yl)imidazo[1,2-a]pyridine-8-carbonitrile (#3)

A 72.5 µl portion (0.57 mmol) of (3S)-dimethylaminopyrrolidine and 145 µl (1.04 mmol) of triethylamine were added to an N,N-dimethylformamide (3.5 ml) solution of 150 mg (0.52 mmol) of 5-chloro-7-methyl-6-(2-methylthiazol-4-yl)imidazo[1,2-a]pyridine-8-carbonitrile (I-6) and heated at 110°C for 10 hours. After concentration of the reaction mixture, the residue was diluted with chloroform, washed with saturated sodium bicarbonate aqueous solution and brine, and then dried over anhydrous sodium sulfate. The solvent was evaporated under a reduced pressure, and the residue was purified by a column chromatography. By eluting with a mixed solvent of chloroform-methanol (10:1, v/v), 110 mg (58%) of the title compound was obtained as a red solid.
MS(ESI)*m*/*z*: 367 (M+1)⁺.
¹H-NMR (CDCl₃) d: 1.74 (1H, m), 2.04 (1H, m), 2.19 (6H, s), 2.34 (3H, s), 2.65 (1H, m), 2.80 (3H, s), 2.86 (1H, t, *J*= 8.0 Hz), 3.04-3.13 (2H, m), 3.25 (1H, dd, *J* = 7.1, 9.3 Hz), 7.07 (1H, s), 7.56 (1H, d, *J=* 1.2 Hz), 7.66 (1H, d, *J=* 1.2 Hz).
IR (ATR): 2871, 2827, 2773, 2221, 1596, 1459 cm-¹.
Elemental analysis values: as C₁₉H₂₂N₆S0.25H₂O
Calcd.: C, 61.51 %; H, 6.11 %; N, 22.65%; S, 8.64%
Found: C, 61.68%; H, 6.13%; N, 22.26%; S, 8.44%

### [Reference Example 7]

### 7-Ethyl-6-hexyl-5-oxo-1,5-dihydroimidazo[1,2-a]pyridine-8-carbonitrile (I-7)

A mixture of 300 mg (2.80 mmol) of (1H-imidazol-2-yl)acetonitrile, 660 mg (3.08 mmol) of 2-propionyloctanoic acid ethyl ester and 432 mg (5.60 mmol) of ammonium acetate was heated at 140°C for 6 hours. After cooling, the reaction mixture was mixed with water and extracted with chloroform, and the organic layer was washed with brine and dried over anhydrous sodium sulfate. The solvent was evaporated under a reduced pressure, and the residue was purified by a column chromatography. By eluting with a mixed solvent of chloroform-methanol (10:1, v/v), 446 mg (59%) of the title compound was obtained as a brown solid.
MS (ESI)*m*/*z*: 272 (M+1)⁺.
¹H-NMR (CDCl₃) d: 0.89-0.91 (3H, m), 1.09 (3H, t, *J=* 7.1 Hz), 1.24-1.92 (8H, m), 2.60-2.66 (2H, m), 2.82 (2H, q, *J=* 7.1 Hz), 7.16 (1H, d, *J=* 2.5 Hz), 7.78 (1H, d, *J=* 2.5 Hz).

### [Reference Example 8]

### 5-Chloro-7-ethyl-6-hexylimidazo[1,2-a]pyridine-8-carbonitrile (I-8)

A mixture of 446 mg (1.64 mmol) of 7-ethyl-6-hexyl-5-oxo-1,5-dihydroimidazo[1,2-a]pyridine-8-carbonitrile (I-7) and 4.0 ml of phosphoryl chloride was heated under reflux for 1 hour. After cooling, the reaction mixture was concentrated under a reduced pressure, and the resulting residue was mixed with ice water, stirred, and then extracted with chloroform. The organic layer was washed with brine and then dried over anhydrous sodium sulfate. The solvent was evaporated under a reduced pressure, and the residue was purified by a column chromatography. By eluting with a mixed solvent of chloroform-methanol (10:1, v/v), 214 mg (45%) of the title compound was obtained as a brown solid.
MS(ESI)*m*/*z*: 290, 292 (M+1)⁺.
¹H-NMR (CDCl₃) d: 0.92 (3H, t, *J=* 6.8 Hz), 1.28-1.39 (9H, m), 1.46 (1H, m), 1.57 (1H, m), 2.78-2.82 (2H, m), 3.01 (2H, q*, J=* 7.6 Hz), 7.73 (1H, d, *J=* 1.2 Hz), 7.78 (1H, d, *J=* 1.2 Hz).

### [Example 4]

### 5-[(3S)-Dimethylaminopyrrolidin-1-yl]-7-ethyl-6-hexylimidazo[1,2-a]pyridine-8-carbonitrile (#4)

A 103 µl portion (0.81 mmol) of (3S)-dimethylaminopyrrolidine and 206 µl (1.48 mmol) of triethylamine were added to an N,N-dimethylformamide (4.0 ml) solution of 214 mg (0.74 mmol) of 5-chloro-7-ethyl-6-hexylimidazo[1,2-a]pyridine-8-carbonitrile (I-8) and heated at 110°C for 3 hours. After concentration of the reaction mixture, the residue was diluted with chloroform, washed with saturated sodium bicarbonate aqueous solution and brine, and then dried over anhydrous sodium sulfate. The solvent was evaporated under a reduced pressure, and the residue was purified by a column chromatography. By eluting with a mixed solvent of chloroform-methanol (10:1, v/v), 139 mg (51%) of the title compound was obtained as a red oily mater. MS(ESI)*m*/*z*: 368 (M+1)⁺.
¹H-NMR (CDCl₃) d: 0.92 (3H, t, *J=* 6.8 Hz), 1.21 (3H, t, *J=* 7.1 Hz), 1.29-1.35 (6H, m), 1.40-1.54 (4H, m), 2.11 (1H, m), 2.32 (1H, m), 2.63-2.67 (2H, m), 2.93-2.98 (3H, m), 3.32 (1H, m), 3.42-3.45 (2H, m), 3.47 (2H, q, *J*= 7.1 Hz), 7.58 (1H, broad s), 7.64 (1H, d, *J=* 1.2 Hz).
IR (ATR): 2937, 2865, 2819, 2767, 2219, 1604, 1477 cm⁻¹.

### [Reference Example 9]

### 6-Benzyl-7-methyl-5-oxo-1,5-dihydroimidazo[1,2-a]pyridine-8-carbonitrile (I-9)

A mixture of 300 mg (2.80 mmol) of (1H-imidazol-2-yl)acetonitrile, 634 µl (3.08 mmol) of 2-benzylacetoacetic acid ethyl ester and 432 mg (5.60 mmol) of ammonium acetate was heated at 120°C for 2 hours. After cooling, the reaction mixture was mixed with water and extracted with chloroform-methanol (5:1, v/v), and the organic layer was washed with brine and dried over anhydrous sodium sulfate. The solvent was evaporated under a reduced pressure, the residue was mixed with acetonitrile and stirred, and then the precipitated solid was collected by filtration and dried to obtain 478 mg (65%) of the title compound as a brown solid.
MS (ESI)*m*/*z*: 264 (M+1)⁺.
¹H-NMR (CDCl₃) d: 2.44 (3H, s), 4.05 (2H, s), 7.08 (1H, d, *J*= 2.4 Hz), 7.14 (1H, m), 7.21-7.25 (4H, m), 7.79 (1H, d, *J* = 2.4 Hz).

### [Reference Example 10]

### 6-Benzyl-5-chloro-7-methylimidazo[1,2-a]pyridine-8-carbonitrile (I-10)

A mixture of 478 mg (1.81 mmol) of 6-benzyl-7-methyl-5-oxo-1,5-dihydroimidazo[1,2-a]pyridine-8-carbonitrile (I-9) and 3.0 ml of phosphoryl chloride was heated under reflux for 1 hour. After cooling, the reaction mixture was concentrated under a reduced pressure, and the resulting residue was mixed with ice water, stirred, and then extracted with chloroform. The organic layer was washed with brine and then dried over anhydrous sodium sulfate. The solvent was evaporated under a reduced pressure, and the residue was purified by a column chromatography. By eluting with a mixed solvent of chloroform-methanol (20:1, v/v), 472 mg (93%) of the title compound was obtained as a brown solid.
MS(ESI)*m*/*z*: 282, 284 (M+1)⁺.
¹H-NMR (CDCl₃) d: 2.56 (3H, s), 4.27 (2H, s), 7.06 (2H, d, *J=* 7.1 Hz), 7.25-7.32 (3H, m), 7.77 (1H, d, *J=* 1.2 Hz), 7.84 (1H, d, *J=* 1.2 Hz).

### [Example 5]

### 6-Benzyl-5-[(3S)-dimethylaminopyrrolidin-1-yl]-7-methylimidazo[1,2-a]pyridine-8-carbonitrile (#5)

A 210 µl portion (1.65 mmol) of (3S)-dimethylaminopyrrolidine and 346 µl (2.48 mmol) of triethylamine were added to an N,N-dimethylformamide (10 ml) solution of 466 mg (1.65 mmol) of 6-benzyl-5-chloro-7-methylimidazo[1,2-a]pyridine-8-carbonitrile (I-11) and heated at 100°C for 18 hours. After concentration of the reaction mixture, the residue was diluted with chloroform, washed with saturated sodium bicarbonate aqueous solution and brine, and then dried over anhydrous sodium sulfate. The solvent was evaporated under a reduced pressure, and the residue was purified by a column chromatography. By eluting with a mixed solvent of chloroform-methanol (20:1, v/v), 364 mg (63%) of the title compound was obtained as a red solid. MS(ESI)*m*/*z*: 360 (M+1)⁺.
¹H-NMR (CDCl₃) d: 1.95 (1H, m), 2.19 (1H, m), 2.23 (6H, s), 2.43 (3H, s), 2.84 (1H, m), 3.34 (1H, m), 3.32-3.36 (3H, m), 4.11 (1H, d, *J =* 6.6 Hz), 4.16 (1H, d, *J =* 6.6 Hz), 7.00 (2H, d, *J*= 7.3 Hz), 7.20 (1H, t, *J* = 7.3 Hz), 7.26 (2H, d, *J* = 7.3 Hz), 7.65 (1H, s), 7.68 (1H, s).
IR (ATR): 2948, 2863, 2819, 2771, 2217, 1612, 1486 cm⁻¹.
Elemental analysis values: as C₂₂H₂₅N₅
Calcd.: C, 73.51%; H, 7.01%; N, 19.48%
Found: C, 73.31%; H, 6.98%; N, 19.38%

### [Reference Example 11]

### 5-Oxo-1,5,6,7,8,9-hexahydroimidazo[1,2-b]isoquinoline-8-carbonitrile (I-11)

A mixture of 300 mg (2.80 mmol) of (1H-imidazol-2-yl)acetonirile, 493 µl (3.08 mmol) of 2-oxocyclohexanecarboxylic acid ethyl ester and 432 mg (5.60 mmol) of ammonium acetate was heated at 110°C for 2 hours. After cooling, the reaction mixture was mixed with water and extracted with chloroform, and the organic layer was washed with brine and dried over anhydrous sodium sulfate. The solvent was evaporated under a reduced pressure, the residue was mixed with acetonitrile and stirred, and then the precipitated solid was collected by filtration and dried to obtain 360 mg (60%) of the title compound as a brown solid.
MS (ESI)*m*/*z*: 214 (M+1)⁺.
¹H-NMR (CDCl₃) d: 1.78-1.83 (4H, m), 2.58-2.64 (2H, m), 2.80-2.85 (2H, m), 7.09 (1H, d, *J*= 2.4 Hz), 7.73 (1H, d, *J =* 2.4 Hz).

### [Reference Example 12]

### 5-Chloro-6,7,8,9-tetrahydroimidazo[1,2-b]isoquinoline-8-carbonitrile (I-12)

A mixture of 360 mg (1.69 mmol) of 5-oxo-1,5,6,7,8,9-hexahydroimidazo[1,2-b]isoquinoline-8-carbonitrile (I-11) and 5.0 ml of phosphoryl chloride was heated under reflux for 1 hour. After cooling, the reaction mixture was concentrated under a reduced pressure, and the resulting residue was mixed with ice water, stirred, and then extracted with chloroform-methanol (5:1, v/v). The organic layer was washed with brine and then dried over anhydrous sodium sulfate. The solvent was evaporated under a reduced pressure, and the residue was purified by a column chromatography. By eluting with a mixed solvent of chloroform-methanol (100:1, v/v), 329 mg (84%) of the title compound was obtained as a pale yellow solid.
MS(ESI)*m*/*z*: 232, 234 (M+1)⁺.
¹H-NMR (CDCl₃) d: 1.86-1.94 (4H, m), 2.86 (2H, m), 3.13 (2H, m), 7.72 (1H, d, *J*= 1.5 Hz), 7.77 (1H, d, *J* = 1.5 Hz).

### [Example 6]

### 5-[(3S)-Dimethylaminopyrrolidin-1-yl]-6,7,8,9-tetrahydroimidazo[1,2-b]isoquinoline-8-carbonitrile (#6)

A 198 µl (1.56 mmol) portion of (3S)-dimethylaminopyrrolidine and 297 µl (2.13 mmol) of triethylamine were added to an N,N-dimethylformamide (5.0 ml) solution of 329 mg (1.42 mmol) of 5-chloro-6,7,8,9-tetrahydroimidazo[1,2-b]isoquinoline-8-carbonitrile (I-13) and heated at 90°C for 4 hours. After concentration of the reaction mixture, the residue was diluted with chloroform, washed with saturated sodium bicarbonate aqueous solution and brine, and then dried over anhydrous sodium sulfate. The solvent was evaporated under a reduced pressure, and the residue was purified by a column chromatography. By eluting with a mixed solvent of chloroform-methanol (10:1, v/v), 266 mg (61%) of the title compound was obtained as a purple solid.
MS(ESI)*m*/*z*: 310 (M+1)⁺.
¹H-NMR (CDCl₃) d: 1.79-1.89 (4H, m), 2.06 (1H, m), 2.29 (1H, m), 2.32 (6H, s), 2.69-2.73 (2H, m), 2.94 (1H, m), 3.09-3.12 (2H, m), 3.33 (1H, m), 3.45-3.53 (3H, m), 7.56 (1H, d*, J=* 1.1 Hz), 7.64 (1H, d, *J* = 1.1 Hz).
IR (ATR): 2937, 2865, 2819, 2767, 2219, 1604, 1477 cm⁻¹.
Elemental analysis values: as C₁₈H₂₃N₅
Calcd.: C, 69.87%; H, 7.49%; N, 22.63%
Found: C, 69.41%; H, 7.45%; N, 22.47%

### [Reference Example 13]

### 4-Methyl-1-trityl-1H-imidazole (I-13)

Under ice-cooling, 20.4 g (73.1 mmol) of trityl chloride was added to a 40 ml N,N-dimethylformamide solution (40ml) of 6.00 g (73.1 mmol) of 4-methyl-1H-imidazole and 11.2 ml (80.4 mmol) of triethylamine. After 18 hours of stirring by returning to room temperature, the reaction mixture was diluted with chloroform, washed with saturated sodium bicarbonate aqueous solution and brine and then dried over anhydrous sodium sulfate. After evaporation of the solvent under a reduced pressure, diethyl ether was added to the residue to effect crystallization, and the crystals were collected by filtration and dried to obtain 21.0 g (89%) of the title compound as a colorless solid.
MS (ESI)*m*/*z*: 325 (M+1)⁺.
¹H-NMR (CDCl₃) d: 2.05 (3H, s), 6.58 (1H, s), 7.03-7.09 (6H, m), 7.21 (1H, s), 7.30-7.42 (9H, m).

### [Reference Example 14]

### (4-Methyl-1-trityl-1H-imidazol-2-yl)methanol (I-14)

Under ice-cooling, 22.5 ml (35.6 mmol) of n-butyl lithium (1.58 M n-hexane solution) was added dropwise to a 300 ml tetrahydrofuran suspension of 10.5 g (32.4 mmol) of 4-methyl-1-trityl-1H-imidazole (I-13), and after cooling to room temperature and stirring for 2 hours, 3.0 g of paraformaldehyde was added thereto in one portion. After 14 hours of stirring, the reaction was quenched by adding saturated ammonium chloride aqueous solution, and water and ethyl acetate were added thereto to carry out extraction. The organic layer was washed with brine and then dried over anhydrous sodium sulfate. The solvent was evaporated under a reduced pressure, and then the residue was purified by a column chromatography. By eluting with a mixed solvent of chloroform-methanol (10:1, v/v), 6.58 g (57%) of the title compound was obtained as a pale yellow solid.
MS (ESI)*m*/*z*: 355 (M+1)⁺.
¹H-NMR (CDCl₃) d: 2.15 (3H, s), 3.64 (2H, s), 6.45 (1H, s), 7.12-7.15 (6H, m), 7.32-7.37 (9H, m).

### [Reference Example 15]

### 2-Chloromethyl-4-methyl-1H-imidazole (I-15)

Under ice-cooling, 4.70 ml (64.4 mmol) of thionyl chloride was added to 5.47 g (15.4 mmol) of (4-methyl-1-trityl-1H-imidazol-2-yl)methanol (I-14), and the mixture was stirred for 0.5 hour, after raising to room temperature the mixture was further stirred for 1.5 hours. The reaction mixture was concentrated under a reduced pressure, and the residue was crystallized by adding tetrahydrofuran and ethyl acetate to obtain 1.59 g (79%) of the title compound as a colorless solid. ¹H-NMR (DMSO-*d*₆) d: 2.29 (3H, s), 4.32 (2H, s), 7.02 (1H, s), 7.11-7.12 (6H, m), 7.43-7.49 (9H, m)

### [Reference Example 16]

### (4-Methyl-1H-imidazol-2-yl)acetonitrile (I-16)

Under ice-cooling, an ethanol (50 ml) solution of 1.59 g (9.52 mmol) of 2-chloromethyl-4-methyl-1H-imidazole (I-15) was added dropwise to an aqueous solution (11 ml) of 2.48 g (38.1 mmol) of potassium cyanide. After raising to room temperature and stirring for 3 hours, the insoluble maerial was removed by filtration, and the filtrate was concentrated. The residue was diluted with saturated sodium bicarbonate aqueous solution, extracted with ethyl acetate, washed with brine and then dried over anhydrous sodium sulfate. The solvent was evaporated under a reduced pressure, and the residue was purified by a column chromatography. By eluting with ethyl acetate, 758 mg (66%) of the title compound was obtained as a yellow solid.
MS (ESI)*m*/*z*: 122 (M+1)⁺.
¹H-NMR (CDCl₃) d: 2.25 (3H, s), 3.91 (2H, s), 6.74 (1H, s).

### [Reference Example 17]

### 2,7-Dimethyl-5-oxo-6-phenyl-1,5-dihydroimidazo[1,2-a]pyridine-8-carbonitrile (I-17)

A mixture of 250 mg (2.05 mmol) of (4-methyl-1H-imidazol-2-yl)acetonitrile (I-16), 426 mg (2.06 mmol) of 2-phenylacetoacetic acid ethyl ester and 318 mg (4.12 mmol) was heated at 140°C for 3 hours. After cooling, the reaction mixture was mixed with water and extracted with chloroform, and the organic layer was washed with brine and then dried over anhydrous sodium sulfate. The solvent was evaporated under a reduced pressure, the residue was mixed with acetonitrile and stirred, and then the thus precipitated solid was collected by filtration to obtain 330 mg (61%) of the title compound as a brown solid.
MS (FAB)*m*/*z*: 264 (M+1)⁺.
¹H-NMR (DMSO-*d*_{*6*}) d: 2.15 (3H, s), 2.31 (3H, s), 7.21-7.23 (2H, m), 7.31 (1H, m), 7.38-7.41 (2H, m), 7.54 (1H, s).
IR (ATR): 3156, 2204, 1650, 1616, 1523, 1317 cm⁻¹.

### [Reference Example 18]

### 5-Chloro-2,7-dimethyl-6-phenylimidazo[1,2-a]pyridine-8-carbonitrile (I-18)

A mixture of 432 mg (1.64 mmol) of 2,7-dimethyl-5-oxo-6-phenyl-1,5-dihydroimidazo[1,2-a]pyridine-8-carbonitrile (I-17) and 2.5 ml (26.8 mmol) of phosphoryl chloride was heated under reflux for 3 hours. After cooling, the reaction mixture was concentrated under a reduced pressure, and the resulting residue was mixed with ice water, stirred, and then extracted with chloroform. The organic layer was washed with brine and then dried over anhydrous sodium sulfate. The solvent was evaporated under a reduced pressure, and the residue was purified by a column chromatography. By eluting with a mixed solvent of chloroform-methanol (6:1, v/v), 462 mg (100%) of the title compound was obtained as a dark brown solid.
MS(ESI)*m*/*z*: 282, 284 (M+1)⁺.
¹H-NMR (DMSO-*d*_{*6*}) d: 2.25 (3H, s), 2.43 (3H, s), 7.35-7.37 (2H, m), 7.47-7.56 (3H, m).

### [Example 7]

### S-[(3S)-Dimethylaminopyrrolidin-1-yl]-2,7-dimethyl-6-phenylimidazo[1,2-a]pyridine-8-carbonitrile (#7)

A 275 µl (1.97 mmol) portion of (3S)-dimethylaminopyrrolidine and 825 µl (5.92 mmol) of triethylamine were added to a dimethyl sulfoxide (5 ml) solution of 556 mg (1.97 mmol) of 5-chloro-2,7-dimethyl-6-phenylimidazo[1,2-a]pyridine-8-carbonitrile (I-18) and heated at 100°C for 8 hours. After concentration of the reaction mixture, the residue was diluted with ethyl acetate, washed with saturated sodium bicarbonate aqueous solution and brine, and then dried over anhydrous sodium sulfate. The solvent was evaporated under a reduced pressure, and the residue was purified by a column chromatography. By eluting with a mixed solvent of chloroform-methanol (10:1, v/v), 167 mg (24%) of the title compound was obtained as a red solid. MS(ESI)*m*/*z*: 360 (M+1)⁺.
¹H-NMR (CDCl₃) d: 1.67 (1H, m), 1.95 (1H, m), 2.14 (6H, s), 2.27 (3H, s), 2.52 (3H, s), 2.56 (1H, m), 2.81 (1H, m), 2.94-3.03 (2H, m), 3.12 (1H, dd, *J*= 7.1, 9.1 Hz), 7.15 (1H, m), 7.19 (1H, m), 7.29 (1H, broad s), 7.43-7.50 (3H, m).
IR (ATR): 2817, 2767, 2221, 1604, 1471 cm⁻¹.
Elemental analysis values: as C₂₂H₂₅N₅
Calcd.: C, 73.51%; H, 7.01%; N, 19.48%
Found: C, 73.24%; H, 6.93%; N, 19.24%

### [Reference Example 19]

### Benzyloxyacetic acid 3-methyl-2-oxobutyl ester (I-19)

Under ice-cooling, 7.35 g (44.5 mmol) of 1-bromo-3-methyl-2-butanone was added to an N,N-dimethylformamide mixed liquid of 7.40 g (44.5 mmol) of benzyloxyacetic acid and 12.3 g (89.1 mmol) of potassium carbonate and then stirred at room temperature for 6 hours. The reaction mixture was filtered through celite, and then the filtrate was mixed with saturated ammonium chloride aqueous solution and extracted with ethyl acetate. The organic layer was washed with brine and then dried over anhydrous sodium sulfate. After filtration, the solvent was evaporated under a reduced pressure, and the residue was purified by a column chromatography. By eluting with a mixed solvent of n-hexane-ethyl acetate (3:2, v/v), 10.5 g (94%) of the title compound was obtained as a pale yellow oily substance.
MS (ESI)*m*/*z*: (M+1)⁺.
¹H-NMR (CDCl₃) d: 1.16 (6H, d, *J*= 6.8 Hz), 2.69 (1H, hep, *J*= 6.8 Hz), 4.24 (2H, s), 4.68 (2H, s), 4.85 (2H, s), 7.29-7.40 (5H, m).

### [Reference Example 20]

### 2-Benzyloxymethyl-4-isopropyl-1H-imidazole (I-20)

A mixture of 10.5 g (42.0 mmol) of benzyloxyacetic acid 3-methyl-2-oxobutyl ester (I-19) and 32.3 g (420 mmol) of ammonium acetate was heated at 140°C for 5 hours. After cooling, the reaction mixture was diluted with chloroform and washed with 1 N sodium hydroxide and brine, and then the organic layer was dried over anhydrous sodium sulfate. After filtration, the solvent was evaporated under a reduced pressure, and the residue was purified by a column chromatography. By eluting with a mixed solvent of n-hexane-ethyl acetate (1:3, v/v), 2.85 g (29%) of the title compound was obtained as a red oily substance.
MS (ESI)*m*/*z:* (M+1)⁺.
¹H-NMR (CDCl₃) d: 1.25 (6H, d, *J*= 6.8 Hz), 2.89 (1H, hep, *J*= 6.8 Hz), 4.00 (1H, s), 4.57 (2H, s), 4.63 (2H, s), 6.68 (1H, s), 7.28-7.40 (5H, m).

### [Reference Example 21]

### (4-Isopropyl-1H-imidazol-2-yl)methanol (I-21)

A 13.6 ml (13.6 mmol) portion of 1 N hydrochloric acid/ethanol solution and 570 mg of 10% Pd/C were added to an ethanol solution of 2.85 g (12.4 mmol) of 2-benzyloxymethyl-4-isopropyl-1H-imidazole (I-20), and subjected to catalytic reduction under a hydrogen atmosphere of 4 atmospheric pressure. After completion of the reaction, the catalyst was removed by filtration, and the filtrate was concentrated to obtain 2.11 g of the title compound as a red oily substance. The thus obtained crude product was subjected to the subsequent reaction without purification.
MS (ESI)*m*/*z:* (M+1)⁺.
¹H-NMR (CD₃OD) d: 1.32 (6H, d, *J=* 7.1 Hz), 3.03 (1H, hep, *J*= 7.1 Hz), 4.02 (1H, s), 4.81 (2H, s), 7.18 (1H, s).

### [Reference Example 22]

### 2-Chloromethyl-4-isopropyl-1H-imidazole (I-22)

Under ice-cooling, 3.50 ml (47.8 mmol) of thionyl chloride was added dropwise to 2.11 g of (4-isopropyl-1H-imidazol-2-yl)methanol (I-21). After stirring at room temperature for 2 hours, the reaction mixture was concentrated under a reduced pressure to obtain 2.45 g of the title compound as a black oily substance. The thus obtained crude product was subjected to the subsequent reaction without purification.
MS (ESI)*m*/*z*: (M+1)⁺.
¹H-NMR (DMSO-*d*_{*6*}) d: 1.26 (6H, d, *J*= 6.8 Hz), 3.01 (1H, hep, *J=* 6.8 Hz), 5.01 (2H, s), 7.43 (1H, s), 8.35 (1H, s).

### [Reference Example 23]

### (4-Isopropyl-1H-imidazol-2-yl)acetonitrile (I-23)

Under ice-cooling, ethanol (60 ml) solution of 2.45 g of 2-chloromethyl-4-isopropyl-1H-imidazole (I-22) was added dropwise to an aqueous solution (14 ml) of 3.27 g (50.2 mmol) of potassium cyanide. After 2 hours of stirring at room temperature, the insoluble material was removed by filtration, the filtrate was concentrated under a reduced pressure, and the residue was diluted with saturated sodium bicarbonate aqueous solution and extracted with ethyl acetate. The organic layer was washed with brine and dried over anhydrous sodium sulfate. The solvent was evaporated under a reduced pressure, and the residue was purified by a column chromatography. By eluting with ethyl acetate, 1.08 g (57%) of the title compound was obtained as a red oily substance.
MS (ESI)*m*/*z*: (M+1)⁺.
¹H-NMR (CDCl₃) d: 1.25 (6H, d, *J =* 6.8 Hz), 2.90 (1H, hep, *J =* 6.8 Hz), 3.92 (2H, s), 6.72 (1H, s).

### [Reference Example 24]

### 2-Isopropyl-7-methyl-5-oxo-6-phenyl-1,5-dihydroimidazo[1,2-a]pyridine-8-carbonitrile (I-24)

A mixture of 373 mg (2.50 mmol) of (4-isopropyl-1H-imidazol-2-yl)acetonitrile (1-23), 567 mg (2.75 mmol) of 2-phenylacetoacetic acid ethyl ester and 386 mg (5.00 mmol) of ammonium acetate was heated at 135°C for 3.5 hours. After cooling, the reaction mixture was mixed with water and extracted with chloroform-methanol (5:1, v/v). The organic layer was washed with brine and dried over anhydrous sodium sulfate. The solvent was evaporated under a reduced pressure, the residue was mixed with acetonitrile and stirred, and the thus precipitated solid was collected by filtration and dried to obtain 387 mg (53%) of the title compound as a pale yellow solid.
MS (ESI)*m*/*z*: (M+1)⁺.
¹H-NMR (DMSO-*d*_{*6*}) d: 1.29 (6H, d, *J*= 6.8 Hz), 2.14 (3H, s), 2.99 (1H, hep, *J =* 6.8 Hz), 7.21 (2H, d, *J*= 7.3 Hz), 7.29 (1H, t, *J*= 7.3 Hz), 7.38 (2H, t, *J*= 7.3 Hz).

### [Reference Example 25]

### 5-Chloro-2-isopropyl-7-methyl-6-phenylimidazo[1,2-a]pyridine-8-carbonitrile (I-25)

A mixture of 387 mg (1.33 mmol) of 2-isopropyl-7-methyl-5-oxo-6-phenyl-1,5-dihydroimidazo[1,2-a]pyridine-8-carbonitrile (I-24) and 2.0 ml (21.5 mmol) of phosphoryl chloride was heated under reflux for 1 hour. After cooling, the reaction mixture was concentrated under a reduced pressure, and the residue was mixed with ice water, stirred and then extracted with chloroform. The organic layer was washed with brine and then dried over anhydrous sodium sulfate. The solvent was evaporated under a reduced pressure, and the thus obtained crude product was dried to obtain 285 mg (69%) of the title compound as a dark brown solid. MS(ESI)*m*/*z*: 247 (M⁺).
¹H-NMR (CDCl₃) d: 1.40 (6H, d, *J=* 6.8 Hz), 2.35 (3H, s), 3.18 (1H, hep, *J*= 6.8 Hz), 7.20-7.23 (2H, m), 7.47-7.56 (4H, m).

### [Example 8]

### 5-[(3S)-Dimethylaminopyrrolidin-1-yl]-2-isopropyl-7-methyl-6-phenylimidazo[1,2-a]pyridine-8-carbonitrile (#8)

A 140 µl (1.10 mmol) portion of (3S)-dimethylaminopyrrolidine and 256 µl (1.83 mmol) of triethylamine were added to a dimethyl sulfoxide (3 ml) solution of 285 mg (0.92 mmol) of 5-chloro-2-isopropyl-7-methyl-6-phenylimidazo[1,2-a]pyridine-8-carbonitrile (I-25) and heated at 90°C for 5.5 hours. After concentration of the reaction mixture, the residue was diluted with chloroform, washed with saturated sodium bicarbonate aqueous solution and brine, and then dried over anhydrous sodium sulfate. The solvent was evaporated under a reduced pressure, and the residue was purified by a column chromatography. By eluting with a mixed solvent of chloroform-methanol (7:1, v/v), 186 mg (52%) of the title compound was obtained as a brown solid.
MS(ESI)*m*/*z*: 388 (M+1)⁺.
¹H-NMR (CDCl₃)d: 1.39 (6H, d, *J*= 6.5 Hz), 1.65 (1H, m), 1.96 (1H, m), 2.14 (6H, s), 2.26 (3H, s), 2.55 (1H, hep, *J*= 6.5 Hz), 2.82 (1H, t, *J*= 8.5 Hz), 2.94-3.02 (2H, m), 3.11 (1H, m), 3.18 (1H, m), 7.14 (1H, m), 7.19 (1H, m), 7.41-7.49 (4H, m).
IR (ATR): 2958, 2863, 2759, 2217, 1602, 1533, 1469, 1440 cm⁻¹.
Elemental analysis values: as C₁₉H₂₇N₅0.25H₂O
Calcd.: C, 73.53%; H, 7.58%; N, 17.86%
Found: C, 73.55%; H, 7.52%; N, 17.89%

### [Reference Example 26]

### Benzyloxyacetic acid 3,3-dimethyl-2-oxo-butyl ester (I-26)

A 1.00 g (5.59 mmol) portion of benzyloxyacetic acid was dissolved in N,N-dimethylformamide (5 ml), and a solution, which had been prepared by dissolving potassium carbonate and 1.0 g (5.59 mmol) of 1-bromo-3,3-dimethyl-2-butanone in N,N-dimethylformamide (5 ml) at 0°C under a stream of nitrogen, was added thereto. After 24 hours of stirring at room temperature, this was poured into 0.5 M hydrochloric acid aqueous solution (40 ml). This solution was extracted with ethyl acetate. The organic layer was washed with brine and dried over anhydrous sodium sulfate. The solvent was evaporated, and the residue was subjected to a column chromatography which uses silica gel, and 1.17 g (80%) of the title compound was obtained from a fraction of hexane:ethyl acetate = 95:5.
HRMS (FAB)*m*/*z*: 265.1446 (Calcd for C₁₅H₂₁O₄ 265.1440).
¹H-NMR (CDCl₃)δ: 1.20 (9H, s), 4.24 (2H, s), 4.67 (2H, s), 4.97 (2H, s), 7.28-7.40 (5H, m). IR (ATR): 1764, 1722, 1192, 1124, 1082, 987, 738, 698 cm⁻¹.

### [Reference Example 27]

### 2-Benzyloxymethyl-4-tert-butyl-1H-imidazole (I-27)

A 6.46 g (24.44 mmol) portion of benzyloxyacetic acid 3,3-dimethyl-2-oxo-butyl ester (I-26) and 18.8 g (244.4 mmol) of ammonium acetate were stirred at 140°C for 12 hours. After cooling to room temperature, the reaction mixture was fractionated with chloroform and 1 M sodium hydroxide aqueous solution. The organic layer was washed with brine and dried over anhydrous sodium sulfate. The solvent was evaporated, and the thus obtained residue was subjected to a column chromatography which uses silica gel, and 4.52 g (76%) of the title compound was obtained from a fraction of chloroform:methanol = 98:2, as a pale yellow oily substance.
HRMS (EI)*m*/*z*: 244.1580 (Calcd for C₁₅H₂₀N₂O 244.1576).
¹H-NMR (CDCl₃)δ: 1.27 (9H, s), 4.55 (2H, s), 4.61 (2H, s), 6.66 (1H, s), 7.28-7.37 (5H, m). IR (ATR): 2960, 1456, 1363, 1092, 1072, 735, 698 cm⁻¹.

### [Reference Example 28]

### 4-tert-Butyl-2-chloromethyl-1H-imidazole hydrochloride (I-28)

A 4.48 g (18.34 mmol) portion of 2-benzyloxymethyl-4-tert-butyl-1H-imidazole (I-27) was dissolved in ethanol (45 ml), and 10% palladium-carbon (896 mg) and 19.3 ml (19.25 mmol) of 1M hydrochloric acid ethanol solution were added thereto at room temperature. This mixture was stirred at room temperature for 12.5 hours in a stream of hydrogen of 4 atmospheric pressure. After removing the catalyst by filtration, the solvent was concentrated to obtain 2-hydroxymethyl-4-tert-butyl-1H-imidazole (3.34 g as crude product). Next, thionyl chloride (6 ml) was slowly added to this compound at 0°C and stirred at room temperature for 9 hours. After the reaction, the solvent evaporated under a reduced pressure, toluene (50 ml) was added to the residue, and the solvent evaporated under a reduced pressure. This process was repeated once. Next, tetrahydrofuran (50 ml) was added thereto, and the solvent evaporated under a reduced pressure. This process was repeated once. When the residue was mixed with diethyl ether and subjected to sonication, a solid was formed. This solid material was collected by filtration and dried under a reduced pressure to obtain 2.91 g (80%) of the title compound as a light brown powder.
MS (EI)*m*/*z*: 172 (M⁺).
HRMS (EI)*m*/*z*: 172.0755 (Calcd for C₈H₁₃N₂Cl 172.0767).
¹H-NMR (CDCl₃)δ: 1.37 (9H, s), 4.91 (2H, s), 7.32 (1H, s).
IR (ATR): 2686, 1682, 1630, 1282, 901, 823, 727 cm⁻¹.

### [Reference Example 29]

### (4-tert-Butyl-1H-imidazol-2-yl)acetonitrile (I-29)

A 3.57 g (54.90 mmol) portion of potassium cyanide was dissolved in water (15 ml), and a solution prepared by dissolving 2.87 g (13.72 mmol) of 4-tert-butyl-2-chloromethyl-1H-imidazole hydrochloride (I-28) in ethanol (61 ml) was slowly added thereto at 0°C, spending 1 hour. After 2 hours of stirring at room temperature, the precipitate was separated by filtration, and the thus obtained solution was concentrated under a reduced pressure. The residue was mixed with 1M sodium hydroxide aqueous solution (100 ml) and extracted with ethyl acetate. The organic layer was washed with brine and dried over anhydrous sodium sulfate. The solvent was evaporated, the thus obtained residue was subjected to a column chromatography which uses silica gel, and 1.51 g (67%) of the title compound was obtained from a fraction of hexane:ethyl acetate = 1:2 as an orange solid.
HRMS (EI)*m*/*z*: 163.1101 (Calcd for C₉H₁₃N₃ 163.1109).
¹H-NMR (CDCl₃)δ: 1.28 (9H, s), 3.90 (2H, s), 6.72 (1H, s).
IR (ATR): 2962, 2258, 1464, 1365, 1203, 754 cm⁻¹.

### [Reference Example 30]

### 2-tert-Butyl-7-methyl-5-oxo-6-phenyl-1,5-dihydroimidazo[1,2-1]pyridine-8-carbonitrile (I-30)

A 300 mg (1.84 mmol) portion of (4-tert-butyl-1H-imidazol-2-yl)acetonitrile (I-29), 386 µl (2.02 mmol) of ethyl 2-phenylacetoacetate and 283 mg (3.68 mmol) of ammonium acetate were heated at 140°C for 6 hours. After cooling to room temperature, this was fractionated with chloroform and saturated sodium bicarbonate aqueous solution. The organic layer was washed with brine and dried over anhydrous sodium sulfate. The solvent was evaporated, the thus obtained residue was subjected to a column chromatography which uses silica gel, and 398 mg (71%) of the title compound was obtained from a fraction of hexane:ethyl acetate = 2:1, as a pale light brown solid.
HREMS (EI)*m*/*z*: 305.1511 (Calcd for C₁₉H₁₉N₃O 305.1528).
¹H-NMR (CDCl₃)δ: 1.38 (9H, s), 2.28 (3H, s), 7.26-7.40 (2H, m), 7.36-7.40 (2H, m), 7.44 (1H, s),11.58 (1H, br s).
IR (ATR): 2208, 1645, 1518, 1317, 748, 717 cm⁻¹.

### [Reference Example 31]

### 2-tert-Butyl-5-chloro-7-methyl-6-phenylimidazo[1,2-a]pyridine-8-carbonitrile (1-31)

A 458 mg (1.50 mmol) portion of 2-tert-butyl-7-methyl-5-oxo-6-phenyl-1,5-dihydroimidazo[1,2-1]pyridine-8-carbonitrile (I-30) was dissolved in phosphorus oxychloride (10 ml) and heated at 120°C for 4 hours in a stream of nitrogen. After cooling to room temperature, the solvent was evaporated under a reduced pressure. The residue was fractionated with chloroform and saturated sodium bicarbonate aqueous solution. The organic layer was washed with brine and dried over anhydrous sodium sulfate. The solvent was evaporated, the thus obtained residue was subjected to a column chromatography which uses silica gel, and 423 mg (87%) of the title compound was obtained from a fraction of chloroform:acetone = 97:3, as a light brown solid.
MS (EI)m/z: 323 (M⁺).
HRMS (EI)*m*/*z*: 323.1187 (Calcd for C₁₉H₁₈N₃Cl 323.1189).
¹H-NMR (CDCl₃)δ: 1.45 (9H, s), 2.35 (3H, s), 7.21-7.54 (2H, m), 7.49-7.54 (3H, m), 7.56 (1H, s). IR (ATR): 2229, 1458, 1236, 773, 704 cm⁻¹.

### [Example 9]

### (3'S)-2-tert-Butyl-5-(3-dimethylaminopyrrolidin-1-yl)-7-methyl-6-phenylimidazo[1,2-a]pyridine-8-carbonitrile (#9)

A 420 mg (1.30 mmol) portion of 2-tert-butyl-5-chloro-7-methyl-6-phenylimidazo[1,2-a]pyridine-8-carbonitrile (I-31) was dissolved in anhydrous dimethyl sulfoxide (10 ml), and 362 µl (2.59 mmol) of triethylamine and 247 µl (1.95 mmol) of (3S)-(-)-(dimethylamino)pyrrolidine were added thereto and heated at 90°C for 7 hours. After cooling to room temperature, the solvent was evaporated under a reduced pressure. The residue was fractionated with chloroform and saturated sodium bicarbonate aqueous solution. The organic layer was washed with brine and dried over anhydrous sodium sulfate. The solvent was evaporated, the thus obtained residue was subjected to a column chromatography which uses silica gel, and 501 mg (96%) of the title compound was obtained from a fraction of chloroform: acetone = 9:1, as a pink solid.
MS (EI)m/z: 401 (M⁺).
¹H-NMR (CDCl₃)δ: 1.42 (9H, s), 1.67 (1H, dq, *J*= 8.8, 12.0), 1.93-2.00 (1H, m), 2.15 (6H, s), 2.24 (3H, s), 2.56 (1H, dq, *J=* 7.1, 8.8 Hz), 2.83 (1H, t, *J*= 8.8 Hz), 2.95-3.06 (2H, m), 3.12 (1H, dd, *J*= 7.1, 8.8 Hz), 7.12-7.21 (1H, m), 7.27 (1H, s), 7.41-7.50 (3H, m).
IR (ATR): 2222, 1606, 1471, 1227, 1201, 1153, 912, 704 cm⁻¹.
Elemental analysis values: as C₂₅H₃₁N₅
Calcd.: C, 74.78%; H, 7.78%; N, 17.44%
Found: C, 74.55%; H, 7.79%; N, 17.40%

### [Reference Example 32]

### 2-tert-Butyl-7-methyl-6-(2-methylthiazol-4-yl)-5-oxo-1,5-dihydroimidazo[1,2-a]pyridine-8-carbonitrile (I-32)

In accordance with the synthesis method of compound I-30, 143 mg (24%) of the title compound was obtained as a light brown amorphous crystal from 300 mg (1.84 mmol) of (4-tert-butyl-1H-imidazol-2-yl)acetonitrile (I-29), 460 mg (2.02 mmol) of ethyl 2-(2-methylthiazol-4-yl)-3-oxo-butanoate and 283 mg (3.68 mmol) of ammonium acetate.
HRMS (EI)*m*/*z*: 326.1206 (Calcd for C₁₇H₁₈N₄OS 326.1201).
¹H-NMR (CDCl₃)δ: 1.05 (9H, s), 2.08 (3H, s), 2.38 (3H, s), 6,92 (1H, s), 6.93 (1H, s), 11.28 (1H, br s).
IR (ATR): 3112, 2204, 1643, 1529, 1173, 748, 731 cm⁻¹.

### [Reference Example 33]

### 2-tert-Butyl-5-chloro-7-methyl-6-(2-methylthiazol-4-yl)imidazo[1,2-a]pyridine-8-carbonitrile (I-33)

In accordance with the synthesis method of compound I-31, 75 mg (50%) of the title compound was obtained as a light brown powder from 142 mg (0.44 mmol) of 2-tert-butyl-7-methyl-6-(2-methylthiazol-4-yl)-5-oxo-1,5-dihydroimidazo[1,2-a]pyridine-8-carbonitrile (I-32) and phosphorus oxychloride (5 ml).
HRMS (EI)*m*/*z*: 344.0857 (Calcd for C₁₇H₁₇N₄ClS 344.0862).
¹H-NMR (CDCl₃)δ: 1.42 (9H, s), 2.41 (3H, s), 2.80 (3H, s), 7.21 (1H, s), 7.54 (1H, s).
IR (ATR): 2229, 1234, 1180, 766 cm⁻¹.

### [Example 10]

### (3'S)-2-tert-Butyl-5-(3-dimethylaminopyrrolidin-1-yl)-7-methyl-6-(2-methylthiazol-4-yl)imidazo[1,2-a]pyridine-8-carbonitrile (# 10)

In accordance with the synthesis method of compound #9, 87 mg (98%) of the title compound was obtained as a colorless solid from 72 mg (0.21 mmol) of 2-tert-butyl-5-chloro-7-methyl-6-(2-methylthiazol-4-yl)imidazo[1,2-a]pyridine-8-carbonitrile (I-33), 58 µl (0.42 mmol) of triethylamine and 32 µl (0.25 mmol) of (3S)-(-)-(dimethylamino)pyrrolidine.
MS (EI)*m*/*z*: 422 (M⁺).
¹H-NMR (CDCl₃)δ: 1.41 (9H, s), 1.74 (1H, dq, *J*= 8.8, 12.2), 2.01-2.09 (1H, m), 2.20 (6H, s), 2.30 (3H, s), 2.64 (1H, dq, *J*= 7.3, 8.8 Hz), 2.80 (3H, s), 2.87 (1H, t, *J*= 8.8 Hz), 3.02-3.14 (2H, m), 3.23 (1H, dd, *J*= 7.3, 8.8 Hz), 7.05 (1H, s), 7.26 (1H, s).
IR (ATR): 2216, 1604, 1495, 1460, 1340, 1234, 1174 cm⁻¹.
Elemental analysis values: as C₂₃H₃₀N₆S
Calcd.: C, 65.37%; H, 7.16%; N, 19.89%
Found: C, 65.18%; H, 7.18%; N, 19.84%

### [Reference Example 34]

### 2-tert-Butyl-7-methyl-5-oxo-6-(2-phenylthiazol-4-yl)-1,5-dihydroimidazo[1,2-a]pyridine-8-carbonitrile (I-34)

In accordance with the synthesis method of compound I-30, 414 mg (58%) of the title compound was obtained as a colorless powder from 300 mg (1.84 mmol) of (4-tert-butyl-1H-imidazol-2-yl)acetonitrile (I-29), 585 mg (2.02 mmol) of ethyl 2-(2-phenylthiazol-4-yl)-3-oxo-butanoate and 283 mg (3.68 mmol) of ammonium acetate.
HRMS (EI)*m*/*z*: 388.1388 (Calcd for C₂₂H₂₀N₄OS 388.1418).
¹H-NMR (CDCl₃)δ: 1.34 (9H, s), 2.39 (3H, s), 7.43-7.51 (5H, m), 7.70 (1H, s), 7.91-7.96 (2H, m). IR (ATR): 3153, 2208, 1645, 1595, 1516, 758, 688 cm⁻¹.

### [Reference Example 35]

### 2-tert-Butyl-5-chloro-7-methyl-6-(2-phenylthiazol-4-yl)imidazo[1,2-a]pyridine-8-carbonitrile (I-35)

In accordance with the synthesis method of compound I-31, 208 mg (50%) of the title compound was obtained as a light brown powder from 400 mg (1.03 mmol) of 2-tert-butyl-7-methyl-5-oxo-6-(2-phenylthiazol-4-yl)-1,5-dihydroimidazo[1,2-a]pyridine-8-carbonitrile (I-34) and phosphorus oxychloride (10 ml).
HRMS (EI)*m*/*z*: 406.1013 (Calcd for C₂₂H₁₉N₄ClS 406.1019).
¹H-NMR (CDCl₃)δ: 1.43 (9H, s), 2.47 (3H, s), 7.37 (1H, s), 7.44-7.50 (3H, m), 7.56 (1H, s), 7.96-8.01 (2H, m).
IR (ATR): 2227, 1606, 1464, 1439, 1238, 983, 764, 685 cm⁻¹.

### [Example 11]

### (3'S)-2-tert-Butyl-5-(3-dimethylaminopyrrolidin-1-yl)-7-methyl-6-(2-phenylthiazol-4-yl)imidazo[1,2-a]pyridine-8-carbonitrile (#11)

In accordance with the synthesis method of compound #9, 188 mg (77%) of the title compound was obtained as a light brown powder from 205 mg (0.50 mmol) of 2-(tert-butyl-5-chloro-7-methyl-6-(2-phenylthiazol-4-yl)imidazo[1,2-a]pyridine-8-carbonitrile (I-35), 141 µl (1.00 mmol) of triethylamine and 77 µl (0.60 mmol) of (3S)-(-)-(dimethylamino)pyrrolidine.
MS(EI)m/z484(M⁺).
¹H-NMR (CDCl₃)δ: 1.42 (9H, s), 1.73 (1H, dq, *J*= 8.8, 12.0), 1.96-2.06 (1H, m), 2.15 (6H, s), 2.36 (3H, s), 2.64 (1H, dq, *J=* 7.1, 8.8 Hz), 2.99 (1H, t, *J*= 8.8 Hz), 3.07 (1H, dt, *J*= 7.1, 8.8 Hz), 3.18 (1H, dt, *J*= 3.4, 8.8 Hz), 3.30 (1H, dd, *J*= 7.1, 8.8 Hz), 7.22 (1H, s), 7.30 (1H, s), 7.45-7.49 (3H, m), 7.96-8.01 (2H, m).
IR (ATR): 2218, 1603, 1471, 768, 688 cm⁻¹.
Elemental analysis values: as C₂₈H₃₂N₆S
Calcd.: C, 69.39%; H, 6.65%; N, 17.34%; S, 6.62%
Found: C, 69.19%, H, 6.616%; N, 17.16%; S, 6.764%

### [Reference Example 36]

### 6-(1-Benzothiophen-3-yl)-2-(tert-butyl)-7-methyl-5-oxo-1,5-dihydroimidazo[1,2-a]pyridine-8-carbonitrile (I-36)

A mixture of 690 mg (2.78 mmol) of methyl 2-(1-benzothiophene-3-yl)acetoacetate synthesized in accordance with the method described in Japanese Patent Application No. 2002-022767, 454 mg (2.78 mmol) of 2-[4-(tert-butyl)-1H-imidazo-2-yl]acetonitrile (I-29) and 428 mg (5.56 mmol) of ammonium acetate was heated at 150°C for 1 hour. After cooling, this was mixed with water and extracted with chloroform. The thus obtained organic layer was dried over anhydrous sodium sulfate, and then the solvent was evaporated under a reduced pressure. The thus obtained residue was subjected to a silica gel column chromatography. By eluting with a mixed solvent of n-hexane-ethyl acetate (2:1), 124 mg (12%) of the title compound was obtained as a pale gray solid.
MS (ESI)m/z: 362 (M+1)⁺.
¹H-NMR (DMSO-d₆)δ: 1.38 (9H, s), 2.12 (3H, s), 7.31-7.37 (3H, m), 7.44 (1H, s), 7.58 (1H, s), 8.01 (1H, d, *J*= 8.0 Hz).

### [Reference Example 37]

### 6-(1-Benzothiophen-3-yl)-2-(tert-butyl)-5-chloro-7-methylimidazo[1,2-a]pyridine-8-carbonitrile (I-37)

A 122 mg (0.34 mmol) portion of 6-(1-benzothiophen-3-yl)-2-(tert-butyl)-7-methyl-5-oxo-1,5-dihydroimidazo[1,2-a]pyridine-8-carbonitrile (I-36) was heated under reflux or 2 hours in phosphoryl chloride (1.20 ml). After cooling, the reaction solution was concentrated under a reduced pressure, mixed with dichloromethane, neutralized with saturated sodium bicarbonate aqueous solution and washed with brine. The thus obtained organic layer was dried over anhydrous sodium sulfate, and then the solvent was evaporated under a reduced pressure. The thus obtained residue was dissolved in a small amount of methanol and purified by recrystallizing from n-hexane-ethyl acetate to obtain 139 mg (quant.) of the title compound as a colorless solid. MS (ESI)m/z: 380 (M+1)⁺.
¹H-NMR (CDCl₃)δ: 1.57 (9H, s), 2.48 (3H, s), 7.31 (1H, s), 7.41 (1H, t, *J*= 7.8 Hz), 7.48 (1H, t, *J* = 7.8 Hz), 7.60 (1H, s), 7.67 (1H, s), 8.00 (1H, d, *J*= 7.8 Hz).

### [Example 12]

### 6-(1-Benzothiophen-3-yl)-2-(tert-butyl)-5-[(3S)-3-(dimethylamino)pyrrolidinyl]-7-methylimidazo[1,2-a]pyridine-8-carbonitrile (#12)

A 139 mg (0.37 mmol) portion of 6-(1-benzothiophen-3-yl)-2-(tert-butyl)-5-chloro-7-methylimidazo[1,2-a]pyridine-8-carbonitrile (I-37) was suspended in dimethyl sulfoxide (3.00 ml), mixed with 0.10 ml (0.73 mmol) of triethylamine and 56 µl (0.44 mmol) of (3S)-dimethylaminopyrrolidine, and stirred by heating at 90°C for 23 hours. After cooling to room temperature, the solvent was evaporated under a reduced pressure. The thus obtained residue was dissolved in chloroform and washed with saturated sodium bicarbonate aqueous solution and brine. The organic layer was dried over anhydrous sodium sulfate, the solvent was evaporated under a reduced pressure, and then the thus obtained residue was subjected to a silica gel column chromatography. By eluting with a mixed solvent of chloroform-methanol (20:1), 26 mg (16%) of the title compound was obtained as a pale red solid.
MS (FAB)m/z: 458 (M+1)⁺.
¹H-NMR (DMSO-*d*_{*6*})δ: 1.44 (9H, s), 1.50-1.70 (1H, m), 1.87 (1H, m), 2.04 (3H, s), 2.07 (3H, s), 2.21 (3H, d, *J=* 7.8 Hz), 2.34-2.39 / 2.51-2.55 (1H, m), 2.78-3.21 (4H, m), 7.23-7.45 (5H, m), 7.95 (1H, t, *J=* 6.6Hz).
IR (ATR): 2769, 1458 cm⁻¹.
Elemental analysis values: as C₂₇H₃₁N₅S·0.5H₂O
Calcd.: C, 69.49%; H, 6.91%; N, 15.01%; S, 6.87%
Found: C, 69.53%; H, 6.73%; N, 14.68%; S, 6.85%

### [Reference Example 38]

### 6-(1-Benzofuran-3-yl)-2-(tert-butyl)-7-methyl-5-oxo-1,5-dihydromidazo[1,2-a]pyridine-8-carbonitrile (I-38)

A mixture of 521 mg (2.24 mmol) of methyl 2-(1-benzofuran-3-yl)acetoacetate synthesized in accordance with the method described in Japanese Patent Application No. 2002-022767, 366 mg (2.24 mmol) of 2-[4-(tert-butyl)-1H-imidazo-2-yl]acetonitrile (I-29) and 356 mg (4.48 mmol) of ammonium acetate was heated at 150°C for 16.5 hours. After cooling, this was mixed with water and extracted with chloroform. The thus obtained organic layer was dried over anhydrous sodium sulfate, and then the solvent was evaporated under a reduced pressure. The thus obtained residue was subjected to a silica gel column chromatography. By eluting with a mixed solvent of chloroform:methanol (20:1), 350 mg (45%) of the title compound was obtained as a brown oily substance.
MS (ESI)m/z: 346 (M+1)⁺.
¹H-NMR (CDCl₃)δ: 1.42 (9H, s), 2.38 (3H, s), 7.21 (1H, t, *J*= 7.6 Hz), 7.28-7.33 (2H, m), 7.47 (1H, s), 7.54 (1H, d, *J*= 8.3 Hz), 7.65 (1H, s), 11.0 (1H, br).

### [Reference Example 39]

### 6-(1-Benzofuran-3-yl)-2-(tert-butyl)-5-chloro-7-methylimidazo[1,2-a]pyridine-8-carbonitrile (I-39)

A 350 mg (1.01 mmol) portion of 6-(1-benzofuran-3-yl)-2-(tert-butyl)-7-methyl-5-oxo-1,5-dihydroimidazo[1,2-a]pyridine-8-carbonitrile (I-38) was heated under reflux or 1.5 hours in phosphoryl chloride (3.50 ml). After cooling, the reaction solution was concentrated under a reduced pressure, mixed with chloroform, neutralized with saturated sodium bicarbonate aqueous solution and washed with brine. The thus obtained organic layer was dried over anhydrous sodium sulfate, and then the solvent was evaporated under a reduced pressure. The thus obtained residue was subjected to a silica gel column chromatography. By eluting with a mixed solvent of n-hexane-ethyl acetate (2:1), 192 mg (52%) of the title compound was obtained as a brown solid. MS (ESI)m/z: 364 (M+1)⁺.
¹H-NMR (CDCl₃)δ: 1.44 (9H, s), 2.44 (3H, s), 7.23-7.43 (3H, m), 7.57 (1H, s), 7.62 (1H, d, *J*= 8.3 Hz), 7.68 (1H, s).

### [Example 13]

### 6-(1-Benzofuran-3-yl)-2-(tert-butyl)-5-[(3S)-3-(dimethylamino)pyrrolidinyl]-7-methylimidazo[1,2-a]pyridine-8-carbonitrile (#13)

A 190 mg (0.52 mmol) portion of 6-(1-benzofuran-3-yl)-2-(tert-butyl)-5-chloro-7-methylimidazo[1,2-a]pyridine-8-carbonitrile (I-39) was suspended in dimethyl sulfoxide (4.00 ml), mixed with 0.14 ml (1.04 mmol) of triethylamine and 79 µl (0.63 mmol) of (3S)-dimethylaminopyrrolidine, and stirred by heating at 90°C for 4 hours. After cooling to room temperature, the solvent was evaporated under a reduced pressure. The thus obtained residue was dissolved in chloroform and washed with saturated sodium bicarbonate aqueous solution and brine. The organic layer was dried over anhydrous sodium sulfate, the solvent was evaporated under a reduced pressure, and then the thus obtained residue was subjected to a silica gel column chromatography. By eluting with a mixed solvent of chloroform-methanol (20:1) and purifying by recrystallization from diethyl ether, 104 mg (45%) of the title compound was obtained as a pale gray solid.
MS (FAB)m/z: 442 (M+1)⁺.
¹H-NMR (CDCl₃)δ: 1.44 (9H, s), 1.59-1.68 (1H, m), 1.91-1.99 (1H, m), 2.05 (3H, s), 2.09 (3H, s), 2.29, 2.31 (3H, s each), 2.35-2.44, 2.58-2.62 (1H, m each), 2.90-3.3 (4H, m), 7.24-7.29 (2H, m), 7.31, 7.32 (1H, s each), 7.37-7.42 (1H, m), 7.50, 7.56 (1H, s each), 7.60 (1H, t, *J*= 7.3 Hz).
IR (ATR): 2220, 1454 cm⁻¹.
Elemental analysis values: as C₂₇H₃₁N₅O
Calcd.: C, 73.44%; H, 7.08%; N, 15.86%
Found: C, 73.10%; H, 7.07%; N, 15.72%

### [Reference Example 40]

### Methyl 2-(1-methyl-1H-indol-3-yl)acetate (I-40)

A 5.00 g (26.43 mmol) portion of 2-(1-methyl-3-indole)acetic acid was dissolved in a mixed solvent of benzene (75 ml) and methanol (25 ml) and 15.90 ml of trimethylsilyl diazomethane (31.71 mmol, 2.0 M n-hexane solution) was added thereto under ice-cooling, and the mixture was stirred at room temperature for 14 hours. The reaction solution was evaporated under a reduced pressure, and the thus obtained residue was subjected to a silica gel column chromatography. By eluting with a mixed solvent of n-hexane-ethyl acetate (5:1), 5.68 g (quant.) of the title compound was obtained as a yellow and transparent oily substance
MS (ESI)m/z: 204 (M+1)⁺.
¹H-NMR (CDCl₃)δ: 3.69 (3H, s), 3.75 (3H, s), 3.77 (2H, s), 7.03 (1H, s), 7.12 (1H, dt, *J*= 0.9, 8.0 Hz), 7.22 (1H, dt, *J=* 0.9, 8.0 Hz), 7.28 (1H, dd, *J=* 0.7, 8.3 Hz), 7.59 (1H, dd, *J=* 0.7, 7.8 Hz).

### [Reference Example 41]

### Methyl 2-(1-methyl-1H-indol-3-yl)-3-oxobutanoate (I-41)

A 8.90 ml portion of n-butyl lithium (1.57 M n-hexane solution) was dissolved in tetrahydrofuran (60 ml), and 1.97 ml (14.02 mmol) of diisopropylamine was added dropwise thereto at -20°C. After 15 minutes of stirring at the same temperature, the reaction solution was cooled to -40°C, mixed with a tetrahydrofuran solution (15 ml) of 3.00 g (14.76 mmol) of methyl 2-(1-methyl-1H-indol-3-yl)acetate (I-40), and stirred at the same temperature for 1 hour. A 0.49 ml (5.17 mmol) portion of acetic anhydride was further added dropwise thereto and stirred for 16 hours while cooling to room temperature. The reaction solution was mixed with saturated ammonium chloride aqueous solution, extracted with diethyl ether and washed with brine. The thus obtained organic layer was dried over anhydrous sodium sulfate, and then the solvent was evaporated under a reduced pressure. The thus obtained residue was applied to a silica gel column chromatography and eluted with a mixed solvent of n-hexane-ethyl acetate (5:1), thereby obtaining 477 mg of the title compound (38% based on acetic anhydride) as a red oily substance of keto-enol mixture.
MS (ESI)m/z: 246 (M+1)⁺.
¹H-NMR (CDCl₃)δ: 1.90 (3H, s), 2.20 (3H, s), 3.66 (3H, s), 3.70 (3H, s), 3.77 (3H, s), 3.80 (3H, s), 4.98 (1H, s), 6.89 (1H, s), 7.10-7.18 (2H, m), 7.24-7.39 (6H, m), 7.57 (1H, d, *J =* 8.1 Hz), 13.26 (1H, s).

### [Reference Example 42]

### 2-(tert-Butyl)-7-methyl-6-(1-methyl-1H-indol-3-yl)-5-oxo-1,5-dihydroimidazo[1,2-a]pyridine-8-carbonitrile (I-42)

A mixture of 316 mg (1.94 mmol) of 2-[4-(tert-butyl)-1H-imidazo-2-yl]acetonitrile (I-29), 475 mg (1.94 mmol) of methyl 2-(1-methyl-1H-indol-3-yl)-3-oxobutanoate (I-41) and 308 mg (3.87 mmol) of ammonium acetate was heated at 150°C for 6 hours. After cooling, this was mixed with water and extracted with chloroform. The thus obtained organic layer was dried over anhydrous sodium sulfate, and then the solvent was evaporated under a reduced pressure. The thus obtained residue was purified by recrystallizing from acetonitrile to obtain 255 mg (37%) of the title compound as pale brown amorphous solid.
MS (ESI)m/z: 359 (M+1)⁺.
¹H-NMR (DMSO-d₆)δ: 1.38 (9H, s), 2.21 (3H, s), 3.84 (3H, s), 6.98 (1H, t, *J*= 6.8 Hz), 7.15 (1H, t, *J*= 6.8 Hz), 7.16 (1H, d, *J* = 8.3 Hz), 7.25 (1H, s), 7.41 (1H, s), 7.46 (1H, d, *J* = 8.3 Hz), 13.07 (1H, s).

### [Reference Example 43]

### 2-(tert-Butyl)-5-chloro-7-methyl-6-(1-methyl-1H-indol-3-yl)imidazo[1,2-a]pyridine-8-carbonitrile (I-43)

A 250 mg (0.70 mmol) portion of 2-(tert-butyl)-7-methyl-6-(1-methyl-1H-indol-3-yl)-5-oxo-1,5-dihydroimidazo[1,2-a]pyridine-8-carbonitrile (I-42) was heated under reflux for 2.5 hours in phosphoryl chloride (2.50 ml). After cooling, the thus obtained reaction solution was poured into ice water (10 ml), neutralized with saturated sodium bicarbonate aqueous solution, extracted with chloroform and dried over anhydrous sodium sulfate, and then the solvent was evaporated under a reduced pressure. After collecting the thus obtained crude crystals by filtration, the residue was applied to a silica gel column chromatography, eluted with a mixed solvent of n-hexane-ethyl acetate (4:1), combined with the crude crystals and then purified by recrystallizing from diethyl ether-ethyl acetate, thereby obtaining 170 mg (65%) of the title compound as a yellow solid.
MS (ESI)m/z: 377 (M+1)⁺.
¹H-NMR (CDCl₃)δ: 1.45 (9H, s), 2.42 (3H, s), 3.91 (3H, s), 7.07 (1H, s), 7.13-7.22 (2H, m), 7.32 (1H, dt, *J =* 1.5, 6.6 Hz), 7.43 (1H, d, *J*= 8.3 Hz), 7.56 (1H, s).

### [Example 14]

### 2-(tert-Butyl)-5-[(3S)-3-(dimethylamino)pyrrolidinyl]-7-methyl-6(1-methyl-1H-indol-3-yl)imidazo[1,2-a]pyridine-8-carbonitrile (#14)

A 168 mg (0.45 mmol) of 2-(tert-butyl)-5-chloro-7-methyl-6-(1-methyl-1H-indol-3-yl)imidazo[1,2-a]pyridine-8-carbonitrile (I-43) was suspended in dimethyl sulfoxide (3.40 ml), mixed with 0.14 ml (0.89 mmol) of triethylamine and 68 µl (0.54 mmol) of (3S)-dimethylaminopyrrolidine, and stirred by heating at 90°C for 2 hours. After cooling to room temperature, the solvent was evaporated under a reduced pressure. The thus obtained residue was dissolved in chloroform and washed with saturated sodium bicarbonate aqueous solution and brine. The organic layer was dried over anhydrous sodium sulfate, the solvent was evaporated under a reduced pressure, and the thus obtained residue was applied to a silica gel column chromatography. By eluting with a mixed solvent of chloroform-methanol (30:1) and purifying by recrystallizing from chloroform-n-hexane-ethyl acetate, 78 mg (38%) of the title compound was obtained as a colorless solid.
MS (ESI)m/z: 455 (M+1)⁺.
¹H-NMR (CDCl₃)δ: 1.44 (9H, s), 1.51-1.65 (1H, m), 1.83-1.90 (0.5H, m), 2.02, 2.03 (6H, s each), 2.25, 2.27 (3H, s each), 2.31-2.35 (0.5H, m), 2.49-2.52 (0.5H, m), 2.79-3.91 (4H, m), 6.85, 6.91 (1H, s each), 7.11-7.25 (1H, m), 7.26-7.31 (1H, m), 7.40 (1H, t, *J* = 7.6 Hz).
IR (ATR): 2220, 1460, 746 cm⁻¹.
Elemental analysis values: as C₂₈H₃₄N₆·0.25H₂O
Calcd.: C, 73.25%; H, 7.57%; N, 18.30%
Found: C, 73.28%; H, 7.51%; N, 18.04%

### [Reference Example 44]

### 2-(6-Methoxy-1-benzofuran-3-yl)acetic acid (I-44)

A 5.00 g (18.58 mmol) portion of 4-bromomethyl-7-methoxycoumarin was suspended in 1 N sodium hydroxide aqueous solution and heated under reflux for 6 hours. The reaction solution was ice-cooled, neutralized with 1 N hydrochloric acid aqueous solution and extracted with chloroform. The thus obtained organic layer was dried over anhydrous sodium sulfate, and then the solvent was evaporated under a reduced pressure to obtain 3.78 g (99%) of the title compound as a crude pale brown solid which was directly subjected to the subsequent reaction.

### [Reference Example 45]

### Methyl 2-(6-methoxy-1-benzofuran-3-yl)acetate (I-45)

At -40°C, 3.97 ml (55.11 mmol) of thionyl chloride was added dropwise to methanol (70 ml) and stirred at the same temperature for 30 minutes. A methanol solution (30 ml) of 3.78 g (18.37 mmol) of 2-(6-methoxy-1-benzofuran-3-yl)acetic acid (I-44) was added dropwise thereto and stirred at room temperature for 7 hours. The reaction solution was evaporated under a reduced pressure, and the residue was diluted with ethyl acetate and washed with saturated sodium bicarbonate aqueous solution and brine. The thus obtained organic layer was dried over anhydrous sodium sulfate, and then the solvent was evaporated under a reduced pressure. The thus obtained residue was applied to a silica gel column chromatography. By eluting with a mixed solvent of n-hexane-ethyl acetate (4:1), 3.89 g (96%) of the title compound was obtained as a colorless solid which was directly subjected to the subsequent reaction.

### [Reference Example 46]

### Methyl 2-(6-methoxy-1-benzofuran-3-yl)-3-oxobutanoate (I-46)

A 10.70 ml portion of n-butyl lithium (16.81 mmol, 1.57 M n-hexane solution) was dissolved in tetrahydrofuran (60 ml), and 2.36 ml (16.81 mmol) of diisopropylamine was added dropwise thereto at -20°C. After 15 minutes of stirring at the same temperature, the reaction solution was cooled to -40°C, mixed with a tetrahydrofuran solution (20 ml) of 3.89 g (17.69 mmol) of methyl 2-(6-methoxy-1-benzofuran-3-yl)acetate (I-45), and stirred at the same temperature for 1 hour. A 1.59 ml (16.81 mmol) portion of acetic anhydride was further added dropwise thereto and stirred for 19 hours while raising to room temperature. The reaction solution was mixed with saturated ammonium chloride aqueous solution, extracted with ethyl acetate and washed with brine. The thus obtained organic layer was dried over anhydrous sodium sulfate, and then the solvent was evaporated under a reduced pressure. The thus obtained residue was applied to a silica gel column chromatography and eluted with a mixed solvent of n-hexane-ethyl acetate (10:1), thereby obtaining 731 mg of the title compound (16% based on acetic anhydride) as a pale yellow and transparent oily substance of keto-enol mixture.
MS (ESI)m/z: 263 (M+1)⁺.
¹H-NMR (CDCl₃)δ: 1.95, 2.23 (3H, s each), 3.68, 3.79 (3H, s each), 3.85, 3.86 (3H, s each), 4.83 (0.2H, s), 6.87-6.92 (1H, m), 7.02-7.04 (1H, m), 7.23 (1H, d, *J* = 8.5 Hz), 7.38 (0.8H, s), 7.40 (0.2H, d, *J*= 8.5 Hz), 7.71 (0.2H, s), 13.25 (0.6H, s).

### [Reference Example 47]

### 2-(tert-Butyl)-6-(6-methoxy-1-benzofuran-3-yl)-7-methyl-5-oxo-1,5-dihydroimidazo[1,2-a]pyridine-8-carbonitrile (I-47)

A mixture of 451 mg (2.76 mmol) of 2-[4-(tert-butyl)-1H-imidazo-2-yl]acetonitrile (I-29), 725 mg (2.76 mmol) of methyl 2-(6-methoxy-1-benzofuran-3-yl)-3-oxobutanoate (I-46) and 439 mg (5.53 mmol) of ammonium acetate was heated at 150°C for 11 hours. After cooling, this was mixed with water and extracted with chloroform. The thus obtained organic layer was dried over anhydrous sodium sulfate, and then the solvent was evaporated under a reduced pressure. The thus obtained residue was applied to a silica gel column chromatography and eluted with a mixed solvent of n-hexane-ethyl acetate (3:1), thereby obtaining 300 mg (29%) of the title compound as a brown solid.
MS (ESI)m/z: 376 (M+1)⁺.
¹H-NMR (CDCl₃)δ: 1.42 (9H, s), 2.38 (3H, s), 3.86 (3H, s), 6.85 (1H, d, *J=* 8.3 Hz), 7.06 (1H, s), 7.18(1H,d, *J*=8.3Hz), 7.47(1H,s), 7.56(1H,s), 11.01(1H,s).

A compound capable of expressing an antifungal activity based on the functional mechanism of 1,6-β-glucan synthesis inhibition, with a broad spectrum and specifically or selectively, is provided, and an antifungal agent which comprises such a compound, a salt thereof or a solvate thereof is provided.
[Reference Example 48]
2-(tert-Butyl)-5-chloro-6-(6-methoxy-1-benzofuran-3-yl)-7-methylimidazo[1,2-a]pyridine-8-carbonitrile (I-48)
A 300 mg (0.80 mmol) portion of 2-(tert-butyl)-6-(6-methoxy-1-benzofuran-3-yl)-7-methyl-5-oxo-1,5-dihydroimidazo[1,2-a]pyridine-8-carbonitrile (I-47) was heated under reflux for 3 hours in phosphoryl chloride (3 ml). After cooling, the solvent was evaporated under a reduced pressure, and the residue was diluted with ethyl acetate, neutralized with saturated sodium bicarbonate aqueous solution and washed with brine. The thus obtained organic layer was dried over anhydrous sodium sulfate, and then the solvent was evaporated under a reduced pressure. The thus obtained crude crystals were purified by recrystallizing from n-hexane-ethyl acetate to obtain 241 mg (77%) of the title compound as a pale brown solid.
MS (ESI)m/z: 394 (M+1)⁺.
¹H-NMR (CDCl₃)δ: 1.44 (9H, s), 2.45 (3H, s), 3.89 (3H, s), 6.92 (1H, dd, *J=* 2.2, 8.8 Hz), 7.09 (1H, d, *J*= 8.3 Hz), 7.13 (1H, d, *J*= 2.2 Hz), 7.57 (1H, s), 7.58 (1H, s).

### [Example 15]

### 2-(tert-Butyl)-5-[(3S)-3-(dimethylamino)pyrrolidinyl]-6-(6-methoxy-1-benzofuran-3-yl)-7-methylimidazo[1,2-a]pyridine-8-carbonitrile (#15)

A 235 mg (0.60 mmol) portion of 2-(tert-butyl)-5-chloro-6-(6-methoxy-1-benzofuran-3-yl)-7-methylimidazo[1,2-a]pyridine-8-carbonitrile (I-48) was suspended in dimethyl sulfoxide (4.70 ml), mixed with 0.17 ml (1.19 mmol) of triethylamine and 91 µl (0.72 mmol) of (3S)-dimethylaminopyrrolidine, and heated at 90°C with stirring for 2 hours. After coolingto room temperature, the solvent was evaporated under a reduced pressure. The thus obtained residue was dissolved in chloroform and washed with saturated sodium bicarbonate aqueous solution and brine. The organic layer was dried over anhydrous sodium sulfate, the solvent was evaporated under a reduced pressure, and then the thus obtained residue was purified by recrystallizing from n-hexane-ethyl acetate, thereby obtaining 163 mg (57%) of the title compound as a colorless solid. MS (ESI)m/z: 472 (M+1)⁺.
¹H-NMR (CDCl₃)δ: 1.43 (9H, s), 1.72 (1H, m), 1.95-1.99 (1H, m), 2.10, 2.14 (6H, s each), 2.29, 2.31 (3H, s each), 2.44-2.50, 2.62-2.66 (1H, m each), 2.97-3.33 (4H, m), 3.88 (3H, s each), 6.90-6.92 (1H, m), 7.07-7.13 (2H, m), 7.31 (0.8H, d, *J*= 4.2 Hz), 7.40, 7.46 (1H, s each), 7.56 (0.2H, d, *J*= 5.4 Hz).
IR (ATR): 2222, 1228cm⁻¹.
Elemental analysis values: as C₂₈H₃₃N₅O₂·0.5H₂O
Calcd.: C, 69.98%; H, 7.13%; N, 14.57%
Found: C, 69.89%; H, 6.91%; N, 14.25%

### [Example 16]

### 2-(tert-Butyl)-7-methyl-6-phenyl-5-(1-piperazinyl)imidazo[1,2-a]pyridine-8-carbonitrile (#16)

A 300 mg (0.93 mmol) portion of 2-(tert-butyl)-5-chloro-7-methyl-6-phenylimidazo[1,2-a]pyridine-8-carbonitrile (I-31) was suspended in dimethyl sulfoxide (3 ml), mixed with 0.34 ml (2.41 mmol) of triethylamine and 104 mg (1.20 mmol) of piperazine, and stirred by heating at 90°C for 20 hours. After cooling to room temperature, the reaction solution was mixed with water, extracted with ethyl acetate, and washed with brine. The thus obtained organic layer was dried over anhydrous sodium sulfate, the solvent was evaporated under a reduced pressure, and then the resulting residue was purified by recrystallizing it from an ethyl acetate-n-hexane mixed solvent, thereby obtaining 30 mg (9%) of the title compound as a red solid.
MS (FAB)m/z: 3 74 (M+1)⁺.
¹H-NMR (DMSO-*d*₆)δ: 1.38 (9H, s), 2.17 (3H, s), 2.71 (4H, br s), 3.10 (4H, br s), 7.29 (2H, d, *J*= 8.1 Hz), 7.44(1H, s), 7.46-7.53 (3H, m).
IR (ATR): 2224, 1604, 1485, 1442 cm⁻¹.
Elemental analysis values: as C₂₃H₂₇N₅.0.5H₂O
Calcd.: C, 72.22%; H, 7.3 8%; N, 18.31%
Found: C, 71.91%; H, 7.24%; N, 17.83%

### [Reference Example 49]

### 2-Acetylhexanoic acid benzyl ester (1-49)

Under an atmosphere of nitrogen, 1.56 g (39.0 mmol) of sodium hydride was added under ice-cooling to a tetrahydrofuran (80 ml) solution of 15.0 g (78.0 mmol) of acetoacetic acid and stirred at the same temperature for 30 minutes, 3.40 ml (30.0 mmol) of 1-iodobutane was added thereto and heated under reflux for 15 hours. After cooling, the reaction solution was mixed with saturated ammonium chloride and extracted with ethyl acetate. The organic layer was washed with brine and dried over magnesium sulfate, and then the solvent was evaporated under a reduced pressure. The thus obtained residue was applied to a silica gel column chromatography, and 6.40 g (86%) of the title compound was obtained as a colorless oily substance from a fraction of n-hexane-ethyl acetate (30:1 v/v).
MS (FAB)*m*/*z*: 249 (M+1)⁺.
¹H-NMR (CDCl₃)δ: 0.87 (3H, t, *J*= 6.9 Hz), 1.19-1.36 (4H, m), 1.79-1.93 (2H, m), 2.17 (3H, s), 3.44 (1H, t, *J*= 7.5 Hz), 5.17 (2H, s), 7.26-7.40 (5H, m).

### [Reference Example 50]

### 2-Acetylhexanoic acid N'-benzoylhydrazide (I-50)

A mixture of 3.70 g (2.89 mmol) of 2-acetylhexanoic acid benzyl ester (I-49), 3.30 ml (30.0 mmol) of trimethyl orthoformate and 0.14 g (0.75 mmol) of tosylic acid monohydrate was heated at 35°C for 21 hours. After concentration under a reduced pressure, the thus obtained residue was applied to a silica gel column chromatography, and 2-(1,1-dimethoxyethyl)-hexanoic acid benzyl ester was obtained as pale yellow crystals (4.0 g, including byproducts) from an n-hexane-ethyl acetate (50:1 v/v) eluate. A 3.5 g portion of the thus obtained crystals were dissolved in methanol (20 ml), mixed with 600 mg of 5% palladium-carbon catalyst and stirred for 2 hours under an atmosphere of hydrogen. After removing the catalyst by filtration, the solvent was evaporated under a reduced pressure to obtain crude 2-(1,1-dimethoxyethyl)-hexanoic acid (2.4 g, including by-products). This mixture (2.3 g) was dissolved in dichloromethane (35 ml), and under ice-cooling, 2.60 g (19.0 mmol) of benzhydrazide, 3.20 g (16.5 mmol) of 1-ethyl-3-(3-dimethylaminopropyl)carbodiimide hydrochloride and 2.0 g (15.0 mmol) of 1-hydroxybenzotriazole were added thereto, and the mixture was stirred at room temperature for 19 hours. After adding 1 N hydrochloric acid, this was stirred for 30 minutes and the precipitated crystals were removed by filtration. The filtrate was washed with saturated sodium bicarbonate aqueous solution and brine, and the thus separated organic layer was dried over magnesium sulfate, and then the solvent was evaporated. The thus obtained residue was applied to a silica gel column chromatography, and 1.50 g (42%, total yield from 2-acetylhexanoic acid benzyl ester) of the title compound was obtained as colorless crystals from a chloroform-methanol (100:1 v/v) eluate.
MS (FAB)m/a: 277 (M+1)⁺.
¹H-NMR (CDCl₃)δ: 0.85 (3H, t, *J*= 6.9 Hz), 1.20-1.37 (4H, m), 1.88 (2H, dd, *J*= 7.5, 14.7 Hz), 2.24 (3H, s), 3.57 (1H, t, *J*= 7.5 Hz), 7.38 (2H, m), 7.51 (1H, m), 7.80 (2H, m), 9.86 (1H, d, *J*= 5.4 Hz), 10.09 (1H, d, *J*= 5.4 Hz).

### [Reference Example 51-1]

### 6-n-Butyl-7-methyl-5-oxo-2-phenyl-1,5-dihydro-[1,2,4]triazolo[1,5-a]pyridine-8-carbonitrile (I-51)

### Synthesis Example 1

A methanol (5 ml) suspension of 830 mg (3.00 mmol) of 2-acetylhexanoic acid N'-benzoylhydrazide (I-50) was mixed with 200 mg (3.00 mmol) of malononitrile and 170 mg (3.00 mmol) of sodium methoxide and heated under reflux for 8 hours. The reaction solution was poured into ice water, mixed with 0.17 ml (3.00 mmol) of acetic acid and stirred for several minutes, and then the resulting crystals were collected by filtration and dried. Subsequently, this product was heated under reflux for 4 hours in acetic acid (3.00 ml). After cooling, this was diluted with the same volume of water, and the crystals were collected by filtration and dried to obtain 754 mg (82%) of the title compound as colorless crystals.
MS (FAB)*m*/*z*: 307 (M+1)⁺.
¹H-NMR (CDCl₃)δ: 0.89 (3H, t, *J =* 6.9 Hz), 1.28-1.41 (4H, m), 2.38 (3H, s), 2.54 (2H, t, *J*= 7.2 Hz), 7.60 (3H, m), 8.15 (2H, m).

### [Reference Example 52]

### Methyl benzimidate hydrochloride (I-52)

A 734 ml (2.94 mol) portion of 4 N hydrochloric acid dioxane solution was added under ice-cooling to a methanol (95.2 ml, 2.36 mol) solution of 200 ml benzonitrile (1.96 mol) and stirred at room temperature for 5 hours, and then the reaction solution was concentrated under a reduced pressure. Ether (500 ml) was added to the thus obtained residue, and the precipitated crystals were collected by filtration and dried to obtain 107 g (32%) of the title compound as a colorless solid.
¹H-NMR (CDCl₃)δ: 4.58 (3H, s), 7.58 (2H, m), 7.72 (1H, m), 8.41 (2H, m).

### [Reference Example 53]

### 5-Cyanomethyl-3-phenyl-1H-[1,2,4]triazole (I-53)

A 1.40 g (33.3 mmol) portion of sodium hydroxide was dissolved in methanol (60 ml) and ice-cooled. This was mixed with 5.70 g (33.2 mmol) of methyl benzimidate hydrochloride (I-52) and 3.39 g (34.2 mmol) of cyanoacetohydrazide and heated under reflux for 2 hours. The reaction solution was concentrated under a reduced pressure, and the thus obtained residue was mixed with brine and extracted with ethyl acetate. The organic layer was dried over magnesium sulfate, and the solvent was evaporated. The thus obtained residue was washed with ether, collected by filtration and dried to obtain 4.93 g (81%) of the title compound as a pale yellow solid.
MS (FAB)*m*/*z:* 185 (M+1)⁺.
¹H-NMR (DMSO-d₆)δ: 4.22 (2H, s), 7.50 (3H, m), 7.97 (2H, m).

### [Reference Example 51-2]

### 6-n-Butyl-7-methyl-5-oxo-2-phenyl-1,5-dihydro-[1,2,4]triazolo[1,5-a]pyridine-8-carbonitrile (I-51)

### Synthesis Example 2

A mixture of 1.00 g (5.43 mmol) of 5-cyanomethyl-3-phenyl-1H-[1,2,4]triazole (I-53), 1.06 g (5.70 mmol) of 2-acetylhexanoic acid ethyl ester and 879 mg (11.4 mmol) of ammonium acetate was heated at 150°C for 1 hour. After cooling, this was mixed with water, and the precipitated crystals were collected by filtration and further washed with acetonitrile. This was collected by filtration and dried to obtain 1.13 g (68%) of the title compound as a colorless solid. Its physical data is identical to those of the sample obtained in Synthesis Example 1.

### [Reference Example 51-3]

### 6-n-Butyl-7-methyl-5-oxo-2-phenyl-1,5-dihydro-[1,2,4]triazolo[1,5-a]pyridine-8-carbonitrile (I-51)

### Synthesis Example 3

A mixture of 2.12 g (12.0 mmol) of 5-cyanomethyl-3-phenyl-1H-[1,2,4]triazole (I-53) and 4.47 g (24.0 mmol) of 2-acetylhexanoic acid ethyl ester was stirred at 75°C for 10 minutes, and then mixed with 2.4 g (13.2 mmol) of sodium methoxide 30% methanol solution and stirred at 115°C for 6 hours (in this case, methanol was evaporated, and the reaction mixture was gradually solidified). After cooling, this was mixed with water (6 ml) and concentrated hydrochloric acid (2.4 ml) and then heated under reflux for 10 minutes. After cooling, the precipitated crystals were collected by filtration, washed with acetonitrile and then dried to obtain 2.11 g (57%) of the title compound as a light brown solid. Its physical data is identical to those of the sample obtained in Synthesis Example 1.

### [Reference Example 54]

### 6-n-Butyl-5-chloro-7-methyl-2-phenyl[1,2,4]triazolo[1,5-a]pyridine-8-carbonitrile (I-54)

A 1.00 g (3.26 mmol) portion of 6-n-butyl-7-methyl-5-oxo-2-phenyl-1,5-dihydro-[1,2,4]triazolo[1,5-a]pyridine-8-carbonitrile (I-51) was heated under reflux for 2 hours in phosphoryl chloride (5 ml). After cooling, phosphoryl chloride was evaporated under a reduced pressure, and the thus obtained residue was mixed with ice water and extracted with chloroform. The organic layer was washed with brine and dried over magnesium sulfate, and the solvent was evaporated, thereby obtaining 1.01 g (95%) of the title compound as a pale yellow solid.
MS (FAB)*m*/*z*: 325 (M+1)⁺.
¹H-NMR (CDCl₃)δ: 1.01 (3H, d, *J* = 7.1 Hz), 1.54 (4H, m), 2.74 (3H, s), 2.89 (2H, t, *J*= 7.8 Hz), 7.50 (3H, m), 8.36 (2H, m).

### [Example 17]

### 6-n-Butyl-7-methyl-5-[(3S)-dimethylaminopyrrolidin-1-yl]-2-phenyl[1,2,4]triazolo[1,5-a]pyridine-8-carbonitrile (#17)

A 215 µl (1.69 mmol) portion of (3S)-dimethylaminopyrrolidine and 429 µl (3.08 mmol) of triethylamine were added to an N,N-dimethylformamide (5 ml) suspension of 500 mg (1.54 mmol) of 6-n-butyl-5-chloro-8-cyano-7-methyl-2-phenyl[1,2,4]triazolo[1,5-a]pyridine (I-54) and stirred at 80 to 90°C for 6.5 hours. After cooling, the reaction solution was concentrated under a reduced pressure, and the thus obtained residue was dissolved in chloroform and washed with saturated sodium bicarbonate aqueous solution and brine. After drying the organic layer over magnesium sulfate, the solvent was evaporated under a reduced pressure. The thus obtained residue was applied to a silica gel column chromatography, and 569 mg (92%) of the title compound was obtained as pale yellow crystals from a chloroform-methanol (98.5:1.5 v/v) eluate. MS (FAB)*m*/*z*: 403 (M+1)⁺.
¹H-NMR (CDCl₃)δ: 0.99 (3H, t, *J=* 7.1 Hz), 1.48 (4H, m), 2.11 (1H, m), 2.33 (1H, m), 2.36 (6H, s), 2.67 (3H, m), 2.78 (2H, m), 3.17 (1H, quint, *J=* 8.0 Hz), 3.49-3.61 (2H, m), 3.71-3.87 (2H, m), 7.48 (3H, m), 8.33 (2H, m).
IR (ATR): 2224, 1620, 1537, 1504, 1475, 1441, 1342 cm⁻¹.
Elemental analysis values: as C₂₄H₃₀N₆
Calcd.: C, 71.61%; H, 7.51%; N, 20.88%
Found: C, 71.39%; H, 7.53%; N, 20.92%

<Reference Examples 55 to 66, 68, 71, 74 and 76 to 125, Examples 18 to 41>

**[Table 1]**

| Substituent R | x | y | z | γ | ε |
|---|---|---|---|---|---|
| Ph | | I-53 | I-93 | I-109 | #18 |
| Me | I-5 | I-77 | I-94 | I-110 | #19 |
| Et | I-56 | I-78 | I-95 | I-111 | #20 |
| Cyclopropyl | I-57 | I-79 | I-96 | I-112 | #21 |
| i-Pr | I-58 | I-80 | I-97 | I-113 | #22 |
| n-Bu | I-59 | I-81 | I-98 | I-114 | #23 |
| i-Bu | I-60 | I-82 | I-99 | I-115 | #24 |
| t-Bu | I-61 | I-83 | I-100 | I-116 | #25 |
| 2-pyridyl | I-62 | I-84 | | | |
| 3-pyridyl | I-63 | I-85 | I-101 | I-117 | #26 |
| 4-pyridyl | I-64 | I-86 | I-102 | I-118 | #27 |
| MeOCH₂ | I-65 | I-87 | I-103 | I-119 | #28 |
| C(Me)₂OH | I-66 | I-88 | I-104 | I-120 | #33 |
| CH₂CH₂OBn (I-67) | I-68 | I-89 | I-105 | I-121 | #34 |
| CH₂CH₂Cl | | | | I-122 | #36 |
| Mixture of C(Me)₂COOEt and C(Me)₂COOMe (I-69) | I-71 | I-90 | I-106 | I-123 | #37 |
| C(Me)₂CH₂OBn (I-73) | I-74 | I-91 | I-107 | I-124 | #39 |
| C(Me)₂CH₂OCH₂(p-F-Ph) (I-75) | I-76 | I-92 | I-108 | I-125 | #41 |

In the above Table 1,
Ph represents phenyl group,
Me represents methyl group,
Et represents ethyl group,
Pr represents propyl group,
Bu represents butyl group, and
Bn represents benzyl group.
In addition, the x, v, z, γ and ε in the above Table 1 are shown by the following formulae.

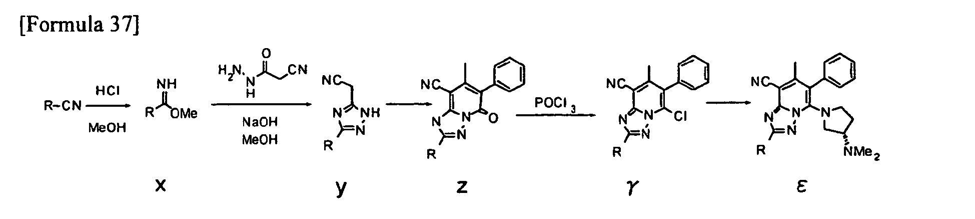

### [Reference Example 55]

### Ethyl acetimidate hydrochloride (I-55)

A 215 ml (0.86 mol) portion of 4 N hydrochloric acid dioxane solution was added, under ice-cooling, to 40.0 ml (0.689 mol) ethanol solution of 30.0 ml (0.574 mol) of acetonitrile and stirred at room temperature for 4.5 hours, and then the reaction solution was concentrated under a reduced pressure. Ether (200 ml) was added to the thus obtained residue, and the precipitated crystals were collected by filtration and dried to obtain 40.6 g (57%) of the title compound as a colorless solid.
¹H-NMR (DMSO-d₆)δ: 1.31 (3H, t, *J*= 7.1 Hz), 2.34 (3H, s), 4.39 (2H, q, *J*= 7.1 Hz).

### [Reference Example 56]

### Methyl propionimidate hydrochloride (I-56)

A methanol (130 ml) solution of 25.0 ml (0.328 mol) of propionitrile was stirred at -10°C for 5 hours while bubbling hydrogen chloride gas into the solution, and then the reaction solution was concentrated under a reduced pressure. Ether was added to the thus obtained residue, and the precipitated crystals were collected by filtration and dried to obtain 41.7 g (100%) of the title compound as a pale yellow solid.
¹H-NMR (CD₃OD)δ: 1.26 (3H, t, *J=* 7.5 Hz), 4.16 (3H, s).

### [Reference Example 57]

### Methyl cyclopropionimidate hydrochloride (I-57)

A methanol (130 ml) solution of 25.0 ml (0.329 mol) of cyclopropyl cyanide was stirred at -10°C for 5 hours while bubbling hydrogen chloride gas into the solution, and then the reaction solution was concentrated under a reduced pressure. Ether was added to the thus obtained residue, and the precipitated crystals were collected by filtration and dried to obtain 43.2 g (97%) of the title compound as colorless crystals.
¹H-NMR (CDCl₃)δ: 1.20-1.32 (4H, m), 2.46 (1H, m), 4.24 (1H, m), 11.35 (1H, brs), 12.48 (1H, brs).

### [Reference Example 58]

### Methyl isobutylimidate hydrochloride (I-58)

A 113 ml (0.45 mol) portion of 4 N hydrochloric acid dioxane solution was added under ice-cooling to a 15.0 ml (0.36 mol) methanol solution of 27.0 ml (0.30 mol) of isobutyronitrile and stirred at room temperature for 5 hours, and then the reaction solution was concentrated under a reduced pressure. Ether was added to the thus obtained residue, and the precipitated crystals were collected by filtration and dried to obtain 25.8 g (63%) of the title compound as a colorless solid.
¹H-NMR (CD₃OD)δ: 1.29 (6H, d, *J=* 6.9 Hz), 1.94 (1H, sep, *J*= 6.9 Hz), 4.16 (3H, s).

### [Reference Example 59]

### Methyl pentanimidate hydrochloride (I-59)

A 108 ml (0.431 mol) portion of 4 N hydrochloric acid dioxane solution was added under ice-cooling to a 13.9 ml (0.344 mol) methanol solution of 30.0 ml (0.287 mol) of valeronitrile and stirred at room temperature for 5.5 hours, and then the reaction solution was concentrated under a reduced pressure. Ether (200 ml) was added to the thus obtained residue, and the precipitated crystals were collected by filtration and dried to obtain 26.0 g (60%) of the title compound as a colorless solid.
¹H-NMR (DMSO-d₆)δ: 0.86 (3H, t, *J*= 7.5 Hz), 1.29 (1H, sex, *J*= 7.5 Hz), 1.57 (2H, quint, *J*= 7.5 Hz), 2.62 (2H, t, *J*= 7.5 Hz), 4.06 (3H, s), 11.6 (2H, brs).

### [Reference Example 60]

### Methyl 3-methylbutylimidate hydrochloride (I-60)

A 90 ml (0.36 mol) portion of 4 N hydrochloric acid dioxane solution was added under ice-cooling to a 12.0 ml (0.29 mol) methanol solution of 25.0 ml (0.24 mol) of isovaleronitrile and stirred at room temperature for 5 hours, and then the reaction solution was concentrated under a reduced pressure. Ether was added to the thus obtained residue, and the precipitated crystals were collected by filtration and dried to obtain 10.8 g (30%) of the title compound as a colorless solid.
¹H-NMR (CD₃OD)δ: 1.02 (6H, d, *J*= 6.6 Hz), 2.13 (1H, m), 2.53 (2H, d, *J*= 7.2 Hz), 4.16 (3H, s).

### [Reference Example 61]

### Methyl 2,2-dimethylpropionimidate hydrochloride (I-61)

A 102 ml (0.407 mol) portion of 4 N hydrochloric acid dioxane solution was added under ice-cooling to a 13.2 ml (0.326 mol) methanol solution of 30.0 ml (0.271 mol) of trimethylacetonitrile and stirred at room temperature for 7.5 hours, and then the reaction solution was concentrated under a reduced pressure. Ether (100 ml) and n-hexane (50 ml) were added to the thus obtained residue, and the precipitated crystals were collected by filtration and dried to obtain 8.06 g (20%) of the title compound as a colorless solid.
¹H-NMR (CDCl₃)δ: 1.25 (9H, s), 4.10 (3H, s), 11.2 (2H, brs).

### [Reference Example 62]

### Ethyl pyridine-2-imidate dihydrochloride (I-62)

Hydrogen chloride gas was bubbled into an ethanol (16 ml)-dichloromethane (200 ml) mixed solution of 20.5 g (0.197 mol) of 2-cyanopyridine for 1 hour under ice-cooling, and then stirred overnight at room temperature (crystals were precipitated). After bubbling nitrogen gas into the reaction suspension for 1 hour, the crystals were washed with ether and then collected by filtration and dried to obtain 40.4 g (92%) of the title compound as colorless crystals.
¹H-NMR (CD₃OD)δ: 1.64 (3H, t, *J*= 7.1 Hz), 4.72 (2H, q, *J=* 7.1 Hz), 7.74-7.84 (1H, m), 8.07-8.17 (1H, m), 8.19-8.31 (1H, m), 8.81-8.86 (1H, m).

### [Reference Example 63]

### Ethyl nicotinimidate dihydrochloride (I-63)

Hydrogen chloride gas was bubbled into an ethanol (16 ml)-dichloromethane (200 ml) mixed solution of 20.0 g (0.192 mol) of 3-cyanopyridine for 1 hour under ice-cooling, and then stirred overnight at room temperature (crystals were precipitated). After bubbling nitrogen gas into the reaction suspension for 1 hour, the crystals were washed with ether and then collected by filtration and dried to obtain 41.4 g (97%) of the title compound as colorless crystals.
¹H-NMR (CD₃OD)δ: 1.17 (3H, t, *J=* 7.0 Hz), 3.60 (2H, q, *J=* 7.0 Hz), 8.17-8.38 (1H, m), 8.96-9.24 (2H, m), 9.30-9.52 (1H, m).

### [Reference Example 64]

### Ethyl isonicotinimidate dihydrochloride (I-64)

Hydrogen chloride gas was bubbled into an ethanol (16 ml)-dichloromethane (200 ml) mixed solution of 20.0 g (0.192 mol) of 4-cyanopyridine for 1 hour under ice-cooling, and then stirred overnight at room temperature (crystals were precipitated). After bubbling nitrogen gas into the reaction suspension for 1 hour, the crystals were washed with ether and then collected by filtration and dried to obtain 42.9 g (100%) of the title compound as colorless crystals.
¹H-NMR (CD₃OD)δ: 1.08 (3H, t, *J =* 7.1 Hz), 3.51 (2H, q, *J =* 7.1 Hz), 8.42-8.51 (2H, m), 8.97-9.02 (1H, m), 9.08-9.15 (1H, m).

### [Reference Example 65]

### Methyl 2-methoxyacetimidate hydrochloride (I-65)

Hydrogen chloride gas was bubbled into a methanol (100 ml) solution of 10.0 ml (0.134 mol) of methoxyacetonitrile at -10°C for 1 hour (internal temperature was -5°C or lower), the internal temperature was raised to 5°C thereafter spending 1 hour, and then this was further stirred for 2.5 hours. After concentration of the reaction solution, ether (100 ml) was added to the thus obtained residue, and the precipitated crystals were collected by filtration and dried to obtain 17.6 g (94%) of the title compound as colorless crystals.
¹H-NMR (CD₃OD)δ: 3.34 (3H, s), 3.51 (3H, s), 4.39 (2H, s).

### [Reference Example 66]

### Methyl 2-hydroxy-2-methylpropionimidate hydrochloride (I-66)

Hydrogen chloride gas was bubbled into a methanol (150 ml) solution of 5.2 ml (97%, 57.0 mmol) of cyanohydrin for 4 hours while stirring by keeping the internal temperature at 3°C or lower. After concentration of the reaction solution (to about 50 ml), ether (300 ml) was added to the thus obtained residue, and the precipitated crystals were collected by filtration and dried to obtain 8.12 g (93%) of the title compound as colorless crystals.
¹H-NMR (CD₃OD)δ: 1.49 (6H, s), 4.12 (3H, s).

### [Reference Example 67]

### 3-Benzyloxypropionitrile (I-67)

A 12.3 g (0.308 mol) portion of sodium hydride was suspended in tetrahydrofuran (200 ml), 20.0 ml (0.293 mol) of 3-hydronypropionitrile was added dropwise thereto while keeping the internal temperature below 0°C, and the mixture was stirred at the same temperature for 10 minutes. Subsequently, 36.6 ml (0.308 mol) of benzyl bromide was added thereto, and then N,N-dimethylformamide (40 ml) was added thereto by taking care of exothermic reaction. After 2 hours of stirring at 0°C, the reaction solution was concentrated, and the resulting residue was poured into saturated ammonium chloride aqueous solution. This was extracted with chloroform, and the organic layer was washed with brine and then dried over magnesium sulfate. The solvent was evaporated, and the thus obtained residue was applied to a silica gel column chromatography, and 33.0 g (70%) of the title compound was obtained as a pale yellow oily substance from a n-hexane-ethyl acetate (5:1 v/v) eluate.
¹H-NMR (CDCl₃)δ: 2.62 (2H, t, *J=* 6.4 Hz), 3.68 (2H, t, *J*= 6.4 Hz), 4.58 (2H, s), 7.25-7.37 (5H, m).

### [Reference Example 68]

### Methyl 3-benzyloxypropionimidate hydrochloride (I-68)

Hydrogen chloride gas was bubbled into a methanol (120 ml) solution of 20.0 g (0.124 mol) of 3-benzyloxypropionitrile (I-67) for 6 hours by keeping the internal temperature below - 5°C, and then the internal temperature was raised to 5°C spending 1.5 hours. After concentration of the reaction solution, ether (150 ml) was added to the thus obtained residue, and the precipitated crystals were collected by filtration and dried to obtain 25.0 g (88%) of the title compound as a pale yellow solid.
¹H-NMR (CDCl₃)δ: 3.05 (2H, t, *J*= 5.9 Hz), 3.83 (2H, t, *J=* 5.9 Hz), 4.28 (3H, s), 4.54 (2H, s), 7.26-7.37 (5H, m), 11.7 (1H, brs), 12.5 (1H, brs).

### [Reference Example 69]

### A mixture of cyano-dimethylacetic acid ethyl ester and cyano-dimethylacetic acid methyl ester (I-69)

Under ice-cooling, 8.8 g (0.22 mol) of sodium hydride was added to an N,N-dimethylformamide (250 ml) solution of 11.3 g (0.10 mol) of ethyl cyanoacetate and stirred at the same temperature for 30 minutes. A tetrahydrofuran (15 ml) solution of 15.6 ml (0.25 mol) of methyl iodide was added dropwise thereto spending 45 minutes, and then this was stirred at room temperature for 71 hours. This was mixed with brine and extracted with ethyl acetate, the organic layer was dried over magnesium sulfate, and the solvent was evaporated. The thus obtained residue was applied to a silica gel column chromatography, and 8.27 g of the title compound was obtained as a colorless oily substance from a n-hexane-ethyl acetate (5:1 v/v) eluate. This was used in the subsequent reaction as such.

### [Reference Example 70]

### Cyano-dimethylacetic acid ethyl ester (I-70)

A 242 g (1.75 mol) portion of potassium carbonate was added to a dimethyl sulfoxide (200 ml) solution of 56.6 g (0.50 mol) of ethyl cyanoacetate, 109 ml (1.75 mol) of methyl iodide was added dropwise thereto spending 1 hour under ice-cooling, and then this was further stirred at room temperature for 2 hours. The reaction solution was filtered, and the filtrate was mixed with brine and extracted with ethyl acetate. The organic layer was dried over magnesium sulfate, and the solvent was evaporated. The thus obtained residue was applied to a silica gel column chromatography, and 57.6 g (82%) of the title compound was obtained as a colorless oily substance from a n-hexane-ethyl acetate (9:1 v/v) eluate.
¹H-NMR (CDCl₃)δ: 1.33 (3H, t, *J*= 7.2 Hz), 1.61 (6H, s), 4.27 (2H, q*, J=* 7.2 Hz).

### [Reference Example 71]

### A mixture of 2-ethoxycarbonyl-2-methylpropionic acid ethyl ester hydrochloride and 2-ethoxycarbonyl-2-methylpropionic acid methyl ester hydrochloride (I-71)

At -10°C, hydrogen chloride gas was bubbled into an ethanol (50 ml) solution of 8.00 g of the mixture of cyano-dimethylacetic acid ethyl ester and cyano-dimethylacetic acid methyl ester (I-69), while stirring for 6 hours. After concentration of the reaction solution, ether was added to the thus obtained residue and stirred for 30 minutes, and the precipitated crystals were collected by filtration and dried to obtain 8.2 g of the title compound as a colorless solid. This was directly used in the subsequent reaction.

### [Reference Example 72]

### 3-Hydroxy-2,2-dimethylpropionitrile (I-72)

Under an atmosphere of nitrogen, a tetrahydrofuran (200 ml) solution of 28.2 g (0.20 mol) of cyano-dimethylacetic acid ethyl ester (1-70) was added dropwise to a tetrahydrofuran (500 ml) solution of 4.8 g (0.22 mol) of lithium borohydride spending 30 minutes, and then the mixture was stirred overnight at room temperature. By adding 6 N hydrochloric acid to the reaction solution, separation of layers was effected, the aqueous layer was extracted with ethyl acetate, combined with the first organic layer, washed with brine and then dried over magnesium sulfate. The solvent was evaporated, ether was added to the resulting residue, the insoluble material was removed by filtration, and then the solvent of the filtrate was evaporated. The thus obtained residue was applied to a silica gel column chromatography, and 12.5 g (63%) of the title compound was obtained as a colorless oily substance from a n-hexane-ethyl acetate (2:1 v/v) eluate.
¹H-NMR (CDCl₃)δ: 1.36 (6H, s), 2.26 (1H, brs), 3.58 (2H, s).

### [Reference Example 73]

### 3-Benzyloxy-2,2-dimethylpropionitrile (I-73)

N,N-dimethylformamide (55 ml) was added to a tetrahydrofuran (330 ml) of 10.9 g (0.11 mol) of 3-hydroxy-2,2-dimethylpropionitrile (I-72), and 5.3 g (0.132 mol) of sodium hydride was added thereto under ice-cooling and then stirred at room temperature for 30 minutes. This was again ice-cooled, 19.6 ml (0.165 mol) of benzyl bromide and 4.1 g (11.0 mmol) of tetra-n-butylammonium iodide were added thereto and then stirred overnight while raising the temperature to room temperature. The reaction solution was mixed with saturated ammonium chloride aqueous solution and extracted with ethyl acetate, the organic layer was washed with brine and then dried over magnesium sulfate, and the solvent was evaporated. The thus obtained residue was applied to a silica gel column chromatography, and 20.0 g (96%) of the title compound was obtained as a colorless oily substance from a n-hexane-ethyl acetate (9:1 v/v) eluate.
¹H-NMR (CDCl₃)δ: 1.36 (6H, s), 3.38 (2H, s), 4.62 (2H, s), 7.29-7.37 (5H, m).

### [Reference Example 74]

### Methyl 3-benzyloxy-2,2-dimethylpropionimidate hydrochloride (1-74)

A 8.1 ml (0.20 mol) portion of methanol was added to a dichloromethane (200 ml) solution of 18.9 g (0.10 mol) of 3-benzyloxy-2,2-dimethylpropionitrile (I-73), and cooling to -5°C, hydrogen chloride gas was bubbled into the mixture for 1 hour. After stirring overnight at 0°C, the reaction solution was concentrated, and the thus obtained residue was mixed with ethanol (150 ml) and stirred for 30 minutes. The precipitated crystals were collected by filtration and dried to obtain 15.2 g (59%) of the title compound as a pale yellow solid.
¹H-NMR (CDCl₃)δ: 1.38 (6H, s), 3.63 (2H, s), 4.33 (3H, s), 4.55 (2H, s), 7.27-7.37 (5H, m).

### [Reference Example 75]

### 3-(4-Fluorobenzyloxy)-2,2-dimethylpropionitrile (I-75)

N,N-dimethylformamide (10 ml) was added to a tetrahydrofuran (60 ml) solution of 2.0 g (20.0 mmol) of 3-hydroxy-2,2-dimethylpropionitrile (I-72), and under ice-cooling, 1.0 g (24.0 mmol) of sodium hydride was added thereto and stirred for 30 minutes. Under ice-cooling, 3.90 ml (24.0 mmol) of 1-bromomethyl-4-fluorobenzene and 700 mg (2.00 mmol) of tetra-n-butylammonium iodide were added thereto, and then this was stirred overnight while raising the temperature to room temperature. The reaction solution was mixed with water and extracted with ethyl acetate, the organic layer was washed with brine and then dried over magnesium sulfate, and the solvent was evaporated. The thus obtained residue was applied to a silica gel column chromatography, and 2.1 g (50%) of the title compound was obtained as a colorless oily substance from a n-hexane-ethyl acetate (15:1 v/v) eluate.
^{l}H-NMR (CDCl₃)δ: 1.36 (6H, s), 3.38 (2H, s), 4.57 (2H, s), 7.04 (2H, m), 7.32 (2H, m).

### [Reference Example 76]

### Methyl 3-(4-fluorophenyl)-2.2-dimethylpropionimidate hydrochloride (1-76)

A 0.8 ml (20 mmol) portion of methanol was added to a dichloromethane (20 ml) solution of 1.90 g (9.20 mmol) of 3-(4-fluorobenzyloxy)-2,2-dimethylpropionitrile (I-75), and after cooling to -10°C, hydrogen chloride gas was bubbled into the mixture for 1.5 hours. After stirring overnight at 0°C, the reaction solution was concentrated, and the thus obtained residue was mixed with ether (150 ml) and stirred for 30 minutes. The precipitated crystals were collected by filtration and dried to obtain 1.10 g (42%) of the title compound as a pale yellow solid.
¹H-NMR (CDCl₃)δ: 1.19 (6H, s), 3.62 (2H, s), 4.33 (3H, s), 4.52 (2H, s), 7.03 (2H, m), 7.28 (2H, m).

### [Reference Example 77]

### 5-Cyanomethyl-3-methyl-1H-[1,2,4]triazole (I-77)

A methanol (60 ml) solution of 3.39 g (95%, 34.2 mmol) of sodium hydroxide was mixed with 4.10 g (33.2 mmol) of ethyl acetimidate hydrochloride (I-55) and 3.39 g (34.2 mmol) of cyanoacetohydrazide and heated under reflux for 2 hours. After cooling, the reaction solution was concentrated under a reduced pressure, the thus obtained residue was mixed with ethanol, the insoluble material was removed by filtration, and the solvent of the filtrate was evaporated. The thus obtained residue was applied to a silica gel column chromatography, and 3.01 g (74%) of the title compound was obtained as a colorless solid from a chloroform-methanol (30:1 v/v) eluate. MS (FAB)*m*/*z:* 123 (M+1)⁺.
¹H-NMR (DMSO-d₆)δ: 2.32 (3H, s), 4.03 (2H, s).

### [Reference Example 78]

### (5-Ethyl-4H-[1,2,4]triazol-3-yl)-acetonitrile (I-78)

A methanol (100 ml) solution of 2.04 g (51.0 mmol) of sodium hydroxide was mixed with 6.18 g (50.0 mmol) of methyl propionimidate hydrochloride (I-56) and 5.46 g (51.5 mmol) of cyanoacetohydrazide and heated under reflux for 2.5 hours. After cooling, the reaction solution was concentrated under a reduced pressure, the thus obtained residue was applied to a silica gel column chromatography, and 6.00 g (88%) of the title compound was obtained as colorless crystals from an eluate of chloroform-methanol (50:1 v/v).
MS (FAB)*m*/*z*: 137 (M+1)⁺.
¹H-NMR (CDCl₃)δ: 1.37 (3H, t, *J=* 7.5 Hz), 2.86 (2H, q, *J=* 7.5 Hz), 3.88 (2H, s), 11.61 (1H, brs).

### [Reference Example 79]

### (5-Cyclopropyl-4H-[1,2,4]triazol-3-yl)-acetonitrile (I-79)

A methanol (100 ml) solution of 2.04 g (51.0 mmol) of sodium hydroxide was mixed with 6.78 g (50.0 mmol) of methyl cyclopropanimidate hydrochloride (I-57) and 5.46 g (51.5 mmol) of cyanoacetohydrazide and heated under reflux for 2.5 hours. After cooling, the reaction solution was concentrated under a reduced pressure, the thus obtained residue was applied to a silica gel column chromatography, and 3.69 g (53%) of the title compound was obtained as colorless crystals from an eluate of chloroform-methanol (50:1 v/v).
MS (FAB)*m*/*z*: 149 (M+1)⁺.
¹H-NMR (CDCl₃)δ: 1.04-1.15 (4H, m), 2.02 (1H, m), 3.83 (2H, s), 11.60 (1H, brs).

### [Reference Example 80]

### (5-Isopropyl-4H-[1,2,4]triazol-3-yl)-acetonitrile (I-80)

A methanol (100 ml) solution of 2.04 g (51.0 mmol) of sodium hydroxide was mixed with 6.88 g (50.0 mmol) of methyl isobutylimidate hydrochloride (I-58) and 5.46 g (51.5 mmol) of cyanoacetohydrazide and heated under reflux for 2.5 hours. After cooling, the reaction solution was concentrated under a reduced pressure, the thus obtained residue was applied to a silica gel column chromatography, and 5.85 g (76%) of the title compound was obtained as colorless crystals from an eluate of chloroform-methanol (75:1 v/v). MS (FAB)*m*/*z*: 151 (M+1)⁺.
¹H-NMR (CDCl₃)δ: 1.39 (6H, d, *J=* 6.9 Hz), 3.16 (1H, sep, *J=* 6.9 Hz), 3.89 (2H, s), 11.04 (1H, brs).

### [Reference Example 81]

### 3-n-Butyl-5-cyanomethyl-1H-[1,2,4]triazole (I-81)

A methanol (60 ml) solution of 1.40 g (33.3 mmol) of sodium hydroxide was mixed with 5.03 g (33.2 mmol) of methyl pentanimidate hydrochloride (I-59) and 3.39 g (34.2 mmol) of cyanoacetohydrazide and heated under reflux for 2 hours. After cooling, the reaction solution was concentrated under a reduced pressure, the thus obtained residue was mixed with chloroform, the insoluble material was removed by filtration, and the solvent of the filtrate was evaporated. The thus obtained residue was applied to a silica gel column chromatography, and 4.11 g (76%) of the title compound was obtained as a colorless solid from an eluate of chloroform-methanol (99:1 v/v).
MS (FAB)*m*/*z*: 165 (M+1)⁺.
¹H-NMR (CDCl₃)δ: 0.94 (3H, t, *J =* 7.4 Hz), 1.04 (2H, sex, *J =* 7.4 Hz), 1.69-1.80 (2H, m), 2.80 (2H, t, *J =* 7.8 Hz), 3.88 (2H, s), 11.6 (1H, brs).

### [Reference Example 82]

### (5-Isobutyl-4H-[1,2,4]triazol-3-yl)-acetonitrile (I-82)

A methanol (100 ml) solution of 2.04 g (51.0 mmol) of sodium hydroxide was mixed with 7.58 g (50.0 mmol) of methyl 3-methylbutylimidate hydrochloride (I-60) and 5.46 g (51.5 mmol) of cyanoacetohydrazide and heated under reflux for 2.5 hours. After cooling, the reaction solution was concentrated under a reduced pressure, the thus obtained residue was applied to a silica gel column chromatography, and 7.36 g (90%) of the title compound was obtained as colorless crystals from an eluate of chloroform-methanol (100: 1 v/v).
MS (FAB)*m*/*z*: 165 (M+1)⁺.
¹H-NMR (CDCl₃)δ: 0.89 (6H, d, *J*= 6.6 Hz), 2.11 (1H, m), 2.69 (2H, d, *J*= 7.5 Hz), 3.89 (2H, s), 11.65 (1H, brs).

### [Reference Example 83]

### 3-tert-Butyl-5-cyanomethyl-1H-[1,2,4]triazole (I-83)

A methanol (84 ml) solution of 1.95 g (46.3 mmol) of sodium hydroxide was mixed with 7.00 g (46.2 mmol) of methyl 2,2-dimethylpropionimidate hydrochloride (I-61) and 4.72 g (47.6 mmol) of cyanoacetohydrazide and heated under reflux for 2 hours. After cooling, the reaction solution was concentrated under a reduced pressure, the thus obtained residue was mixed with chloroform, the insoluble material was removed by filtration, and the solvent of the filtrate was evaporated. The thus obtained residue was applied to a silica gel column chromatography, and 4.74 g (63%) of the title compound was obtained as a colorless solid from an eluate of chloroform-methanol (99:1 v/v).
MS (FAB)*m*/*z*: 165 (M+1)⁺.
¹H-NMR (CDCl₃)δ: 1.42 (9H, s), 3.87 (2H, s), 11.0 (1H, brs).

### [Reference Example 84]

### [5-(2-Pyridyl)-2H-[1,2,4]triazol-3-yl]-acetonitrile (I-84)

A 6.69 g (30.0 mmol) portion of ethyl pyridine-2-imidate dihydrochloride (I-62) and 3.82 g (38.5 mmol) of cyanoacetohydrazide were added to a 0.554 N sodium hydroxide methanol solution (77 ml) and heated under reflux for 2 hours. After cooling, the reaction solution was concentrated under a reduced pressure, the thus obtained residue was mixed with water, and the insoluble material was collected by filtration to obtain the title compound as crude crystals. This was applied to a flash silica gel column chromatography, and 1.06 g (19%) of the title compound was obtained as colorless crystals from an eluate of chloroform-methanol (30:1 → 20:1 v/v).
MS (FAB)*m*/*z*: 186 (M+1)⁺.
¹H-NMR (DMSO-d₆)δ: 4.22 (2H, s), 7.48-7.60 (1H, m), 7.93-8.15 (2H, m), 8.66-8.75 (1H, m).

### [Reference Example 85]

### [5-(3-pyridyl)-2H-[1,2,4]triazol-3-yl]-acetonitrile (I-85)

A 6.69 g (30.0 mmol) portion of ethyl nicotinimidate dihydrochloride (I-63) and 3.82 g (38.5 mmol) of cyanoacetohydrazide were added to a 0.554 N sodium hydroxide methanol solution (77 ml) and heated under reflux for 2 hours. After cooling, the reaction solution was concentrated under a reduced pressure, the thus obtained residue was mixed with water, and the insoluble material was collected by filtration to obtain the title compound as crude crystals. This was applied to a flash silica gel column chromatography, and 2.24 g (40%) of the title compound was obtained as colorless crystals from an eluate of chloroform-methanol (30:1 v/v).
MS (FAB)*m*/*z*: 186 (M+1)⁺.
¹H-NMR (DMSO-d₆)δ: 4.29 (2H, s), 7.47-7.65 (1H, m), 8.25-8.40 (1H, m), 8.58-8.78 (1H, m), 9.10-9.26 (1H, m).

### [Reference Example 86]

### [5-(4-Pyridyl)-2H-[1,2,4]triazol-3-yl]-acetonitrile (I-86)

A 6.69 g (30.0 mmol) portion of ethyl isonicotinimidate dihydrochloride (I-64) and 3.82 g (38.5 mmol) of cyanoacetohydrazide were added to a 0.554 N sodium hydroxide methanol solution (77 ml) and heated under reflux for 2.5 hours. After cooling, the reaction solution was concentrated under a reduced pressure, the thus obtained residue was mixed with water, and the insoluble material was collected by filtration to obtain the title compound as crude crystals. This was applied to a flash silica gel column chromatography, and 1.86 g (34%) of the title compound was obtained as pale yellow crystals from an eluate of chloroform-methanol (30:1 → 20:1 v/v). MS (FAB)*m*/*z*: 186 (M+1)⁺.
¹H-NMR (DMSO-d₆)δ: 4.31 (2H, s), 7.86-7.93 (1H, m), 8.67-8.77 (2H, m).

### [Reference Example 87]

### 5-Cyanomethyl-3-methoxymethyl-1H-[1,2,4] triazole (I-87)

A 17.0 g (0.122 mol) portion of methyl 2-methoxyacetimidate hydrochloride (I-65) and 12.4 g (0.125 mol) of cyanoacetohydrazide were added to a methanol (220 ml) solution of 5.13 g (0.122 mol) of sodium hydroxide and heated under reflux for 2.5 hours. After cooling, the reaction solution was concentrated under a reduced pressure, the thus obtained residue was mixed with chloroform, and the insoluble material was removed by filtration. The solvent of the filtrate was evaporated, and the thus obtained residue was applied to a silica gel column chromatography to obtain 14.3 g (77%) of the title compound as a colorless solid from an eluate of chloroform-methanol (200:3 → 50:1 v/v). MS (FAB)*m*/*z:* 153 (M+1)⁺.
¹H-NMR (CDCl₃)δ: 3.52 (3H, s), 3.88 (2H, s), 4.67 (2H, s), 11.18 (1H, brs).

### [Reference Example 88]

### [5-(1-Hydroxy-1-methylethyl)-2H-[1,2,4]triazol-3-yl]-acetonitrile (I-88)

A 4.61 g (30.0 mmol) portion of methyl 2-hydroxy-2-methylpropionimidate hydrochloride (1-66) and 3.82 g (30.9 mmol) of cyanoacetohydrazide were added to a 0.554 N sodium hydroxide methanol solution (54.2 ml) and heated under reflux for 2.5 hours. After cooling, the reaction solution was concentrated under a reduced pressure, the thus obtained residue was mixed with water, and the insoluble material was collected by filtration to obtain the title compound as crude crystals. This was applied to a flash silica gel column chromatography, and 4.10 g (82%) of the title compound was obtained as a colorless solid from an eluate of chloroform-methanol (30:1 → 10:1 v/v).
MS (FAB)*m*/*z*: 167 (M+1)⁺.
¹H-NMR (CD₃OD)δ: 1.56 (6H, s), 3.96 (2H, s).

### [Reference Example 89]

### 3-(2-Benzyloxyethyl)-5-cyanomethyl-1H-[1,2,4]triazole (I-89)

A 10.0 g (43.5 mmol) portion of methyl 3-benzyloxypropionimidate hydrochloride (I-68) and 4.44 g (44.8 mmol) of cyanoacetohydrazide were added under ice-cooling to a methanol (80 ml) solution of 1.83 g (43.5 mmol) of sodium hydroxide and heated under reflux for 2 hours. After cooling, the reaction solution was concentrated under a reduced pressure, the thus obtained residue was applied to a silica gel column chromatography, and 9.39 g (89%) of the title compound was obtained as a colorless solid from an eluate of chloroform-methanol (1:0 → 100:1 → 50:1 v/v).
MS (FAB)*m*/*z*: 243 (M+1)⁺.
¹H-NMR (CDCl₃)δ: 3.08 (2H, t, *J*= 5.7 Hz), 3.79 (2H, t, *J=* 5.7 Hz), 3.81 (2H, s), 4.55 (2H, s), 7.26-7.40 (5H, m), 11.50 (1H, brs).

### [Reference Example 90]

### A mixture of 2-(5-cyanomethyl-4H-[1,2,4]triazol-3-yl)-2-methylpropionic acid ethyl ester and 2-(5-cyanomethyl-4H-[1,2,4]triazol-3-yl)-2-methylpropionic acid methyl ester (I-90)

A 8.0 g portion of the mixture of 2-ethoxycarbonyl-2-methylpropionic acid ethyl ester hydrochloride and 2-ethoxycarbonyl-2-methylpropionic acid methyl ester hydrochloride (I-71) and 3.96 g (40.0 mmol) of cyanoacetohydrazide were added to a methanol (75 ml) solution of 0.91 g (38.0 mmol) of sodium hydroxide and heated under reflux for 2.5 hours. After cooling, the insoluble material was removed by filtration, the filtrate was concentrated under a reduced pressure, and the thus obtained residue was applied to a silica gel column chromatography. A 1.32 g portion of the title compound was obtained as colorless crystals from an eluate of chloroform-methanol (75:1 v/v). This was directly used in the subsequent reaction.

### [Reference Example 91]

### [5-(2-Benzyloxy-1,1-dimethyl-ethyl)-4H-[1,2,4]triazolo-3-yl]-acetonitrile (I-91)

A 14.0 g (54.3 mmol) portion of methyl 3-benzyloxy-2,2-dimethylpropionimidate hydrochloride (I-74) and 5.90 g (59.0 mmol) of cyanoacetohydrazide were added to a methanol (114 ml) solution of 2.30 g (57.0 mmol) of sodium hydroxide and heated under reflux for 3 hours. After cooling, the insoluble material was removed by filtration, the filtrate was concentrated under a reduced pressure, the thus obtained residue was applied to a silica gel column chromatography, and 10.0 g (68%) of the title compound was obtained as colorless crystals from an eluate of chloroform-methanol (40:1 v/v).
MS (FAB)*m*/*z*: 271 (M+1)⁺.
¹H-NMR (CDCl₃)δ: 1.39 (6H, s), 3.53 (2H, s), 3.82 (2H, s), 4.59 (2H, s), 7.29-7.41 (5H, m), 11.23 (1H, brs).

### [Reference Example 92]

### {5-[2-(4-Fluorobenzyloxy)-1,1-dimethyl-ethyl]-4H-[1,2,4]triazolo-3-yl}-acetonitrile (I-92)

A 1.07 g (3.90 mmol) portion of methyl 3-(4-fluorophenyl)-2,2-dimethylpropionimidate hydrochloride (I-76) and 450 mg (4.50 mmol) of cyanoacetohydrazide were added to a methanol (10 ml) solution of 170 mg (4.30 mmol) of sodium hydroxide and heated under reflux for 3.5 hours. After cooling, the reaction solution was concentrated under a reduced pressure, and the thus obtained residue was dissolved in water and extracted with chloroform. The organic layer was washed with brine and dried over magnesium sulfate, and then the solvent was evaporated. The thus obtained residue was applied to a silica gel column chromatography, and 406 mg (36%) of the title compound was obtained as colorless crystals from an eluate of chloroform-methanol (50:1 v/v).
MS (FAB)*m*/*z*: 271 (M+1)⁺.
¹H-NMR (CDCl₃)δ: 1.38 (6H, s), 3.51 (2H, s), 3.83 (2H, s), 4.56 (2H, s), 7.06 (2H, m), 7.29 (2H, m), 11.11 (1H, brs).

### [Reference Example 93]

### 7-Methyl-5-oxo-2,6-diphenyl-1,5-dihydro-[1,2,4]triazolo[1,5-a]pyridine-8-carbonitrile (I-93)

A mixture of 1.00 g (5.43 mmol) of 5-cyanomethyl-3-phenyl-1H-[1,2,4]triazole (I-53), 1.18 g (5.72 mmol) of 2-phenylacetoacetic acid ethyl ester and 879 mg (11.4 mmol) of ammonium acetate was heated at 150°C for 3 hours. After cooling, water was added thereto, and the thus precipitated crystals were collected by filtration and further washed with acetonitrile. This was collected by filtration and dried to obtain 885 mg (50%) of the title compound as a colorless solid. MS (FAB)*m*/*z*: 327 (M+1)⁺.
¹H-NMR (DMSO-d₆)δ: 2.13 (3H, s), 7.13 (1H, brs), 7.24 (3H, m), 7.36 (2H, m), 7.46 (3H, m), 8.15 (2H, m).

### [Reference Example 94]

### 2,7-Dimethyl-5-oxo-6-phenyl-1,5-dihydro-[1,2,4]triazolo[1,5-a]pyridine-8-carbonitrile (I-94)

A mixture of 730 mg (6.00 mmol) of 5-cyanomethyl-3-methyl-1H-[1,2,4]triazole (I-77), 1.28 g (6.20 mmol) of 2-phenylacetoacetic acid ethyl ester and 960 mg (12.4 mmol) of ammonium acetate was heated at 150°C for 2.5 hours. After cooling, this was applied to a silica gel column chromatography and eluted with chloroform-methanol (5:1 v/v), the solvent was evaporated, the resulting residue was mixed with acetonitrile and then the thus precipitated crystals were collected by filtration and dried to obtain 183 mg (12%) of the title compound as colorless crystals.
MS (FAB)*m*/*z*: 265 (M+1)⁺.
¹H-NMR (DMSO-d₆)δ: 2.16 (3H, s), 2.51 (3H, s), 7.23 (2H, m), 7.38 (3H, m).

### [Reference Example 95]

### 2-Ethyl-7-methyl-5-oxo-6-phenyl-1.5-dihydro-[1,2,41triazolo[1,5-alpyridine-8-carbonitrile (1-95)

A mixture of 820 mg (6.00 mmol) of (5-ethyl-4H-[1,2,4]triazol-3-yl)-acetonitrile (I-78), 1.28 g (6.20 mmol) of 2-phenylacetoacetic acid ethyl ester and 960 mg (12.4 mmol) of ammonium acetate was heated at 150°C for 4.5 hours. After cooling, this was applied to a silica gel column chromatography and eluted with chloroform-methanol (25:1 v/v), the solvent was evaporated, the resulting residue was mixed with acetonitrile and then the thus precipitated crystals were collected by filtration and dried to obtain 350 mg (21%) of the title compound as pale yellow crystals.
MS (FAB)*m*/*z*: 279 (M+1)⁺.
¹H-NMR (DMSO-d₆)δ: 1.26 (3H, t, *J=* 7.5 Hz), 2.13 (3H, s), 2.76 (2H, q, *J=* 7.5 Hz), 7.20 (2H, m), 7.29 (1H, m), 7.39 (2H, m).

### [Reference Example 96]

### 2-Cyclopropyl-7-methyl-5-oxo-6-phenyl-1,5-dihydro-[1,2,4]triazolo[1,5-a]pyridine-8-carbonitrile (I-96)

A mixture of 890 mg (6.00 mmol) of (5-cyclopropyl-4H-[1,2,4]triazol-3-yl)-acetonitrile (I-79), 1.28 g (6.20 mmol) of 2-phenylacetoacetic acid ethyl ester and 960 mg (12.4 mmol) of ammonium acetate was heated at 150°C for 5 hours. After cooling, this was applied to a silica gel column chromatography and eluted with chloroform-methanol (25:1 v/v), the solvent was evaporated, the resulting residue was mixed with acetonitrile and then the thus precipitated crystals were collected by filtration and dried to obtain 366 mg (21 %) of the title compound as yellow crystals.
MS (FAB)ndz: 291 (M+1)⁺.
¹H-NMR (DMSO-d₆)δ: 1.01 1 (4H, m), 2.11 (3H, s), 2.17 (1H, m), 7.19 (2H, m), 7.29 (1 H, m), 7.38 (2H, m).

### [Reference Example 97]

### 7-Methyl-5-oxo-6-phenyl-2-i-propyl-1,5-dihydro-[1,2,4]triazolo[1,5-a]pyridine-8-carbonitrile (I-97)

A mixture of 900 mg (6.00 mmol) of (5-isopropyl-4H-[1,2,4]triazol-3-yl)-acetonitrile (I-80), 1.28 g (6.20 mmol) of 2-phenylacetoacetic acid ethyl ester and 960 mg (12.4 mmol) of ammonium acetate was heated at 150°C for 6 hours. After cooling, this was applied to a silica gel column chromatography and eluted with chloroform-methanol (25:1 v/v), the solvent was evaporated, the resulting residue was mixed with acetonitrile and then the thus precipitated crystals were collected by filtration and dried to obtain 780 mg (45%) of the title compound as pale yellow crystals.
MS (FAB)*m*/*z*: 293 (M+I)⁺.
¹H-NMR (DMSO-d₆)δ: 1.33 (6H, d, *J*= 6.9 Hz), 1.13 (3H, s), 3.15 (1H, sep, *J*= 6.9 Hz), 7.19 (2H, m), 7.28-7.42 (3H, m).

### [Reference Example 98]

### 2-n-Butyl-7-methyl-5-oxo-6-phenyl-1,5-dihydro-[1,2,4]triazolo[1,5-a]pyridine-8-carbonitrile (I-98)

A mixture of 1.00 g (6.09 mmol) of 3-n-butyl-5-cyanomethyl-1H-[1,2,4]triazole (I-81), 1.32 g (6.39 mmol) of 2-phenylacetoacetic acid ethyl ester and 986 mg (12.8 mmol) of ammonium acetate was heated at 150°C for 2.5 hours. After cooling, this was applied to a silica gel column chromatography and eluted with chloroform-methanol (98:2 v/v), the solvent was evaporated, the resulting residue was mixed with acetonitrile and then the thus precipitated crystals were collected by filtration and dried to obtain 311 mg (17%) of the title compound as a pale yellow solid (additional 148 mg (7.9%) was obtained from the mother liquor).
MS (FAB)*m*/*z*: 307 (M+1)⁺.
¹H-NMR (DMSO-d₆)δ: 0.92 (3H, t, *J=* 7.5 Hz), 1.36 (2H, sex, *J=* 7.5 Hz), 1.74 (2H, quint, *J=* 7.5 Hz), 2.14 (3H, s), 2.81 (2H, t, *J*= 7.5 Hz), 7.19-7.22 (2H, m), 7.30-7.43 (3H, m).

### [Reference Example 99]

### 2-i-Butyl-7-methyl-5-oxo-6-phenyl-1,5-dihydro-[1,2,4]triazolo[1,5-a]pyridine-8-carbonitrile (I-99)

A mixture of 990 mg (6.00 mmol) of (5-isobutyl-4H-[1,2,4]triazol-3-yl)-acetonitrile (I-82), 1.28 g (6.20 mmol) of 2-phenylacetoacetic acid ethyl ester and 960 mg (12.4 mmol) of ammonium acetate was heated at 150°C for 6 hours. After cooling, this was applied to a silica gel column chromatography and eluted with chloroform-methanol (98:2 v/v), the solvent was evaporated, the resulting residue was mixed with acetonitrile and then the thus precipitated crystals were collected by filtration and dried to obtain 363 mg (20%) of the title compound as a pale yellow solid.
MS (FAB)*m*/*z*: 307 (M+1)⁺.
¹H-NMR (DMSO-d₆)δ: 0.96 (6H, t, *J=* 6.6 Hz), 2.14 (3H, s), 2.16 (1H, m), 2.69 (2H, d, *J=* 7.2 Hz), 7.19-7.29 (2H, m), 7.30-7.43 (3H, m).

### [Reference Example 100]

### 2-tert-Butyl-7-methyl-5-oxo-6-phenyl-1,5-dihydro-[1,2,4]triazolo[1,5-a]pyridine-8-carbonitrile (I-100)

A mixture of 1.00 g (6.09 mmol) of 3-tert-butyl-5-cyanomethyl-1H-[1,2,4]triazole (I-83), 1.32 g (6.39 mmol) of 2-phenylacetoacetic acid ethyl ester and 986 mg (12.8 mmol) of ammonium acetate was heated at 150°C for 3.5 hours. After cooling, this was applied to a silica gel column chromatography and eluted with chloroform-methanol (100:1 → 99:1 → 95:5 v/v), the solvent was evaporated, the resulting residue was mixed with acetonitrile and then the thus precipitated crystals were collected by filtration and dried to obtain 621 mg (33%) of the title compound as a pale yellow solid.
MS (FAB)*m*/*z*: 307 (M+1)⁺.
¹H-NMR (CDCl₃)δ: 1.38 (9H, s), 2.24 (3H, s), 7.26-7.43 (5H, m).

### [Reference Example 101]

### 7-Methyl-5-oxo-6-phenyl-2-(3-pyridyl)-1,5-dihydro-[1,2,4]triazolo[1,5-a]pyridine-8-carbonitrile (I-101)

A mixture of 1.00 g (5.40 mmol) of [5-(3-pyridyl)-2H-[1,2,4]triazol-3-yl]-acetonitrile (I-85), 1.23 g (5.96 mmol) of 2-phenylacetoacetic acid ethyl ester and 866 mg (11.2 mmol) of ammonium acetate was heated at 150°C for 2 hours. After cooling, water was added thereto, the resulting solid material was pulverized and then subjected to decantation, and the solid was subsequently washed with acetonitrile and ethanol. The thus obtained solid was collected by filtration and dried to obtain 883 mg (50%) of the title compound as a colorless solid.
MS (FAB)*m*/*z*: 328 (M+1)⁺.
¹H-NMR (DMSO-d₆)δ: 2.14 (3H, s), 7.20-7.42 (5H, m), 7.47-7.55 (1H, m), 8.43-8.49 (1H, m), 8.61-8.67 (1H, s), 9.28-9.32 (1H, m).

### [Reference Example 102]

### 7-Methyl-5-oxo-6-phenyl-2-(4-pyridyl)-1,5-dihydro-[1,2,4]triazolo[1,5-a]pyridine-8-carbonitrile (I-102)

A mixture of 800 mg (4.32 mmol) of [5-(4-pyridyl)-2H-[1,2,4]triazol-3-yl]-acetonitrile (I-86), 927 mg (4.49 mmol) of 2-phenylacetoacetic acid ethyl ester and 693 mg (8.99 mmol) of ammonium acetate was heated at 150°C for 3 hours. After cooling, water was added thereto, the resulting solid material was pulverized and then subjected to decantation, and the solid was subsequently washed with acetonitrile and ethanol. The thus obtained solid was collected by filtration and dried to obtain 971 mg (69%) of the title compound as a yellow solid.
MS (FAB)*m*/*z*: 328 (M+1)⁺.
¹H-NMR (DMSO-d₆)δ: 2.14 (3H, s), 7.10-7.43 (5H, m), 8.03-8.12 (2H, m), 8.15-8.64 (2H, m).

### [Reference Example 103]

### 2-Methoxymethyl-7-methyl-5-oxo-6-phenyl-1,5-dihydro-[1,2,4]triazolo[1,5-a]pyridine-8-carbonitrile (I-103)

A mixture of 7.00 g (46.0 mmol) of 5-cyanomethyl-3-methoxymethyl-1H-[1,2,4]triazole (I-87), 9.96 g (48.3 mmol) of 2-phenylacetoacetic acid ethyl ester and 7.45 g (96.6 mmol) of ammonium acetate was heated at 150°C for 4 hours. After cooling, this was mixed with water and then subjected to toluene azeotrope, and the precipitated solid was washed with chloroform. The thus obtained solid was collected by filtration and dried to obtain 4.80 g (35%) of the title compound as a pale yellow solid.
MS (FAB)*m*/*z*: 295 (M+1)⁺.
¹H-NMR (DMSO-d₆)δ: 2.22(3H, s), 3.43 (3H, s), 4.59 (2H, s), 7.23-7.43 (5H, m).

### [Reference Example 104]

### 2-(1-Hydroxy-1-methylethyl)-7-methyl-5-oxo-6-phenyl-1,5-dihydro[1,2,4]triazolo[1,5-a]piridine-8-carbonitrile (I-104)

A mixture of 2.00 g (12.0 mmol) of [5-(1-hydroxy-1-methylethyl)-2H-[1,2,4]triazol-3-yl]-acetonitrile (I-88), 2.72 g (13.2 mmol) of 2-phenylacetoacetic acid ethyl ester and 2.07 g (26.5 mmol) of ammonium acetate was heated at 150°C for 3.5 hours. After cooling, this was mixed with water, acidified with 1 N hydrochloric acid and then extracted with ethyl acetate. The organic layer was washed with brine and water and dried over magnesium sulfate. This was concentrated, and the thus precipitated solid was collected by filtration and dried to obtain 389 mg (11%) of the title compound as a colorless solid.
MS (FAB)*m*/*z*: 309 (M+1)⁺.
¹H-NMR (DMSO-d₆)δ: 1.59 (6H, s), 2.14 (3H, s), 7.16-7.24 (2H, m), 7.28-7.46 (3H, m).

### [Reference Example 105]

### 2-(2-Benzyloxyethyl)-7-methyl-5-oxo-6-phenyl-1,5-dihydro-[1,2,4]triazolo[1,5-a]pyridine-8-carbonitrile (I-105)

A mixture of 5.00 g (20.6 mmol) of 3-(2-benzyloxyethyl)-5-cyanomethyl-1H-[1,2,4]triazole (I-89), 4.47 g (21.7 mmol) of 2-phenylacetoacetic acid ethyl ester and 3.34 g (43.3 mmol) of ammonium acetate was heated at 150°C for 4.5 hours. After cooling, this was applied to a silica gel column chromatography and eluted with chloroform-methanol (100:0 → 50:1 → 25:1 v/v), the solvent was evaporated, the resulting residue was mixed with acetonitrile, and the thus precipitated crystals were collected by filtration and dried to obtain 2.04 g (26%) of the title compound as a pale ocherous solid.
MS (FAB)*m*/*z*: 385 (M+1)⁺.
¹H-NMR (DMSO-d₆)δ: 2.23 (3H, s), 3.05 (2H, t, *J*= 6.5 Hz), 3.81 (2H, t, *J*= 6.5 Hz), 4.48 (2H, s), 7.20-7.45(10H, m).

### [Reference Example 106]

### A mixture of 2-(8-cyano-7-methyl-5-oxo-6-phenyl-1,5-dihydro-[1,2,4]triazolo[1,5-a]pyridin-2-yl)-2-methylpropionic acid ethyl ester and 2-(8-cyano-7-methyl-5-oxo-6-phenyl-1,5-dihydro-[1,2,4]triazolo[1,5-a]pyridin-2-yl)-2-methylpropionic acid methyl ester (I-106)

A mixture of 1.07 g (5.00 mmol) of the mixture of 2-(5-cyanomethyl-4H-[1,2,4]triazol-3-yl)-2-methylpropionic acid ethyl ester and 2-(5-cyanomethyl-4H-[1,2,4]triazol-3-yl)-2-methylpropionic acid methyl ester (I-90), 1.07 g (5.20 mmol) of 2-phenylacetoacetic acid ethyl ester and 800 mg (10.4 mmol) of ammonium acetate was heated at 150°C for 4 hours. After cooling, this was applied to a silica gel column chromatography and eluted with chloroform-methanol (50:1 v/v), the solvent was evaporated, the resulting residue was mixed with acetonitrile, and the thus precipitated crystals were collected by filtration and dried to obtain 202 mg of the title compound as colorless crystals. This was directly used in the subsequent reaction.

### [Reference Example 107]

### 2-(2-Benzyloxy-1,1-dimethylethyl)-7-methyl-5-oxo-6-phenyl-1,5-dihydro-[1,2,4]triazolo[1,5-a]pyridine-8-carbonitrile (I-107)

A mixture of 5.00 g (18.5 mmol) of [5-(2-benzyloxy-1,1-dimethyl-ethyl)-4H-[1,2,4]triazolo-3-yl]-acetonitrile (I-91), 3.90 g (18.8 mmol) of 2-phenylacetoacetic acid ethyl ester and 2.90 g (37.6 mmol) of ammonium acetate was heated at 150°C for 6.5 hours. After cooling, this was applied to a silica gel column chromatography and eluted with chloroform-methanol (50:1 v/v), the solvent was evaporated, the resulting residue was mixed with acetonitrile, and the thus precipitated crystals were collected by filtration and dried to obtain 2.00 g (26%) of the title compound as pale yellow crystals.
MS (FAB)*m*/*z*: 413 (M+1)⁺.
¹H-NMR (CDCl₃)δ: 1.35 (6H, s), 2.21 (3H, s), 3.56 (2H, s), 4.42 (2H, s), 7.18-7.42 (10H, m).

### [Reference Example 108]

### 2-[2-(4-Fluorobenzyloxy)-1,1-dimethylethyl]-7-methyl-5-oxo-6-phenyl-1,5-dihydro-[1,2,4]triazolo[1,5-a]pyridine-8-carbonitrile (I-108)

A mixture of 380 mg (1.30 mmol) of {5-[2-(4-fluorobenzyloxy)-1,1-dimethyl-ethyl]-4H-[1,2,4]triazolo-3-yl}-acetonitrile (I-92), 290 mg (1.40 mmol) of 2-phenylacetoacetic acid ethyl ester and 220 mg (2.80 mmol) of ammonium acetate was heated at 150°C for 6 hours. After cooling, this was applied to a silica gel column chromatography and eluted with chloroform-methanol (50:1 v/v) to obtain 369 mg of crude product of the title compound as a yellow oily substance.
MS (FAB)*m*/*z:* 431 (M+1)⁺.

### [Reference Example 109]

### 5-Chloro-7-methyl-2,6-diphenyl[1,2,4]triazolo[1,5-a]pyridine-8-carbonitrile (I-109)

An 813 mg (2.49 mmol) portion of 7-methyl-5-oxo-2,6-diphenyl-1,5-dihydro-[1,2,4]triazolo[1,5-a]pyridine-8-carbonitrile (I-93) was heated under reflux for 2.5 hours in phosphoryl chloride (5 ml). After cooling, phosphoryl chloride was evaporated under a reduced pressure, and the thus obtained residue was mixed with ice water and extracted with chloroform. The organic layer was washed with brine and dried over magnesium sulfate, and the solvent was evaporated, thereby obtaining 815 mg (95%) of the title compound as a colorless solid.
MS (FAB)*m*/*z*: 345 (M+1)⁺.
¹H-NMR (CDCl₃)δ: 2.45 (3H, s), 7.26 (2H, m), 7.54 (6H, m), 8.39 (2H, m).

### [Reference Example 110]

### 5-Chloro-2,7-dimethyl-6-phenyl[1,2,4]triazolo[1,5-a]pyridine-8-carbonitrile (I-110)

A 120 mg (0.45 mmol) portion of 2,7-dimethyl-5-oxo-6-phenyl-1,5-dihydro-[1,2,4]triazolo[1,5-a]pyridine-8-carbonitrile (I-94) was heated under reflux for 2 hours in phosphoryl chloride (5 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated, thereby obtaining 120 mg (93%) of the title compound as a colorless solid.
MS (FAB)*m*/*z*: 283 (M+1)⁺.
¹H-NMR (CDCl₃)δ: 2.43 (3H, s), 2.71 (3H, s), 7.24 (2H, m), 7.55 (3H, m).

### [Reference Example 111]

### 5-Chloro-2-ethyl-7-methyl-6-phenyl[1,2,4]triazolo[1,5-a]pyridine-8-carbonitrile (I-111)

A 250 mg (0.90 mmol) portion of 2-ethyl-7-methyl-5-oxo-6-phenyl-1,5-dihydro-[1,2,4]triazolo[1,5-a]pyridine-8-carbonitrile (I-95) was heated under reflux for 2 hours in phosphoryl chloride (5 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated, thereby obtaining 257 mg (96%) of the title compound as a pale yellow solid. MS (FAB)*m*/*z*: 297 (M+1)⁺.
¹H-NMR (CDCl₃)δ: 1.46 (3H, t, *J*= 7.5 Hz), 2.43 (3H, s), 3.05 (2H, q, *J*= 7.5 Hz), 7.23 (2H, m), 7.49-7.58 (3H, m).

### [Reference Example 112]

### 5-Chloro-2-cyclopropyl-7-methyl-6-phenyl[1,2,4]triazolo[1,5-a]pyridine-8-carbonitrile (I-112)

A 250 mg (0.86 mmol) portion of 2-cyclopropyl-7-methyl-5-oxo-6-phenyl-1,5-dihydro-[1,2,4]triazolo[1,5-a]pyridine-8-carbonitrile (I-96) was heated under reflux for 3 hours in phosphoryl chloride (5 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated, thereby obtaining 205 mg (77%) of the title compound as pale brown crystals. MS (FAB)*m*/*z*: 309 (M+1)⁺.
¹H-NMR (CDCl₃)δ: 1.12-1.19 (2H, m), 1.24-1.29 (2H, m), 2.31 (1H, m), 2.41 (3H, s), 7.20-7.26 (2H, m), 7.48-7.58 (3H, m).

### [Reference Example 113]

### 5-Chloro-7-methyl-6-phenyl-2-i-propyl-[1,2.4]triazolo[1,5-a]pyridine-8-carbonitrile (I-113)

A 500 mg (1.70 mmol) portion of 7-methyl-5-oxo-6-phenyl-2-i-propyl-1,5-dihydro-[1,2,4]triazolo[1,5-a]pyridine-8-carbonitrile (1-97) was heated under reflux for 1 hour in phosphoryl chloride (5 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated, thereby obtaining 478 mg (91 %) of the title compound as pale yellow crystals. MS (FAB)*m*/*z*: 311 (M+1)⁺.
¹H-NMR (CDCl₃)δ: 1.48 (6H, d, *J=* 6.9 Hz), 2.42 (3H, s), 3.37 (1H, sep, *J=* 6.9 Hz), 7.23 (2H, m), 7.48-7.59 (3H, m).

### [Reference Example 114]

### 2-n-Butyl-5-chloro-7-methyl-6-phenyl-[1,2,4]triazolo[1,5-a]pyridine-8-carbonitrile (I-114)

A 300 mg (0.98 mmol) portion of 2-n-butyl-7-methyl-5-oxo-6-phenyl-1,5-dihydro-[1,2,4]triazolo[1,5-a]pyridine-8-carbonitrile (I-98) was heated under reflux for 1.5 hours in phosphoryl chloride (2 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated, thereby obtaining 319 mg (100%) of the title compound as a pale yellow solid. MS (FAB)*m*/*z:* 325 (M+1)⁺.
¹H-NMR (CDCl₃)δ: 0.97 (3H, t, *J*= 7.4 Hz), 1.46 (2H, sex, *J*= 7.4 Hz), 1.83-1.93 (2H, m), 2.43 (3H, s), 3.01 (2H, t, *J*= 7.8 Hz), 7.22-7.26 (2H, m), 7.50-7.58 (3H, m).

### [Reference Example 115]

### 2-i-Butyl-5-chloro-7-methyl-6-phenyl-[1,2,4]triazolo[1,5-a]pyridine-8-carbonitrile (I-115)

A 250 mg (0.82 mmol) portion of 2-i-butyl-7-methyl-5-oxo-6-phenyl-1,5-dihydro-[1,2,4]triazolo[1,5-a]pyridine-8-carbonitrile (I-99) was heated under reflux for 3 hours in phosphoryl chloride (5 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated, thereby obtaining 271 mg (100%) of the title compound as pale yellow crystals. MS (FAB)*m*/*z*: 325 (M+1)⁺.
¹H-NMR (CDCl₃)δ: 1.03 (6H, d, *J=* 6.9 Hz), 2.33 (3H, s), 2.89 (2H, d, *J*= 7.2 Hz), 7.26 (2H, m), 7.54 (3H, m).

### [Reference Example 116]

### 2-tert-Butyl-5-chloro-7-methyl-6-phenyl-[1,2,4]triazolo[1,5-a]pyridine-8-carbonitrile (I-116)

A 676 mg (2.21 mmol) portion of 2-tert-butyl-7-methyl-5-oxo-6-phenyl-1,5-dihydro-[1,2,4]triazolo[1,5-a]pyridine-8-carbonitrile (I-100) was heated under reflux for 2.5 hours in phosphoryl chloride (4 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated, thereby obtaining 716 mg (100%) of the title compound as pale yellow crystals. MS (FAB)*m*/*z*: 325 (M+1)⁺.
¹H-NMR (CDCl₃)δ:1.52 (9H, s), 2.41 (3H, s), 7.20-7.24 (2H, m), 7.50-7.57 (3H, m).

### [Reference Example 117]

### 5-Chloro-7-methyl-6-phenyl-2-(3-pyridyl)-[1,2,4]triazolo[1,5-a]pyridine-8-carbonitrile (I-117)

A 750 mg (2.29 mmol) portion of 7-methyl-5-oxo-6-phenyl-2-(3-pyridyl)-1,5-dihydro-[1,2,4]triazolo[1,5-a]pyridine-8-carbonitrile (I-101) was heated under reflux for 2.5 hours in phosphoryl chloride (5 ml) (in the course of the reaction, phosphoryl chloride (5 ml) was supplemented). After cooling, phosphoryl chloride was evaporated under a reduced pressure, and the thus obtained residue was adjusted to pH 7 by adding saturated sodium bicarbonate aqueous solution and then extracted with chloroform. The organic layer was washed with brine and dried over magnesium sulfate, and then the solvent was evaporated, thereby obtaining 599 mg (76%) of the title compound as a yellow solid.
MS (FAB)*m*/*z*: 346 (M+1)⁺.
¹H-NMR (CDCl₃)δ: 2.40 (3H, s), 7.16-7.24 (2H, m), 7.35-7.43 (1H, m), 7.45-7.56 (3H, m), 8.55-8.61 (1H, m), 8.66-8.70 (1H, m), 9.49-9.53 (1H, m).

### [Reference Example 118]

### 5-Chloro-7-methyl-6-phenyl-2-(4-pyridyl)-[1,2,4]triazolo[1,5-a]pyridine-8-carbonitrile (I-118)

A 900 mg (2.75 mmol) portion of 7-methyl-5-oxo-6-phenyl-2-(4-pyridyl)-1,5-dihydro-[1,2,4]triazolo[1,5-a]pyridine-8-carbonitrile (I-102) was heated under reflux for 3.5 hours in phosphoryl chloride (10 ml). After cooling, phosphoryl chloride was evaporated under a reduced pressure, and the thus obtained residue was adjusted to pH 8 by adding saturated sodium bicarbonate aqueous solution, and extracted with chloroform. The organic layer was washed with brine and dried over magnesium sulfate, and then the solvent was evaporated, thereby obtaining 1.00 g of a crude title compound. This was directly used in the subsequent reaction.
MS (FAB)*m*/*z*: 346 (M+1)⁺.

### [Reference Example 119]

### 5-Chloro-2-methoxymethyl-7-methyl-6-phenyl-[1,2,4]triazolo[1,5-a]pyridine-8-carbonitrile (I-119)

A 4.98 g (16.9 mmol) portion of 2-methoxymethyl-7-methyl-5-oxo-6-phenyl-1,5-dihydro-[1,2,4]triazolo[1,5-a]pyridine-8-carbonitrile (I-103) was heated under reflux for 2 hours in phosphoryl chloride (50 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated, thereby obtaining 4.87 g (92%) of the title compound as a pale yellow solid.
MS (FAB)*m*/*z*: 313 (M+1)⁺.
¹H-NMR (CDCl₃)δ: 2.45 (3H, s), 3.57 (3H, s), 4.83 (2H, s), 7.22-7.30 (2H, m), 7.49-7.60 (3H, m).

### [Reference Example 120]

### 5-Chloro-7-methyl-6-phenyl-2-i-propenyl-[1,2,4]triazolo[1,5-a]pyridine-8-carbonitrile (I-120)

A 230 mg (0.75 mmol) portion of 2-(1-hydroxy-1-methylethyl)-7-methyl-5-oxo-6-phenyl-1,5-dihydro-[1,2,4]triazolo[1,5-a]pyridine-8-carbonitrile (I-104) was heated under reflux for 2 hours in phosphoryl chloride (4 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated, thereby obtaining 224 mg (97%) of the title compound as a pale brown solid.
MS (FAB)*m*/*z*: 309 (M+1)⁺.
¹H-NMR (CDCl₃)δ: 2.32 (3H, s), 2.43 (3H, s), 5.56 (1H, m), 6.44 (1H, s), 7.21-7.30 (2H, m), 7.53-7.62 (3H, m).

### [Reference Examples 121 and 122]

### 2-(2-Benzyloxyethyl)-5-chloro-7-methyl-6-phenyl-[1,2,4]triazolo[1,5-a]pyridine-8-carbonitrile (I-121) and 5-chloro-2-(2-chloroethyl)-7-methyl-6-phenyl-[1,2,4]triazolo[1,5-a]pyridine-8-carbonitrile (I-122)

A 1.98 g (5.15 mmol) portion of 2-(2-benzyloxyethyl)-7-methyl-5-oxo-6-phenyl-1,5-dihydro-[1,2,4]triazolo[1,5-a]pyridine-8-carbonitrile (I-105) was heated under reflux for 2 hours in phosphoryl chloride (10 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated. The thus obtained residue was applied to a silica gel column chromatography and eluted with n-hexane-ethyl acetate (9:2 v/v) to obtain 98 mg (6%: I-122) and 1.19 g (57%: I-121) of the title compounds as pale yellow solids, respectively.
I-121:
MS (FAB)*m*/*z*: 403 (M+1)⁺.
¹H-NMR (CDCl₃)δ: 2.42 (3H, s), 3.33 (2H, t, *J=* 6.8 Hz), 4.03 (2H, t, *J=* 6.8 Hz), 4.59 (2H, s), 7.21-7.37 (7H, m), 7.49-7.58 (3H, m).
I-122:
MS (FAB)*m*/*z*: 331 (M+1)⁺.
¹H-NMR (CDCl₃)δ: 2.44 (3H, s), 3.48 (2H, t, *J=* 6.9 Hz), 4.06 (2H, t, *J=* 6.9 Hz), 7.21-7.27 (2H, m), 7.50-7.58 (3H, m).

### [Reference Example 123]

### 2-[5-Chloro-8-cyano-7-methyl-6-phenyl-[1,2,4]triazolo[1,5-a]pyridin-2-yl]-2-methylpropionic acid methyl ester (I-123)

A 117 mg (0.50 mmol) portion of the mixture of 2-(8-cyano-7-methyl-5-oxo-6-phenyl-1,5-dihydro-[1,2,4]triazolo[1,5-a]pyridin-2-yl)-2-methylpropionic acid ethyl ester and 2-(8-cyano-7-methyl-5-oxo-6-phenyl-1,5-dihydro-[1,2,4]triazolo[1,5-a]pyridin-2-yl)-2-methylpropionic acid methyl ester (I-106) was heated under reflux for 3 hours in phosphoryl chloride (5 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated. The thus obtained residue was applied to a silica gel column chromatography and eluted with n-hexane-ethyl acetate (9:1 v/v) to obtain 87 mg of the title compound as colorless crystals (91 mg of a mixture of the title compound and ethyl ester thereof (I-123-1) was also obtained).
MS (FAB)*m*/*z:* 369 (M+1)⁺.
¹H-NMR (CDCl₃)δ: 1.78 (6H, s), 2.42 (3H, s), 3.72 (3H, s), 7.21-7.24 (2H, m), 7.51-7.57 (3H, m).

### [Reference Example 124]

### 2-(2-Benzyloxy-1,1-dimethylethyl)-5-chloro-7-methyl-6-phenyl-[1,2,4]triazolo[1,5-a]pyridine-8-carbonitrile (1-124)

A 1.80 g (4.40 mmol) portion of 2-(2-benzyloxy-1,1-dimethylethyl)-7-methyl-5-oxo-6-phenyl-1,5-dihydro-[1,2,4]triazolo[1,5-a]pyridine-8-carbonitrile (I-107) was heated under reflux for 2.5 hours in phosphoryl chloride (10 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated. The thus obtained residue was applied to a silica gel column chromatography and eluted with n-hexane-ethyl acetate (4:1 v/v) to obtain 1.76 g (93%) of the title compound as colorless crystals.
MS (FAB)*m*/*z*: 431 (M+1)⁺.
¹H-NMR (CDCl₃)δ: 1.53 (6H, s), 2.41 (3H, s), 3.80 (2H, s), 4.56 (2H, s), 7.20-7.32 (7H, m), 7.50-7.56 (3H, m).

### [Reference Example 125]

### 5-Chloro-2-[2-(4-fluorobenzyloxy)-1,1-dimethylethyl]-7-methyl-6-phenyl-[1,2,4]triazolo[1,5-a]pyridine-8-carbonitrile (I-125)

A 369 mg portion of 2-[2-(4-fluorobenzyloxy)-1,1-dimethylethyl]-7-methyl-5-oxo-6-phenyl-1,5-dihydro-[1,2,4]triazolo[1,5-a]pyridine-8-carbonitrile (I-108) was heated under reflux for 2 hours in phosphoryl chloride (10 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated. The thus obtained residue was applied to a silica gel column chromatography and eluted with n-hexane-ethyl acetate (10:1 v/v) to obtain 216 mg (37%, yield from I-92) of the title compound as pale yellow crystals.
MS (FAB)*m*/*z:* 449 (M+1)⁺.
¹H-NMR (CDCl₃)δ: 1.52 (6H, s), 2.41 (3H, s), 3.79 (2H, s), 4.51 (2H, s), 6.94-7.00 (2H, m), 7.21-7.29 (4H, m), 7.50-7.57 (3H, m).

### [Example 18]

### 7-Methyl-5-[(3S)-dimethylaminopyrrolidin-1-yl]-2,6-diphenyl[1,2,4]triazolo[1,5-a]pyridine-8-carbonitrile (#18)

A 202 µl (1.60 mmol) portion of (3S)-dimethylaminopyrrolidine and 404 µl (2.90 mmol) of triethylamine were added to an N,N-dimethylformamide (5 ml) solution of 500 mg (1.45 mmol) of 5-chloro-7-methyl-2,6-diphenyl[1,2,4]triazolo[1,5-a]pyridine-8-carbonitrile (I-109) and stirred at 80 to 90°C for 4 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 559 mg (91%) of the title compound was obtained as a pale yellow solid from an eluate of chloroform-methanol (99:1 v/v).
MS (FAB)*m*/*z*: 423 (M+1)⁺.
¹H-NMR (CDCl₃)δ: 1.60-1.74 (1H, m), 1.95-2.08 (1H, m), 2.18 (6H, s), 2.23 (4H, s), 2.59-2.70 (1H, m), 3.31 (1H, t, *J*= 9.3 Hz), 3.40 (1H, m), 3.52 (1H, dd, *J*= 6.9, 9.9 Hz), 3.61-3.70 (1H, m), 7.14-7.19 (1H, m), 7.26-7.30 (1H, m), 7.40-7.53 (6H, m), 8.30-8.37 (2H, m).
IR (KBr): 2962, 2766, 2218, 1610, 1508, 1442 cm⁻¹.
Elemental analysis values: as C₂₆H₂₆N₆·0.25H₂O
Calcd.: C, 73.13%; H, 6.25%; N, 19.68%
Found: C, 72.99%; H, 6.11%; N, 19.74%

### [Example 19]

### 2,7-Dimethyl-5-[(3S)-dimethylaminopyrrolidin-1-yl]-6-phenyl[1,2,4]triazolo[1,5-a]pyridine-8-carbonitrile (#19)

A 51 µl (0.40 mmol) portion of (3S)-dimethylaminopyrrolidine and 112 µl (0.80 mmol) of triethylamine were added to an N,N-dimethylformamide (5 ml) solution of 94 mg (0.33 mmol) of 5-chloro-2,7-dimethyl-6-phenyl[1,2,4]triazolo[1,5-a]pyridine-8-carbonitrile (I-110) and stirred at 80 to 90°C for 5.5 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 49 mg (41%) of the title compound was obtained as pale yellow crystals from an eluate of chloroform-methanol (100:1 v/v).
MS (FAB)*m*/*z*: 361 (M+1)⁺.
¹H-NMR (CDCl₃)δ: 1.63 (1H, quint, *J=* 9.9 Hz), 1.74 (1H, brs), 1.98 (1H, m), 2.14 (6H, s), 2.26 (3H, s), 2.60 (3H, s), 3.16 (1H, t, *J=* 9.6 Hz), 3.31 (1H, dt, *J=* 2.4, 9.3 Hz), 3.42 (1H, dd, *J =* 6.9, 9.9 Hz), 3.62 (1H, dd, *J=* 7.2, 10.2 Hz), 7.13 (1H, d, *J=* 6.6 Hz), 7.25 (1H, m), 7.44 (3H, m). IR(KBr): 2768, 2216, 1607, 1541, 1510, 1485, 1351 cm⁻¹.
Elemental analysis values: as C₂₁H₂₄N₆
Calcd.: C, 69.97%; H, 6.71%; N, 23.32%
Found: C, 69.70%; H, 6.73%; N, 23.02%

### [Example 20]

### 2-Ethyl-7-methyl-5-[(3S)-dimethylaminopyrrolidin-1-yl]-6-phenyl[1,2,4]triazolo[1,5-a]pyridine-8-carbonitrile (#20)

A compound capable of expressing an antifungal activity based on the functional mechanism of 1,6-β-glucan synthesis inhibition, with a broad spectrum and specifically or selectively, is provided, and an antifungal agent which comprises such a compound, a salt thereof or a solvate thereof is provided.
A 120 µl (0.93 mmol) portion of (3S)-dimethylaminopyrrolidine and 240 µl (1.70 mmol) of triethylamine were added to an N,N-dimethylformamide (5 ml) solution of 180 mg (0.61 mmol) of 5-chloro-2-ethyl-7-methyl-6-phenyl[1,2,4]triazolo[1,5-a]pyridine-8-carbonitrile (I-111) and stirred at 80 to 90°C for 6 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 224 mg (98%) of the title compound was obtained as pale yellow crystals from an eluate of chloroform-methanol (50:1 v/v). MS (FAB)*m*/*z*: 375 (M+1)⁺.
¹H-NMR (CDCl₃)δ: 1.42 (3H, t, *J* = 7.5 Hz), 1.63 (1H, m), 1.98 (1H, m), 2.15 (6H, s), 2.26 (3H, s), 2.61 (1H, m), 2.95 (2H, q, *J* = 7.5 Hz), 3.21 (1H, dd, *J* = 8.7, 9.9 Hz), 3.32 (1H, m), 3.45 (1H, dd, *J=* 7.2, 9.6 Hz), 3.60 (1H, dt, *J=* 6.9, 10.2 Hz), 7.13 (1H, m), 7.24 (1H, m), 7.34-7.49 (3H, m). IR (KBr): 2770, 2211, 1603, 1541, 1508, 1480, 1358, 1268 cm⁻¹.
Elemental analysis values: as C₂₂H₂₆N₆·0.25H₂O
Calcd.: C, 69.72%; H, 7.05%; N, 22.17%
Found: C, 70.15%; H, 6.99%; N, 21.97%

### [Example 21]

### 2-Cyclopropyl-7-methyl-5-[(3S)-dimethylaminopyrrolidin-1-yl]-6-phenyl[1,2,4]triazolo[1,5-a]pyridine-8-carbonitrile (#21)

A 100 µl (0.79 mmol) portion of (3S)-dimethylaminopyrrolidine and 200 µl (1.40 mmol) of triethylamine were added to an N,N-dimethylformamide (5 ml) solution of 150 mg (0.49 mmol) of 5-chloro-2-cyclopropyl-7-methyl-6-phenyl[1,2,4]triazolo[1,5-a]pyridine-8-carbonitrile (I-1112) and stirred at 80 to 90°C for 6 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 188 mg (98%) of the title compound was obtained as pale yellow crystals from an eluate of chloroform-methanol (50:1 v/v). MS (FAB)*m*/*z*: 387 (M+1)⁺.
¹H-NMR (CDCl₃)δ: 1.03-1.09 (2H, m), 1.14-1.19 (2H, m), 1.58-1.68 (1H, m), 1.96 (1H, m), 2.14 (6H, s), 2.22 (1H, m), 2.24 (3H, s), 2.60 (1H, m), 3.17 (1H, dd, *J=* 8.7, 9.9 Hz), 3.29 (1H, m), 3.42 (1H, dd, *J*= 7.2, 9.9 Hz), 3.57 (1H, dt, *J =* 6.9, 10.2 Hz), 7.12 (1H, m), 7.24 (1H, m), 7.38-7.49 (3H, m).
IR (KBr): 2771, 2210, 1606, 1542, 1508, 1477, 1360, 1270 cm⁻¹.
Elemental analysis values: as C₂₃H₂₆N₆·0.5H₂O
Calcd.: C, 69.85%; H, 6.88%; N, 21.25%
Found: C, 69.92%; H, 6.68%; N, 21.19%

### [Example 22]

### 7-Methyl-5-[(3S)-dimethylaminopyrrolidin-1-yl]-6-phenyl-2-i-propyl[1,2,4]triazolo[1,5-a]pyridine-8-carbonitrile (#22)

A 190 µl (1.50 mmol) portion of (3S)-dimethylaminopyrrolidine and 420 µl (1.50 mmol) of triethylamine were added to an N,N-dimethylformamide (5 ml) solution of 311 mg (1.00 mmol) of 5-chloro-7-methyl-6-phenyl-2-i-propyl-[1,2,4]triazolo[1,5-a]pyridine-8-carbonitrile (I-113) and stirred at 80 to 90°C for 6 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 360 mg (93%) of the title compound was obtained as yellow crystals from an eluate of chloroform-methanol (50:1 v/v).
MS (FAB)*m*/*z*: 389 (M+1)⁺.
¹H-NMR (CDCl₃)δ: 1.44 (6H, d, *J* = 6.9 Hz), 1.63 (1H, m), 1.98 (1H, m), 2.15 (6H, s), 2.25 (3H, s), 2.63 (1H, m), 3.21-3.36 (3H, m), 3.48 (1H, dd, *J =* 6.9, 9.9 Hz), 3.57 (1H, dt, *J =* 6.9, 10.2 Hz), 7.13 (1H, m), 7.24 (1H, m), 7.38-7.49 (3H, m).
IR (KBr): 2770, 2213, 1607, 1539, 1512, 1481, 1359 cm⁻¹.
Elemental analysis values: as C₂₃H₂₈N₆
Calcd.: C, 71.10%; H, 7.26%; N, 21.63%
Found: C, 71.01%; H, 7.25%; N, 21.63%

### [Example 23]

### 2-n-Butyl-7-methyl-5-[(3S)-dimethylaminopyrrolidin-1-yl]-6-phenyl[1,2,4]triazolo[1,5-a]pyridine-8-carbonitrile (#23)

A 107 µl (0.85 mmol) portion of (3S)-dimethylaminopyrrolidine and 215 µl (1.54 mmol) of triethylamine were added to an N,N-dimethylformamide (2.5 ml) solution of 250 mg (0.77 mmol) of 2-n-butyl-5-chloro-7-methyl-6-phenyl-[1,2,4]triazolo[1,5-a]pyridine-8-carbonitrile (I-114) and stirred at 80 to 90°C for 3.5 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 277 mg (89%) of the title compound was obtained as a ocherous solid from an eluate of chloroform-methanol (50:1 v/v).
MS (FAB)*m*/*z:* 403 (M+1)⁺.
¹H-NMR (CDCl₃)δ: 0.97 (3H, t, *J=* 7.4 Hz), 1.46 (2H, sex, *J*= 7.4 Hz), 1.56-1.69 (1H, m), 1.80-1.90 (2H, m), 1.93-2.02 (1H, m), 2.15 (6H, s), 2.25 (3H, s), 2.55-2.66 (1H, m), 2.92 (2H, t, *J*= 7.8 Hz), 3.21 (1H, t, *J*= 9.3 Hz), 3.28-3.35 (1H, m), 3.45 (1H, dd, *J*= 6.6, 9.9 Hz), 3.54-3.63 (1H, m), 7.11-7.15 (1H, m), 7.23-7.26 (1H, m), 7.40-7.60 (3H, m).
IR (KBr): 2956, 2771, 2209, 1610, 1508 cm⁻¹.
Elemental analysis values: as C₂₄H₃₀N₆
Calcd.: C, 71.61%; H, 7.51%; N, 20.88%
Found: C, 71.49%; H, 7.48%; N, 20.70%

### [Example 24]

### 2-i-Butyl-7-methyl-5-[(3S)-dimethylaminopyrrolidin-1-yl]-6-phenyl[1,2,4]triazolo[1,5-a]pyridine-8-carbonitrile (#24)

A 120 µl (0.93 mmol) portion of (3S)-dimethylaminopyrrolidine and 240 µl (1.70 mmol) of triethylamine were added to an N,N-dimethylformamide (5 ml) solution of 200 mg (0.62 mmol) of 2-i-butyl-5-chloro-7-methyl-6-phenyl-[1,2,4]triazolo[1,5-a]pyridine-8-carbonitrile (I-115) and stirred at 80 to 90°C for 6 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 226 mg (91%) of the title compound was obtained as yellow crystals from an eluate of chloroform-methanol (50:1 v/v).
MS (FAB)*m*/*z*: 403 (M+1)⁺.
¹H-NMR (CDCl₃)δ: 1.02 (6H, d, *J*= 6.6 Hz), 1.63 (1H, m), 1.97 (1H, m), 2.14 (6H, s), 2.26 (1H, m), 2.61 (1H, m), 2.79 (2H, d, *J* = 7.2 Hz), 3.22 (1H, dd, *J=* 8.7, 9.9 Hz), 3.33 (1H, m), 3.45 (1H, dd, *J=* 6.6, 9.9 Hz), 3.58 (1H, dt, *J=* 6.9, 10.2 Hz), 7.14 (1H, m), 7.25 (1H, m), 7.39-7.50 (3H, m). IR (KBr): 2766, 2211, 1602, 1537, 1504, 1474, 1361 cm⁻¹.
Elemental analysis values: as C₂₄H₃₀N₆
Calcd.: C, 71.61%; H, 7.51%; N, 20.88%
Found: C, 71.68%; H, 7.67%; N, 20.43%

### [Example 25]

### 2-tert-Butyl-7-methyl-5-[(3S)-dimethylaminopyrrolidin-1-yl]-6-phenyl[1,2,4]triazolo[1,5-a]pyridine-8-carbonitrile (#25)

A 172 µl (1.35 mmol) portion of (3S)-dimethylaminopyrrolidine and 343 µl (2.46 mmol) of triethylamine were added to an N,N-dimethylformamide (4 ml) solution of 400 mg (1.23 mmol) of 2-tert-butyl-5-chloro-7-methyl-6-phenyl-[1,2,4]triazolo[1,5-a]pyridine-8-carbonitrile (I-116) and stirred at 80 to 90°C for 2.5 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 479 mg (99%) of the title compound was obtained as pale yellow crystals from an eluate of chloroform-methanol (99:1 v/v). MS (FAB)*m*/*z*: 403 (M+1)⁺.
¹H-NMR (CDCl₃)δ: 1.47 (9H, s), 1.56-1.69 (1H, m), 1.92-2.00 (1H, m), 2.16 (6H, s), 2.24 (3H, s), 2.57-2.67 (1H, m), 3.23-3.36 (2H, m), 3.50-3.59 (2H, m), 7.11-7.15 (1H, m), 7.22-7.26 (1H, m), 7.38-7.49 (3H, m).
IR (KBr): 2964, 2772, 2210, 1606, 1508 cm⁻¹.
Elemental analysis values: as C₂₄H₃₀N₆
Calcd.: C, 71.61%; H, 7.51%; N, 20.88%
Found: C, 71.44%; H, 7.49%; N, 21.01%

### [Example 26]

### 7-Methyl-5-[(3S)-dimethylaminopyrrolidin-1-yl]-6-phenyl-2-(3-pyridyl)-[1,2,4]triazolo[1,5-a]pyridine-8-carbonitrile (#26)

A 199 mg (1.74 mmol) portion of (3S)-dimethylaminopyrrolidine and 385 µl (2.90 mmol) of triethylamine were added to an N,N-dimethylformamide (10 ml) solution of 311 mg (1.00 mmol) of 5-chloro-7-methyl-6-phenyl-2-(3-pyridyl)-[1,2,4]triazolo[1,5-a]pyridine-8-carbonitrile (I-117) and stirred at 80 to 90°C for 6 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 532 mg (87%) of the title compound was obtained as a pale yellow solid from an eluate of chloroform-methanol (100:1 → 97:3 v/v).
MS (FAB)*m*/*z*: 424 (M+1)⁺.
¹H-NMR (CDCl₃)δ: 1.59-1.82 (2H, m), 1.94-2.09 (1H, m), 2.18 (6H, s), 2.30 (3H, s), 2.59-2.73 (1H, m), 3.24-3.35 (1H, m), 3.37-3.48 (1H, m), 3.49-3.58 (1H, m), 3.61-3.73 (1H, m), 7.13-7.22 (1H, m), 7.24-7.33 (1H, m), 7.38-7.55 (4H, m), 8.57-8.64 (1H, m), 8.68-8.76 (1H, m), 9.48-9.55 (1H, m).
IR (KBr): 2951, 2820, 2770, 2206, 1612, 1596, 1573, 1538, 1508, 1473 cm⁻¹.
Elemental analysis values: as C₂₅H₂₅N₇-0.5H₂O
Calcd.: C, 69.42%; H, 6.06%; N, 22.67%
Found: C, 69.10%; H, 5.94%; N, 22.55%

### [Example 27]

### 7-Methyl-5-[(3S)-dimethylaminopyrrolidin-1-yl]-6-phenyl-2-(4-pyridyl)-[1,2,4]triazolo[1,5-a]pyridine-8-carbonitrile (#27)

A 377 mg (3.30 mmol) portion of (3S)-dimethylaminopyrrolidine and 730 µl (5.50 mmol) of triethylamine were added to an N,N-dimethylformamide (20 ml) solution of 311 mg (1.00 mmol) of 5-chloro-7-methyl-6-phenyl-2-(4-pyridyl)-[1,2,4]triazolo[1,5-a]pyridine-8-carbonitrile (I-118) and stirred at 80 to 90°C for 6 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 300 mg (26%, yield from I-102) of the title compound was obtained as a pale yellow solid from an eluate of chloroform-methanol (100: 1 → 97:3 v/v).
MS (FAB)*m*/*z:* 424 (M+1)⁺.
¹H-NMR (CDCl₃)δ: 1.60-1.77 (1H, m), 1.94-2.10 (1H, m), 2.19 (6H, s), 2.29 (3H, s), 2.57-2.72 (1H, m), 3.27-3.71 (4H, m), 7.10-7.22 (1H, m), 7.22-7.34 (1H, m), 7.40-7.58 (3H, m), 8.13-8.22 (2H, m), 8.71-8.81 (2H, m).
IR (KBr): 2982, 2950, 2815, 2770, 2214, 1610, 1532, 1511, 1470, 1450 cm⁻¹.
Elemental analysis values: as C₂₅H₂₅N₇·0.25H₂O
Calcd.: C, 70.15%; H, 6.00%; N, 22.91%
Found: C, 70.15%; H, 5.84%; N, 22.93%

### [Example 28]

### 2-Methoxymethyl-7-methyl-5-[(3S)-dimethylaminopyrrolidin-1-yl]-6-phenyl-[1,2,4]triazolo[1,5-a]pyridine-8-carbonitrile (#28)

An 893 µl (1.03 mmol) portion of (3S)-dimethylaminopyrrolidine and 1.78 ml (12.8 mmol) of triethylamine were added to an N,N-dimethylformamide (20 ml) solution of 2.00 g (6.39 mmol) of 5-chloro-2-methoxymethyl-7-methyl-6-phenyl-[1,2,4]triazolo[1,5-a]pyridine-8-carbonitrile (I-119) and stirred at 80 to 90°C for 2.5 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 1.20 g of crude title compound was obtained from an eluate of chloroform-methanol (1:0 → 100:1 → 20:1 v/v). This was again applied to a silica gel column chromatography, and 1.11 g (44%) of the title compound was obtained as an orange solid from an eluate of chloroform-methanol (1:0 → 100:1 → 100:3 v/v).
MS (FAB)*m*/*z*: 391 (M+1)⁺.
¹H-NMR (CDCl₃)δ: 1.56-1.69 (1H, m), 1.90-2.03 (1H, m), 2.13 (6H, s), 2.28 (3H, s), 2.54-2.65 (1H, m), 3.14 (1H, t, *J*= 9.3 Hz), 3.34-3.44 (2H, m), 3.56 (3H, s), 3.65-3.74 (1H, m), 4.74 (2H, s), 7.12-7.15 (1H, m), 7.23-7.27 (1H, m), 7.40-7.51 (3H, m).
IR (KBr): 2952, 2816, 2770, 2210, 1608, 1538, 1507, 1456, 1106 cm⁻¹.
Elemental analysis values: as C₂₂H₂₆N₆·0.25H₂O
Calcd.: C, 66.90%; H, 6.76%; N, 21.28%
Found: C, 67.09%; H, 6.67%; N, 21.26%

### [Example 29]

### 2-Hydroxymethyl-7-methyl-5-[(3S)-dimethylaminopyrrolidin-1-yl]-6-phenyl-[1,2,4]triazolo[1,5-a]pyridine-8-carbonitrile (#29)

A 1.82 ml (12.8 mmol) portion of iodomethylsilane was added to a chloroform (5 ml) solution of 500 mg (1.28 mmol) of 2-methoxymethyl-7-methyl-5-[(3S)-dimethylaminopyrrolidin-1-yl]-6-phenyl-[1,2,4]triazolo[1,5-a]pyridine-8-carbonitrile (#28) and stirred at room temperature for 5.5 hours. The reaction solution was poured into 2 N sodium hydroxide aqueous solution, mixed with brine and extracted with chloroform. The organic layer was washed with 1 N hydrochloric acid, 2 N sodium hydroxide aqueous solution and brine and dried over magnesium sulfate, and then the solvent was evaporated. The thus obtained residue was applied to a silica gel column chromatography, and 246 mg (51 %) of the title compound was obtained as a pale yellow solid from an eluate of chloroform-methanol (100:1 → 50:1 → 25:1 v/v).
MS (FAB)*m*/*z*: 377 (M+1)⁺.
¹H-NMR (CDCl₃)δ: 1.60-1.73 (1H, m), 1.93-2.03 (1H, m), 2.15 (6H, s), 2.27 (3H, s), 2.55-2.65 (1H, m), 3.27 (1H, t, *J*= 9.3 Hz), 3.34-3.44 (2H, m), 3.53-3.62 (1H, m), 4.93 (2H, s), 7.12-7.15 (1H, m), 7.26-7.31 (1H, m), 7.40-7.51 (3H, m).
IR (KBr): 3350, 2972, 2869, 2773, 2218, 1610, 1540, 1507, 1487 cm⁻¹.
Elemental analysis values: as C₂₁H₂₄N₆O·0.25H₂O
Calcd.: C, 66.21%; H, 6.48%; N, 22.06%
Found: C, 66.42%; H, 6.42%; N, 21.83%

### [Example 30]

### 2-Benzyloxymethyl-7-methyl-5-[(3S)-dimethylaminopyrrolidin-1-yl]-6-phenyl-[1,2,4]triazolo[1,5-a]pyridine-8-carbonitrile (#30)

A 17.5 mg (60%, 0.44 mmol) portion of sodium hydride was added at -15°C to a tetrahydrofuran (3 ml) solution of 150 mg (0.40 mmol) of 2-hydroxymethyl-7-methyl-5-[(3S)-dimethylaminopyrrolidin-1-yl]-6-phenyl-[1,2,4]triazolo[1,5-a]pyridine-8-carbonitrile (#29) and stirred at the same temperature for 5 minutes. Next, 56.9 µl (0.48 mmol) of benzyl bromide was added thereto and then stirred at room temperature for 4 hours. The reaction solution was concentrated under a reduced pressure, the resulting residue was dissolved in chloroform, and the organic layer was washed with water and brine and dried over magnesium sulfate. The solvent was evaporated, the thus obtained residue was applied to a silica gel column chromatography, and 75 mg (40%) of the title compound was obtained as a yellow solid from an eluate of chloroform-methanol (1:0 → 100:1 v/v).
MS (FAB)*m*/*z:* 467 (M+1)⁺.
¹H-NMR (CDCl₃)δ: 1.56-1.70 (1H, m), 1.91-2.01 (1H, m), 2.13 (6H, s), 2.28 (3H, s), 2.54-2.67 (1H, m), 3.16 (1H, t, *J=* 9.5 Hz), 3.32-3.46 (2H, m), 3.61-3.71 (1H, m), 4.76 (2H, s), 4.83 (2H, s), 7.10-7.18 (1H, m), 7.23-7.51 (9H, m).
IR (KBr): 2944, 2866, 2214, 1609, 1508, 1093 cm⁻¹.
Elemental analysis values: as C₂₈H₃₀N₆O
Calcd.: C, 72.08%; H, 6.48%; N, 18.01%
Found: C, 72.02%; H, 6.54%; N, 17.52%

### [Example 31]

### 2-Fluoromethyl-7-methyl-5-[(3S)-dimethylaminopyrrolidin-1-yl]-6-phenyl-[1,2,4]triazolo[1,5-a]pyridine-8-carbonitrile (#31)

At -10°C, a dichloromethane (5 ml) solution of 100 mg (0.27 mmol) of 2-hydroxymethyl-7-methyl-5 -[(3S)-dimethylaminopyrrolidin-1-yl]-6-phenyl-[1,2,4]triazolo[1,5-a]pyridine-8-carbonitrile (#29) was added dropwise to a dichloromethane (5 ml) solution of 53.3 mg (0.33 mmol) of diethylaminosulfur trifluoride. After 1 hour of stirring at -10°C, the reaction solution was mixed with water (1 ml) and extracted with chloroform. The organic layer was washed with brine and dried over magnesium sulfate. The solvent was evaporated, the thus obtained residue was applied to a silica gel column chromatography, and 25 mg (20%) of the title compound was obtained as a pale yellow solid from an eluate of chloroform-methanol (10:1 v/v). MS (FAB)*m*/*z*: 379 (M+1)⁺.
¹H-NMR (CDCl₃)δ: 1.57-1.71 (1H, m), 1.93-2.04 (1H, m), 2.14 (6H, s), 2.29 (3H, s), 2.55-2.67 (1H, m), 3.18 (1H, t, *J=* 9.5 Hz), 3.34-3.45 (2H, m), 3.61-3.71 (1H, m), 5.54 (1H s), 5.70 (1H, s), 7.12-7.17 (1H, m), 7.23-7.30 (1H, m), 7.42-7.53 (3H, m).
IR (KBr): 2946, 2770, 2216, 1608, 1514, 1372 cm⁻¹.

### [Example 32]

### 2-Cyanomethyl-7-methyl-5-[(3S)-dimethylaminopyrrolidin-1-yl]-6-phenyl-[1,2,4]triazolo [1,5-a]pyridine-8-carbonitrile (#32)

A 186 mg (0.49 mmol) portion of 2-hydroxymethyl-7-methyl-5-[(3S)-dimethylaminopyrrolidin-1-yl]-6-phenyl-[1,2,4]triazolo[1,5-a]pyridine-8-carbonitrile (#29) was mixed with 1.24 ml (4.94 mmol) of 4 N hydrochloric acid dioxane solution and dissolved by further adding chloroform and methanol thereto. This was stirred at room temperature for 10 minutes and then concentrated under a reduced pressure, and the thus obtained residue was mixed with 10 ml of thionyl chloride and heated under reflux for 40 minutes. After cooling, this was evaporated to dryness under a reduced pressure. An ethanol (3 ml) solution of the thus obtained residue was added dropwise to a water (0.5 ml) solution of 225 mg (3.46 mmol) of potassium cyanide under ice-cooling, and this was stirred at the same temperature for 1 hour, warmed up to room temperature and stirred overnight (in the course of the reaction, water (5 ml) was added). This was further stirred at 60°C for 6 hours (in the course of the reaction, 96.5 mg (1.48 mmol) of potassium cyanide was supplemented). A 17.5 mg (60%, 0.44 mmol) portion of sodium hydride was added thereto and stirred at the same temperature for 5 minutes. After cooling, the reaction solution was evaporated under a reduced pressure, the resulting residue was washed with a chloroform-methanol mixed solvent (10:1 v/v), and the filtrate was concentrated under a reduced pressure. The thus obtained residue was applied to a silica gel column chromatography (twice) and eluted with chloroform-methanol (200:1 → 100:1 → 50:1 v/v) and further with chloroform-methanol (100:1 v/v), and then 66 mg (35%) of the title compound was obtained by isolating and purifying it by a preparative TLC (developed with chloroform-methanol (10:1 v/v)).
MS (FAB)*m*/*z*: 386 (M+1)⁺.
¹H-NMR (CDCl₃)δ: 1.50-1.64 (1H, m), 1.88-1.99 (1H, m), 2.07 (6H, s), 2.21 (3H, s), 2.49-2.60 (1H, m), 3.08 (1H, t, *J*= 9.5 Hz), 3.31-3.40 (2H, m), 3.59-3.69 (1H, m), 3.99 (2H, s), 7.05-7.09 (1H, m), 7.16-7.22 (1H, m), 7.34-7.45 (3H, m).
IR (KBr): 2950, 2769, 2210, 1607, 1517, 1365 cm⁻¹.
Elemental analysis values: as C₂₂H₂₃N₇·0.25H₂O
Calcd.: C, 67.76%; H, 6.07%; N, 25.14%
Found: C, 67.86%; H, 5.96%; N, 24.82%

### [Example 33]

### 7-Methyl-5-[(3S)-dimethylaminopyrrolidin-1-yl]-6-phenyl-2-i-propenyl-[1,2,4]triazolo[1,5-a]pyridine-8-carbonitrile (#33)

A 81.4 mg (0.713 mmol) portion of (3S)-dimethylaminopyrrolidine and 173 µl (1.30 mmol) of triethylamine were added to an N,N-dimethylformamide (3 ml) solution of 200 mg (0.648 mmol) of 5-chloro-7-methyl-6-phenyl-2-i-propenyl[1,2,4]triazolo[1,5-a]pyridine-8-carbonitrile (I-120) and stirred at 80 to 90°C for 6 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 195 mg (78%) of the title compound was obtained as a pale yellow solid from an eluate of chloroform-methanol (100:1 v/v).
MS (FAB)*m*/*z*: 387 (M+1)⁺.
¹H-NMR (CDCl₃)δ: 1.55-1.71 (1H, m), 1.92-2.03 (1H, m), 2.15 (6H, s), 2.27 (6H, s), 2.56-2.67 (1H, m), 3.22-3.37 (2H, m), 3.47-3.61 (2H, m), 5.46 (1H, m), 6.36 (1H, m), 7.12-7.18 (1H, m), 7.23-7.30 (1H, m), 7.39-7.50 (3H, m).
IR (KBr): 3055, 2978, 2950, 2867, 2817, 2768, 2211, 1610, 1538, 1509, 1476 cm⁻¹.
Elemental analysis values: as C₂₃H₂₆N₆·0.25H₂O
Calcd.: C, 70.65%; H, 6.83%; N, 21.49%
Found: C, 71.01%; H, 6.74%; N, 21.73%

### [Example 34]

### 2-(2-Benzyloxyethyl)-7-methyl-5-[(3S)-dimethylaminopyrrolidin-1-yl]-6-phenyl-[1,2,4]triazolo[1,5-a]pyridine-8-carbonitrile (#34)

A 86.6 µl (0.683 mmol) portion of (3S)-dimethylaminopyrrolidine and 173 µl (1.24 mmol) of triethylamine were added to an N,N-dimethylformamide (2.5 ml) solution of 250 mg (0.621 mmol) of 2-(benzyloxyethyl)-5-chloro-7-methyl-6-phenyl-[1,2,4]triazolo[1,5-a]pyridine-8-carbonitrile (I-121) and stirred at 80 to 90°C for 1 hour. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 256 mg (86%) of the title compound was obtained as a ocherous solid from an eluate of chloroform-methanol (100:1 v/v).
MS (FAB)*m*/*z*: 481 (M+1)⁺.
¹H-NMR (CDCl₃)δ: 1.54-1.68 (1H, m), 1.91-2.00 (1H, m), 2.13 (6H, s), 2.26 (3H, s), 2.54-2.64 (1H, m), 3.15 (1H, t, *J*= 9.5 Hz), 3.25 (2H, t, *J=* 6.9 Hz), 3.28-3.36 (1H, m), 3.42 (1H, dd, *J=* 6.6, 9.9 Hz), 3.57-3.64 (1H, m), 4.01 (2H, t, *J*= 6.9 Hz), 4.59 (2H, s), 7.10-7.15 (1H, m), 7.23-7.50 (9H, m).
IR (KBr): 2970, 2779, 2210, 1606, 1505 cm⁻¹.
Elemental analysis values: as C₂₉H₃₂N₆O·0.25H₂O
Calcd.: C, 71.80%; H, 6.75%; N, 17.32%
Found: C, 71.91 %; H, 6.65%; N, 17.32%

### [Example 35]

### 2-(2-Hydroxyethyl)-7-methyl-5-[(3S)-dimethylaminopyrrolidin-1-yl]-6-phenyl-[1,2,4]triazolo[1,5-a]pyridine-8-carbonitrile (#35)

In a mixed solution of methanol (9 ml)-tetrahydrofuran (9 ml), 900 mg (1.87 mmol) of 2-(2-benzyloxyethyl)-7-methyl-5-[(3S)-dimethylaminopyrrolidin-1-yl]-6-phenyl-[1,2,4]triazolo[1,5-a]pyridine-8-carbonitrile (#34) was mixed with 2.81 ml (11.2 mmol) of 4 N hydrochloric acid dioxane solution, stirred for 5 minutes and then concentrated under a reduced pressure. The thus obtained residue was dissolved in methanol (8 ml), mixed with 360 mg of 5% palladium-carbon catalyst and then stirred at room temperature for 1.25 hours in an atmosphere of hydrogen (4.5 atm). After removing the catalyst by filtration, the solvent was evaporated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 383 mg (53%) of the title compound was obtained as a yellow solid from an eluate of chloroform-methanol (100:1 → 50:1 → 20:1 v/v).
MS (FAB)*m*/*z*: 391 (M+1)⁺.
¹H-NMR (CDCl₃)δ: 1.54-1.69 (1H, m), 1.91-2.02 (1H, m), 2.14 (6H, s), 2.27 (3H, s), 2.54-2.65 (1H, m), 3.12-3.19 (1H, m), 3.17 (2H, t, *J=* 5.6 Hz), 3.31-3.38 (1H, m), 3.43 (1H, dd, *J =* 6.9, 9.9 Hz), 3.57-3.66 (1H, m), 4.09 (2H, t, *J =* 5.6 Hz), 7.11-7.15 (1H, m), 7.23-7.26 (1H, m), 7.40-7.51 (3H, m).
IR (KBr): 3145, 2957, 2877, 2213, 1608, 1508 cm⁻¹.
Elemental analysis values: as C₂₂H₂₆N₆O-1.25H₂O
Calcd.: C, 63.98%; H, 6.96%; N, 20.35%
Found: C, 64.06%; H, 6.46%; N, 20.13%

### [Example 36]

### 2-(2-Benzyloxyethyl)-7-methyl-5-[(3S)-dimethylaminopyrrolidin-1-yl]-6-phenyl-[1,2,4]triazolo[1,5-a]pyridine-8-carbonitrile (#36)

A 15.2 µl (0.12 mmol) portion of (3S)-dimethylaminopyrrolidine and 30.3 µl (0.22 mmol) of triethylamine were added to an N,N-dimethylformamide (0.4 ml) solution of 36 mg (0.11 mmol) of 5-chloro-2-(2-chloroethyl)-7-methyl-6-phenyl-[1,2,4]triazolo[1,5-a]pyridine-8-carbonitrile (I-122) and stirred at 80 to 90°C for 40 minutes. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 26 mg (59%) of the title compound was obtained as a yellow solid from an eluate of chloroform-methanol (100:3 v/v).
MS (FAB)*m*/*z*: 409 (M+1)⁺.
¹H-NMR (CDCl₃)δ: 1.54-1.70 (1H, m), 1.91-2.03 (1H, m), 2.15 (6H, s), 2.26 (3H, s), 2.55-2.66 (1H, m), 3.22 (1H, t, *J=* 9.5 Hz), 3.30-3.48 (2H, m), 3.39 (2H, t, *J=* 6.9 Hz), 3.28-3.36 (1H, m), 3.42 (1H, dd, *J=* 6.6, 9.9 Hz), 3.57-3.64 (1H, m), 4.01 (2H, t, *J=* 6.9 Hz), 3.53-3.64 (1H, m), 4.03 (2H, t, *J*= 6.9 Hz), 7.10-7.16 (1H, m), 7.23-7.26 (1H, m), 7.40-7.53 (3H, m).
IR (KBr): 295 8, 2769, 2211, 1607, 1508, 1271, 702 cm⁻¹.

### [Example 37]

### 2{5-[(3S)-dimethylaminopyrrolidin-1-yl]-8-cyano-7-methyl-6-phenyl-[1,2,4]triazolo[1,5-al]pyridin-2-yl}-2-methylpropionic acid methyl ester (#37)

A 60 µl (0.45 mmol) portion of (3 S)-dimethylaminopyrrolidine was added to an N,N-dimethylformamide (5 ml) solution of 70 mg (9.19 mmol) of 2-[5-chloro-8-cyano-7-methyl-6-phenyl-[1,2,4]triazolo[1,5-a]pyridin-2-yl]-2-methylpropionic acid methyl ester (I-123) and stirred at 80 to 90°C for 5 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 76 mg (90%) of the title compound was obtained as a ocherous solid from an eluate of chloroform-methanol (50: v/v).
MS (FAB)*m*/*z*: 447 (M+1)⁺.
¹H-NMR (CDCl₃)δ: 1.60 (1H, m), 1.75 (6H, s), 1.97 (1H,m), 2.14 (6H, s), 2.24 (3H, s), 2.60 (1H, m), 3.20 (1H, dd, *J*= 8.7, 9.9 Hz), 3.36 (1H, m), 3.46-3.61 (2H, m), 3.72 (3H, s), 7.13 (1H, m), 7.24 (1H, m), 7.38-7.49 (3H, m).
IR (KBr): 2765, 2215, 1734, 1607, 1508, 1475, 1355, 1266, 1145 cm⁻¹.
Elemental analysis values: as C₂₅H₃₀N₆O₂
Calcd.: C, 67.24%; H, 6.77%; N, 18.82%
Found: C, 67.14%; H, 6.77%; N, 18.74%

### [Example 38]

### 2-{5-[(3S)-dimethylaminonyrrolidin-1-yl]-8-cyano-7-methyl-6-phenyl-[1,2,4]triazolo[1,5-a]pyridin-2-yl}-2-methylpropionic acid (#38)

A 70 µl (0.55 mmol) portion of (3S)-dimethylaminopyrrolidine was added to an N,N-dimethylformamide (5 ml) solution of 82 mg (0.22 mmol) of a methyl ester-ethyl ester mixture of 2-[5-chloro-8-cyano-7-methyl-6-phenyl-[1,2,4]triazolo[1,5-a]pyridin-2-yl]-2-methylpropionic acid (I-123) and stirred at 80 to 90°C for 5 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 108 mg of ester form (mixture of ethyl ester and methyl ester) of the title compound was obtained as pale brown crystals from an eluate of chloroform-methanol (50:1 v/v). A 90 mg portion thereof was dissolved in methanol (4 ml) and mixed with water (1 ml) and then heated under reflux for 1 hour. After cooling, this was mixed with 54 mg (1.35 mmol) of sodium hydroxide at room temperature and stirred at the same temperature for 5 days. After concentration under a reduced pressure, the thus obtained residue was dissolved in water and washed with ether, and then the water layer was adjusted to pH 7.0 with 1 N hydrochloric acid and extracted with chloroform. This was dried over magnesium sulfate, and the solvent was evaporated to obtain 81 mg of the title compound as a colorless solid.
MS (FAB)*m*/*z*: 433 (M+1)⁺.
¹H-NMR (CDCl₃)δ:1.58 (3H, s), 1.64 (3H, s), 1.74 (1H, m), 1.94 (1H, m), 2.20 (3H, s), 2.43 (6H, s), 2.97 (1H, m), 3.23 (2H, m), 3.50 (1H, dd, *J =* 7.2, 11.7 Hz), 4.00 (4H, brs), 4.09 (1H, dd, *J =* 6.0, 11.7 Hz), 7.21-7.25 (2H, m), 7.42-7.50 (3H, m).
IR (KBr): 3434, 2976, 2216, 1606, 1536, 1508, 1481, 1351, 1267 cm⁻¹.
Elemental analysis values: as C₂₄H₂₈N₆O₂·1.25H₂O
Calcd.: C, 63.35%; H, 6.76%; N, 18.47%
Found: C, 63.58%; H, 6.42%; N, 18.24%

### [Example 39]

### 2-(2-Benzyloxy-1,1-dimethylethyl)-7-methyl-5-[(3S)-dimethylaminopyrrolidin-1-yl]-6-phenyl[1,2,4]triazolo[1,5-a]pyridine-8-carbonitrile (#39)

A 600 µl (4.6 mmol) portion of (3S)-dimethylaminopyrrolidine and 1.3 ml (9.20 mmol) of triethylamine were added to an N,N-dimethylformamide (15 ml) solution of 1.60 g (3.70 mmol) of 2-(2-benzyloxy-1,1-dimethylethyl)-5-chloro-7-methyl-6-phenyl-[1,2,4]triazolo[1,5-a]pyridine-8-carbonitrile (I-124) and stirred at 80 to 90°C for 4 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 1.78 g (95%) of the title compound was obtained as pale yellow crystals from an eluate of chloroform-methanol (100:1 v/v).
MS (FAB)*m*/*z*: 509 (M+1)⁺.
¹H-NMR (CDCl₃)δ: 1.49 (6H, s), 1.61 (1H, m), 1.93 (1H, m), 2.14 (6H, s), 2.41 (3H, s), 2.60 (1H, m), 3.23 (1H, dd, *J*= 9.0, 9.9 Hz), 3.31 (1H, m), 3.48-3.59 (2H, m), 3.78 (2H, s), 4.55 (2H, m), 7.10-7.48 (10H, m).
IR (KBr): 2869, 2207, 1604, 1537, 1504, 1468, 1349, 1091 cm⁻¹.
Elemental analysis values: as C₃₁H₃₆N₆O
Calcd.: C, 73.20%; H, 7.13%; N, 16.52%
Found: C, 73.04%; H, 7.11%; N, 16.38%

### [Example 40]

### 2-(2-Hydroxy-1,1-dimethylethyl)-7-methyl-5-[(3S)-dimethylaminopyrrolidin-1-yl]-6-phenyl-[1,2,4]triazolo[1,5-a]pyridine-8-carbonitrile (#40)

A 1.40 g (2.80 mmol) portion of 2-(2-benzyloxy-1,1-dimethylethyl)-7-methyl-5-[(3S)-dimethylaminopyrrolidin-1-yl]-6-phenyl[1,2,4]triazolo[1,5-a]pyridine-8-carbonitrile (#39) was dissolved in a mixed solution of methanol (14 ml) and tetrahydrofuran (14 ml), mixed with 4.1 ml (16.4 mmol) of 4 N hydrochloric acid dioxane solution and stirred at room temperature for 10 minutes, and then concentrated under a reduced pressure (toluene azeotropy was carried out twice) The thus obtained residue was dissolved in methanol (30 ml), mixed with 560 mg of 5% palladium-carbon catalyst and then stirred at room temperature for 30 minutes in an atmosphere of hydrogen (4.5 atm). After removing the catalyst by filtration, the solvent was evaporated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 1.0 g (87%) of the title compound was obtained as pale yellow crystals from an eluate of chloroform-methanol (50:1 v/v). MS (FAB)*m*/*z*: 419 (M+1)⁺.
¹H-NMR (CDCl₃)δ: 1.44 (6H, s), 1.63 (1H, m), 1.98 (1H, m), 2.15 (6H, s), 2.25 (3H, s), 2.61 (1H, m), 3.22 (1H, dd, *J*= 8.7, 10.2 Hz), 3.35 (1H, m), 3.44-3.60 (2H, m), 3.76 (1H, s), 3.86 (1H, brs), 7.11-7.14 (1H, m), 7.23-7.26 (1H, m), 7.39-7.50 (3H, m).
IR (KBr): 3155, 2965, 2214, 1603, 1536, 1503, 1469, 1347, 1065 cm⁻¹.
Elemental analysis values: as C₂₄H₃₀N₆O·0.25H₂O
Calcd.: C, 68.14%; H, 7.27%; N, 19.87%
Found: C, 68.17%; H, 7.21%; N, 19.39%

### [Example 41]

### 2-[2-(4-Fluorobenzyloxy)-1,1-dimethylethyl]-7-methyl-5-[(3S)-dimethylaminopyrrolidin-1-yl]-6-phenyl-[1,2,4]triazolo[1,5-a]pyridine-8-carbonitrile (#41)

A 120 µl (0.95 mmol) portion of (3S)-dimethylaminopyrrolidine was added to an N,N-dimethylformamide (5 ml) solution of 170 mg (0.38 mmol) of 5-chloro-2-[2-(4-fluorobenzyloxy)-1,1-dimethylethyl]-7-methyl-6-phenyl-[1,2,4]triazolo[1,5-a]pyridine-8-carbonitrile (I-125) and stirred at 70 to 80°C for 6 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 170 mg (85%) of the title compound was obtained as pale yellow crystals from an eluate of chloroform-methanol (50:1 v/v). MS (FAB)*m*/*z*: 527 (M+1)⁺.
¹H-NMR (CDCl₃)δ: 1.49 (6H, s), 1.61 (1H, m), 1.95 (1H, m), 2.14 (6H, s), 2.25 (3H, s), 2.59 (1H, m), 3.24 (1H, m), 3.32 (1H, m), 3.47-3.57 (2H, m), 3.76 (2H, s), 4.50 (2H, m), 6.95-7.02 (2H, m), 7.10-7.14 (1H, m), 7.21-7.29 (3H, m), 7.40-7.49 (3H, m).
IR (KBr): 2969, 2210, 1604, 1537, 1508, 1476, 1350, 1086 cm⁻¹.
Elemental analysis values: as C₃₁H₃₅FN₆O
Calcd.: C, 70.70%; H, 6.70%; N, 15.96%
Found: C, 70.54%; H, 6.70%; N, 15.87%

### [Reference Example 126]

### 2-(4-Fluorophenyl)-3-oxobutyronitrile (I-126)

A 22.8 ml (0.19 mmol) portion of 4-fluorophenylacetonitrile and 29.7 ml of ethyl acetate were added to an ethanol solution of sodium ethoxide prepared from ethanol (130 ml) and 5.68 g (0.247 mol) of metallic sodium and then heated under reflux for 6 hours. After cooling, this was concentrated under a reduced pressure, and the thus obtained residue was mixed with brine and chloroform to separate the organic layer. This was washed with 1 N hydrochloric acid and brine and dried over magnesium sulfate, and then the solvent was evaporated. The thus obtained residue was mixed with n-hexane-isopropyl ether (1:1 v/v), and the precipitated crystals were collected by filtration to obtain 9.83 g (29%) of the title compound as a pale yellow solid. In addition, the solvent of the filtrate was evaporated, and the resulting residue was applied to a silica gel column chromatography to obtain 5.11 g (15%) of the title compound from a chloroform eluate.
MS (FAB)*m*/*z*: 178 (M+1)⁺.
¹H-NMR (CDCl₃)δ: 2.29 (3H, s), 4.67 (1H, s), 7.10-7.17 (2H, m), 7.35-7.42 (2H, m).

### [Reference Example 127]

### 2-(4-Fluorophenyl)-3-methoxybut-2-ene nitrile (I-127)

A trimethyl orthoacetate (20 ml) solution of 1.0 g (5.64 mmol) of 2-(4-fluorophenyl)-3-oxobutyronitrile (I-126) was heated under reflux for 7 hours (in the course of the reaction, trimethyl orthoacetate (20 ml) was supplemented). After cooling, the reaction solution was concentrated under a reduced pressure, the resulting residue was applied to a silica gel column chromatography, and 809 mg (75%) of the title compound was obtained as a yellow oily substance from an eluate of n-hexane-ethyl acetate (5:1 → 4:1 v/v).
MS (FAB)*m*/*z*: 192 (M+1)⁺.
¹H-NMR (CDCl₃)δ: 2.05 (0.6H, s), 2.45 (2.4H, s), 3.87 (3H, s), 3.92 (3H, s), 6.98-7.09 (2H, m), 7.21-7.28 (0.4H, m), 7.54-7.61 (1.6H, m).

### [Reference Example 128]

### 5-Amino-2-tert-butyl-6-(4-fluorophenyl)-7-methyl[1,2,4]triazolo[1,5-a]pyridine-8-carbonitrile (I-128)

A tetrahydrofuran (50 ml) solution of 500 mg (3.04 mmol) of 3-tert-butyl-5-cyanomethyl-1H-[1,2,4]triazole (I-83) was cooled to -30°C, and 3.35 ml (6.70 mmol) of a heptane-tetrahydrofuran-ethylbenzene mixed solution of 2.0 M lithium diisopropyl amide was added dropwise thereto. After 30 minutes of stirring at the same temperature, a tetrahydrofuran (10 ml) solution of 582 mg (3.04 mmol) of 2-(4-fluorophenyl)-3-methoxybut-2-ene nitrile (I-127) was added dropwise thereto, and this was stirred as such at -30°C overnight. The reaction solution was poured into saturated ammonium chloride aqueous solution and extracted with ethyl acetate. The organic layer was washed with brine and dried over magnesium sulfate, and then the solvent was evaporated. The thus obtained residue was applied to a silica gel column chromatography, and 175 mg (17%) of the title compound was obtained as a yellowish brown solid from a chloroform eluate.
MS (FAB)*m*/*z:* 324 (M+1)⁺.
¹H-NMR (CDCl₃)δ: 1.47 (9H, s), 2.30 (3H, s), 5.44 (2H, brs), 7.21-7.32 (4H, m).

### [Reference Example 129]

### 2-tert-Butyl-5-chloro-6-(4-fluorophenyl)-7-methyl[1,2,4]triazolo[1,5-a]pyridine-8-carbonitrile (I-129)

A 93.8 µl (0.789 mmol) portion of tert-butyl nitrite and 84.8 mg (0.631 mmol) of copper(II) chloride were added to acetonitrile (3 ml) and stirred at 75°C for 5 minutes. This was mixed with 170 mg (0.526 mmol) of 5-amino-2-tert-butyl-6-(4-fluorophenyl)-7-methyl[1,2,4]triazolo[1,5-a]pyridine-8-carbonitrile (I-128) and stirred at 75°C for 1 hour. After cooling, this was concentrated under a reduced pressure, and the resulting residue was mixed with 0.5 N hydrochloric acid and extracted with chloroform. The organic layer was washed with brine and dried over magnesium sulfate, and then the solvent was evaporated. The thus obtained residue was applied to a silica gel column chromatography, and 99 mg (55%) of the title compound was obtained as a yellow solid from chloroform-n-hexane (10:1 v/v).
MS (FAB)*m*/*z:* 343 (M+1)⁺.
¹H-NMR (CDCl₃)δ: 1.51 (9H, s), 2.41 (3H, s), 7.17-7.29 (4H, m).

### [Example 42]

### 2-tert-Butyl-6-(4-fluorophenyl)-7-methyl-5-[(3S)-dimethylaminopyrrolidin-1-yl-[1,2,4]triazolo[1,5-a]pyridine-8-carbonitrile (#42)

A 37.5 µl (0.295 mmol) portion of (3S)-dimethylaminopyrrolidine and 74.8 µl (0.537 mmol) of triethylamine were added to an N,N-dimethylformamide (1 ml) solution of 92 mg (0.268 mmol) of 2-tert-butyl-5-chloro-6-(4-fluorophenyl)-7-methyl-[1,2,4]triazolo[1,5-a]pyridine-8-carbonitrile (I-129) and stirred at 80 to 90°C for 30 minutes. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 97 mg (86%) of the title compound was obtained as a yellow solid from an eluate of chloroform-methanol (1:0 → 100:1 v/v).
MS (FAB)*m*/*z*: 403 (M+1)⁺.
¹H-NMR (CDCl₃)δ: 1.47 (9H, s), 1.56-1.71 (1H, m), 1.93-2.04 (1H, m), 2.18 (6H, s), 2.23 (3H, s), 2.58-2.69 (1H, m), 3.27-3.36 (2H, m), 3.49-3.60 (2H, m), 7.07-7.26 (4H, m).
IR (KBr): 2965, 2209, 1606, 1494, 1225 cm⁻¹.
Elemental analysis values: as C₂₄H₂₉FN₆
Calcd.: C, 68.55%; H, 6.95%; N, 19.98%
Found: C, 68.45%; H, 6.98%; N, 19.78%

### [Reference Examples 130 and 131]

### 2-tert-Butyl-5-chloro-7-methyl-6-(4-nitrophenyl)-[1,2,4]triazolo[1,5-a]pyridine-8-carbonitrile (I-130) and 2-tert-butyl-5-chloro-7-methyl-6-(3-nitrophenyl)-[1,2,4]triazolo[1,5-a]pyridine-8-carbonitrile (I-131)

A 163 µl (1.73 mmol) portion of acetic anhydride was added to a carbon tetrachloride (1 ml) suspension of 510 mg (1.57 mmol) of 2-tert-butyl-5-chloro-7-methyl-6-phenyl-[1,2,4]triazolo[1,5-a]pyridine-8-carbonitrile (I-116) and cooled to 0°C. While vigorously stirring, 510 µl of fuming nitric acid was slowly added dropwise thereto (in the course of the reaction, carbon tetrachloride (2 ml) was supplemented). After the dropwise addition, this was stirred at 0°C for 20 minutes and then stirred at room temperature for 15 minutes. This was supplemented with 510 µl of fuming nitric acid at room temperature and stirred as such for 10 minutes, and the reaction solution was poured into ice water. This was neutralized with saturated sodium bicarbonate aqueous solution and then extracted with chloroform. The organic layer was washed with brine and dried over magnesium sulfate, and then the solvent was evaporated under a reduced pressure. The resulting residue was applied to a silica gel column chromatography (carried out twice), and 185 mg (32%) and 286 mg (49%) of the title compounds (I-130) and (I-131) were respectively obtained as pale yellow solids from an eluate of n-hexane-ethyl acetate (5:1 v/v). (I-130)
MS (FAB)*m*/*z*: 370 (M+1)⁺.
¹H-NMR(CDCl₃)δ: 1.52 (9H, s), 2.42 (3H, s), 7.44-7.50 (4H, m), 8.40-8.47 (2H, m). (I-131)
MS (FAB)*m*/*z*: 370 (M+1)⁺.
¹H-NMR (CDCl₃)δ: 1.52 (9H, s), 2.43 (3H, s), 7.60 (1H, dt, *J =* 1.4, 8.0 Hz), 7.78 (1H, t, *J=* 8.0 Hz), 8.16 (1H, t, *J =* 1.8 Hz), 8.39-8.43 (1H, m).

### [Example 43]

### 2-tert-Butyl-7-methyl-5-[(3S)-dimethylaminopyrrolidin-1-yl]-6-(4-nitrophenyl)-[1,2,4]triazolo[1,5-a]pyridine-8-carbonitrile (#43)

A 64.2 µl (0.506 mmol) portion of (3S)-dimethylaminopyrrolidine and 128 µl (0.910 mmol) of triethylamine were added to an N,N-dimethylformamide (2 ml) solution of 170 mg (0.46 mmol) of 2-tert-butyl-5-chloro-7-methyl-6-(4-nitrophenyl)-[1,2,4]triazolo[1,5-a]pyridine-8-carbonitrile (I-130) and stirred at 80 to 90°C for 45 minutes. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 175 mg (85%) of the title compound was obtained as a yellow solid from an eluate of chloroform-methanol (1:0 → 100:1 v/v).
MS (FAB)*m*/*z*: 448 (M+1)⁺.
¹H-NMR (CDCl₃)δ: 1.47 (9H, s), 1.60-1.75 (1H, m), 1.95-2.04 (1H, m), 2.18 (6H, s), 2.24 (3H, s), 2.60-2.70 (1H, m), 3.27-3.53 (3H, m), 3.61 (1H, dd, *J*= 6.6, 9.9 Hz), 7.34-7.39 (1H, m), 7.47-7.51 (1H, m), 8.30-8.40 (2H, m).
IR (KBr): 2968, 2212, 1606, 1514, 1493,1455, 1350 cm⁻¹.
Elemental analysis values: as C₂₄H₂₉N₇O₂
Calcd.: C, 64.41%; H, 6.53%; N, 21.91%
Found: C, 64.33%; H, 6.55%; N, 21.57%

### [Example 44]

### 2-tert-Butyl-7-methyl-5-[(3S)-dimethylaminopyrrolidin-1-yl]-6-(3-nitrophenyl)-[1,2,4]triazolo[1,5-a]pyridine-8-carbonitrile (#44)

A 96.6 µl (0.761 mmol) portion of (3S)-dimethylaminopyrrolidine and 193 µl (1.38 mmol) of triethylamine were added to an N,N-dimethylformamide (2.5 ml) solution of 256 mg (0.692 mmol) of 2-tert-butyl-5-chloro-7-methyl-6-(3-nitrophenyl)-[1,2,4]triazolo[1,5-a]pyridine-8-carbonitrile (I-131) and stirred at 80 to 90°C for 40 minutes. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 289 mg (93%) of the title compound was obtained as a yellow solid from an eluate of chloroform-methanol (1:0 → 100:1 v/v).
MS (FAB)*m*/*z*: 448 (M+1)⁺.
¹H-NMR (CDCl₃)δ: 1.47 (9H, s), 1.60-1.75 (1H, m), 1.93-2.04 (1H, m), 2.15 (3H, s), 2.17 (3H, s), 2.24 (1.5H, s), 2.25 (1.5H, s), 2.58-2.71 (1H, m), 3.24-3.37 (2H, m), 3.43-3.55 (1H, m), 3.57-3.69 (1H, m), 7.50-7.55 (0.5H, m), 7.63-7.73 (1.5H, m), 8.05 (0.5H, t, *J*= 1.8 Hz), 8.16 (0.5H, t, *J=* 1.8 Hz), 8.27-8.35 (1H, m).
IR (KBr): 2963, 2211, 1605, 1530, 1513, 1495, 1351 cm⁻¹.
Elemental analysis values: as C₂₄H₂₉N₇O₂·H₂O
Calcd.: C, 61.92%; H, 6.71%; N, 21.06%
Found: C, 62.05%; H, 6.28%; N, 20.97%

### [Example 45]

### 6-(4-Aminophenyl)-2-tert-butyl-7-methyl-5-[(3S)-dimethylaminopyrrolidin-1-yl]-[1,2,4]triazolo[1,5-a]ipyridine-8-carbonitrile (#45)

A 90 mg (0.201 mmol) portion of 2-tert-butyl-7-methyl-5-[(3S)-dimethylaminopyrrolidin-1-yl]-6-(4-nitrophenyl)-[1,2,4]triazolo[1,5-a]pyridine-8-carbonitrile (#43) was dissolved in 4 N hydrochloric acid dioxane solution (1 ml) by subjecting it to several minutes of ultrasonic treatment. After concentrated under a reduced pressure, the resulting residue was dissolved in methanol (5 ml), mixed with 26 mg of 5% palladium-carbon catalyst and then stirred at room temperature for 1.5 hours under an atmosphere of hydrogen under ordinary pressure. After removing the catalyst by filtration, the solvent was evaporated under a reduced pressure, and the resulting residue was mixed with ethyl acetate and washed with 10% sodium carbonate aqueous solution and brine. The organic layer was dried over magnesium sulfate, and then the solvent was evaporated under a reduced pressure. The thus obtained residue was applied to a silica gel column chromatography, and 61 mg (73%) of the title compound was obtained as a pale yellow solid from an eluate of chloroform-methanol (100:1 → 50:1 v/v). MS (FAB)*m*/*z*: 418 (M+1)⁺.
¹H-NMR (CDCl₃)δ: 1.47 (9H, s), 1.54-1.70 (1H, m), 1.92-2.03 (1H, m), 2.19 (6H, s), 2.26 (3H, s), 2.57-2.68 (1H, m), 3.28-3.56 (4H, m), 3.80 (2H, brs), 6.68-6.77 (2H, m), 6.84-6.88 (1H, m), 6.93-6.98 (1H, m). IR (KBr): 3462, 3356, 3223, 2964, 2866, 2819, 2771, 2217, 1628, 1602, 1535, 1507, 1474, 1458 cm⁻¹.
Elemental analysis values: as C₂₄H₃₁N₇·0.75H₂O
Calcd.: C, 66.87%; H, 7.60%; N, 22.75%
Found: C, 66.80%; H, 7.27%; N, 22.48%

### [Example 46]

### 6-(3-Aminophenyl)-2-tert-butyl-7-methyl-5-[(3S)-dimethylaminopyrrolidin-1-yl]-[1,2,4]triazolo[1,5-a]pyridine-8-carbonitrile (#46)

A 58 mg (0.13 mmol) portion of 2-tert-butyl-7-methyl-5-[(3S)-dimethylaminopyrrolidin-1-yl]-6-(3-nitrophenyl)-[1,2,4]triazolo[1,5-a]pyridine-8-carbonitrile (#44) was dissolved in 4 N hydrochloric acid dioxane solution (1 ml) by subjecting it to several minutes of ultrasonic treatment. After concentrated under a reduced pressure, the resulting residue was dissolved in methanol (5 ml), mixed with 16 mg of 5% palladium-carbon catalyst and then stirred at room temperature for 2.5 hours under an atmosphere of hydrogen under ordinary pressure. After removing the catalyst by filtration, the solvent was evaporated under a reduced pressure, and the resulting residue was mixed with ethyl acetate and washed with 10% sodium carbonate aqueous solution and brine. The organic layer was dried over magnesium sulfate, and then the solvent was evaporated under a reduced pressure. The thus obtained residue was applied to a silica gel column chromatography, and 33.2 mg (61%) of the title compound was obtained as a pale yellow solid from an eluate of chloroform-methanol (100:1 → 50:1 v/v).
MS (FAB)*m*/*z*: 418 (M+1)⁺.
¹H-NMR (CDCl₃)δ: 1.47 (9H, s), 1.58-1.73 (1H, m), 1.94-2.05 (1H, m), 2.18 (3H, s), 2.20 (3H, s), 2.27 (1.5H, s), 2.28 (1.5H, s), 2.59-2.72 (1H, m), 3.34-3.68 (4H, m), 3.77 (2H, brs), 6.41-6.44 (0.5H, m), 6.47-6.53 (1H, m), 6.58-6.62 (0.5H, m), 6.68-6.73 (1H, m), 7.16-7.24 (1H, m).
IR (KBr): 3468, 3365, 3229, 2966, 2775, 2217, 1631, 1602, 1535, 1508, 1475, 1453 cm⁻¹. Elemental analysis values: as C₂₄H₃₁N₇·H₂O
Calcd.: C, 66.18%; H, 7.64%; N, 22.51 %
Found: C, 66.62%; H, 7.28%; N, 22.19%

### [Example 47]

### 2-tert-Butyl-5-[(2-N',N'-diethylamino)ethylamino]-7-methyl-6-phenyl[1,2,4]triazolo[1,5-a]pyridine-8-carbonitrile (#47)

A 143 mg (1.23 mmol) portion of N,N-diethyl ethylenediamine was added to an N,N-dimethylformamide (2 ml) solution of 200 mg (0.62 mmol) of 2-tert-butyl-5-chloro-7-methyl-6-phenyl-[1,2,4]triazolo[1,5-a]pyridine-8-carbonitrile (I-116) and stirred at 80 to 90°C for 2.5 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 160 mg (64%) of the title compound was obtained as a pale yellow solid from an eluate of chloroform-methanol (200:1 v/v).
MS (FAB)*m*/*z:* 405 (M+1)⁺.
¹H-NMR (CDCl₃)δ: 0.96 (6H, t, *J=* 7.2 Hz), 1.47 (9H, s), 2.22 (3H, s), 2.38-2.45 (6H, m), 2.85 (2H, q, *J*= 5.3 Hz), 7.19 (1H, brs), 7.23-7.27 (2H, m), 7.43-7.48 (3H, m).
IR (KBr): 2964, 2816, 2215, 1614, 1556 cm⁻¹.
Elemental analysis values: as C₂₄H₃₂N₆
Calcd.: C, 71.25%; H, 7.97%; N, 20.77%
Found: C, 71.07%; H, 8.0 1 %; N, 20.69%

### [Example 48]

### 2-tert-Butyl-7-methyl-5-(piperazin-1-yl)-6-phenyl[1,2,4]triazolo[1,5-a]pyridine-8-carbonitrile (#48)

A 530 mg (6.16 mmol) portion of piperazine and 434 µl (2.46 mmol) of triethylamine were added to an N,N-dimethylformamide (40 ml) solution of 400 mg (1.23 mmol) of 2-tert-butyl-5-chloro-7-methyl-6-phenyl-[1,2,4]triazolo[1,5-a]pyridine-8-carbonitrile (I-116) and stirred at 80 to 90°C for 2.5 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 432 mg (94%) of the title compound was obtained as a pale yellow solid from an eluate of chloroform-methanol (1:0 → 100: 1 → 100:3 v/v).
MS (FAB)*m*/*z*: 375 (M+1)⁺.
¹H-NMR (CDCl₃)δ: 1.48 (9H, s), 2.23 (3H, s), 2.85-2.90 (4H, m), 3.00-3.05 (4H, m), 7.16-7.19 (2H, m), 7.38-7.51 (3H, m).
IR (KBr): 2965, 2219, 1607, 1512, 1440, 1204 cm⁻¹.
Elemental analysis values: as C₂₂H₂₆N₆·1.5H₂O
Calcd.: C, 65.81%; H, 7.28%; N, 20.93%
Found: C, 65.25%; H, 6.48%; N, 20.54%

### [Example 49]

### 2-tert-Butyl-7-methyl-5-[(3S)-dimethylaminopyrrolidin-1-yl]-6-phenyl[1,2,4]triazolo[1,5-a]pyridine-8-carbonitrile (#49)

A 51 mg (0.507 mmol) portion of (3S)-methylaminopyrrolidine and 122 µl (0.922 mmol) of triethylamine were added to an N,N-dimethylformamide (10 ml) solution of 150 mg (0.461 mmol) of 2-tert-butyl-5-chloro-7-methyl-6-phenyl-[1,2,4]triazolo[1,5-a]pyridine-8-carbonitrile (I-116) and stirred at 80 to 90°C for 3 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 95 mg (53%) of the title compound was obtained as pale green crystals from an eluate of chloroform-methanol (50:1 → 30:1 v/v).
MS (FAB)*m*/*z*: 389 (M+1)⁺.
¹H-NMR (CDCl₃)δ: 1.47 (9H, s), 1.57-1.70 (1H, m), 1.78-2.03 (2H, m), 2.16 (6H, s), 2.25 (3H, s), 2.37 (3H, s), 3.10-3.19 (1H, m), 3.23-3.34 (2H, m), 3.37-3.50 (2H, m), 7.12-7.22 (2H, m), 7.36-7.50 (3H, m).
IR (KBr): 2967, 2206, 1606, 1537, 1509, 1495, 1458, 1440 cm⁻¹.
Elemental analysis values: as C₂₃H₂₈N₆·0.5H₂O
Calcd.: C, 69.49%; H, 7.35%; N, 21.14%
Found: C, 69.71%; H, 7.17%; N, 21.07%

<Reference Examples 132 to 147 and Examples 50 to 57>

**[Table 2]**

| Substituent R | y | φ | δ | ε |
|---|---|---|---|---|
| Me | I-77 | I-132 | I-140 | #50 |
| i-Pr | I-80 | I-133 | I-141 | #51 |
| n-Bu | I-81 | I-134 | I-142 | #52 |
| i-Bu | I-82 | I-135 | I-143 | #53 |
| t-Bu | I-83 | I-136 | I-144 | #54 |
| 2-pyridyl | I-84 | I-137 | I-145 | #55 |
| 3-pyridyl | I-85 | I-138 | I-146 | #56 |
| 4-pyridyl | I-86 | I-139 | I-147 | #57 |

In the above Table 2,
Me represents methyl group,
Pr represents propyl group, and
Bu represents butyl group.
Also, y, θ, δ and ε in Table 2 are those which are represented by the following formulae.

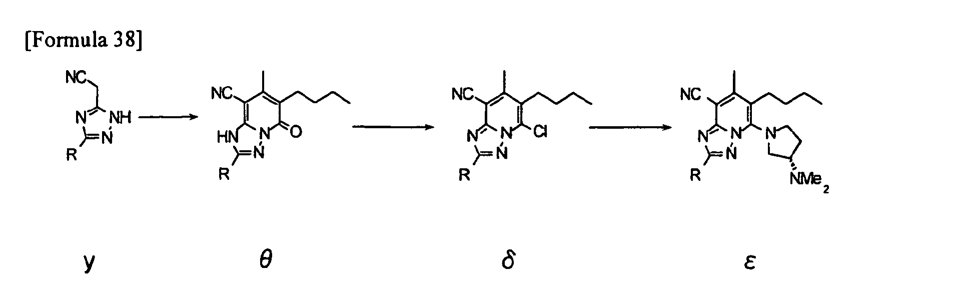

### [Reference Example 132]

### 6-n-Butyl-5-hydroxy-2,7-dimethyl[1,2,4]triazolo[1,5-a]pyridine-8-carbonitrile (I-132)

A mixture of 1.00 g (8.19 mmol) of 5-cyanomethyl-3-methyl-1H-[1,2,4]triazole (I-77), 1.59 g (8.52 mmol) of 2-acetylhexanoic acid ethyl ester and 1.31 g (17.0 mmol) of ammonium acetate was heated at 150°C for 1 hour. After cooling, water was added thereto, and the precipitated crystals were collected by filtration and further washed with acetonitrile. This was collected by filtration and dried to obtain 884 mg (44%) of the title compound as a colorless solid.
MS (FAB)*m*/*z*: 245 (M+1)⁺.
¹H-NMR (CD₃OD)δ: 0.95 (3H, t, *J=* 7.1 Hz), 1.32-1.54 (4H, m), 2.41 (3H, s), 2.43 (3H, s), 2.61 (2H, t, *J*= 7.6 Hz).

### [Reference Example 133]

### 6-n-Butyl-7-methyl-2-i-propyl-5-oxo-1,5-dihydro[1,2,4]triazolo[1,5-a]pyridine-8-carbonitrile (I-133)

A mixture of 750 mg (5.0 mmol) of (5-isopropyl-4H-[1,2,4]triazol-3-yl)-acetonitrile (I-80), 970 mg (5.2 mmol) of 2-acetylhexanoic acid ethyl ester and 800 mg (10.4 mmol) of ammonium acetate was heated at 150°C for 1 hour. After cooling, water was added thereto, and the precipitated crystals were collected by filtration and further washed with acetonitrile. This was collected by filtration and dried to obtain 640 mg (47%) of the title compound as pale pink crystals.
MS (FAB)*m*/*z:* 273 (M+1)⁺.
¹H-NMR (DMSO-d₆)δ: 0.87 (3H, t, *J*= 6.9 Hz), 1.25 (6H, d, *J=* 6.9 Hz), 1.31 (4H, m), 2.26 (3H, s), 2.46 (2H, m), 2.96 (1H, sep, *J*= 6.9 Hz).

### [Reference Example 134]

### 2,6-Di-n-butyl-7-methyl-5-oxo-1,5-dihydro[1,2,4]triazolo[1,5-a]pyridine-8-carbonitrile (I-134)

A mixture of 1.0 g (6.09 mmol) of 3-n-butyl-5-cyanomethyl-1H-[1,2,4]triazole (I-81), 1.19 g (6.39 mmol) of 2-acetylhexanoic acid ethyl ester and 986 mg (12.8 mmol) of ammonium acetate was heated at 150°C for 2 hours. After cooling, water was added thereto, and the precipitated crystals were collected by filtration and further washed with acetonitrile. This was collected by filtration and dried to obtain 703 mg (40%) of the title compound as pale pink crystals.
MS (FAB)*m*/*z*: 287 (M+1)⁺.
¹H-NMR (CD₃OD)δ: 0.93-0.97 (6H, m), 1.35-1.47 (6H, m), 1.73-1.83 (2H, m), 2.43 (3H, s), 2.62 (2H, t, *J =* 7.5 Hz), 2.81 (2H, t, *J =* 7.7 Hz).

### [Reference Example 135]

### 2-i-Butyl-6-n-butyl-7-methyl-5-oxo-1,5-dihydro[1,2,4]triazolo[1,5-a]pyridine-8-carbonitrile (I-135)

A mixture of 750 mg (5.00 mmol) of (5-isobutyl-4H-[1,2,4]triazol-3-yl)-acetonitrile (I-82), 970 mg (5.20 mmol) of 2-acetylhexanoic acid ethyl ester and 800 mg (10.4 mmol) of ammonium acetate was heated at 150°C for 1 hour. After cooling, water was added thereto, and the precipitated crystals were collected by filtration and further washed with acetonitrile. This was collected by filtration and dried to obtain 610 mg (43%) of the title compound as pale pink crystals.
MS (FAB)*m*/*z*: 287 (M+1)⁺.
¹H-NMR (DMSO-d₆)δ: 0.88 (3H, t, *J=* 6.9 Hz), 0.94 (6H, d, *J=* 6.6 Hz), 1.31 (4H, m), 2.13 (1H, m), 2.33 (3H, s), 2.53 (2H, m), 2.65 (2H, *d, J=* 7.2 Hz).

### [Reference Example 136]

### 2-i-Butyl-6-n-butyl-7-methyl-5-oxo-1,5-dihydro[1,2,4]triazolo[1,5-a]pyridine-8-carbonitrile (I-136)

A mixture of 1.0 g (6.09 mmol) of 3-tert-butyl-5-cyanomethyl-1H-[1,2,4]triazole (I-83), 1.19 g (6.39 mmol) of 2-acetylhexanoic acid ethyl ester and 986 mg (12.8 mmol) of ammonium acetate was heated at 150°C for 3.5 hours. After cooling, the reaction residue was applied to a silica gel column chromatography, eluted with chloroform-methanol (100:0 → 98:2 → 95:5 v/v) and further washed with acetonitrile. This was collected by filtration and dried to obtain 1.09 g (63%) of the title compound as a pale ocherous solid.
MS (FAB)*m*/*z*: 287 (M+1)⁺.
¹H-NMR (CDCl₃)δ: 0.93 (3H, t, *J=* 7.2 Hz), 1.35-1.53 (13H, m), 2.41 (3H, s), 2.70 (2H, t, *J=* 7.4 Hz).

### [Reference Example 137]

### 6-n-Butyl-5-hydroxy-7-methyl-2-(2-pyridyl)-[1,2,4]triazolo[1,5-a]pyridine-8-carbonitrile (I-137)

A mixture of 500 mg (2.70 mmol) of [5-(2-pyridyl)-2H-[1,2,4]triazol-3-yl]-acetonitrile (I-84), 523 mg (2.81 mmol) of 2-acetylhexanoic acid ethyl ester and 433 mg (5.62 mmol) of ammonium acetate was heated at 150°C for 20 minutes. This was further supplemented with 950 mg (5.10 mmol) of 2-acetylhexanoic acid ethyl ester and 433 mg (5.62 mmol) of ammonium acetate and heated at 150°C for 1 hour. After cooling, water and ethanol were added thereto, and the precipitated crystals were heated at 80°C for 5 minutes in a mixed solution of methanol (72 ml) and chloroform (36 ml). Subsequently, this was stirred at room temperature for 1 hour, and the precipitated crystals were collected by filtration and dried to obtain 227 mg (27%) of the title compound as a colorless solid.
MS (FAB)*m*/*z*: 308 (M+1)⁺.
¹H-NMR (DMSO-d₆)δ: 0.90 (3H, t, *J=* 6.9 Hz), 1.22-1.50 (4H, m), 2.33 (3H, s), 2.43-2.65 (2H, m), 7.38-7.49 (1H, m), 7.84-7.95 (1H, m), 8.13-8.21 (1H, m), 8.63-8.71 (1H, m).

### [Reference Example 138]

### 6-n-Butyl-5-hydroxy-7-dimethyl-2-(3-pyridyl)-[1,2,4]triazolo[1,5-a]pyridine-8-carbonitrile (I-138)

A mixture of 700 mg (3.78 mmol) of [5-(3-pyridyl)-2H-[1,2,4]triazol-3-yl]-acetonitrile (I-85), 733 mg (3.93 mmol) of 2-acetylhexanoic acid ethyl ester and 606 mg (7.86 mmol) of ammonium acetate was heated at 150°C for 1.5 hours. After cooling, acetonitrile and ethanol were added thereto, and the precipitated crystals were collected by filtration and dried to obtain 887 mg (76%) of the title compound as a colorless solid.
MS (FAB)*m*/*z*: 308 (M+1)⁺.
¹H-NMR (DMSO-d₆)δ: 0.89 (3H, t, *J =* 6.9 Hz), 1.24-1.48 (4H, m), 2.32 (3H, s), 2.42-2.61 (2H, m), 7.44-7.58 (1H, m), 8.38-8.49 (1H, m), 8.57-8.68 (1H, m), 9.21-9.32 (1H, m).

### [Reference Example 139]

### 6-n-Butyl-5-hydroxy-7-dimethyl-2-(4-pyridyl)-[1,2,4]triazolo[1,5-a]pyridine-8-carbonitrile (I-139)

A mixture of 800 mg (4.32 mmol) of [5-(4-pyridyl)-2H-[1,2,4]triazol-3-yl]-acetonitrile (I-86), 837 mg (4.50 mmol) of 2-acetylhexanoic acid ethyl ester and 693 mg (8.99 mmol) of ammonium acetate was heated at 150°C for 30 minutes. After cooling, ethanol, acetonitrile and water were added thereto, and the precipitated crystals were collected by filtration and dried to obtain 1.01 g (76%) of the title compound as a colorless solid.
MS (FAB)*m*/*z*: 308 (M+1)⁺.
¹H-NMR (DMSO-d₆)δ: 0.89 (3H, t, *J*= 7.0 Hz), 1.24-1.46 (4H, m), 2.33 (3H, s), 2.45-2.60 (2H, m), 7.99-8.09 (2H, m), 8.60-8.73 (2H, m).

### [Reference Example 140]

### 6-n-Butyl-5-chloro-2,7-dimethyl-[1.2.4]triazolo[1.5-a]pyridine-8-carbonitrile (I-140)

An 800 mg (3.27 mmol) portion of 6-n-butyl-5-hydroxy-2,7-dimethyl[1,2,4]triazolo[1,5-a]pyridine-8-carbonitrile (I-132) was heated under reflux for 1 hour in phosphoryl chloride (5 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated to obtain 768 mg (89%) of the title compound as a pale yellow solid.
MS (FAB)*m*/*z*: 263 (M+1)⁺.
¹H-NMR (CDCl₃)δ: 1.00 (3H, t, *J=* 7.2 Hz), 1.42-1.64 (4H, m), 2.67 (3H, s), 2.73 (3H, s), 2.81-2.93 (3H, m).

### [Reference Example 141]

### 6-n-Butyl-5-chloro-7-methyl-2-i-propyl-[1,2,4]triazolo[1,5-a]pyridine-8-carbonitrile (I-141)

A 500 mg (1.80 mmol) portion of 6-n-butyl-7-methyl-2-i-propyl-5-oxo-1,5-dihydro[1,2,4]triazolo[1,5-a]pyridine-8-carbonitrile (I-133) was heated under reflux for 1 hour in phosphoryl chloride (5 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated to obtain 768 mg (89%) of the title compound as brown crystals.
MS (FAB)*m*/*z:* 291 (M+1)⁺.
¹H-NMR (CDCl₃)δ: 1.00 (3H, t, *J*= 6.9 Hz), 1.45 (6H, d, *J*= 6.9 Hz), 1.52 (4H, m), 2.72 (3H, s), 2.87 (2H, m), 3.33 (1H, sep, *J*= 6.9 Hz).

### [Reference Example 142]

### 2,6-Di-n-butyl-5-chloro-7-methyl-[1,2,4]triazolo[1,5-a]pyridine-8-carbonitrile (I-142)

A 500 mg (1.75 mmol) portion of 2,6-di-n-butyl-7-methyl-5-oxo-1,5-dihydro[1,2,4]triazolo[1,5-a]pyridine-8-carbonitrile (I-134) was heated under reflux for 2.5 hours in phosphoryl chloride (3 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated to obtain 532 mg (100%) of the title compound as pale yellow crystals.
MS (FAB)*m*/*z*: 305 (M+1)⁺.
¹H-NMR (CDCl₃)δ: 0.94-1.03 (6H, m), 1.38-1.58 (6H, m), 1.80-1.90 (2H, m), 2.72 (3H, s), 2.87 (2H, t, *J*= 8.0 Hz), 2.97 (2H, t, *J=* 7.8 Hz).

### [Reference Example 143]

### 2-i-Butyl-6-n-butyl-5-chloro-7-methyl-[1,2,4]triazolo[1,5-a]pyridine-8-carbonitrile (I-143)

A 500 mg (1.70 mmol) portion of 2-i-butyl-6-n-butyl-7-methyl-5-oxo-1,5-dihydro[1,2,4]triazolo[1,5-a]pyridine-8-carbonitrile (I-135) was heated under reflux for 2.5 hours in phosphoryl chloride (3 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated to obtain 546 mg (100%) of the title compound as brown crystals. MS (FAB)*m*/*z*: 305 (M+1)⁺.
¹H-NMR (CDCl₃)δ: 1.00 (3H, t, *J*= 6.9 Hz), 1.01 (6H, d, *J*= 6.6 Hz), 1.53 (4H, m), 2.30 (2H, m), 2.73 (3H, s), 2.85 (2H, d, *J=* 7.2 Hz), 2.87 (2H, t, *J=* 7.2 Hz).

### [Reference Example 144]

### 2-tert-Butyl-6-n-butyl-5-chloro-7-methyl-[1,2,4]triazolo[1,5-a]pyridine-8-carbonitrile (I-144)

A 900 mg (3.14 mmol) portion of 2-i-butyl-6-n-butyl-7-methyl-5-oxo-1,5-dihydro[1,2,4]triazolo[1,5-a]pyridine-8-carbonitrile (I-136) was heated under reflux for 1.5 hours in phosphoryl chloride (5 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated to obtain 939 mg (98%) of the title compound as pale brown crystals.
MS (FAB)*m*/*z*: 305 (M+1)⁺.
¹H-NMR (CDCl₃)δ: 1.00 (3H, t, *J*= 7.1 Hz), 1.45-1.60 (13H, m), 2.71 (3H, s), 2.86 (2H, t, *J=* 7.8 Hz).

### [Reference Example 145]

### 6-n-Butyl-5-chloro-7-methyl-2-(2-pyridyl)-[1,2,4]triazolo[1,5-a]pyridine-8-carbonitrile (I-145)

A 200 mg (0.651 mmol) portion of 6-n-butyl-5-chloro-7-methyl-2-(2-pyridyl)-[1,2,4]triazolo[1,5-a]pyridine-8-carbonitrile (I-137) was heated under reflux for 2 hours in phosphoryl chloride (8 ml). After cooling, phosphoryl chloride was evaporated under a reduced pressure, and the thus obtained residue was mixed with ice water, further adjusted to pH 7 with saturated sodium bicarbonate aqueous solution and then extracted with chloroform. The organic layer was washed with brine and dried over magnesium sulfate, and then the solvent was evaporated to obtain 233 mg (100%) of the title compound as a yellow oily substance.
MS (FAB)*m*/*z*: 326 (M+1)⁺.
¹H-NMR (CDCl₃)δ: 1.02 (3H, t, *J=* 7.1 Hz), 1.43-1.67 (4H, m), 2.77 (3H, s), 2.87-2.95 (2H, m), 7.42-7.51 (1H, m), 7.86-7.97 (1H, m), 8.42-8.50 (1H, m), 8.82-8.93 (1H, m).

### [Reference Example 146]

### 6-n-Butyl-5-chloro-7-methyl-2-(3-pyridyl)-[1,2,4]triazolo[1,5-a]pyridine-8-carbonitrile (I-146)

A 700 mg (2.28 mmol) portion of 6-n-butyl-5-chloro-7-methyl-2-(3-pyridyl)-[1,2,4]triazolo[1,5-a]pyridine-8-carbonitrile (I-138) was heated under reflux for 2 hours in phosphoryl chloride (5 ml). After cooling, phosphoryl chloride was evaporated under a reduced pressure, and the thus obtained residue was mixed with ice water, further adjusted to pH 7 with saturated sodium bicarbonate aqueous solution and then extracted with chloroform. The organic layer was washed with brine and dried over magnesium sulfate, and then the solvent was evaporated. The resulting residue was applied to a silica gel column chromatography to obtain 201 mg (27%) of the title compound as a yellow solid from an eluate of chloroform-methanol (50:1 v/v).
¹H-NMR (CDCl₃)δ: 0.98-1.07 (3H, m), 1.45-1.65 (4H, m), 2.76 (3H, s), 2.86-2.96 (2H, m), 7.41-7.48 (1H, m), 8.58-8.65 (1H, m), 8.70-8.77 (1H, m), 9.53-9.58 (1H, m).

### [Reference Example 147]

### 6-n-Butyl-5-chloro-7-methyl-2-(4-pyridyl)-[1,2,4]triazolo[1,5-a]pyridine-8-carbonitrile (I-147)

A 910 mg (2.96 mmol) portion of 6-n-butyl-5-chloro-7-methyl-2-(4-pyridyl)-[1,2,4]triazolo[1,5-a]pyridine-8-carbonitrile (I-139) was heated under reflux for 2 hours in phosphoryl chloride (10 ml). After cooling, phosphoryl chloride was evaporated under a reduced pressure, and the thus obtained residue was mixed with ice water, further adjusted to pH 8 with saturated sodium bicarbonate aqueous solution and then extracted with chloroform. The organic layer was washed with brine and dried over magnesium sulfate, and then the solvent was evaporated to obtain 1.09 g of the title compound as a crude product. This was directly used in the subsequent reaction.

### [Example 50]

### 2-tert-Butyl-2,7-dimethyl-5-[(3S)-dimethylaminopyrrolidin-1-yl]-[1,2,4]triazolo[1,5-a]pyridine-8-carbonitrile (#50)

A 239 mg (2.09 mmol) portion of (3S)-dimethylaminopyrrolidine and 530 µl (3.80 mmol) of triethylamine were added to an N,N-dimethylformamide (6 ml) solution of 500 mg (1.90 mmol) of 6-n-butyl-5-chloro-2,3-dimethyl-[1,2,4]triazolo[1,5-a]pyridine-8-carbonitrile (I-140) and stirred at 80 to 90°C for 5.5 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 515 mg (80%) of the title compound was obtained as a yellow solid from an eluate of chloroform-methanol (100:1 v/v).
MS (FAB)*m*/*z*: 341 (M+1)⁺.
¹H-NMR (CDCl₃)δ: 0.93 (3H, t, *J* = 7.1 Hz), 1.36-1.54 (4H, m), 1.97-2.12 (1H, m), 2.22-2.39 (7H, m), 2.56 (3H, s), 2.63 (3H, s), 2.68-2.80 (2H, m), 3.02-3.14 (1H, m), 3.43-3.54 (2H, m), 3.57-3.66 (1H, m), 3.73-3.84 (1H, m).
IR (KBr): 2953, 2868, 2815, 2768, 2222, 1616, 1508, 1475 cm⁻¹.
Elemental analysis values: as C₁₉H₂₈N₆
Calcd.: C, 67.03%; H, 8.29%; N, 24.68%
Found: C, 66.99%; H, 8.38%; N, 25.01%

### [Example 51]

### 6-n-Butyl-7-methyl-5-[(3S)-dimethylaminopyrrolidin-1-yl]-2-i-propyl-[1,2,4]triazolo[1,5-a]pyridine-8-carbonitrile (#51)

A 220 µl (1.70 mmol) portion of (3S)-dimethylaminopyrrolidine and 480 µl (3.40 mmol) of triethylamine were added to an N,N-dimethylformamide (4 ml) solution of 400 mg (1.40 mmol) of 6-n-butyl-5-chloro-7-methyl-2-i-propyl[1,2,4]triazolo[1,5-a]pyridine-8-carbonitrile (I-141) and stirred at 80 to 90°C for 5.5 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 515 mg (83%) of the title compound was obtained as a red oily substance from an eluate of chloroform-methanol (50:1 v/v). MS (FAB)*m*/*z*: 369 (M+1)⁺.
¹H-NMR (CDCl₃)δ: 0.97 (3H, t, *J*= 6.9 Hz), 1.43 (6H, d, *J=* 6.9 Hz), 1.45 (4H, m), 2.04 (1H, m), 2.27 (1H, m), 2.33 (6H, s), 2.68 (3H, s), 2.74 (2H, m), 3.12 (1H, m), 3.26 (1H, sep, *J*= 6.9 Hz), 3.43 (1H, t, *J*= 8.1 Hz), 3.52 (3H, dt, *J=* 2.7, 8.7 Hz), 3.71 (2H, m).
IR (neat): 2920, 2233, 1613, 1532, 1504, 1470, 1348 cm⁻¹.

### [Example 52]

### 2,6-di-n-Butyl-7-methyl-5-[(3S)-dimethylaminopyrrolidin-1-yl]-[1,2,4]triazolo[1,5-a]pyridine-8-carbonitrile (#52)

A 183 µl (1.44 mmol) portion of (3S)-dimethylaminopyrrolidine and 366 µl (2.62 mmol) of triethylamine were added to an N,N-dimethylformamide (4 ml) solution of 400 mg (1.31 mmol) of 2,6-di-n-butyl-5-chloro-7-methyl-[1,2,4]triazolo[1,5-a]pyridine-8-carbonitrile (I-142) and stirred at 80 to 90°C for 3.5 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 461 mg (92%) of the title compound was obtained as a yellow oily substance from an eluate of chloroform-methanol (99:1 v/v).
MS (FAB)*m*/*z*: 383 (M+1)⁺.
¹H-NMR (CDCl₃)δ: 0.94-0.99 (6H, m), 1.38-1.51 (6H, m), 1.78-1.89 (2H, m), 1.98-2.11 (1H, m), 2.22-2.33 (1H, s), 2.33 (6H, s), 2.64 (3H, s), 2.68-2.77 (2H, m), 2.92 (2H, t, *J*= 7.7 Hz), 3.05-3.15 (1H, m), 3.43-3.53 (2H, m), 3.64 (1H, t, *J*= 7.8 Hz), 3.71-3.80 (1H, m).
IR (neat): 2918, 2843, 2223, 1614, 1461 cm⁻¹.

### [Example 53]

### 2-i-Butyl-6-n-butyl-7-methyl-5-[(3S)-dimethylaminopyrrolidin-1-yl]-[1,2,4]triazolo[1,5-a]pyridine-8-carbonitrile (#53)

A 190 µl (1.50 mmol) portion of (3S)-dimethylaminopyrrolidine and 420 µl (3.00 mmol) of triethylamine were added to an N,N-dimethylformamide (4 ml) solution of 400 mg (1.31 mmol) of 2-i-butyl-6-n-butyl-5-chloro-7-methyl-[1,2,4]triazolo[1,5-a]pyridine-8-carbonitrile (I-143) and stirred at 80 to 90°C for 5 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 480 mg (97%) of the title compound was obtained as a red oily substance from an eluate of chloroform-methanol (75:1 v/v).
MS (FAB)*m*/*z*: 383 (M+1)⁺.
¹H-NMR (CDCl₃)δ: 0.97 (3H, t, *J*= 6.9 Hz), 1.00 (6H, d, *J*= 6.6 Hz), 1.46 (4H, m), 2.05 (1H, m), 2.26 (1H, m), 2.33 (6H, s), 2.64 (3H, s), 2.75 (2H, m), 2.79 (2H, d, *J=* 7.2 Hz), 3.11 (1H, m), 3.45 (2H, m), 3.70 (2H, m).
IR (neat): 2956, 2905, 2223, 1612, 1504, 1469, 1349 cm⁻¹.

### [Example 54]

### 2-tert-Butyl-6-n-butyl-7-methyl-5-[(3S)-dimethylaminopyrrolidin-1-yl]-[1,2,4]triazolo[1,5-a]pyridine-8-carbonitrile (#54)

A 275 µl (2.17 mmol) portion of (3S)-dimethylaminopyrrolidine and 549 µl (3.94 mmol) of triethylamine were added to an N,N-dimethylformamide (6 ml) solution of 600 mg (1.97 mmol) of 2-tert-butyl-6-n-butyl-5-chloro-7-methyl-[1,2,4]triazolo[1,5-a]pyridine-8-carbonitrile (I-144) and stirred at 80 to 90°C for 4 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 723 mg (97%) of the title compound was obtained as a red solid from an eluate of chloroform-methanol (99:1 v/v).
MS (FAB)*m*/*z*: 383 (M+1)⁺.
¹H-NMR (CDCl₃)δ: 0.97 (3H, t, *J* = 7.1 Hz), 1.36-1.55 (13H, m), 1.97-2.11 (1H, m), 2.21-2.34 (1H, m), 2.34 (6H, s), 2.63 (3H, s), 2.68-2.77 (2H, m), 3.14 (1H, quint, *J =* 8.1 Hz), 3.40 (1H, t, *J*= 8.6 Hz), 3.53 (1H, dt, *J =* 2.4, 8.6 Hz), 3.66-3.74 (2H, m).
IR (KBr): 2956, 2869, 2772, 2216, 1607 cm⁻¹.
Elemental analysis values: as C₂₂H₃₄N₆·0.25H₂O
Calcd.: C, 68.27%; H, 8.98%; N, 21.71%
Found: C, 68.45%; H, 8.89%; N, 21.85%

### [Example 55]

### 6-n-Butyl-7-methyl-5-[(3S)-dimethylaminopyrrolidin-1-yl]-2-(2-pyridyl)-[1,2,4]triazolo[1,5-a]pyridine-8-carbonitrile (#55)

A 68.6 mg (0.60 mmol) portion of (3S)-dimethylaminopyrrolidine and 133 µl (1.00 mmol) of triethylamine were added to an N,N-dimethylformamide (5 ml) solution of 163 mg (0.50 mmol) of 6-n-butyl-5-chloro-7-methyl-2-(2-pyridyl)-[1,2,4]triazolo[1,5-a]pyridine-8-carbonitrile (I-145) and stirred at 80 to 90°C for 6 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 63.7 mg (32%) of the title compound was obtained as a yellow solid from an eluate of chloroform-methanol (100:1 → 97:3 v/v).
MS (FAB)*m*/*z*: 404 (M+1)⁺.
¹H-NMR (CDCl₃)δ: 0.99 (3H, t, *J=* 7.1 Hz), 1.39-1.60 (4H, m), 2.03-2.24 (2H, m), 2.28-2.45 (7H, m), 2.69 (3H, s), 2.72-2.86 (2H, m), 3.09-3.25 (1H, m), 3.53-3.66 (2H, m), 3.66-3.79 (1H, m), 3.88-4.01 (1H, m), 7.35-7.44 (1H, m), 7.79-7.91 (1H, m), 8.32-8.42 (1H, m), 8.76-8.89 (1H, m).
IR (KBr): 2957, 2822, 2773, 2218, 1611, 1532, 1504, 1478, 1415 cm⁻¹.
Elemental analysis values: as C₂₃H₂₉N₇·0.25H₂O
Calcd.: C, 67.70%; H, 7.29%; N, 24.03%
Found: C, 67.66%; H, 7.24%; N, 23.86%

### [Example 56]

### 6-n-Butyl-7-methyl-5-[(3S)-dimethylaminopyrrolidin-1-yl]-2-(3-pyridyl)-[1,2,4]triazolo[1,5-a]pyridine-8-carbonitrile (#56)

A 54.7 mg (0.479 mmol) portion of (3S)-dimethylaminopyrrolidine and 106 µl (0.798 mmol) of triethylamine were added to an N,N-dimethylformamide (3 ml) solution of 130 mg (0.399 mmol) of 6-n-butyl-5-chloro-7-methyl-2-(3-pyridyl)-[1,2,4]triazolo[1,5-a]pyridine-8-carbonitrile (I-146) and stirred at 80 to 90°C for 6 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 145 mg (90%) of the title compound was obtained as a yellow solid from an eluate of chloroform-methanol (100:1 → 97:3 v/v).
MS (FAB)*m*/*z*: 404 (M+1)⁺.
¹H-NMR (CDCl₃)δ: 0.94 (3H, t, *J=* 7.0 Hz), 1.38-1.60 (4H, m), 2.03-2.19 (1H, m), 2.28-2.49 (7H, m), 2.68 (3H, s), 2.72-2.86 (2H, m), 3.08-3.23 (1H, m), 3.46-3.65 (2H, m), 3.69-3.91 (2H, m), 7.3 8-7.47 (1H, m), 8.55-8.64 (1H, m), 8.67-8.75 (1H, m).
IR (KBr): 2957, 2822, 2773, 2218, 1611, 1532, 1504, 1478, 1415 cm⁻¹.
Elemental analysis values: as C₂₃H₂₉N₇
Calcd.: C, 68.46%; H, 7.24%; N, 24.30%
Found: C, 68.26%; H, 7.24%; N, 24.28%

### [Example 57]

### 6-n-Butyl-7-methyl-5-[(3S)-dimethylaminopyrrolidin-1-yl-]-2-(4-pyridyl)-[1,2,4]triazolo[1,5-a]pyridine-8-carbonitrile (#57)

A 405 mg (3.55 mmol) portion of (3S)-dimethylaminopyrrolidine and 786 µl (5.92 mmol) of triethylamine were added to an N,N-dimethylformamide (20 ml) solution of 1.09 g (3.34 mmol) of 6-n-butyl-5-chloro-7-methyl-2-(4-pyridyl)-[1,2,4]triazolo[1,5-a]pyridine-8-carbonitrile (I-147) and stirred at 80 to 90°C for 6 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 388 mg (32%, yield from I-139) of the title compound was obtained as a yellow solid from an eluate of chloroform-methanol (100:1 → 97:3 v/v).
MS (FAB)*m*/*z*: 404 (M+1)⁺.
¹H-NMR (CDCl₃) δ: 0.99 (3H, t, *J=* 6.9 Hz), 1.38-1.58 (4H, m), 2.03-2.21 (1H, m), 2.26-2.48 (7H, m), 2.68 (3H, s), 2.73-2.85 (2H, m), 3.07-3.22 (1H, m), 3.50-3.64 (2H, m), 3.68-3.77 (1H, m), 3.78-3.91 (1H, m), 8.14-8.22 (2H, m), 8.72-8.81 (2H, m).
IR (KBr): 2949, 2868, 2812, 2778, 2754, 2225, 1617, 1502, 1476 cm⁻¹.
Elemental analysis values: as C₂₃H₂₉N₇·0.25H₂O
Calcd.: C, 67.70%; H, 7.29%; N, 24.03%
Found: C, 67.71%; H, 7.05%; N, 24.02%

### [Reference Example 148]

### 2-tert-Butyl-5-hydroxy-6-phenyl-[1,2,4]triazolo[1,5-a]pyridine-8-carbonitrile (I-148)

At -40°C and under an atmosphere of nitrogen, 3.3 ml (6.6 mmol) of a heptane-tetrahydrofuran-ethylbenzene mixed solution of 2.0 M lithium diisopropyl amide was added dropwise to a tetrahydrofuran (30 ml) solution of 490 mg (3.00 mmol) of 3-tert-butyl-5-cyanomethyl-1H-[1,2,4]triazole (I-83). After 30 minutes of stirring at the same temperature, a tetrahydrofuran (10 ml) solution of 0.58 g (3.0 mmol) of ethyl 3-methoxy-2-phenylacrylate was added dropwise thereto, and this was stirred overnight at the same temperature and then warmed up to room temperature and further stirred overnight at room temperature. The reaction solution was mixed with saturated ammonium chloride aqueous solution and extracted with ethyl acetate. The organic layer was washed with brine and dried over magnesium sulfate, and then the solvent was evaporated. The thus obtained residue was applied to a silica gel column chromatography, and 112 mg (13%) of the title compound was obtained as a colorless solid from an eluate of chloroform-methanol (10:1 v/v).
MS (FAB)*m*/*z*: 293 (M+1)⁺.
¹H-NMR (DMSO-d₆)δ: 1.42 (9H, s), 7.24-7.29 (1H, m), 7.35-7.40 (2H, m), 7.68-7.71 (2H, m), 7.99 (1H, s).

### [Reference Example 149]

### 2-tert-Butyl-5-chloro-6-phenyl-[1,2,4]triazolo[1,5-a]pyridine-8-carbonitrile (I-149)

An 80 mg (0.27 mmol) portion of 2-tert-butyl-5-hydroxy-6-phenyl-[1,2,4]triazolo[1,5-a]pyridine-8-carbonitrile (I-148) was heated under reflux for 5.5 hours in phosphoryl chloride (5 ml). After cooling, phosphoryl chloride was evaporated under a reduced pressure, and the thus obtained residue was mixed with ice water and extracted with chloroform. The organic layer was washed with brine and dried over magnesium sulfate, and the solvent was evaporated, thereby obtaining 95 mg (100%) of the title compound as a yellow solid.
MS (FAB)*m*/*z*: 311 (M+1)⁺.
¹H-NMR (CDCl₃)δ: 1.54 (9H, s), 7.44-7.54 (5H, m), 7.96 (1H, s).

### [Example 58]

### 2-tert-Butyl-5-[(3S)-dimethylaminopyrrolidin-1-yl]-6-phenyl-[1,2,4]triazolo[1,5-a]pyridine-8-carbonitrile (#58)

A 41 µl (0.32 mmol) portion of (3S)-dimethylaminopyrrolidine and 56 µl (0.40 mmol) of triethylamine were added to an N,N-dimethylformamide (5 ml) solution of 85 mg (0.27 mmol) of 2-tert-butyl-5-chloro-6-phenyl-[1,2,4]triazolo[1,5-a]pyridine-8-carbonitrile (I-149) and stirred at 80 to 90°C for 3.5 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and the title compound was obtained as a crude product from an eluate of chloroform-methanol (50:1 v/v). This was dissolved in diethyl ether (5 ml), mixed with 0.25 ml (1.0 mmol) of 4 N hydrochloric acid ethyl acetate solution and then stirred at room temperature for 5 minutes. The precipitated crystals were collected by filtration to obtain 84 mg (73%) of hydrochloride of the title compound as a colorless solid.
MS (FAB)*m*/*z*: 389 (-HCl) (M+1)⁺.
¹H-NMR (CDCl₃)δ: 1.56 (9H, s), 2.32 (1H, m), 2.74 (1H, m), 2.79 (3H, t,J= 4.2 Hz), 2.87 (3H, d, *J*= 4.2 Hz), 3.47 (1H, m), 3.68 (1H, m), 3.97 (1H, m), 4.08 (1H, m), 4.41 (1H, m), 7.38-7.53 (5H, m), 7.77 (1H, m), 12.84 (1H, brs).
IR (KBr): 3417, 2965, 2222, 1604, 1513, 1444, 1357, 1207 cm⁻¹.
Elemental analysis values: as C₂₃H₂₈N₆·HCl·1.25H₂O
Calcd.: C, 61.73%; H, 7.10%; N, 18.57%; Cl, 7.92%
Found: C, 61.34%; H, 6.69%; N, 18.57%; Cl, 8.70%

### [Reference Example 150]

### 6-Benzyl-2-tert-butyl-5-hydroxy-7-methyl-[1,2,4]triazolo[1,5-a]pyridine-8-carbonitrile (I-150)

A mixture of 990 mg (6.00 mmol) of 3-tert-butyl-5-cyanomethyl-1H-[1,2,4]triazole (I-83), 1.45 g (6.60 mmol) of 2-benzyl-3-oxoacetic acid ethyl ester and 1.02 g (13.2 mmol) of ammonium acetate was heated at 160°C for 30 minutes. After cooling, acetonitrile was added thereto, and the precipitated crystals were collected by filtration and dried to obtain 726 mg (38%) of the title compound as colorless crystals.
MS (FAB)*m*/*z*: 321 (M+1)⁺.
¹H-NMR (DMSO-d₆)δ: 1.40 (9H, s), 2.29 (3H, s), 3.92 (2H, s), 7.10-7.24 (5H, m).

### [Reference Example 151]

### 6-Benzyl-2-tert-butyl-5-chloro-7-methyl-[1,2,4]triazolo[1,5-a]pyridine-8-carbonitrile (I-151)

A 650 mg (2.00 mmol) portion of 6-benzyl-2-tert-butyl-5-hydroxy-7-methyl-[1,2,4]triazolo[1,5-a]pyridine-8-carbonitrile (I-150) was heated under reflux for 4 hours in phosphoryl chloride (10 ml). After cooling, phosphoryl chloride was evaporated under a reduced pressure, and the thus obtained residue was mixed with ice water and extracted with chloroform. The organic layer was washed with brine and dried over magnesium sulfate, and then the solvent was evaporated to obtain 687 mg (100%) of the title compound as pale yellow crystals.
MS (FAB)*m*/*z*: 339 (M+1)⁺.
¹H-NMR (CDCl₃)δ: 1.52 (9H, s), 2.59 (3H, s), 4.30 (2H, s), 7.05-7.08 (2H, m), 7.21-7.33 (3H, m).

### [Example 59]

### 6-Benzyl-2-tert-butyl-7-methyl-5-[(3S)-dimethylaminopyrrolidin-1-yl]-[1,2,4]triazolo[1,5-a]pyridine-8-carbonitrile (#59)

A 67 µl (0.53 mmol) portion of (3S)-dimethylaminopyrrolidine and 92 µl (0.66 mmol) of triethylamine were added to an N,N-dimethylformamide (5 ml) solution of 150 mg (0.44 mmol) of 6-benzyl-2-tert-butyl-5-chloro-7-methyl-[1,2,4]triazolo[1,5-a]pyridine-8-carbonitrile (I-151) and stirred at 70 to 80°C for 6 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 161 mg (88%) of the title compound was obtained as a purple solid from an eluate of chloroform-methanol (50:1 v/v).
MS (FAB)*m*/*z*: 417 (M+1)⁺.
¹H-NMR (CDCl₃)δ: 1.48 (9H, s), 1.88-2.00 (1H, m), 2.11-2.21 (1H, m), 2.29 (6H, s), 2.47 (3H, s), 3.08 (1H, brs), 3.38-3.52 (2H, m), 3.62-3.71 (2H, m), 4.18 (2H, s), 7.02 (2H, d, *J*= 6.6 Hz), 7.14-7.30 (3H, m).
IR (KBr): 2962, 2218, 1606, 1508, 1472, 1450, 1209 cm⁻¹.
Elemental analysis values: as C₂₅H₃₂N₆·0.25H₂O
Calcd.: C, 71.31%; H, 7.78%; N, 19.96%
Found: C, 71.35%; H, 7.74%; N, 19.48%

### [Reference Example 152]

### 2-Benzyl-3-oxopentanoic acid methyl ester (I-152)

Potassium carbonate (10 g) and 7.96 ml (66.9 mmol) of benzyl bromide were added to an acetone (100 ml) solution of 7.26 g (55.8 mmol) of 3-oxopentanoic acid methyl ester and heated under reflux for 15 hours. After cooling, the reaction solution was filtered, and the solvent of the filtrate was evaporated. The thus obtained residue was applied to a silica gel column chromatography, and 7.08 g (58%) of the title compound was obtained as a colorless oily substance from an eluate of n-hexane-ethyl acetate (9:1 v/v). This was directly used in the subsequent reaction.
¹H-NMR (CDCl₃)δ: 0.99 (3H, t, *J* = 7.3 Hz), 2.25-2.37 (1H, m), 2.50-2.62 (1H, m), 3.16 (2H, d, *J* = 7.6 Hz), 3.68 (3H, s), 3.80 (3H, t, *J*= 7.6 Hz), 7.14-7.29 (5H, m).

### [Reference Example 153]

### 6-Benzyl-2-tert-butyl-7-ethyl-5-hydroxy-[1,2,4]triazolo[1,5-a]pyridine-8-carbonitrile (I-153)

A mixture of 990 mg (6.00 mmol) of 3-tert-butyl-5-cyanomethyl-1H-[1,2,4]triazole (I-83), 1.45 g (6.60 mmol) of 2-benzyl-3-oxopentanoic acid methyl ester (I-152) and 1.02 g (13.2 mmol) of ammonium acetate was heated at 160°C for 40 minutes. After cooling, this was applied to a silica gel column chromatography, and 442 mg (22%) of the title compound was obtained as colorless crystals (crystallized from acetonitrile), from an eluate of chloroform-methanol (100:1 v/v).
MS (FAB)*m*/*z*: 335 (M+1)⁺.
¹H-NMR (DMSO-d₆)δ: 1.01 (3H, t, *J=* 7.5 Hz), 1.40 (9H, s), 2.65 (2H, q, *J*= 7.5 Hz), 3.92 (2H, s), 7.10-7.24 (5H, m).

### [Reference Example 154]

### 6-Benzyl-2-tert-butyl-5-chloro-7-ethyl-[1,2,4]triazolo[1,5-a]pyridine-8-carbonitrile (I-154)

A 400 mg (1.20 mmol) portion of 6-benzyl-2-tert-butyl-7-ethyl-5-hydroxy-[1,2,4]triazolo[1,5-a]pyridine-8-carbonitrile (I-153) was heated under reflux for 4 hours in phosphoryl chloride (10 ml). After cooling, phosphoryl chloride was evaporated under a reduced pressure, and the thus obtained residue was mixed with ice water and extracted with chloroform. The organic layer was washed with brine and dried over magnesium sulfate, and the solvent was evaporated, thereby obtaining 444 mg (100%) of the title compound as colorless crystals.
MS (FAB)*m*/*z*: 353 (M+1)⁺.
¹H-NMR (CDCl₃)δ:1.19 (3H, t, *J*= 7.5 Hz), 1.52 (9H, s), 2.97 (2H, q, *J*= 7.5 Hz), 4.32 (2H, s), 7.04-7.07 (2H, m), 7.23-7.32 (3H, m).

### [Example 60]

### 6-Benzyl-2-tert-butyl-7-ethyl-5-[(3S)-dimethylaminopyrrolidin-1-yl]-[1,2,4]triazolo[1,5-a]pyridine-8-carbonitrile (#60)

A 67 µl (0.53 mmol) portion of (3S)-dimethylaminopyrrolidine and 92 µl (0.66 mmol) of triethylamine were added to an N,N-dimethylformamide (5 ml) solution of 150 mg (0.43 mmol) of 6-benzyl-2-tert-butyl-5-chloro-7-ethyl-[1,2,4]triazolo[1,5-a]pyridine-8-carbonitrile (I-154) and stirred at 70 to 80°C for 6 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and 144 mg (78%) of the title compound was obtained as a colorless solid from an eluate of chloroform-methanol (50:1 v/v).
MS (FAB)*m*/*z*: 431 (M+1)⁺.
¹H-NMR (CDCl₃)δ: 1.19 (3H, t, *J=* 7.5 Hz), 1.48 (9H, s), 1.84-1.97 (1H, m), 2.08-2.17 (1H, m), 2.29 (6H, s), 2.87 (2H, q, *J=* 7.5 Hz), 3.05 (1H, brs), 3.34-3.45 (2H, m), 3.58-3.66 (2H, m), 4.19. (2H, s), 7.00-7.04 (2H, m), 7.17-7.29 (3H, m).
IR (KBr): 2966, 2221, 1604, 1507, 1492, 1458, 1209 cm⁻¹.

### [Reference Example 155]

### 2-tert-Butyl-6-ethyl-5-hydroxy-7-phenyl-[1,2,4]triazolo[1,5-a]pyridine-8-carbonitrile (I-155)

A mixture of 1.00 g (6.00 mmol) of 3-tert-butyl-5-cyanomethyl-1H-[1,2,4]triazole (I-83), 1.45 g (6.60 mmol) of 2-benzoylbutyric acid ethyl ester and 1.02 g (13.2 mmol) of ammonium acetate was heated at 150°C for 5 hours. After cooling, this was dissolved in chloroform, washed with water and brine and dried over magnesium sulfate. The resulting residue was applied to a silica gel column chromatography, and 152 mg (8%) of the title compound was obtained as a pale blue solid from an eluate of chloroform-methanol (100:1 v/v).
MS (FAB)*m*/*z*: 321 (M+1)⁺.
¹H-NMR (DMSO-d₆)δ: 0.87 (3H, t, *J=* 7.2 Hz), 1.41 (9H, s), 2.22 (2H, q, *J=* 7.2 Hz), 7.28-7.31 (2H, m), 7.43-7.54 (3H, m).

### [Reference Example 156]

### 2-tert-Butyl-5-chloro-6-ethyl-7-phenyl-[1,2,4]triazolo[1,5-a]pyridine-8-carbonitrile (I-156)

A 120 mg (0.37 mmol) portion of 2-tert-butyl-6-ethyl-5-hydroxy-7-phenyl-[1,2,4]triazolo[1,5-a]pyridine-8-carbonitrile (I-155) was heated under reflux for 5 hours in phosphoryl chloride (5 ml). After cooling, phosphoryl chloride was evaporated under a reduced pressure, and the thus obtained residue was mixed with ice water and extracted with chloroform. The organic layer was washed with brine and dried over magnesium sulfate, and the solvent was evaporated. The thus obtained residue was applied to a silica gel column chromatography, and 90 mg (72%) of the title compound was obtained as colorless crystals from an eluate of chloroform-methanol (100:1 v/v).
MS (FAB)*m*/*z*: 339 (M+1)⁺.
¹H-NMR (CDCl₃)δ: 1.03 (3H, t, *J=* 7.5 Hz), 1.52 (9H, s), 2.70 (2H, q, *J=* 7.5 Hz), 7.30-7.34 (2H, m), 7.53-7.57 (3H, m).

### [Example 61]

### 2-tert-Butyl-6-ethyl-5-[(3S)-dimethylaminopyrrolidin-1-yl]-7-phenyl-[1,2,4]triazolo[1,5-a]pyridine-8-carbonitrile (#61)

A 31 µl (0.24 mmol) portion of (3S)-dimethylaminopyrrolidine and 46 µl (0.33 mmol) of triethylamine were added to an N,N-dimethylformamide (5 ml) solution of 76 mg (0.22 mmol) of 2-tert-butyl-5-chloro-6-ethyl-7-phenyl-[1,2,4]triazolo[1,5-a]pyridine-8-carbonitrile (I-156) and stirred at 70 to 80°C for 3 hours. After cooling, the reaction solution was concentrated under a reduced pressure, and the thus obtained residue was dissolved in chloroform and washed with saturated sodium bicarbonate aqueous solution and brine. The organic layer was dried over magnesium sulfate, and then the solvent was evaporated under a reduced pressure. The thus obtained residue was applied to a silica gel column chromatography, and the title compound was obtained as a crude product from an eluate of chloroform-methanol (50:1 v/v). This was dissolved in diethyl ether (5 ml), mixed with 0.25 ml (1.0 mmol) of 4 N hydrochloric acid ethyl acetate solution and then stirred at room temperature for 5 minutes. The precipitated crystals were collected by filtration to obtain 80 mg (80%) of hydrochloride of the title compound as a colorless solid.
MS (FAB)*m*/*z*: 417 (-HCl) (M+1)⁺.
¹H-NMR (CDCl₃)δ: 0.91 (3H, t, *J*= 7.5 Hz), 1.50 (9H, s), 2.59-2.78 (4H, m), 2.93 (6H, dd, *J*= 4.5, 18.3 Hz), 3.64-3.72 (1H, m), 3.79-3.87 (1H, m), 4.01-4.25 (3H, m), 7.27-7.31 (2H, m), 7.51-7.53 (3H, m), 13.09 (1H, brs).
IR (KBr): 3402, 2966, 2224, 1607, 1505, 1443, 1247 cm⁻¹.
Elemental analysis values: as C₂₅H₃₂N₆·1.25HCl·1.5H₂O
Calcd.: C, 61.38%; H, 7.47%; N, 17.18%; Cl, 9.06%
Found: C, 61.50%; H, 7.18%; N, 17.12%; Cl, 9.19%

### [Reference Example 157]

### 5-tert-Butyl-1H-pyrazole-3-carboxylic acid ethyl ester (L-157)

Under ice-cooling, 12.7 ml (0.1 mol) of pinacolone and 13.6 ml (0.1 mol) of diethyl oxalate were added dropwise to an ethanol solution of sodium ethoxide prepared from ethanol (150 ml) and 2.30 g (0.1 mol) of metallic sodium, and this was stirred at room temperature for 3.5 hours. The reaction solution was concentrated under a reduced pressure, and the resulting residue was mixed under ice-cooling with acetic acid (46 ml). Subsequently, 6.69 ml (80% min., 0.11 mol) of hydrazine monohydrate was added dropwise thereto under ice-cooling, and then stirred at 40°C for 3 hours. After cooling, this was concentrated to dryness under a reduced pressure, the resulting residue was mixed with water, and the resulting insoluble material was collected by filtration and dried to obtain 12.6 g (64%) of the title compound as a colorless solid.
MS (FAB)*m*/*z*: 197 (M+1)⁺.
¹H-NMR (CDCl₃)δ: 1.25-1.42 (12H, m), 4.36 (2H, q, *J=* 7.1 Hz), 6.64 (1H, s).

### [Reference Example 158]

### 5-tert-Butyl-3-hydroxymethyl-1H-pyrazole (I-158)

At -5°C, a tetrahydrofuran (180 ml) solution of 3.48 g (purity 80%, 73.3 mmol) of lithium aluminum hydride was added dropwise to a tetrahydrofuran (150 ml) solution of 12.0 g (61.1 mmol) of 5-tert-butyl-1H-pyrazole-3-carboxylic acid ethyl ester (I-157), while keeping the inner temperature below 15°C. After stirring overnight at room temperature, hydrous tetrahydrofuran was added thereto, and the solvent was evaporated under a reduced pressure. The thus obtained residue was mixed with ethyl acetate and saturated (+)-sodium tartarate aqueous solution, and the insoluble material was removed by filtration. The organic layer of the filtrate was separated, washed with brine and dried over magnesium sulfate, and then the solvent was evaporated to obtain 8.33 g (88%) of the title compound as a colorless solid.
MS (FAB)*m*/*z*: 155 (M+1)⁺.
¹H-NMR (CDCl₃)δ: 1.29 (9H, s), 4.65 (2H, s), 6.03 (1H, s), 6.71 (2H, brs).

### [Reference Example 159]

### 5-tert-Butyl-3-chloromethyl-1H-pyrazole monohydrochloride (I-159)

A 8.33 g (54 mmol) portion of 5-tert-butyl-3-hydroxymethyl-1H-pyrazole (I-158) was dissolved in 134 ml (0.54 mol) of 4 N hydrochloric acid dioxane solution and stirred for 10 minutes, and then this was concentrated under a reduced pressure. The thus obtained residue was mixed with thionyl chloride (60 ml) and stirred at 75°C for 20 minutes. After cooling, this was concentrated under a reduced pressure, and the resulting residue was washed with diethyl ether to obtain 9.42 g (83%) of the title compound as a pale yellow solid.
MS (FAB)*m*/*z*: 173 (-HCl) (M+1)⁺.
¹H-NMR (CD₃OD)δ: 1.41 (9H, s), 4.80 (2H, s), 6.73 (1H, s).

### [Reference Example 160]

### (5-tert-Butyl-1H-pyrazol-3-yl)acetonitrile (I-160)

Under ice-cooling, an ethanol (80 ml) solution of 8.80 g (42.1 mmol) of 5-tert-butyl-3-chloromethyl-1H-pyrazole monohydrochloride (I-159) was added dropwise to an aqueous (23 ml) solution of 19.2 g (0.295 mmol) of potassium cyanide, and the mixture was stirred at the same temperature for 1 hour and further stirred at room temperature for 4.5 hours. The insoluble material was removed by filtration, and the solvent of the filtrate was evaporated. The thus obtained residue was washed with chloroform, the insoluble material was again removed by filtration, and the solvent of the filtrate was evaporated. The resulting residue was applied to a silica gel column chromatography, and 5.87 g (85%) of the title compound was obtained as a yellow solid from an eluate of chloroform-methanol (100:1 v/v).
MS (FAB)*m*/*z*: 164 (M+1)⁺.
¹H-NMR (CDCl₃)δ: 1.33 (9H, s), 3.74 (2H, s), 6.10 (1H, s).

### [Reference Example 161]

### α-Acetylphenylacetonitrile (I-161)

A 25 g (0.213 mol) portion of phenylacetonitrile and 33 ml of ethyl acetate were added to an ethanol solution of sodium ethoxide prepared from ethanol (150 ml) and 6.38 g (0.277 mol) of metallic sodium and then heated under reflux for 3 hours. After cooling, this was concentrated under a reduced pressure, and the thus obtained residue was mixed with water and dichloromethane to separate the organic layer. This was washed with 1 N hydrochloric acid and brine and dried over magnesium sulfate, and then the solvent was evaporated. The thus obtained residue was applied to a silica gel column chromatography to obtain 3.15 g (9%) of the title compound as a colorless oily substance from a n-hexane-ethyl acetate (5:1→3:1 v/v) eluate.
MS (FAB)*m*/*z*: 160 (M+1)⁺.
¹H-NMR (CDCl₃)δ: 2.27 (3H, s), 4.68 (1H, s), 7.36-7.52 (5H, m).

### [Reference Example 162]

A compound capable of expressing an antifungal activity based on the functional mechanism of 1,6-β-glucan synthesis inhibition, with a broad spectrum and specifically or selectively, is provided, and an antifungal agent which comprises such a compound, a salt thereof or a solvate thereof is provided.
3-Methoxy-2-phenyl-2-butenenitrile (I-162)
A 25.0 g (0.157 mol) portion of α-acetylphenylacetonitrile (I-161) was heated under reflux for 6 hours in 140 ml (1.21 mol) of trimethyl orthoacetate. After cooling, this was concentrated under a reduced pressure, and the resulting residue was applied to a silica gel column chromatography to obtain 15.4 g (57%) of the title compound as a pale yellow oily substance from an eluate of n-hexane-ethyl acetate (10:1 → 5: v/v).
MS (FAB)*m*/*z*: 174 (M+1)⁺.
¹H-NMR (CDCl₃)δ: 2.45 (3H, s), 3.86 (3H, s), 7.18-7.26 (1H, m), 7.26-7.37 (2H, m), 7.58-7.64 (2H, m).

### [Reference Example 163]

### 7-Amino-2-tert-butyl-5-methyl-6-phenylpyrazolo[1,5-a]pyridine-4-carbonitrile (I-163)

At -30°C and under an atmosphere of nitrogen, 2.02 ml (4.05 mmol) of a heptane-tetrahydrofuran-ethylbenzene mixed solution of 2.0 M lithium diisopropyl amide was added dropwise to a tetrahydrofuran (30 ml) solution of 300 mg (1.84 mmol) of (5-tert-butyl-1H-pyrazol-3-yl)acetonitrile (I-160), and this was stirred at the same temperature for 30 minutes. A tetrahydrofuran (5 ml) solution of 318 mg (1.84 mmol) of 3-methoxy-2-phenyl-2-butenenitrile (I-162) was added dropwise thereto at -30°C, and this was stirred at the same temperature for 4 hours and then raised up to 0°C and further stirred for 1 hours. This was mixed with ethyl acetate, the organic layer was washed with saturated ammonium chloride aqueous solution and dried over magnesium sulfate, and then the solvent was evaporated. The thus obtained residue was applied to a silica gel column chromatography, and 235 mg (42%) of the title compound was obtained as a pale yellow solid from an eluate of n-hexane-ethyl acetate (8:1 → 6:1 v/v).
MS (FAB)*m*/*z*: 305 (M+1)⁺.
¹H-NMR (CDCl₃)δ: 1.42 (9H, s), 2.25 (3H, s), 5.48 (2H, brs), 6.44 (1H, s), 7.23-7.30 (2H, m), 7.42-7.56 (3H, m).

### [Examples 62 and 63]

### 2-tert-Butyl-5-methyl-7-[(3S)-dimethylaminopyrrolidin-1-yl]-6-phenylpyrazolo[1,5-a]piridine-4-carbonitrile (#62) and 2-tert-butyl-3-chloro-5-methyl-7-[(3S)-dimethylaminopyrrolidin-1-yl]-6-phenylpyrazolo[1,5-a]pyridine-4-carbonitrile (#63)

An 88 µl (0.74 mmol) portion of tert-butyl nitrite and 80 mg (0.59 mmol) of copper(II) chloride were heated at 75°C for several minutes in acetonitrile (3 ml) in an atmosphere of nitrogen. This was mixed with 150 mg (0.49 mmol) of 7-amino-2-tert-butyl-5-methyl-6-phenylpyrazolo[1,5-a]pyridine-4-carbonitrile (I-163) and stirred at the same temperature for 1 hour. This was mixed with chloroform, the organic layer was washed with 1 N hydrochloric acid and brine and dried over magnesium sulfate, and then the solvent was evaporated. The thus obtained residue was applied to a silica gel column chromatography to obtain the main products from a n-hexane-ethyl acetate (8:1 v/v) eluate. This was dissolved in N,N-dimethylformamide (2 ml), mixed with 21 mg (0.185 mmol) of (3S)-dimethylaminopyrrolidine and 41 µl (0.308 mmol) of triethylamine, and stirred at 80 to 90°C for 4 hours. After cooling, the reaction solution was mixed with ethyl acetate and washed with brine, the organic layer was dried over magnesium sulfate, and then the solvent was evaporated under a reduced pressure. The thus obtained residue was applied to a silica gel column chromatography, and the title compounds (#62: 23 mg, yellow solid; #63: 24.8 mg, yellow solid) were respectively obtained from an eluate of chloroform-methanol (200:1 → 100:1 v/v).

# 62:
MS (FAB)*m*/*z*: 402 (M+1)⁺.
¹H-NMR (CDCl₃)δ: 1.42 (9H, s), 1.50-1.70 (1H, m), 1.88-1.97 (1H, m), 2.17 (6H, s), 2.20 (3H, s), 2.63-2.75 (1H, m), 3.16-3.26 (2H, m), 3.3 8-3.59 (2H, m), 6.45 (1H, s), 7.09-7.23 (2H, m), 7.35-7.47 (3H, m).
IR (KBr): 2960, 2865, 2817, 2768, 2209, 1603, 1515, 1468, 1443 cm⁻¹.
# 63:
MS (FAB)*m*/*z:* 436 (M+1)⁺.
¹H-NMR (CDCl₃)δ: 1.50 (9H, s), 1.55-1.75 (1H, m), 1.87-2.02 (1H, m), 2.15 (6H, s), 2.23 (3H, s), 2.58-2.70 (1H, m), 3.09-3.23 (2H, m), 3.37-3.58 (2H, m), 7.07-7.23 (2H, m), 7.36-7.47 (3H, m). IR (KBr): 2965, 2864, 2816, 2770, 2212, 1599, 1514, 1470, 1442 cm⁻¹.

### [Reference Example 164]

### Ethyl 3-chloromethyl-5-methyl-1H-pyrazole-4-carboxylate hydrochloride (I-164)

A 16.0 g (86.9 mmol) portion of ethyl 3-hydroxymethyl-5-methyl-1H-pyrazole-4-carboxylate synthesized in accordance with the method described in a reference *(Synthesis,* 448, (1978)) was mixed with 4 N hydrochloric acid dioxane solution (220 ml) and stirred at room temperature for 20 minutes. The reaction solution was mixed with ether and stirred for 20 minutes, and then the precipitated crystals were collected by filtration (hydrochloride of the material, 18.4 g, 96%). An 18 g (81.6 mmol) of this hydrochloride was mixed with thionyl chloride and stirred at 70°C for 3 hours. After cooling, the reaction solution was concentrated under a reduced pressure, the resulting residue was mixed with ether, and the precipitated crystals were collected by filtration to obtain 18.7 g (96%) of the title compound.
MS (FAB)*m*/*z*: 203 (-HCl) (M+1)⁺.
¹H-NMR (CDCl₃)δ: 1.42 (3H, t, *J=* 7.2 Hz), 2.75 (3H, s), 4.41 (2H, q, *J*=7.2 Hz), 4.99 (2H, s), 13.22 (2H, brs).

### [Reference Example 165]

### Ethyl 5-cyanomethyl-3-methyl-1H-pyrazolo-4-carboxylate (I-165)

An ethanol (150 ml) solution of 18.0 g (75.3 mmol) of ethyl 3-chloromethyl-5-methyl-1H-pyrazole-4-carboxylate hydrochloride (I-164) was added dropwise to an aqueous (50 ml) solution of 34.5 g (0.53 mol) of potassium cyanide spending 1 hour under ice-cooling, and this was stirred at the same temperature for 1 hour and further at room temperature for 4 hours. The reaction solution was filtered, the filtrate was concentrated under a reduced pressure, and then the thus obtained residue was applied to a silica gel column chromatography to obtain 12.2 g (84%) of the title compound as colorless crystals from an eluate of chloroform-methanol (40:1 v/v).
MS (FAB)*m*/*z*: 194 (M+1)⁺.
¹H-NMR (CDCl₃)δ: 1.39 (3H, t, *J=* 7.2 Hz), 2.56 (3H, s), 4.01 (2H, s), 4.33 (2H, q, *J=* 7.2 Hz), 11.04 (1H, brs).

### [Reference Example 166]

### Ethyl 7-amino-4-cyano-2,5-dimethyl-6-phenylpyrazolo[1,5-a]pyridine-3-carboxylate (I-166)

At -30°C, 21.3 ml (33.0 mmol) of n-hexane solution of 1.55 mol/l of n-butyl lithium was added to a tetrahydrofuran (170 ml) solution of 4.7 ml (33.0 mmol) diisopropylamine, and this was stirred at the same temperature for 1 hour. Next, a tetrahydrofuran (100 ml) solution of 2.9 g (15.0 mmol) of ethyl 5-cyanomethyl-3-methyl-1H-pyrazolo-4-carboxylate (I-165) was added thereto spending 10 minutes or more and this was stirred at the same temperature for 1 hour, and then a tetrahydrofuran (30 ml) solution of 2.60 g (15.0 mmol) of 3-methoxy-2-phenyl-2-butenenitrile (I-162) was added dropwise thereto spending 10 minutes or more and this was stirred at the same temperature for 2 hours and further at 0°C for 15 hours. The reaction solution was mixed with saturated ammonium chloride aqueous solution and extracted with ethyl acetate. The organic layer was washed with brine and dried over magnesium sulfate, and then the solvent was evaporated. The resulting residue was applied to a silica gel column chromatography, and 750 mg (15%) of the title compound was obtained as a brown solid from an eluate of n-hexane-ethyl acetate (1:1 v/v).
MS (FAB)*m*/*z*: 335 (M+1)⁺.
¹H-NMR (CDCl₃)δ: 1.45 (3H, q, *J=* 7.2 Hz), 2.35 (3H, s), 2.67 (3H, s), 4.48 (2H, q, *J=* 7.2 Hz), 5.61 (2H, brs), 7.27-7.30 (2H, m), 7.45-7.58 (3H, m).

### [Reference Example 167]

### Ethyl 7-chloro-4-cyano-2,5-dimethyl-6-phenylpyrazolo[1,5-a]pyridine-3-carboxylate (I-167)

A 320 µl (2.69 mmol) portion of tert-butyl nitrite and 289 mg (2.15 mmol) of copper(II) chloride were heated at 70°C for several minutes in acetonitrile (12 ml) under an atmosphere of nitrogen. This was mixed with 600 mg (1.79 mmol) of ethyl 7-amino-4-cyano-2,5-dimethyl-6-phenylpyrazolo[1,5-a]pyridine-3-carboxylate (I-166) and stirred at the same temperature for 1 hour. After cooling, the solvent was evaporated under a reduced pressure, the thus obtained residue was mixed with chloroform, the resulting organic layer was washed with 1 N hydrochloric acid and brine and dried over magnesium sulfate, and then the solvent was evaporated. The thus obtained residue was applied to a silica gel column chromatography, and 274 mg (41%) of the title compound was obtained as a pale yellow solid from an eluate of n-hexane-ethyl acetate (8:1 v/v). MS (FAB)*m*/*z*: 354 (M+1)⁺.
¹H-NMR (CDCl₃)δ: 1.46 (3H, q, *J=* 7.2 Hz), 2.45 (3H, s), 2.74 (3H, s), 4.50 (2H, q, *J=* 7.2 Hz), 7.19-7.28 (2H, m), 7.48-7.59 (3H, m).

### [Example 64]

### Ethyl 4-cyano-7-[(3S)-dimethylaminopyrrolidin-1-yl]-2,5-dimethyl-6-phenylpyrazolo[1,5-a]pyridine-3-carboxylate (#64)

A 102 mg (0.889 mmol) portion of (3S)-dimethylaminopyrrolidine and 197 µl (1.48 mmol) of triethylamine were added to an N,N-dimethylformamide (10 ml) solution of 262 mg (0.741 mmol) of ethyl 7-chloro-4-cyano-2,5-dimethyl-6-phenylpyrazolo[1,5-a]pyridine-3-carboxylate (I-167) and stirred at 80 to 90°C for 4.5 hours. After cooling, the reaction solution was mixed with ethyl acetate and washed with brine, the resulting organic layer was dried over magnesium sulfate, and then the solvent was evaporated under a reduced pressure. The thus obtained residue was applied to a silica gel column chromatography, and 281 mg (88%) of the title compound was obtained as a pale light brown solid from an eluate of chloroform-methanol (100:1 v/v).
MS (FAB)*m*/*z*: 432 (M+1)⁺.
¹H-NMR (CDCl₃)δ: 1.44 (3H, q, *J*= 7.1 Hz), 1.56-1.72 (1H, m), 1.88-2.00 (1H, m), 2.14 (6H, s), 2.31(9H, s), 2.60-2.70 (4H, m), 3.11-3.23 (2H, m), 3.33-3.53 (2H, m), 4.49 (2H, q, *J*= 7.1 Hz), 7.08-7.14 (1H, m), 7.20-7.28 (1H, m), 7.35-7.51 (3H, m).
IR (KBr): 2970, 2953, 2814, 2763, 2214, 1706, 1595, 1512, 1483, 1465 cm⁻¹.
Elemental analysis values: as C₂₅H₂₉N₅O₂
Calcd.: C, 69.58%; H, 6.77%; N, 16.23%
Found: C, 69.43%; H, 6.77%; N, 16.22%

### [Test Example 1]

Measuring method of the antifungal activities of the compounds of the present invention was carried out in accordance with Japanese Journal of Medical Mycology, 40, 243 - 246, 1999, The Japanese Society for Medical Mycology Standardization Committee, (alamar Blue), and the 50% or 80% growth inhibition (GI50 or GI80) concentration (µg/ml) for drug non-addition growth used as the positive control was measured. The results are shown in Table 3.

**[Table 3]**

| Ex. | D-No. | *Saccharomyces* cerevisiae AY-14 | *Candida albicans* ATCC MYA-573 | *Candida glabrata* ATCC 48435 | *Candida krusei* ATCC 44507 |
|---|---|---|---|---|---|
| 8 | D21-7677 | 2 | 1 | 0.125 | >4 |
| 9 | D21-5964 | 0.125 | 0.25 | 0.25 | >4 |
| 25 | D21-1791 | 0.063 | 4 | 0.125 | >4 |
| 33 | D21-3166 | 0.004 | 4 | 0.008 | 4 |
| 39 | D21-7628 | 0.063 | 0.5 | 0.25 | >4 |
| 62 | D31-1733 | 2 | 4 | 2 | 4 |
| | AMPH | 0.016 | 0.063 | 0.016 | 0.063 |
| | Fluconazole | 4 | >4 | 4 | >4 |
| | | GI80 | GI50 | GI80 | GI50 |

While the invention has been describe in detail and with reference to specific embodiments thereof, it will be apparent to one skilled in the art that various changes and modifications can be made therein without departing from the spirit and scope of the invention.
This application is based on a Japanese patent application filed on February 16, 2004 (Japanese Patent Application No. 2004-038918), the entire contents thereof being thereby incorporated by reference.

### Industrial Applicability

The present invention provides a compound capable of expressing an antifungal activity based on the functional mechanism of 1,6-β-glucan synthesis inhibition, with a broad spectrum and specifically or selectively, and provides an antifungal agent which comprises such a compound, a salt thereof or a solvate thereof.

## Claims

1. A compound represented by the following formula (I), a salt thereof, or a solvate thereof 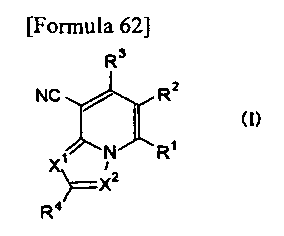 [in the formula,
R¹ means a basic group which may have a substituent,
R² means
hydrogen atom,
halogen atom,
carboxy group,
a group represented by the following formula 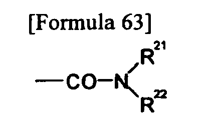 (in the formula, R²¹ and R²² each independently represents hydrogen atom, an alkyl group having from 1 to 6 carbon atoms or an aryl group having from 6 to 10 carbon atoms),
an alkyl group having from 1 to 6 carbon atoms,
an alkenyl group having from 2 to 6 carbon atoms,
an alkynyl group having from 2 to 6 carbon atoms,
an acyl group having from 2 to 7 carbon atoms,
an alkoxycarbonyl group having from 2 to 7 carbon atoms,
a cycloalkyl group having from 3 to 6 carbon atoms,
a cycloalkenyl group having 5 or 6 carbon atoms,
a cycloalkylalkyl group having from 4 to 12 carbon atoms,
an aryl group having from 6 to 10 carbon atoms,
an aralkyl group having from 7 to 12 carbon atoms,
a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom),
a heteroaryl group having from 3 to 10 carbon atoms, or
a heteroarylalkyl group having from 3 to 12 carbon atoms,
wherein when R² is an alkyl group, an alkenyl group, an alkynyl group, an acyl group or an alkoxycarbonyl group, these may have 1 or more groups of 1 or more species selected from [substituent group 2-1] as the substituent;
[substituent group 2-1]:
halogen atom,
amino group,
imino group,
nitro group,
hydroxy group,
mercapto group,
carboxy group,
cyano group,
sulfo group,
a dialkyl phosphoryl group,
a group represented by the following formula 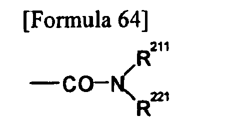 (in the formula, R²¹¹ and R²²¹ each independently represents hydrogen atom, an alkyl group having from 1 to 6 carbon atoms or an aryl group having from 6 to 10 carbon atoms),
an alkoxy group having from 1 to 6 carbon atoms,
an alkylthio group having from 1 to 6 carbon atoms,
an acyl group having from 2 to 7 carbon atoms,
an alkoxycarbonyl group having from 2 to 7 carbon atoms,
a cycloalkyl group having from 3 to 6 carbon atoms,
an aryl group having from 6 to 10 carbon atoms, and
an arylthio group having from 6 to 10 carbon atoms
wherein amino group of the [substituent group 2-1] may have 1 or 2 groups, as the substituent, selected from the group consisting of formyl group, an alkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, an aralkyl group having from 7 to 12 carbon atoms, an aromatic heterocyclic group, an alkylsulfonyl group having from 1 to 6 carbon atoms and an arylsulfonyl group having from 6 to 10 carbon atoms, in addition, when said amino group has 2 substituents, they may be bonded together to form a cyclic structure;
hydroxy group of the [substituent group 2-1] or mercapto group of the [substituent group 2-1] may have a substituent selected from the group consisting of an alkyl group having from 1 to 6 carbon atoms, an aminoalkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, a cycloalkyl group having from 3 to 6 carbon atoms, an aryl group having from 6 to 10 carbon atoms, an aralkyl group having from 7 to 12 carbon atoms and an aromatic heterocyclic group;
when R² is a cycloalkyl group, these may have 1 or more groups of 1 or more species selected from [substituent group 2-2] as the substituent;
[substituent group 2-2]:
halogen atom,
amino group,
imino group,
nitro group,
hydroxy group,
mercapto group,
carboxy group,
cyano group,
sulfo group,
a group represented by the following formula 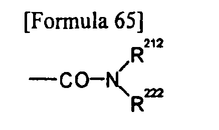 (in the formula, R²¹² and R²²² each independently represents hydrogen atom, an alkyl group having from 1 to 6 carbon atoms or an aryl group having from 6 to 10 carbon atoms),
an alkoxy group having from 1 to 6 carbon atoms,
an alkylthio group having from 1 to 6 carbon atoms,
an acyl group having from 2 to 7 carbon atoms, and
an alkoxycarbonyl group having from 2 to 7 carbon atoms;
amino group of the [substituent group 2-2] may have 1 or 2 groups, as the substituent, selected from the group consisting of formyl group, an alkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, an aralkyl group having from 7 to 12 carbon atoms, an aromatic heterocyclic group, an alkylsulfonyl group having from 1 to 6 carbon atoms and an arylsulfonyl group having from 6 to 10 carbon atoms, in addition, when said amino group has 2 substituents, they may be bonded together to form a cyclic structure;
when R² is an aryl group, an aralkyl group, a heteroaryl group or a heteroarylalkyl group, these may have 1 or more groups of 1 or more species selected from [substituent group 2-3] as the substituent;
[substituent group 2-3]:
halogen atom,
amino group,
imino group,
nitro group,
hydroxy group,
mercapto group,
carboxy group,
cyano group,
sulfo group,
a group represented by the following formula 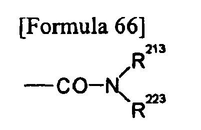 (in the formula, R²¹³ and R²²³ each independently represents hydrogen atom, an alkyl group having from 1 to 6 carbon atoms or an aryl group having from 6 to 10 carbon atoms), an alkoxy group having from 1 to 6 carbon atoms, an alkylthio group having from 1 to 6 carbon atoms, an acyl group having from 2 to 7 carbon atoms, an alkoxycarbonyl group having from 2 to 7 carbon atoms, an aralkyloxy group having from 7 to 12 carbon atoms, an aralkyloxycarbonyl group having from 8 to 15 carbon atoms, an aryl group and a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom);
amino group of the [substituent group 2-3] may have 1 or 2 groups, as the substituent, selected from the group consisting of formyl group, an alkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, an aralkyl group having from 7 to 12 carbon atoms, an aromatic heterocyclic group, an alkylsulfonyl group having from 1 to 6 carbon atoms and an arylsulfonyl group having from 6 to 10 carbon atoms, in addition, when said amino group has 2 substituents, they may be bonded together to form a cyclic structure;
when R² is a heterocyclic group, it may have 1 or 2 groups selected from the next [substituent group 2-4] as the substituent;
[substituent group 2-4]:
halogen atom,
amino group,
hydroxy group,
mercapto group,
carboxy group,
sulfo group,
a group represented by the following formula 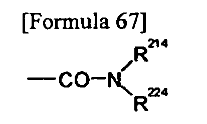 (in the formula, R²¹⁴ and R²²⁴ each independently represents hydrogen atom, an alkyl group having from 1 to 6 carbon atoms or an aryl group having from 6 to 10 carbon atoms),
an alkyl group having from 1 to 6 carbon atoms,
an alkenyl group having from 2 to 6 carbon atoms,
an alkynyl group having from 2 to 6 carbon atoms,
an alkoxy group having from 1 to 6 carbon atoms,
an alkylthio group having from 1 to 6 carbon atoms,
a halogenoalkyl group having from 1 to 6 carbon atoms,
an acyl group having from 2 to 7 carbon atoms,
an alkoxycarbonyl group having from 2 to 7 carbon atoms, and
an aryl group having from 6 to 10 carbon atoms;
wherein amino group of the [substituent group 2-4] may have 1 or 2 groups, as the substituent, selected from the group consisting of formyl group, an alkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, an aralkyl group having from 7 to 12 carbon atoms, a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom), an aromatic heterocyclic group, an alkylsulfonyl group having from 1 to 6 carbon atoms and an arylsulfonyl group having from 6 to 10 carbon atoms, in addition, when said amino group has 2 substituents, they may be bonded together to form a cyclic structure;
in addition, R¹ and R² may together form a cyclic structure including the carbon atoms to which these are bonded, wherein this ring contains 1 or 2 hetero atoms of 1 or more species selected from the group consisting of nitrogen atom, oxygen atom and sulfur atom, and the structural moiety to be formed herein may be saturated or unsaturated;
R³ means
hydrogen atom,
halogen atom,
amino group,
hydroxy group,
mercapto group,
nitro group,
cyano group,
formyl group,
carboxy group,
a group represented by the following formula 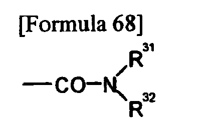 (in the formula, R³¹ and R³² each independently represents hydrogen atom, an alkyl group having from 1 to 6 carbon atoms or an aryl group having from 6 to 10 carbon atoms),
an alkyl group having from 1 to 6 carbon atoms,
an alkenyl group having from 2 to 6 carbon atoms,
an alkynyl group having from 2 to 6 carbon atoms,
an alkoxy group having from 1 to 6 carbon atoms,
an alkylthio group having from 1 to 6 carbon atoms
an acyl group having from 2 to 5 carbon atoms,
an alkoxycarbonyl group having from 2 to 5 carbon atoms,
a cycloalkyl group having from 3 to 7 carbon atoms,
a cycloalkenyl group having from 4 to 7 carbon atoms,
an aryl group having from 6 to 10 carbon atoms,
an aralkyl group having from 7 to 12 carbon atoms,
a heteroaryl group having from 3 to 10 carbon atoms;
wherein said amino group, said hydroxy group or said mercapto group may be protected by a protecting group;
when R³ is an alkyl group, an alkenyl group, an alkynyl group, an alkoxy group, an alkylthio group, an acyl group, an alkoxycarbonyl group, a cycloalkyl group, a cycloalkenyl group, an aryl group, an aralkyl group or a heteroaryl group, these may have 1 or more groups of 1 or more species selected from [substituent group 3-1] as the substituent;
[substituent group 3-1]:
amino group,
hydroxy group,
mercapto group,
halogen atom,
an alkoxy group having from 1 to 6 carbon atoms,
an alkylthio group having from 1 to 6 carbon atoms,
an acyl group having from 2 to 5 carbon atoms, and
an alkoxycarbonyl group having from 2 to 5 carbon atoms;
amino group of the [substituent group 3-1] may have 1 or 2 groups, as the substituent, selected from the group consisting of formyl group, an alkyl group having from 1 to 6 carbon atoms, a cycloalkyl group having from 3 to 6 carbon atoms, an aryl group having from 6 to 10 carbon atoms, an aromatic heterocyclic group, an acyl group having from 2 to 5 carbon atoms and an alkoxycarbonyl group having from 2 to 5 carbon atoms, wherein when said amino group has 2 substituents, they may be bonded together to form a cyclic structure;
in addition, R² and R³ may together form a polymethylene chain structure and form a 5-membered or 6-membered cyclic structure by including the carbon atoms to which R² and R³ are to be bonded, this polymethylene chain may contain 1 or 2 hetero atoms of 1 or more species selected from the group consisting of nitrogen atom, oxygen atom and sulfur atom, and the polymethylene chain formed herein may have 1 or more groups of 1 or more species selected from [substituent group 3-2] as the substituent;
[substituent group 3-2]:
amino group,
hydroxy group,
mercapto group,
halogen atom,
an alkoxy group having from 1 to 6 carbon atoms,
an alkylthio group having from 1 to 6 carbon atoms,
an acyl group having from 2 to 5 carbon atoms, and
an alkoxycarbonyl group having from 2 to 5 carbon atoms;
amino group of the [substituent group 3-2] may have 1 or 2 groups, as the substituent, selected from the group consisting of formyl group, an alkyl group having from 1 to 6 carbon atoms, a cycloalkyl group having from 1 to 6 carbon atoms, an aryl group having from 6 to 10 carbon atoms, an aromatic heterocyclic group, an acyl group having from 2 to 5 carbon atoms and an alkoxycarbonyl group having from 2 to 5 carbon atoms, wherein when said amino group has 2 substituents, they may be bonded together to form a cyclic structure;
in addition, R² and R³ may together form a polymethylene chain structure and form a 5-membered or 6-membered cyclic structure by including the carbon atoms to which R² and R³ are to be bonded, and this polymethylene chain may contain 1 or 2 hetero atoms of 1 or more species selected from the group consisting of nitrogen atom, oxygen atom and sulfur atom,
wherein the polymethylene chain formed herein may have 1 or more groups of 1 or more species selected from [substituent group 3-2] as the substituent;
[substituent group 3-2]: amino group, hydroxy group, mercapto group, halogen atom, an alkoxy group having from 1 to 6 carbon atoms, an alkylthio group having from 1 to 6 carbon atoms, an acyl group having from 2 to 5 carbon atoms, and an alkoxycarbonyl group having from 2 to 5 carbon atoms;
amino group of the [substituent group 3-2] may have 1 or 2 groups, as the substituent, selected from the group consisting of formyl group, an alkyl group having from 1 to 6 carbon atoms, a cycloalkyl group having from 1 to 6 carbon atoms, an aryl group having from 6 to 10 carbon atoms, an aromatic heterocyclic group, an acyl group having from 2 to 5 carbon atoms and an alkoxycarbonyl group having from 2 to 5 carbon atoms, wherein when said amino group has 2 substituents, they may be bonded together to form a cyclic structure; and
R⁴ means
hydrogen atom,
halogen atom,
amino group,
hydroxy group,
mercapto group,
nitro group,
cyano group,
formyl group,
carboxy group,
a group represented by the following formula 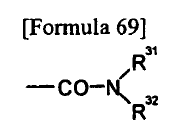 (in the formula, R³¹ and R³² each independently represents hydrogen atom, an alkyl group having from 1 to 6 carbon atoms or an aryl group having from 6 to 10 carbon atoms),
an alkyl group having from 1 to 4 carbon atoms,
an cyclic alkyl group having from 3 to 8 carbon atoms,
an aryl group having from 6 to 10 carbon atoms,
a heteroaryl group having from 5 to 9 carbon atoms,
an alkynyl group having from 2 to 6 carbon atoms, or
a group represented by 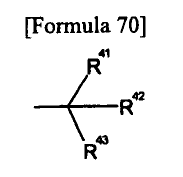 (in the formula, R⁴¹ and R⁴² each independently represents hydrogen atom, an alkyl group having from 1 to 6 carbon atoms or an alkoxy group having from 1 to 6 carbon atoms, or both may together form an exomethylene structure, and this exomethylene structure may further have an alkyl group having from 1 to 6 carbon atoms, an alkoxy group having from 1 to 6 carbon atoms or a halogenoalkyl group having from 1 to 6 carbon atoms, as a substituent, and
R⁴³ means hydrogen atom, a halogen atom, hydroxy group, mercapto group, nitrile group, nitro group, carboxy group, an alkoxycarbonyl group having from 2 to 7 carbon atoms, an alkylaminocarbonyl group having from 2 to 7 carbon atoms, an arylaminocarbonyl group having from 7 to 11 carbon atoms, a cycloalkylaminocarbonyl group having from 4 to 7 carbon atoms, an aralkylaminocarbonyl group having from 8 to 12 carbon atoms, an alkyl group having from 1 to 6 carbon atoms, a halogenoalkyl group having from 1 to 6 carbon atoms, a hydroxyalkyl group having from 1 to 6 carbon atoms, an aminoalkyl group having from 1 to 6 carbon atoms, an alkoxy group having from 1 to 6 carbon atoms, a cycloalkyl group having from 3 to 8 carbon atoms, a cycloalkyloxy group having from 3 to 8 carbon atoms, an aralkyl group having from 7 to 11 carbon atoms, or an aralkyloxy group having from 7 to 11 carbon atoms);
when R⁴ is an alkyl group, a cyclic alkyl group, an aryl group or a heteroaryl group, and when R⁴³ is an alkyl group, these may have 1 or more groups of 1 or more species selected from [substituent group 4] as the substituent;
[substituent group 4]:
halogen atom,
amino group,
nitro group,
hydroxy group,
mercapto group,
carboxy group,
cyano group,
sulfo group,
a group represented by the following formula 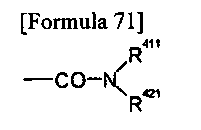 (in the formula, R⁴¹¹ and R⁴²¹ each independently mean hydrogen atom, an alkyl group having from 1 to 6 carbon atoms or an aryl group having from 6 to 10 carbon atoms),
an alkoxy group having from 1 to 6 carbon atoms,
an alkylthio group having from 1 to 6 carbon atoms,
an acyl group having from 2 to 7 carbon atoms,
an alkoxycarbonyl group having from 2 to 7 carbon atoms,
an aralkyloxy group having from 7 to 12 carbon atoms,
an aralkyloxycarbonyl group having from 8 to 15 carbon atoms,
an aryl group having from 6 to 10 carbon atoms, and
a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom);
amino group of the [substituent group 4] may have 1 or 2 groups, as the substituent, selected from the group consisting of formyl group, an alkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, an alkoxycarbonyl group having from 2 to 7 carbon atoms, a cycloalkyl group having from 3 to 6 carbon atoms, an aryl group having from 6 to 10 carbon atoms, an aralkyl group having from 7 to 12 carbon atoms, an aromatic heterocyclic group, an alkylsulfonyl group having from 1 to 6 carbon atoms and an arylsulfonyl group having from 6 to 10 carbon atoms, wherein when said amino group has 2 substituents, they may be bonded together to form a cyclic structure;
hydroxy group or mercapto group of the [substituent group 4] may have a substituent selected from the group consisting of an alkyl group having from 1 to 6 carbon atoms, an aminoalkyl group having from 1 to 6 carbon atoms, a hydroxyalkyl group having from 1 to 6 carbon atoms, a mercaptoalkyl group having from 1 to 6 carbon atoms, an acyl group having from 2 to 7 carbon atoms, a cycloalkyl group having from 3 to 6 carbon atoms, an aryl group having from 6 to 10 carbon atoms, an aralkyl group having from 7 to 12 carbon atoms and an aromatic heterocyclic group, wherein when R⁴ is an alkynyl group, it may have an alkyl group having from 1 to 6 carbon atoms, an alkoxyalkyl group having from 1 to 6 carbon atoms, a halogenoalkyl group having from 1 to 6 carbon atoms or carboxy group as a substituent;
X¹ and X² each independently mean
nitrogen atom or
carbon atom which may be substituted with
a halogen atom,
an alkoxy group having from 1 to 6 carbon atoms,
an alkyl group having from 1 to 6 carbon atoms which may have a substituent,
an ester group, wherein either one of X¹ and X² is nitrogen atom;
wherein the substituent of alkyl group is 1 or 1 or more groups selected from the following group of substituents;
halogen atom,
amino group,
nitro group,
hydroxy group,
mercapto group,
carboxy group,
cyano group,
an alkoxy group having from 1 to 6 carbon atoms,
an alkylthio group having from 1 to 6 carbon atoms,
an acyl group having from 2 to 7 carbon atoms,
an alkoxycarbonyl group having from 2 to 7 carbon atoms,
a cycloalkyl group having from 3 to 6 carbon atoms, and
an aryl group having from 6 to 10 carbon atoms;
when the substituents on carbon atoms are esters, these may be
an alkyl ester having from 1 to 6 carbon atoms,
an aryl ester having from 6 to 10 carbon atoms,
or an aralkyl ester consisting of an alkyl group having from 1 to 6 carbon atoms and an aryl group having from 6 to 10 carbon atoms;
in addition, the aryl moiety of these aryl esters and aralkyl groups may be substituted with 1 or 1 or more groups selected from the following group of substituents;
halogen atom,
amino group,
nitro group,
hydroxy group,
mercapto group,
carboxy group,
cyano group,
an alkyl group having from 1 to 6 carbon atoms,
an alkoxy group having from 1 to 6 carbon atoms,
an alkylthio group having from 1 to 6 carbon atoms,
an acyl group having from 2 to 7 carbon atoms,
an alkoxycarbonyl group having from 2 to 7 carbon atoms,
a cycloalkyl group having from 3 to 6 carbon atoms, and
an aryl group having from 6 to 10 carbon atoms].

2. The compound, a salt thereof, or a solvate thereof described in claim 1, wherein the basic group of R¹ is
(1) an amino substituted alkyl group having from 1 to 6 carbon atoms, which may have a substituent,
(2) an amino substituted cyclic alkyl group having from 3 to 6 carbon atoms, which may have a substituent,
(3) an aminocycloalkenyl group having from 3 to 6 carbon atoms, which may have a substituent,
(4) an amino substituted aralkyl group wherein the binding region with the bicyclic nucleus is an aromatic ring, which may have a substituent,
(5) an aminoalkyl substituted amino group having from 1 to 6 carbon atoms, which may have a substituent,
(6) an amino substituted cyclic alkylamino group having from 3 to 6 carbon atoms, which may have a substituent,
(7) an aminocycloalkenylamino group having from 3 to 6 carbon atoms, which may have a substituent,
(8) an amino substituted aralkylamino group wherein the binding region with the bicyclic nucleus is an aromatic ring, which may have a substituent, or
(9) a nitrogen-containing heterocyclic substituent, which may have a substituent;
wherein the amino group as the basic nature expressing group in the substituents of (1) to (8) may have 1 or 2 (may be the same or different when 2) of the substituents selected from the following substituent group [1-1];
substituent group [1-1]:
an alkyl group having from 1 to 6 carbon atoms, an alkenyl group having from 2 to 6 carbon atoms, an alkynyl group having from 2 to 6 carbon atoms, an alkoxycarbonyl group having from 2 to 7 carbon atoms, a cycloalkyl group having from 3 to 10 carbon atoms, a cycloalkenyl group having from 4 to 10 carbon atoms, and a group derived from an amino acid, a dipeptide or a polypeptide consisting of 3 to 5 amino acids;
also, when the substituent selected from the substituent group [1-1] is an alkyl group, an alkenyl group, an alkynyl group, an alkoxycarbonyl group, a cycloalkyl group or a cycloalkenyl group, these may have 1 or more of 1 or more groups selected from [substituent group 1-1-1]; [substituent group 1-1-1]: hydroxy group, mercapto group, a halogen atom, an alkoxy group having from 1 to 6 carbon atoms, an alkylthio group having from 1 to 6 carbon atoms and a cycloalkyl group having from 3 to 10 carbon atoms;
in addition, the nitrogen-containing heterocyclic substituent of (9) preferably uses a carbon atom as the binding position, is saturated or partially saturated, and is a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom), and the substituent on this heterocyclic group may be selected from [substituent group 1-2]; [substituent group 1-2]: a halogen atom, amino group, hydroxy group, oxo group, a group represented by the following formula 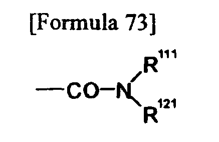 (in the formula, R¹¹¹ and R¹²¹ each independently represents hydrogen atom, an alkyl group having from 1 to 6 carbon atoms or an aryl group having from 6 to 10 carbon atoms), an alkyl group having from 1 to 6 carbon atoms, an aminoalkyl group having from 1 to 8 carbon atoms, an aminocycloalkyl group having from 3 to 8 carbon atoms, an alkoxy group having from 1 to 6 carbon atoms, an alkylthio group having from 1 to 6 carbon atoms, a halogenoalkyl group having from 1 to 6 carbon atoms and an alkylamino group having from 1 to 6 carbon atoms;
wherein the alkyl moiety of the alkyl group, alkylamino group, cycloalkylamino group, alkoxy group, alkylthio group, halogenoalkyl group or aminoalkyl group of the [substituent group 1-2] may have 1 or more groups of 1 or more species selected from [substituent group 1-2-1]; [substituent group 1-2-1]: a halogen atom, hydroxy group, an alkyl group having from 1 to 6 carbon atoms, an alkoxy group having from 1 to 6 carbon atoms, an alkoxycarbonyl group having from 2 to 7 carbon atoms, an alkylcarbonylamino group having from 2 to 7 carbon atoms and an aryl group having from 6 to 10 carbon atoms;
wherein the amino group moiety of the amino group, aminoalkyl group, aminocycloalkyl group and alkylamino group of the [substituent group 1-2] may be protected with a protecting group, and also may have 1 or 2 of alkyl groups having from 1 to 6 carbon atoms (may have 1 or more groups of 1 or more species selected from the group of groups consisting of hydroxy group, a halogen atom, and an alkoxy group and alkylthio group having from 1 to 6 carbon atoms) as the substituent, and also, an amino acid, a dipeptide or a polypeptide consisting of 3 to 5 amino acids may be bonded thereto.

3. The compound, a salt thereof, or a solvate thereof described in claim 2, wherein R¹ is a nitrogen-containing heterocyclic group which may have a substituent.

4. The compound, a salt thereof, or a solvate thereof described in claim 3, wherein R¹ is a nitrogen-containing heterocyclic group which may have a substituent, and said nitrogen-containing heterocyclic group is a saturate or partially saturated nitrogen-containing heterocyclic group.

5. The compound, a salt thereof or a solvate thereof described in claim 4, wherein R¹ is a group represented by the following formula; 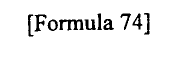 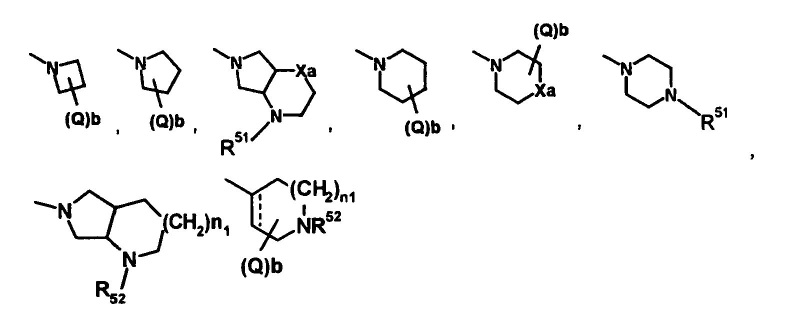 [in the formula, Xa means oxygen atom, sulfur atom, a substituent or NR⁵²,
R⁵¹ and R⁵² each independently means hydrogen atom, an alkyl group having from 1 to 6 carbon atoms, a halogenoalkyl group having from 1 to 6 carbon atoms or a cycloalkyl group having from 3 to 6 carbon atoms,
the substituent Q means a substituent represented by the following formula, 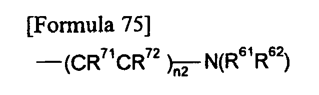 b means an integer of 0, 1 or 2,
n1 means an integer of 0 or 1,
n2 means an integer of 0, 1 or 2,
R⁶¹ and R⁶² each independently means hydrogen atom, an alkyl group having from 1 to 6 carbon atoms or a halogenoalkyl group having from 1 to 6 carbon atoms, or a group derived from an amino acid, a dipeptide or a polypeptide consisting of 3 to 5 amino acids,
R⁷¹ and R⁷² each independently means hydrogen atom, an alkyl group having from 1 to 6 carbon atoms, a halogenoalkyl group having from 1 to 6 carbon atoms, a hydroxyalkyl group having from 3 to 6 carbon atoms, an aminoalkyl group having from 1 to 6 carbon atoms, an alkoxyalkyl group having from 2 to 12 carbon atoms, a cycloalkyl group having from 3 to 6 carbon atoms, a phenyl group which may have a substituent or a heteroaryl group having from 3 to 10 carbon atoms which may have a substituent,
and the dotted line means that said binding region may form a double bond].

6. The compound, a salt thereof, or a solvate thereof described in any one of claims 1 to 5, wherein R² is an aryl group having from 6 to 10 carbon atoms, which may have a substituent, or a monocyclic, bicyclic or spiro cyclic heterocyclic group having from 2 to 10 carbon atoms (contains from 1 to 4 hetero atoms of 1 or more species selected from the group consisting of nitrogen atom, oxygen atom and sulfur atom).

7. The compound, a salt thereof, or a solvate thereof described in claim 6, wherein R² is a group represented by the following formula; 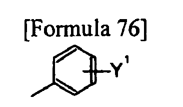 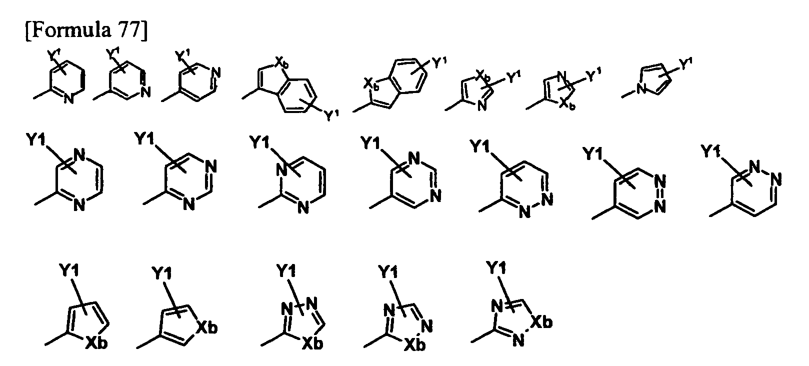 (in the formula, Xb means oxygen atom, sulfur atom, a substituent or NR⁸, wherein R⁸ means hydrogen atom, an alkyl group having from 1 to 6 carbon atoms or a halogenoalkyl group having from 1 to 6 carbon atoms, and the substituent Y¹ has the same meaning as described in the aforementioned [substituent group 2-2]).

8. The compound, a salt thereof, or a solvate thereof described in claim 7, wherein R³ is a halogen atom, amino group, hydroxy group, mercapto group, an alkyl group having from 1 to 4 carbon atoms which may have a substituent, an alkoxy group having from 1 to 6 carbon atoms which may have a substituent, an alkylthio group having from 1 to 6 carbon atoms, an acyl group having from 2 to 5 carbon atoms or an alkoxycarbonyl group having from 2 to 5 carbon atoms; wherein the amino group among them may have 1 or 2 groups, as the substituent, selected from the group consisting of formyl group, an alkyl group having from 1 to 6 carbon atoms, a cycloalkyl group having from 1 to 6 carbon atoms, an aryl group having from 6 to 10 carbon atoms, a heteroaryl group having from 3 to 10 carbon atoms, an acyl group having from 2 to 5 carbon atoms and an alkoxycarbonyl group having from 2 to 5 carbon atoms, and when said amino group has 2 substituents, they may be bonded together to form a cyclic structure.

9. The compound, a salt thereof, or a solvate thereof described in claim 7, wherein R³ is a group represented by the following formula; 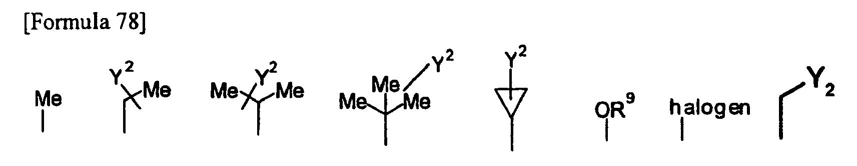 (in the formula, R⁹ means hydrogen atom, an alkyl group having from 1 to 6 carbon atoms, a cycloalkyl group having from 3 to 7 carbon atoms, an aryl group having from 6 to 10 carbon atoms, an aralkyl group having from 7 to 12 carbon atoms or an aromatic heterocyclic group, and the substituent Y² means amino group, hydroxy group, mercapto group, a halogen atom, an alkoxy group having from 1 to 6 carbon atoms, an alkylthio group having from 1 to 6 carbon atoms, an acyl group having from 2 to 5 carbon atoms or an alkoxycarbonyl group having from 2 to 5 carbon atoms, wherein the amino group among them may have 1 or 2 groups, as the substituent, selected from the group consisting of formyl group, an alkyl group having from 1 to 6 carbon atoms, a cycloalkyl group having from 1 to 6 carbon atoms, an aryl group having from 6 to 10 carbon atoms, an aromatic heterocyclic group, an acyl group having from 2 to 5 carbon atoms and an alkoxycarbonyl group having from 2 to 5 carbon atoms, and when said amino group has 2 substituents, they may be bonded together to form a cyclic structure).

10. The compound, a salt thereof, or a solvate thereof described in claim 7, wherein R³ is a group represented by the following formula; 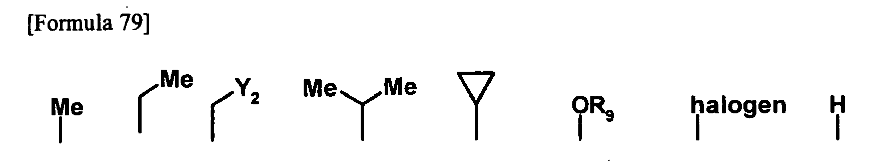 (in the formula, R⁹ means hydrogen atom, an alkyl group having from 1 to 6 carbon atoms, a cycloalkyl group having from 3 to 7 carbon atoms, an aryl group having from 6 to 10 carbon atoms, an aralkyl group having from 7 to 12 carbon atoms or an aromatic heterocyclic group, and the substituent Y² means amino group, hydroxy group, mercapto group, a halogen atom, an alkoxy group having from 1 to 6 carbon atoms, an alkylthio group having from 1 to 6 carbon atoms, an acyl group having from 2 to 5 carbon atoms or an alkoxycarbonyl group having from 2 to 5 carbon atoms, wherein the amino group among them may have 1 or 2 groups, as the substituent, selected from the group consisting of formyl group, an alkyl group having from 1 to 6 carbon atoms, a cycloalkyl group having from 1 to 6 carbon atoms, an aryl group having from 6 to 10 carbon atoms, an aromatic heterocyclic group, an acyl group having from 2 to 5 carbon atoms and an alkoxycarbonyl group having from 2 to 5 carbon atoms, and when said amino group has 2 substituents, they may be bonded together to form a cyclic structure).

11. The compound, a salt thereof, or a solvate thereof described in claim 9 or 10, wherein Y² is a halogen atom, alkoxy group having from 1 to 6 carbon atoms, hydroxy group or amino group, and R⁹ is hydrogen atom, an alkyl group having from 1 to 6 carbon atoms, a cycloalkyl group having from 3 to 7 carbon atoms, an aryl group having from 6 to 10 carbon atoms or an aralkyl group having from 7 to 12 carbon atoms.

12. The compound described in claim 9 or 10, wherein Y² is fluorine atom, chlorine atom, methoxy group or hydroxy group, and R⁹ is hydrogen atom, methyl group, ethyl group or isopropyl group.

13. The compound, a salt thereof, or a solvate thereof described in any one of claims 1 to 12, wherein R⁴ is an alkyl group having from 1 to 4 carbon atoms which may have a substituent, or a compound represented by the following formula; 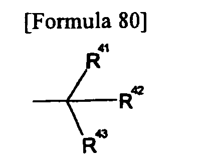 (R⁴¹, R⁴² and R⁴³ are as defmed in the foregoing).

14. The compound, a salt thereof, or a solvate thereof described in any one of claims 1 to 12, wherein R⁴ is a substituent having a structure represented by the following formula; 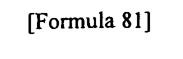 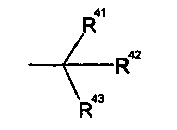 (R⁴¹, R⁴² and R⁴³ are as defmed in the foregoing).

15. A compound, a salt thereof, or a solvate thereof, which is a compound represented by the formula (I) having a combination in which
R² is an aryl group;
R¹ is a cyclic substituent having a saturated or partially saturated substituent;
R³ is an alkyl group having from 1 to 3 carbon atoms;
R⁴ is a substituent selected from the group consisting of (1) an alkyl or alkylene group having from 2 to 5 carbon atoms which may take a branched chain form, (2) a cyclic alkyl group having 3 or 4 carbon atoms, (3) an alkyl group having from 2 to 5 carbon atoms having fluorine atom or chlorine atom, which may take a branched chain form, (4) an alkoxyalkyl group having from 2 to 5 carbon atoms, and (6) a substituted benzyloxyethyl group which may have 1 or 2 methyl groups on the ethyl group.

16. A compound, a salt thereof, or a solvate thereof, which is a compound represented by the formula (I) having a combination in which
R² is an aryl group;
R¹ is a saturated or partially saturated nitrogen-containing heterocyclic group substituted with amino group, an alkylamino group or a dialkylamino group;
R³ is an alkyl group having from 1 to 3 carbon atoms;
R⁴ is a substituent selected from the group consisting of (1) an alkyl or alkylene group having from 2 to 5 carbon atoms which may take a branched chain form, (2) a cyclic alkyl group having 3 or 4 carbon atoms, (3) an alkyl group having from 2 to 5 carbon atoms having fluorine atom or chlorine atom, which may take a branched chain form, (4) an alkoxyalkyl group having from 2 to 5 carbon atoms, and (6) a substituted benzyloxyethyl group which may have 1 or 2 methyl groups on the ethyl group.

17. A compound, a salt thereof, or a solvate thereof, which is a compound represented by the formula (I) having a combination in which
R² is phenyl group;
R¹ is pyrrolidinyl group substituted with amino group, an alkylamino group or a dialkylamino group;
R³ is methyl group;
R⁴ is a substituent selected from the group consisting of ethyl group, isopropyl group, normal butyl group, tertiary butyl group, cyclopropyl group, propylen-2-yl group, methoxymethyl group, fluoromethyl group, 2-chloroethyl group, 2-hydroxyethyl group, 1,1-dimethyl-2-hydroxyethyl group, 2-benzyloxyethyl group, 2-benzyloxy-1,1-dimethyl-ethyl group and 2-(4-fluorophenylmethyl)oxyethyl group.

18. A compound, a salt thereof or a solvate thereof, which is a compound represented by the formula (I) having a combination in which
R² is phenyl group;
R¹ is pyrrolidinyl group substituted with amino group, methylamino group or dimethylamino group;
R³ is methyl group;
R⁴ is a substituent selected from the class consisting of ethyl group, isopropyl group, normal butyl group, tertiary butyl group, cyclopropyl group, propylen-2-yl group, methoxymethyl group, fluoromethyl group, 2-chloroethyl group, 2-hydroxyethyl group, 1,1-dimethyl-2-hydroxyethyl group, 2-benzyloxyethyl group, 2-benzyloxy-1,1-dimethyl-ethyl group and 2-(4-fluorophenylmethyl)oxyethyl group.

19. A medicine which comprises the compound, a salt thereof, or a solvate thereof described in any one of claims 1 to 18.

20. An infection treating agent which comprises the compound, a salt thereof, or a solvate thereof described in any one of claims 1 to 18.

21. An antifungal agent which comprises the compound, a salt thereof, or a solvate thereof described in any one of claims 1 to 18.

22. A method for treating an infection, which uses the compound, a salt thereof, or a solvate thereof described in any one of claims 1 to 18.

23. Use of the compound, a salt thereof or a solvate thereof described in any one of claims 1 to 18 for infection treatment.
